# EUROPEAN PATENT SPECIFICATION

(11) **EP 3 217 179 B1**
(45) Date of publication and mention of the grant of the patent: **08.01.2020**
(21) Application number: 17167637.2
(22) Date of filing: 02.10.2013
(51) Int. Cl.: G01N 33/68

(54) **METHODS FOR PREDICTING AND MONITORING MUCOSAL HEALING**
VERFAHREN ZUR VORAUSSAGE UND ÜBERWACHUNG VON MUKOSALER WUNDHEILUNG
MÉTHODES DE PRÉDICTION ET DE SURVEILLANCE DE CICATRISATION DES MUQUEUSES

(30) Priority: 05.10.2012 US 201261710491 P; 17.05.2013 US 201361824959 P
(43) Date of publication of application: 13.09.2017
(62) Divisional of application: 13779638.9
(73) Proprietor: Prometheus Biosciences, Inc., San Diego, CA 92121 (US)
(72) Inventor: SINGH, Sharat, Rancho Santa Fe, CA 92127 (US); LIU, Xinjun, San Diego, CA 92130 (US); HAUENSTEIN, Scott, San Diego, CA 92130 (US); KIRKLAND, Richard, San Diego, CA 92111 (US); DRAKE, Katherine, San Diego, CA 92126 (US)
(74) Representative: J A Kemp LLP

(56) References cited:
- US-A1- 2004 197 304
- Maneesh Dave ET AL: "Mucosal healing in inflammatory bowel disease-a true paradigm of success?", Gastroenterology & hepatology, 1 January 2012 (2012-01-01), pages 29-38, XP055089992, United States Retrieved from the Internet: URL:http://www.pubmedcentral.nih.gov/artic lerender.fcgi?artid=3277196&tool=pmcentrez &rendertype=abstract [retrieved on 2013-11-25]
- DATABASE MEDLINE [Online] US NATIONAL LIBRARY OF MEDICINE (NLM), BETHESDA, MD, US; November 2005 (2005-11), GEBOES KAREL ET AL: "Endoscopic and histologic evidence of persistent mucosal healing and correlation with clinical improvement following sustained infliximab treatment for Crohn's disease.", XP009174582, Database accession no. NLM16307694 & CURRENT MEDICAL RESEARCH AND OPINION NOV 2005, vol. 21, no. 11, November 2005 (2005-11), pages 1741-1754, ISSN: 0300-7995
- MARCO DAPERNO ET AL: "Results of the 2nd part Scientific Workshop of the ECCO (II): Measures and markers of prediction to achieve, detect, and monitor intestinal healing in Inflammatory Bowel Disease", JOURNAL OF CROHN'S AND COLITIS, vol. 5, no. 5, 5 October 2011 (2011-10-05) , pages 484-498, XP028297232, ISSN: 1873-9946, DOI: 10.1016/J.CROHNS.2011.07.003 [retrieved on 2011-07-14]
- FRANCO SCALDAFERRI: "Mucosal biomarkers in inflammatory bowel disease: Key pathogenic players or disease predictors?", WORLD JOURNAL OF GASTROENTEROLOGY, vol. 16, no. 21, 1 January 2010 (2010-01-01), page 2616, XP055090265, ISSN: 1007-9327, DOI: 10.3748/wjg.v16.i21.2616 [retrieved on 2013-11-25]

## Description

### BACKGROUND OF THE INVENTION

Inflammatory bowel disease (IBD) which includes Crohn's disease (CD) and ulcerative colitis (UC) is a chronic idiopathic inflammatory disorder affecting the gatrointestine tract. Disease progression of CD and UC includes repeated episodes of inflammation and ulceration of the intestine, leading to complications requiring hospitalization, surgery and escalation of therapy (Peyrin-Biroulet et al., Am. J. Gastroenterol,. 105: 289-297 (2010); Langholz E., Dan. Med. Bull., 46: 400-415 (1999)). Current treatments such as anti-tumor necrosis factor-alpha (TNF-α) biologics (*e.g*., infliximab (IFX), etanercept, adalimumab (ADL) and certolizumab pegol), thiopurine drugs (*e.g*., azathioprine (AZA), 6-mercaptopurin (6-MP)), anti-inflammatory drugs *(e.g.,* mesalazine), and steroids *(e.g.,* corticosteroids) have been shown to reduce disease activity. In some clinical trials of CD, mucosal healing (MH) which is described as the absence of intestinal ulcers, was induced in patients receiving combination therapy of corticosteroids and IFX or ADL. Furthermore, MH was maintained in patients receiving IFX.

Other studies have shown that mucosal healing can be a hallmark of suppression of bowel inflammation and can predict long-term disease remission (Froslie et al., Gastroenterology, 133: 412-422 (2007); Baert et al., Gastroenterology, (2010)). Long-term mucosal healing has been associated with a decreased risk of colectomy and colorectal cancer in UC patients, a decreased need for corticosteroid treatment in CD patients, and possibly a decreased need for hospitalization (Dave et al., Gastroenterology & Hepatology, 8(1): 29-38 (2012)).

The process of mucosal healing can be divided into three phase, beginning with bleeding (*e.g*., degradation of the endothelial layers of the blood vessels) and inflammation, then progression to cell and tissue proliferation, and finally tissue remodeling. At the inflammation stage, cytokines, chemokines and other inflammatory signaling molecules are secreted by immune cells in the gut mucosa. During proliferation, tissue repair and remodeling growth factors activate intestinal epithelial cells to proliferate, migrate to the sites of injury and repair the damaged tissue. At the remodeling phase, structural and functional improvements occur to the intestinal mucosal barrier. The present invention is based on the identification of novel markers of mucosal healing that are predictive of the phases of the process.

There is an unmet need in the art for non-invasive methods for predicting the likelihood of mucosal healing and/or monitoring the progression of mucosal healing in patients with IBD or other inflammatory diseases. This information enables the personalized therapeutic management of the disease, such as allowing for appropriate selection and/or administration of therapy. The present invention satisfies this need and provides related advantages as well.

### BRIEF SUMMARY OF THE INVENTION

Provided herein is an in vitro method for predicting the likelihood of mucosal healing in a subject having or suspected of having an inflammatory bowel disease (IBD), the method comprising:
(a) measuring the level of a first set of markers to form a first marker score, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBir1, Fla2, FlaX, OmpC, and an anti-drug antibody (ADA), wherein lower levels of SAA and VCAM relative to the level of the same marker in patients with IBD but no mucosal healing are predictive of mucosal healing;
(b) measuring the level of a second set of markers to form a second marker score, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFα antibody, wherein a lower level of TGFA relative to the level of the same marker in patients with IBD but no mucosal healing is predictive of mucosal healing;
(c) comparing the first marker score to the second marker score; and
(d) predicting the likelihood of mucosal healing based upon the comparison in step (c).

In some embodiments, the subject has an inflammatory bowel disease (IBD). In some instances, the inflammatory bowel disease is Crohn's disease or ulcerative colitis.

In some embodiments, the first set of markers comprises one or more of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, an anti-drug antibody (ADA), and combinations thereof. In some instances, the first set of markers comprises one or more of GMCSF, IL-2, and VCAM.

In some embodiments, the second set of markers comprises one or more of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody. In some instances, the second set of markers comprises HGF.

In some embodiments, each marker is assigned a value of from 0 to 6 based upon the concentration or level of the marker.

In some embodiments, the concentration or level of the marker is relative to the level of the same marker in a patient population without mucosal healing.

In some embodiments, the value for each marker in the first set of markers is summed to form the inflammatory phase marker score. In some embodiments, the value for each marker in the second set of markers is summed to form the proliferation phase marker score.

In some embodiments, the comparison in step (c) comprises applying an algorithm incorporating the inflammatory phase marker score and the proliferation phase marker score.

In some embodiments, the algorithm comprises subtracting the inflammatory phase marker score from the proliferation phase marker score to form a biomarker score of the subject.

In some embodiments, the subject has an increased likelihood of having complete improvement of mucosal healing without relapse when the biomarker score of the subject is higher than the biomarker score of a patient population without mucosal healing.

In some embodiments, the algorithm predicts the likelihood of mucosal healing independent of clinical confounders. In some instances, the clinical confounders comprise one or more selected from the group consisting of age of diagnosis, age of last sample, disease location, anal involvement, smoking, and surgery.

In some embodiments, the algorithm predicts the likelihood of mucosal healing by excluding serology markers. In other words, the algorithm can predict the likelihood of mucosal healing without the use of serology markers. In particular embodiments, the excluded serology markers comprise one or more selected from the group consisting of ASCA-A, ASCA-G, CBir1, Fla2, FlaX, and OmpC.

In some embodiments, the subject is receiving an anti-TNFa antibody. In some embodiments, the anti-TNFa antibody comprises one or more of REMICADE™ (infliximab), ENBREL™ (etanercept), HUMIRA™ (adalimumab), and CIMZIA® (certolizumab pegol).

In some embodiments, the marker is measured in a sample selected from the group consisting of serum, plasma, whole blood, stool, peripheral blood mononuclear cells (PBMC), polymorphonuclear (PMN) cells, a tissue biopsy, and combinations thereof.

Also provided herein is a method for monitoring the progression of mucosal healing in a subject. The method comprises the steps of: (a) measuring the level of a first set of markers at a plurality of time points to form a plurality of first marker scores, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBir1, Fla2, FlaX, OmpC, and an anti-drug antibody (ADA), wherein lower levels of SAA and VCAM relative to the level of the same marker in patients with IBD but no mucosal healing are predictive of mucosal healing; (b) measuring the level of a second set of markers at a plurality of time points to form a plurality of second marker scores, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody,wherein a lower level of TGFA relative to the level of the same marker in patients with IBD but no mucosal healing is predictive of mucosal healing; (c) comparing the first marker score to the second marker score at each time point and across the plurality of time points; and (d) monitoring the progression of mucosal healing based upon the comparison in step (c).

In some embodiments, the subject has an inflammatory bowel disease (IBD). In some instances, the inflammatory bowel disease is Crohn's disease or ulcerative colitis.

In some embodiments, the first set of markers comprises one or more of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, and an anti-drug antibody (ADA). In some instances, the first set of markers comprises one or more of GMCSF, IL-2, and VCAM.

In some embodiments, the second set of markers comprises one or more of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody. In some instances, the second set of markers comprises HGF.

In some embodiments, each marker is assigned a value of from 0 to 6 based upon the concentration or level of the marker.

In some embodiments, the concentration or level of the marker is relative to the level of the same marker in a patient population without mucosal healing.

In some embodiments, the value for each marker in the first set of markers is summed to form the inflammatory phase marker score. In some embodiments, the value for each marker in the second set of markers is summed to form the proliferation phase marker score.

In some embodiments, the comparison in step (c) comprises applying an algorithm incorporating the inflammatory phase marker score and the proliferation phase marker score.

In some embodiments, the algorithm comprises subtracting the inflammatory phase marker score from the proliferation phase marker score to form a biomarker score of the subject at each time point.

In some embodiments, the subject is progressing through the phases of mucosal healing when the biomarker score of the subject increases at each time point over the plurality of time points. In some instances, the subject is progressing from a phase of mucosal healing selected from an inflammatory phase and a proliferation phase onto the next phase of mucosal healing.

In some embodiments, the algorithm monitors the progression of mucosal healing independent of clinical confounders. In some instances, the clinical confounders comprise one or more selected from the group consisting of age of diagnosis, age of last sample, disease location, anal involvement, smoking, surgery, and combinations thereof.

In some embodiments, the algorithm monitors the progression of mucosal healing by excluding serology markers. In other words, the algorithm can predict the progression of mucosal healing without the use of serology markers. In certain embodiments, the excluded serology markers comprise one or more selected from the group consisting of ASCA-A, ASCA-G, CBir1, Fla2, FlaX, and OmpC.

In some embodiments, the subject is receiving an anti-TNFa antibody. In some embodiments, the anti-TNFa antibody comprises one or more of REMICADE™ (infliximab), ENBREL™ (etanercept), HUMIRA™ (adalimumab), and CIMZIA® (certolizumab pegol).

In some embodiments, the marker at each time point is measured in a sample selected from the group consisting of serum, plasma, whole blood, stool, peripheral blood mononuclear cells (PBMC), polymorphonuclear (PMN) cells, and a tissue biopsy.

In some embodiments, the method further comprises optimizing therapeutic efficacy of an anti-TNFa antibody therapy based upon the progression of mucosal healing in the subject.

In yet other embodiments, the present invention relates to an anti-TNFa antibody for the use in the treatment of IBD, wherein the amount of anti-TNFa antibody is based upon the progression of mucosal healing in the subject as determined by the in vitro method comprising:
a) measuring the level of a first set of markers at a plurality of time points to form a plurality of first marker scores, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBir1, Fla2, FlaX, OmpC, and an anti-drug antibody (ADA); (b) measuring the level of a second set of markers at a plurality of time points to form a plurality of second marker scores, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody, wherein a lower level of TGFA relative to the level of the same marker in patients with IBD but no mucosal healing is predictive of mucosal healing; (c) comparing the first marker score to the second marker score at each time point and across the plurality of time points; and (d) monitoring the progression of mucosal healing based upon the comparison in step (c).

Other objects, features, and advantages of the present invention will be apparent to one of skill in the art from the following detailed description and figures.

### BRIEF DESCRIPTION OF THE DRAWINGS

FIG. 1 illustrates the three phases of mucosal healing - inflammatory phase, proliferation phase and remodeling phase.
FIG. 2 shows representative data of clinical outcome for an individual who exhibited mucosal improvement (FIG. 2A) and an individual who experienced relapse after exhibiting complete improvement (FIG 2B).
FIG. 3 depicts the clinical status of individuals who have healed (FIG. 3A) and individuals who have never healed (FIG. 3B).
FIG. 4 shows the marker distributions between two extremes in the mucosal healing continuum - individuals who have never healed and individuals who have completely healed.
FIG. 5 depicts a table of inflammatory markers that are associated with mucosal healing. For GM-CSF, IL2, VCAM and HGF, lower marker values are predictive of mucosal healing.
FIG. 6A-B show a schematic of the individual patient analysis strategy that compares the expression of various markers in "true" healed indivudals (FIG. 6A) to not healed individuals (FIG. 6B).
FIG. 7A-F show inflammatory marker data used to identify "true" healed individuals. Individuals exhibiting low inflammation were selected for the individual patient analysis study.
FIG. 8A-D show clinical data (*e.g.,* ATI and/or IFX status) and the levels (*e.g.,* concentration) of repair factor markers in samples from Patient #1, a "true" healed individual.
FIG. 9A-D show data of inflammatory, anti-inflammatory and serology markers for Patient #1.
FIG. 10A-D show clinical data and the levels of repair factor markers in samples from Patient #2, a "true" healed individual.
FIG. 11A-D show data of inflammatory, anti-inflammatory and serology markers for Patient #2.
FIG. 12A-D show clinical data and the levels of repair factor markers in samples from Patient #3, a "true" healed individual.
FIG. 13A-D show data of inflammatory, anti-inflammatory and serology markers for Patient #3.
FIG. 14A-D show clinical data and the levels of repair factor markers in samples from Patient #4, a "true" healed individual.
FIG. 15A-D show data of inflammatory, anti-inflammatory and serology markers for Patient #4.
FIG. 16A-D shows clinical data and the levels of repair factor markers in samples from Patient #5, a not healed individual.
FIG. 17A-D show data of inflammatory, anti-inflammatory and serology markers for Patient #5.
FIG. 18A-D show clinical data and the levels of repair factor markers in samples from Patient #6, a not healed individual.
FIG. 19A-D show data of inflammatory, anti-inflammatory and serology markers for Patient #6.
FIG. 20A-D show clinical data and the levels of repair factor markers in samples from Patient #7, a not healed individual.
FIG. 21A-D show data of inflammatory, anti-inflammatory and serology markers for Patient #7.
FIG. 22A-D show clinical data and the levels of repair factor markers in samples from Patient #8, a not healed individual.
FIG. 23A-D show data of inflammatory, anti-inflammatory and serology markers for Patient #8.
FIG. 24A-D show illustrative data of repair factor markers (*e.g*., HER ligands, FGFs, PDGFs, and VEGFs) and clinical outcome from a theoretical patient progressing from the inflammatory phase (year 1), proliferation phase (year 2), and remodeling phase (year 3).
FIG. 25A-C show illustrative data of inflammatory and anti-inflammatory markers (*e.g*., markers detected by CEER and inflammatory markers) and clinical outcome from a theoretical patient progressing from the inflammatory phase (year 1), proliferation phase (year 2), and remodeling phase (year 3).
FIG. 26 shows the number of individuals in the study missing a particular marker.
FIG. 27 is a graph of the distribution of biomarker scores in the patient population of the study.
FIG. 28 shows that the biomarker score was predictive of having complete improvement without relapse.
FIG. 29 shows that after controlling for potential clinical confounders, the biomarker score was predictive of having complete improvement without relapse.
FIG. 30 shows the distribution of the biomarker scores for the two populations analyzed: individuals with complete improvement without relapse and individuals who never healed.
FIG. 31 shows that the biomarker score, determined without serology marker data was predictive of having complete improvement without relapse.
FIG. 32 shows that after controlling for potential clinical confounders, the biomarker score of FIG. 31 was predictive of having complete improvement without relapse.
FIG. 33 shows the distribution of the biomarker scores of FIG. 32 for the two populations analyzed.
FIG. 34A-C show statistical data from the combined marker analysis wherein removal of a single marker from the marker set was tested.

### DETAILED DESCRIPTION OF THE INVENTION

### I. Introduction

Anti-TNFα drugs such as infliximab (IFX) and adalimumab (ADA), promote mucosal healing in inflammatory bowel disease (IBD) patients. In fact, in clinical trials, endoscopic mucosal healing is a marker of the anti-inflammatory action of biological anti-TNFa drugs.

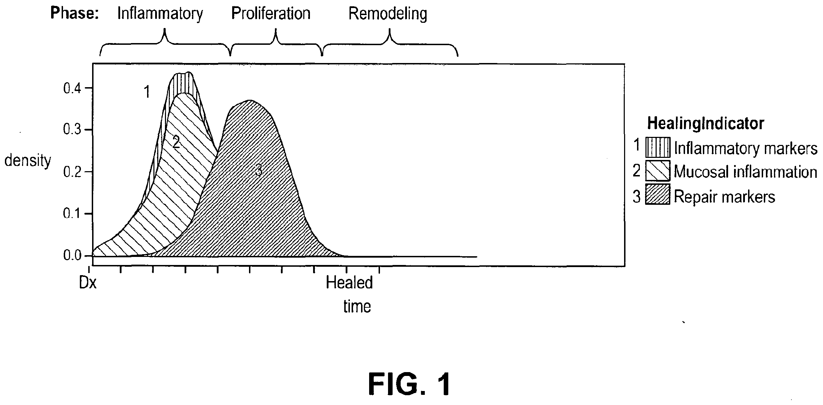

One underlying principle of the present invention is that IBD such as Crohn's disease and ulcerative colitis is better understood when viewed as a wound of the intestinal mucosal. Like other wounds of epithelial tissue, three phases of healing occur. As is shown in FIG. 1, a subject having IBD and being treated with an anti-TNF α drug, will progress through an inflammatory phase, a proliferation phase, and finally a remodeling phase. This mucosal healing mechanism occurs over time and is facilitated by anti-TNF α drugs while being treated.

The inflammatory phase occurs first and during this phase, bacteria and foreign debris are removed from the wound. Inflammatory markers are present at their highest concentration levels during this phase. Next, the proliferative phase is characterized by tissue formation, epithelialization, and wound contraction. In this phase, epithelial cells that are activated by growth factors and repair factors proliferate and provide cover for the new tissue. During the remodeling phase, the wound contracts and is made smaller by the action of myofibroblasts, which establish a grip on the wound edges and contract themselves, thereby healing the wound. A person just beginning therapy will most likely be in the inflammation phase and progress with a proper therapeutic regimen to the remodeling phase. And eventually, the intestinal mucosa will be restored.

Using the invention provided herein, it is possible to identify and determine the "phase" a particular subject is in at any particular time and predict the likelihood of mucosal healing (*e.g.,* progression through the phases of mucosal healing towards complete improvement thereof). In addition, the present invention can be used to monitor a subject's progression through the phases of mucosal healing over a plurality of time points. The present invention can also be used to determine whether a patient has an increased likelihood of having complete improvement of mucosal healing without relapse. Furthermore, the present invention provides methods for optimizing therapeutic efficiency for an anti-TNFa antibody therapy and for selecting an appropriate therapeutic regimen based on the progression of mucosal healing in the subject.

### II. Definitions

As used herein, the following terms have the meanings ascribed to them unless specified otherwise.

The term "mucosal healing" refers to restoration of normal mucosal appearance of a previously inflamed region, and complete absence of ulceration and inflammation at the endoscopic and microscopic levels. Mucosal healing includes repair and restoration of the mucosa, submucosa, and muscularis layers. It can also include neuronal and lymphangiogenic elements of the intestinal wall.

The term "progression of mucosal healing" refers to a transition through the phases (*e.g.,* stages) of mucosal healing from inflammatory phase, proliferation phase and remodeling phase towards complete improvement (*e.g*., complete repair) of the intestinal mucosa.

The term "complete improvement of mucosal healing without relapse" refers to a disease state wherein a patient having a disease such as IBD is undergoing or has undergone complete repair of the mucosa such that it is free of inflammation and/or an ulceration.

The terms "marker" and "biomarker" include any biochemical markers, serological markers, protein markers, genetic markers, and/or other clinical or echographic characteristics, that can be measured in a sample. In certain embodiments, a marker of the invention can be used to detect mucosal healing in a sample from an individual with a disease such as IBD including Crohn's disease and ulcerative colitis.

The term "marker score" or "biomarker score" includes an empirically derived score that is based upon an analysis of a plurality of markers such as, e.g., inflammatory markers, anti-inflammatory markers, repair factor markers, serology markers, level of anti-TNFa antibody, and level of anti-drug antibody. In one aspect, a first set of markers such as the concentration of the markers or their measured concentration values are transformed into an inflammatory phase marker score by an algorithm resident on a computer. In another aspect, a second set of markers such as the concentration of the markers or their measured concentration values are transformed into a proliferation phase marker score by an algorithm resident on a computer. A marker score can be determined multiple times over the course of different time points. In certain aspects, the marker score comprises or corresponds to a synthetic or human derived output, value, or cut off value(s) which expresses the biological data in numerical terms.

The term "TNFα" is intended to include a human cytokine that exists as a 17 kDa secreted form and a 26 kDa membrane associated form, the biologically active form of which is composed of a trimer of noncovalently bound 17 kDa molecules. The structure of TNFα is described further in, for example, Jones et al., Nature, 338:225-228 (1989). The term TNFα is intended to include human TNFα, a recombinant human TNFα (rhTNF-α), or TNFα that is at least about 80% identity to the human TNFα protein. Human TNFα consists of a 35 amino acid (aa) cytoplasmic domain, a 21 aa transmembrane segment, and a 177 aa extracellular domain (ECD) (Pennica, D. et al. (1984) Nature 312:724). Within the ECD, human TNFα shares 97% aa sequence identity with rhesus TNFα, and 71% to 92% aa sequence identity with bovine, canine, cotton rat, equine, feline, mouse, porcine, and rat TNFα. TNFα can be prepared by standard recombinant expression methods or purchased commercially (R & D Systems, Catalog No. 210-TA, Minneapolis, Minn.).

In certain embodiments, "TNFα" is an "antigen," which includes a molecule or a portion of the molecule capable of being bound by an anti-TNF-a drug. TNFα can have one or more than one epitope. In certain instances, TNFα will react, in a highly selective manner, with an anti-TNFa antibody. Preferred antigens that bind antibodies, fragments, and regions of anti-TNFa antibodies include at least 5 amino acids of human TNFα. In certain instances, TNFα is a sufficient length having an epitope of TNFα that is capable of binding anti-TNFa antibodies, fragments, and regions thereof.

The terms "TNF inhibitor", "TNF-α inhibitor," "TNFα inhibitor" and anti TNFα drug" are intended to encompass agents including proteins, antibodies, antibody fragments, fusion proteins (*e.g.,* Ig fusion proteins or Fc fusion proteins), multivalent binding proteins (*e.g.,* DVD Ig), small molecule TNF-α antagonists and similar naturally- or nonnaturally-occurring molecules, and/or recombinant and/or engineered forms thereof, that, directly or indirectly, inhibits TNF α activity, such as by inhibiting interaction of TNF-α with a cell surface receptor for TNF-α, inhibiting TNF-α protein production, inhibiting TNF-α gene expression, inhibiting TNFα secretion from cells, inhibiting TNF-α receptor signaling or any other means resulting in decreased TNF-α activity in a subject. The term "TNFα inhibitor" preferably includes agents which interfere with TNF-α activity. Examples of TNF-α inhibitors (anti-TNFa drug) include etanercept (ENBREL™, Amgen), infliximab (REMICADE™, Johnson and Johnson), human anti-TNF monoclonal antibody adalimumab (D2E7/HUMIRA™, Abbott Laboratories), CDP 571 (Celltech), and CDP 870 (Celltech), as well as other compounds which inhibit TNF-α activity, such that when administered to a subject suffering from or at risk of suffering from a disorder in which TNF-α activity is detrimental (*e.g*., IBD), the disorder is treated.

The terms "anti-drug antibody" and "ADA" are intended to encompass a human anti-chimeric antibody (HACA), a human anti-humanized antibody (HAHA), a human anti-mouse antibody (HAMA). The terms "antibodies to infliximab" and "ATI" refer to antibodies against the anti-TNFa antibody drug infliximab.

The term "subject," "patient," or "individual" typically refers to humans, but also to other animals including, *e.g.,* other primates, rodents, canines, felines, equines, ovines, porcines, and the like.

The term "patient population without mucosal healing" includes a group of patients wherein the patient has IBD and inflammation and/or an ulceration in the intestinal mucosa. In some instances, the intestinal mucosa of such a patient has not or has never healed. For example, the patient can be in the inflammatory phase of mucosal healing.

The term "clinical confounder" refers to an extraneous variable based on clinical observations that can be statistically related to or correlated with an independent variable, *e.g.,* the concentration or level of a marker. Clinical confounders for predicting mucosal healing in inflammatory bowel disease patients can include one or more of age of diagnosis, age of last sample, disease location, anal involvement, smoking, surgery, socioeconomic status, gender, diet, *etc.*

The term "sample" as used herein includes any biological specimen obtained from a patient. Samples include, without limitation, whole blood, plasma, serum, red blood cells, white blood cells (*e.g*., peripheral blood mononuclear cells (PBMC), polymorphonuclear (PMN) cells), ductal lavage fluid, nipple aspirate, lymph (*e.g*., disseminated tumor cells of the lymph node), bone marrow aspirate, saliva, urine, stool (*i.e.,* feces), sputum, bronchial lavage fluid, tears, fine needle aspirate (*e.g*., harvested by random periareolar fine needle aspiration), any other bodily fluid, a tissue sample such as a biopsy of a site of inflammation (*e.g*., needle biopsy), and cellular extracts thereof. In some embodiments, the sample is whole blood or a fractional component thereof such as plasma, serum, or a cell pellet. In other embodiments, the sample is obtained by isolating PBMCs and/or PMN cells using any technique known in the art. In other embodiments, the sample is a tissue biopsy, *e.g.,* tissue obtained from a site of inflammation such as a portion of the gastrointestinal tract or synovial tissue.

### III. Description of the Embodiments

The methods described herein can be used for predicting the likelihood of mucosal healing in a subject. In some embodiments, the subject has an inflammatory bowel disease. In some instances, the inflammatory bowel disease is Crohn's disease or ulcerative colitis.

In one aspect of the invention provided herein, the method includes (a) measuring a first set of markers to form an inflammatory phase marker score; (b) measuring a second set of markers to form a proliferation phase marker score; (c) comparing the inflammatory phase marker score to the proliferation phase marker score; and (d) predicting the likelihood of mucosal healing based upon the comparison in step (c).

In some embodiments, the first set of markers includes one or more of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, an anti-drug antibody (ADA), and combinations thereof. In one embodiment, the first set of markers includes one or more of GMCSF, IL-2, VCAM, and combinations thereof.

In some embodiments, the second set of markers includes one or more of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, an anti-TNFa antibody, and combinations thereof. In one embodiment, the second set of markers includes HGF.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6 based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In certain embodiments, the value of the marker is based upon 6 standard samples for each marker.

In some embodiments, one or more markers selected from the group consisting of an anti-TNFa antibody and ADA is measured using a homogeneous mobility shift assay (HMSA). In certain embodiments, the value of the marker is based on a quantile level for each marker.

In some embodiments, one or more markers selected from the group consisting of IL10, CRP, ICAM, SAA, VCAM, IL2, IL8, IL12p70, IL1B, GMCSF, IFNγ, IL6, TNFα, ASCAA, ASCAG, CBir1, Fla2, FlaX, and OmpC is measured using an immunoassay. In some instances, the value of the marker is based on a quantile level for each marker.

In some embodiments, the concentration or level of the marker is relative to the level of the same marker in a patient population without mucosal healing *(e.g.,* an IBD patient population without mucosal healing). The value for each marker in the first set of markers can be summed to form the inflammatory phase marker score. The value for each marker in the second set of markers can be summed to form the proliferation phase marker score.

In some embodiments, the comparison in step (c) includes applying an algorithm incorporating the inflammatory phase marker score and the proliferation phase marker score. In some instances, the algorithm includes subtracting the inflammatory phase marker score from the proliferation phase marker score to form a biomarker score of the subject.

In some embodiments, if the biomarker score of the subject is higher than the biomarker score of a patient population without mucosal healing, the subject has an increased likelihood of having complete improvement of mucosal healing without relapse.

In some embodiments, the algorithm predicts the likelihood of mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm predicts the likelihood of mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some embodiments, the algorithm used for predicting the likelihood of mucosal healing does not include (*e.g*., excludes, is without, or is independent of) values (*e.g*., scores or measurements, such as, concentrations, amounts or levels) of serology markers, such as ASCA-A, ASCA-G, CBir1, Fla2, FlaX, OmpC, or combinations thereof.

In some embodiments, the subject is receiving an anti-TNFa antibody. In some instances, the anti-TNFa antibody includes one or more of REMICADE™ (infliximab), ENBREL™ (etanercept), HUMIRA™ (adalimumab), CIMZIA® (certolizumab pegol), and combinations thereof.

In some embodiments, the marker is measured in a sample from the subject selected from the group consisting of serum, plasma, whole blood, stool, peripheral blood mononuclear cells (PBMC), polymorphonuclear (PMN) cells, a tissue biopsy, and combinations thereof.

Also, the methods provided herein can be used for monitoring the progression of mucosal healing in a subject. In some embodiments, the subject has an inflammatory bowel disease. In some instances, the inflammatory bowel disease is Crohn's disease or ulcerative colitis.

In one aspect of the invention provided herein, the method includes the steps of: (a) measuring a first set of markers at a plurality of time points to form a plurality of inflammatory phase marker scores; (b) measuring a second set of markers at a plurality of time points to form a plurality of proliferation phase marker scores; (c) comparing the inflammatory phase marker score to the proliferation phase marker score at each time point and across the plurality of time points; and (d) monitoring the progression of mucosal healing based upon the comparison in step (c).

In some embodiments, the first set of markers includes one or more of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, an anti-drug antibody (ADA), and combinations thereof. In some instances, the first set of markers includes one or more of GMCSF, IL-2, VCAM, and combinations thereof.

In some embodiments, the second set of markers includes one or more of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, an anti-TNFa antibody, and combinations thereof. In some instances, the second set of markers includes HGF.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6 based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In certain embodiments, the value of the marker is based upon 6 standard samples for each marker.

In some embodiments, one or more markers selected from the group consisting of an anti-TNFa antibody and ADA is measured using a homogeneous mobility shift assay (HMSA). In certain embodiments, the value of the marker is based on a quantile level for each marker.

In some embodiments, one or more markers selected from the group consisting of IL10, CRP, ICAM, SAA, VCAM, IL2, IL8, IL12p70, IL1B, GMCSF, IFNγ, IL6, TNFα, ASCAA, ASCAG, CBir1, Fla2, FlaX, and OmpC is measured using an immunoassay. In some instances, the value of the marker is based on a quantile level for each marker.

In some embodiments, the concentration or level of the marker is relative to the level of the same marker in a patient population without mucosal healing (*e.g.,* an IBD patient population without mucosal healing). The value for each marker in the first set of markers can be summed to form the inflammatory phase marker score. The value for each marker in the second set of markers can be summed to form the proliferation phase marker score.

In some embodiments, the comparison in step (c) includes applying an algorithm incorporating the inflammatory phase marker score and the proliferation phase marker score. In some instances, the algorithm includes subtracting the inflammatory phase marker score from the proliferation phase marker score to form a biomarker score of the subject at each time point.

In some embodiments, the subject is progressing through the phases of mucosal healing when the biomarker score of the subject increases at each time point over the plurality of time points. In some instances, the subject is progressing from a phase of mucosal healing selected from an inflammatory phase and a proliferation phase onto the next phase of mucosal healing.

The algorithm of the method provided herein can monitor the progression of mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm monitors the progression of mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some instances, the algorithm used for monitoring the progression of mucosal healing does not include (*e.g*., excludes, is without, or is independent of) values (*e.g*., scores or measurements, such as, concentrations, amounts or levels) of serology markers, such as ASCA-A, ASCA-G, CBir1, Fla2, FlaX, OmpC, or combinations thereof.

The subject of the methods provided herein can be receiving an anti-TNFa antibody. In some instances, the anti-TNFa antibody includes one or more of REMICADE™ (infliximab), ENBREL™ (etanercept), HUMIRA™ (adalimumab), CIMZIA® (certolizumab pegol), and combinations thereof.

In some embodiments, the marker at each time point is measured in a sample selected from the group consisting of serum, plasma, whole blood, stool, peripheral blood mononuclear cells (PBMC), polymorphonuclear (PMN) cells, a tissue biopsy, and combinations thereof.

In some embodiments, the plurality of time points comprises at least 2, 3, 4, 5, 6, 7, 8, 9, 10, 11, 12, 13, 14, 15, 16, 17, 18, 19, 20, 25, 30, 35, 40, 45, 50, or more time points.

In some embodiments, the method further includes optimizing therapeutic efficacy of an anti-TNFa antibody therapy based upon the progression of mucosal healing in the subject.

In yet other embodiments, the method further includes selecting an appropriate therapeutic regimen based upon the progression of mucosal healing in the subject.

### A. Additional Embodiments of the Invention

The methods described herein are also useful for identifying the phase of mucosal healing, such as an inflammatory phase, a proliferation phase, or a remodeling phase, in a subject, *(e.g.,* an individual having IBD and receiving anti-TNFa therapy). As such, in one embodiment, the method can be further used to select or administer an appropriate therapy.

In some embodiments, the method includes the steps of: (a) measuring a first set of markers to form an inflammatory phase marker score; (b) measuring a second set of markers to form a proliferation phase marker score; (c) identifying the phase of mucosal healing of the subject by using an algorithm incorporating the inflammatory phase marker score and the proliferation phase marker score; and (d) selecting an appropriate therapy based upon the phase of mucosal healing of the subject.

In some embodiments, the subject has inflammatory bowel disease. In some aspects, inflammatory bowel disease is Crohn's disease or ulcerative colitis.

In some embodiments, the first set of markers comprises one or more selected from the group consisting of TWEAK, CRP, ICAM, SAA, VCAM, IL2, IL8, IL12p70, IL1β, GMCSF, IFNγ, IL6, TNFα, ASCAA, ASCAG, CBir1, Fla2, FlaX, OmpC, an anti-drug antibody (ADA), and combinations thereof. In some embodiments, the second set of markers comprises one or more selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL10, an anti-TNFa antibody, and combinations thereof.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6 based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In some instances, the value of the marker described herein is based upon 6 standard samples for each marker.

In some embodiments, one or more markers selected from the group consisting of an anti-TNFa antibody and ADA is measured using a homogeneous mobility shift assay (HMSA). In some instances, the value of the marker is based on a quantile level for each marker.

In some embodiments, one or more markers selected from the group consisting of IL10, CRP, ICAM, SAA, VCAM, IL2, IL8, IL12p70, IL1B, GMCSF, IFNγ, IL6, TNFα, ASCAA, ASCAG, CBir1, Fla2, FlaX, and OmpC is measured using an immunoassay. In some instances, the value of the marker is based on a quantile level for each marker.

In some embodiments, each marker is measured in a sample selected from the group consisting of serum, plasma, whole blood, stool, peripheral blood mononuclear cells (PBMC), polymorphonuclear (PMN) cells, a tissue biopsy, and combinations thereof.

In some embodiments, the concentration or level of the marker is relative to the level of the same marker in a patient population without mucosal healing (*e.g.,* an IBD patient population without mucosal healing). The value for each marker in the first set of markers can be summed to form the inflammatory phase marker score. The value for each marker in the second set of markers can be summed to form the proliferation phase marker score. In some embodiments, the algorithm is the summation of the proliferation phase marker values minus the summation of the inflammatory phase marker values.

The algorithm of the method provided herein can identify the phase of mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm identifies the phase of mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some instances, the algorithm used for identifying the phase of mucosal healing does not include (*e.g.,* excludes, is without, or is independent of) values (*e.g.,* scores or measurements, such as, concentrations, amounts or levels) of serology markers, such as ASCA-A, ASCA-G, CBir1, Fla2, FlaX, OmpC, or combinations thereof.

In certain embodiments, the method of identifying the phase of mucosal healing in a subject can be used to select or administer an anti-TNFa antibody. In some instances, the anti-TNFα antibody includes one or more of REMICADE™ (infliximab), ENBREL™ (etanercept), HUMIRA™ (adalimumab), CIMZIA® (certolizumab pegol), and combinations thereof.

In addition, the methods provided herein can be used for monitoring mucosal healing in a subject in order to optimize therapeutic efficacy. In some embodiments, the method includes (a) measuring a first set of markers to form an inflammatory phase marker score; (b) measuring a second set of markers to form a proliferation phase marker score; (c) monitoring mucosal healing in the subject by using an algorithm incorporating the inflammatory phase marker score and the proliferation phase marker score; and (d) optimizing therapeutic efficacy of an anti-TNFa antibody therapy based upon the mucosal healing in the subject.

In some embodiments, the subject has inflammatory bowel disease. In some aspects, inflammatory bowel disease is Crohn's disease or ulcerative colitis.

In some embodiments, the first set of markers comprises one or more selected from the group consisting of TWEAK, CRP, ICAM, SAA, VCAM, IL2, IL8, IL12p70, IL1β, GMCSF, IFNγ, IL6, TNFα, ASCAA, ASCAG, CBir1, Fla2, FlaX, OmpC, an anti-drug antibody (ADA), and combinations thereof. In some embodiments, the second set of markers comprises one or more selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL10, an anti-TNFa antibody, and combinations thereof.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6, *e.g.,* 0, 1, 2, 3, 4, 5, or 6, based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In some instances, the value of the marker described herein is based upon 6 standard samples for each marker.

In some embodiments, one or more markers selected from the group consisting of an anti-TNFa antibody and ADA is measured using a homogeneous mobility shift assay (HMSA). In some instances, the value of the marker is based on a quantile level for each marker.

In some embodiments, one or more markers selected from the group consisting of IL-10, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, and OmpC is measured using an immunoassay. In some instances, the value of the marker is based on a quantile level for each marker.

In some embodiments, each marker is measured in a sample selected from the group consisting of serum, plasma, whole blood, stool, peripheral blood mononuclear cells (PBMC), polymorphonuclear (PMN) cells, and a tissue biopsy.

In some embodiments, the concentration or level of the marker is relative to the level of the same marker in a patient population without mucosal healing (*e.g.,* an IBD patient population without mucosal healing). The value for each marker in the first set of markers can be summed to form the inflammatory phase marker score. The value for each marker in the second set of markers can be summed to form the proliferation phase marker score. In some embodiments, the algorithm is the summation of the proliferation phase marker values minus the summation of the inflammatory phase marker values.

The algorithm of the method provided herein can monitor mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm monitors mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some instances, the algorithm used for monitoring mucosal healing does not include (*e.g.,* excludes, is without, or is independent of) values (*e.g.,* scores or measurements, such as, concentrations, amounts or levels) of serology markers, such as ASCA-A, ASCA-G, CBir1, Fla2, FlaX, OmpC, or combinations thereof.

In certain embodiments, the method of monitoring mucosal healing in a subject can be used to optimize therapeutic efficacy by selecting or administering an appropriate anti-TNFa antibody. In some instances, the anti-TNFa antibody includes one or more of REMICADE™ (infliximab), ENBREL™ (etanercept), HUMIRA™ (adalimumab), CIMZIA® (certolizumab pegol), and combinations thereof.

Also, the methods provided herein are useful for selecting a therapeutic regimen for a subject by monitoring mucosal healing. In some embodiments, the method includes the steps of: (a) measuring a first set of markers to form an inflammatory phase marker score, wherein the inflammatory phase marker score is measured at a plurality of time points over the course of therapy;(b) measuring a second set of markers to form a proliferation phase marker score, wherein the proliferation phase marker score is measured at a plurality of time points over the course of therapy; (c) monitoring mucosal healing in the subject by using an algorithm incorporating the inflammatory phase marker score and the proliferation phase marker score; and (d) selecting an appropriate therapeutic regimen for the individual, wherein the therapeutic regimen promotes mucosal healing and is based upon the algorithm.

In some embodiments, the subject has inflammatory bowel disease. In some aspects, inflammatory bowel disease is Crohn's disease or ulcerative colitis.

In some embodiments, the first set of markers is one or more selected from the group consisting of TWEAK, CRP, ICAM, SAA, VCAM, IL-2, IL-8, IL-12p70, IL1-β, GMCSF, IFNγ, IL6, TNFα, ASCA-A, ASCA-G, CBir1, Fla2, FlaX, OmpC, an anti-drug antibody (ADA), and combinations thereof. In some embodiments, the second set of markers is one or more selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, an anti-TNFa antibody, and combinations thereof.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6 based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In some instances, the value of the marker described herein is based upon 6 standard samples for each marker.

In some embodiments, one or more markers selected from the group consisting of an anti-TNFa antibody and ADA is measured using a homogeneous mobility shift assay (HMSA). In some instances, the value of the marker is based on a quantile level for each marker.

In some embodiments, one wherein one or more markers selected from the group consisting of IL10, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, and OmpC is measured using an immunoassay. In some instances, the value of the marker is based on a quantile level for each marker.

In some embodiments, each marker is measured in a sample selected from the group consisting of serum, plasma, whole blood, stool, peripheral blood mononuclear cells (PBMC), polymorphonuclear (PMN) cells, and a tissue biopsy.

In some embodiments, the concentration or level of the marker is relative to the level of the same marker in a patient population without mucosal healing (*e.g.,* an IBD patient population without mucosal healing). The value for each marker in the first set of markers can be summed to form the inflammatory phase marker score. The value for each marker in the second set of markers can be summed to form the proliferation phase marker score. In some embodiments, the algorithm is the summation of the proliferation phase marker values minus the summation of the inflammatory phase marker values.

The algorithm of the method provided herein can monitor mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm monitors mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some instances, the algorithm used for monitoring mucosal healing does not include *(e.g.,* excludes, is without, or is independent of) values (*e.g.,* scores or measurements, such as, concentrations, amounts or levels) of serology markers, such as ASCA-A, ASCA-G, CBir1, Fla2, FlaX, OmpC, or combinations thereof.

In certain embodiments, the method of monitoring mucosal healing in a subject can be used to select an appropriate anti-TNFa antibody for the subject. In some instances, the anti-TNFa antibody includes one or more of REMICADE™ (infliximab), ENBREL™ (etanercept), HUMIRA™ (adalimumab), CIMZIA® (certolizumab pegol), and combinations thereof.

In some embodiments, the plurality of time points comprises at least 2, 3, 4, 5, 6, 7, 8, 9, 10, 11, 12, 13, 14, 15, 16, 17, 18, 19, 20, 25, 30, 35, 40, 45, 50, or more time points.

In addition, the methods provided herein are useful for predicting the likelihood of mucosal healing in a subject. In some embodiments, the method includes the steps of: (a) measuring a first set of markers to form an inflammatory phase marker score; (b) measuring a second set of markers to form a proliferation phase marker score; (c) monitoring mucosal healing in the subject by using an algorithm incorporating the inflammatory phase marker score and the proliferation phase marker score; and (d) predicting the likelihood of mucosal healing based upon the algorithm.

In some embodiments, the subject has inflammatory bowel disease. In some aspects, inflammatory bowel disease is Crohn's disease or ulcerative colitis.

In some embodiments, the first set of markers is one or more selected from the group consisting of TWEAK, CRP, ICAM, SAA, VCAM, IL-2, IL-8, IL-12p70, IL1-β, GMCSF, IFNγ, IL6, TNFα, ASCA-A, ASCA-G, CBir1, Fla2, FlaX, OmpC, an anti-drug antibody (ADA), and combinations thereof. In some embodiments, the second set of markers is one or more selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, an anti-TNFa antibody, and combinations thereof.

In some embodiments, the anti-drug antibody (ADA) is a member selected from the group consisting of a human anti-chimeric antibody (HACA), a human anti-humanized antibody (HAHA), a human anti-mouse antibody (HAMA), and combinations thereof.

In some embodiments, the anti-TNFa antibody is a member selected from the group consisting of REMICADE™ (infliximab), ENBREL™ (etanercept), HUMIRA™ (adalimumab), CIMZIA® (certolizumab pegol), and combinations thereof.

Each marker of the first set and/or the second set may be assigned a value based upon the concentration or level of the marker. In particular embodiments, each marker of the first set and/or the second set is assigned a value of from 0 to 6, *e.g.,* 0, 1, 2, 3, 4, 5, or 6, based upon the concentration or level of the marker.

In some embodiments, one or more markers selected from the group consisting of EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, and TWEAK is measured using a CEER assay. In some instances, the value of the marker described herein is based upon 6 standard samples for each marker.

In some embodiments, one or more markers selected from the group consisting of an anti-TNFa antibody and ADA is measured using a homogeneous mobility shift assay (HMSA). In some instances, the value of the marker is based on a quantile level for each marker.

In some embodiments, one or more markers selected from the group consisting of IL10, CRP, ICAM, SAA, VCAM, IL-2, IL-8, IL-12p70, IL1B, GMCSF, IFNγ, IL-6, TNFα, ASCA-A, ASCA-G, CBir1, Fla2, FlaX, and OmpC is measured using an immunoassay. In some instances, the value of the marker is based on a quantile level for each marker.

In some embodiments, each marker is measured in a sample selected from the group consisting of serum, plasma, whole blood, stool, peripheral blood mononuclear cells (PBMC), polymorphonuclear (PMN) cells, and a tissue biopsy.

In some embodiments, the concentration or level of the marker is relative to the level of the same marker in a patient population without mucosal healing (*e.g.,* an IBD patient population without mucosal healing). The value for each marker in the first set of markers can be summed to form the inflammatory phase marker score. The value for each marker in the second set of markers can be summed to form the proliferation phase marker score. In some embodiments, the algorithm is the summation of the proliferation phase marker values minus the summation of the inflammatory phase marker values.

The algorithm of the method provided herein can predict the likelihood of mucosal healing independent of clinical confounders. In some instances, the clinical confounders include one or more selected from the group consisting of age of diagnosis, age of last sample, disease location, anal involvement, smoking, surgery, and combinations thereof.

In other embodiments, the algorithm predicts the likelihood of mucosal healing by excluding serology markers. In some embodiments, the excluded serology markers include one or more selected from the group consisting of ASCA-A, ASCA-G, CBir1, Fla2, FlaX, OmpC, and combinations thereof. For example, in some instances, the algorithm used for predicting the likelihood of mucosal healing does not include (*e.g*., excludes, is without, or is independent of) values (*e.g*., scores or measurements, such as, concentrations, amounts or levels) of serology markers, such as ASCA-A, ASCA-G, CBir1, Fla2, FlaX, OmpC, or combinations thereof.

In certain embodiments, the method of predicting the likelihood of mucosal healing in a subject can be used to select an appropriate therapeutic regimen such as an anti-TNFa antibody. In some instances, the anti-TNFa antibody includes one or more of REMICADE™ (infliximab), ENBREL™ (etanercept), HUMIRA™ (adalimumab), CIMZIA® (certolizumab pegol), and combinations thereof.

In some embodiments, the algorithm is used to predict relapse of a disease.

The methods described herein can be used to evaluate the phase of mucosal healing, such as an inflammatory phase, a proliferation phase, or a remodeling phase, in an IBD individual receiving anti-TNFa therapy. In certain aspects, in both UC and CD patients, as shown herein, mucosal healing can change the natural course of the disease by decreasing relapse rates, and/or the need for surgery. Still further, mucosal healing can reduce the development of long-term disease complications, such as bowel damage in CD and colorectal cancer in UC. Thus, it is important to continually monitor a subject while on therapies to ensure the progress of mucosal healing.

### B. Measuring Markers

As described herein, the methods for assessing mucosal healing in a subject include measuring the concentration or level of a first set of markers used to form the inflammatory phase marker score, wherein at least one or a plurality (*e.g*., at least 2, 3, 4, 5, 6, 7, 8, 9, 10, 11, 12, 13, 14, 15, 16, 17, 18, 19, or 20) of the markers are selected from the group consisting of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, an anti-drug antibody (ADA), and combinations thereof. In some embodiments, the methods include measuring a combination of at least two markers selected from the group consisting of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, and an ADA, *e.g.,* TWEAK and CRP, TWEAK and ICAM, TWEAK and SAA, TWEAK and VCAM, TWEAK and IL-2, TWEAK and IL-8, TWEAK and IL-12p70, TWEAK and IL-1β, TWEAK and GMCSF, TWEAK and IFNγ, TWEAK and IL-6, TWEAK and TNFα, TWEAK and ASCA-A, TWEAK and ASCA-G, TWEAK and CBir1, TWEAK and Fla2, TWEAK and FlaX, TWEAK and OmpC, TWEAK and ADA, CRP and ICAM, CRP and SAA, CRP and VCAM, CRP and IL-2, CRP and IL-8, CRP and IL-12p70, CRP and IL-1β, CRP and GMCSF, CRP and IFNγ, CRP and IL-6, CRP and TNFα, CRP and ASCA-A, CRP and ASCA-G, CRP and CBir1, CRP and Fla2, CRP and FlaX, CRP and OmpC, CRP and ADA, ICAM and SAA, ICAM and VCAM, ICAM and IL-2, ICAM and IL-8, ICAM and IL-12p70, ICAM and IL-1β, ICAM and GMCSF, ICAM and IFNγ, ICAM and IL-6, ICAM and TNFα, ICAM and ASCA-A, ICAM and ASCA-G, ICAM and CBir1, ICAM and Fla2, ICAM and FlaX, ICAM and OmpC, ICAM and ADA, SAA and VCAM, SAA and IL-2, IL-8, SAA and IL-12p70, SAA and IL-1β, SAA and GMCSF, SAA and IFNγ, SAA and IL-6, SAA and TNFα, SAA and ASCA-A, SAA and ASCA-G, SAA and CBir1, SAA and Fla2, SAA and FlaX, SAA and OmpC, SAA and ADA, VCAM and IL-2, VCAM and IL-8, VCAM and IL-12p70, VCAM and IL-1β, VCAM and GMCSF, VCAM and IFNγ, VCAM and IL-6, VCAM and TNFα, VCAM and ASCA-A, VCAM and ASCA-G, VCAM and CBir1, VCAM and Fla2, VCAM and FlaX, VCAM and OmpC, VCAM and ADA, IL-2 and IL-8, IL-2 and IL-12p70, IL-2 and IL-1β, IL-2 and GMCSF, IL-2 and IFNγ, IL-2 and IL-6, IL-2 and TNFα, IL-2 and ASCA-A, IL-2 and ASCA-G, IL-2 and CBir1, IL-2 and Fla2, IL-2 and FlaX, IL-2 and OmpC, IL-2 and ADA, IL-8 and IL-12p70, IL-8 and IL-1β, IL-8 and GMCSF, IL-8 and IFNγ, IL-8 and IL-6, IL-8 and TNFα, IL-8 and ASCA-A, IL-8 and ASCA-G, IL-8 and CBir1, IL-8 and Fla2, IL-8 and FlaX, IL-8 and OmpC, IL-8 and ADA, IL-12p70 and IL-1β, IL-12p70 and GMCSF, IL-12p70 and IFNγ, IL-12p70 and IL-6, IL-12p70 and TNFα, IL-12p70 and ASCA-A, IL-12p70 and ASCA-G, IL-12p70 and CBir1, IL-12p70 and Fla2, IL-12p70 and FlaX, IL-12p70 and OmpC, IL-12p70 and ADA, , IL-1β and GMCSF, IL-Iβ and IFNγ, IL-Iβ and IL-6, IL-Iβ and TNFα, IL-Iβ and ASCA-A, IL-1β and ASCA-G, IL-Iβ and CBir1, IL-Iβ and Fla2, IL-Iβ and FlaX, IL-Iβ and OmpC, IL-Iβ and ADA, GMCSF and IFNγ, GMCSF and IL-6, GMCSF and TNFα, GMCSF and ASCA-A, GMCSF and ASCA-G, GMCSF and CBir1, GMCSF and Fla2, GMCSF and FlaX, GMCSF and OmpC, GMCSF and ADA, IFNγ and IL-6, IFNγ and TNFα, IFNγ and ASCA-A, IFNγ and ASCA-G, IFNγ and CBir1, IFNγ and Fla2, IFNγ and FlaX, IFNγ and OmpC, IFNγ and ADA, IL-6 and TNFα, IL-6 and ASCA-A, IL-6 and ASCA-G, IL-6 and CBir1, IL-6 and Fla2, IL-6 and FlaX, IL-6 and OmpC, IL-6 and ADA, TNFα and ASCA-A, TNFα and ASCA-G, TNFα and CBir1, TNFα and Fla2, TNFα and FlaX, TNFα and OmpC, TNFα and ADA, ASCA-A and ASCA-G, ASCA-A and CBir1, ASCA-A and Fla2, ASCA-A and FlaX, ASCA-A and OmpC, ASCA-A and ADA, ASCA-G and CBir1, ASCA-G and Fla2, ASCA-G and FlaX, ASCA-G and OmpC, ASCA-G and ADA, CBirl and Fla2, CBirl and FlaX, CBirl and OmpC, CBirl and ADA, Fla2 and FlaX, Fla2 and OmpC, Fla2 and ADA, FlaX and OmpC, FlaX and ADA, OmpC and ADA, and the like. In some instances, the combination of at least two markers can further include at least 1, 2, 3, 4, 5, 6, 7, 8, 9, 10, 11, 12, 13, 14, 15, 16, 17, or 18 of the other markers selected from the first set of markers to form an inflammatory phase marker score.

In some embodiments, the methods include measuring a combination of at least three markers selected from the group consisting of TWEAK, CRP, ICAM, SAA, VCAM, IL-2, IL-8, IL-12p70, IL-1β, GMCSF, IFNγ, IL-6, TNFα, ASCA-A, ASCA-G, CBir1, Fla2, FlaX, OmpC, and an ADA, *e.g.,* TWEAK, CRP and ICAM; TWEAK, CRP and SAA; TWEAK, CRP and VCAM; TWEAK, CRP and IL-2; TWEAK, CRP and IL-8; TWEAK, CRP and IL-12p70; TWEAK, CRP and IL-1β; TWEAK, CRP and GMCSF; TWEAK, CRP and IFNγ; TWEAK, CRP and IL-6; TWEAK, CRP and TNFα; TWEAK, CRP and ASCA-A; TWEAK, CRP and ASCA-G; TWEAK, CRP and CBir1; TWEAK, CRP and Fla2; TWEAK, CRP and FlaX; TWEAK, CRP and OmpC; TWEAK, CRP and ADA; TWEAK, ICAM and SAA; TWEAK, ICAM and VCAM; TWEAK, ICAM and IL-2; TWEAK, ICAM and IL-8; TWEAK, ICAM and IL-12p70; TWEAK, ICAM and IL-1β; TWEAK, ICAM and GMCSF; TWEAK, ICAM and IFNγ; TWEAK, ICAM and IL-6; TWEAK, ICAM and TNFα; TWEAK, ICAM and ASCA-A; TWEAK, ICAM and ASCA-G; TWEAK, ICAM and CBir1; TWEAK, ICAM and Fla2; TWEAK, ICAM and FlaX; TWEAK, ICAM and OmpC; TWEAK, ICAM and ADA; TWEAK, SAA and VCAM; TWEAK, SAA and IL-2; TWEAK, SAA and IL-8; TWEAK, SAA and IL-12p70; TWEAK, SAA and IL-1β; TWEAK, SAA and GMCSF; TWEAK, SAA and IFNγ; TWEAK, SAA and IL-6; TWEAK, SAA and TNFα; TWEAK, SAA and ASCA-A; TWEAK, SAA and ASCA-G; TWEAK, SAA and CBir1; TWEAK, SAA and Fla2; TWEAK, SAA and FlaX; TWEAK, SAA and OmpC; TWEAK, SAA and ADA; TWEAK, VCAM and IL-2; TWEAK, VCAM and IL-8; TWEAK, VCAM and IL-12p70; TWEAK, VCAM and IL-1β; TWEAK, VCAM and GMCSF; TWEAK, VCAM and IFNγ; TWEAK, VCAM and IL-6; TWEAK, VCAM and TNFα; TWEAK, VCAM and ASCA-A; TWEAK, VCAM and ASCA-G; TWEAK, VCAM and CBir1; TWEAK, VCAM and Fla2; TWEAK, VCAM and FlaX; TWEAK, VCAM and OmpC; TWEAK, VCAM and ADA; TWEAK, IL-2 and IL-8; TWEAK, IL-2 and IL-12p70; TWEAK, IL-2 and IL-1β; TWEAK, IL-2 and GMCSF; TWEAK, IL-2 and IFNγ; TWEAK, IL-2 and IL-6; TWEAK, IL-2 and TNFα; TWEAK, IL-2 and ASCA-A; TWEAK, IL-2 and ASCA-G; TWEAK, IL-2 and CBir1; TWEAK, IL-2 and Fla2; TWEAK, IL-2 and FlaX; TWEAK, IL-2 and OmpC; TWEAK, IL-2 and ADA; TWEAK, IL-8 and IL-12p70; TWEAK, IL-8 and IL-1β; TWEAK, IL-8 and GMCSF; TWEAK, IL-8 and IFNγ; TWEAK, IL-8 and IL-6; TWEAK, IL-8 and TNFα; TWEAK, IL-8 and ASCA-A; TWEAK, IL-8 and ASCA-G; TWEAK, IL-8 and CBir1; TWEAK, IL-8 and Fla2; TWEAK, IL-8 and FlaX; TWEAK, IL-8 and OmpC; TWEAK, IL-8 and ADA; TWEAK, IL-12p70 and IL-1β; TWEAK, IL-12p70 and GMCSF; TWEAK, IL-12p70 and IFNγ; TWEAK, IL-12p70 and IL-6; TWEAK, IL-12p70 and TNFα; TWEAK, IL-12p70 and ASCA-A; TWEAK, IL-12p70 and ASCA-G; TWEAK, IL-12p70 and CBir1; TWEAK, IL-12p70 and Fla2; TWEAK, IL-12p70 and FlaX; TWEAK, IL-12p70 and OmpC; TWEAK, IL-12p70 and ADA; TWEAK, IL-1β and GMCSF; TWEAK, IL-Iβ and IFNγ; TWEAK, IL-1β and IL-6; TWEAK, IL-1β and TNFα; TWEAK, IL-1β and ASCA-A; TWEAK, IL-Iβ and ASCA-G; TWEAK, IL-Iβ and CBir1; TWEAK, IL-Iβ and Fla2; TWEAK, IL-1β and FlaX; TWEAK, IL-Iβ and OmpC; TWEAK, IL-1β and ADA; TWEAK, GMCSF and IFNγ; TWEAK, GMCSF and IL-6; TWEAK, GMCSF and TNFα; TWEAK, GMCSF and ASCA-A; TWEAK, GMCSF and ASCA-G; TWEAK, GMCSF and CBir1; TWEAK, GMCSF and Fla2; TWEAK, GMCSF and FlaX; TWEAK, GMCSF and OmpC; TWEAK, GMCSF and ADA; TWEAK, IFNγ and IL-6; TWEAK, IFNγ and TNFα; TWEAK, IFNγ and ASCA-A; TWEAK, IFNγ and ASCA-G; TWEAK, IFNγ and CBir1; TWEAK, IFNγ and Fla2; TWEAK, IFNγ and FlaX; TWEAK, IFNγ and OmpC; TWEAK, IFNγ and ADA; TWEAK, IL-6 and TNFα; TWEAK, IL-6 and ASCA-A; TWEAK, IL-6 and ASCA-G; TWEAK, IL-6 and CBir1; TWEAK, IL-6 and Fla2; TWEAK, IL-6 and FlaX; TWEAK, IL-6 and OmpC; TWEAK, IL-6 and ADA; TWEAK, TWEAK, TNFα and ASCA-A; TWEAK, TNFα and ASCA-G; TWEAK, TNFα and CBir1; TWEAK, TNFα and Fla2; TWEAK, TNFα and FlaX; TWEAK, TNFα and OmpC; TWEAK, TNFα and ADA; TWEAK, ASCA-A and ASCA-G; TWEAK, ASCA-A and CBir1; TWEAK, ASCA-A and Fla2; TWEAK, ASCA-A and FlaX; TWEAK, ASCA-A and OmpC; TWEAK, ASCA-A and ADA; TWEAK, ASCA-G and CBir1; TWEAK, ASCA-G and Fla2; TWEAK, ASCA-G and FlaX; TWEAK, ASCA-G and OmpC; TWEAK, ASCA-G and ADA; TWEAK, CBirl and Fla2; TWEAK, CBirl and FlaX; TWEAK, CBirl and OmpC; TWEAK, CBirl and ADA; TWEAK, F1a2 and FlaX; TWEAK, F1a2 and OmpC; TWEAK, F1a2 and ADA; TWEAK, FlaX and OmpC; TWEAK, FlaX and ADA; TWEAK, OmpC and ADA; CRP, TWEAK and ICAM; CRP, TWEAK and SAA; CRP, TWEAK and VCAM; CRP, TWEAK and IL-2; CRP, TWEAK and IL-8; CRP, TWEAK and IL-12p70; CRP, TWEAK and IL-1β; CRP, TWEAK and GMCSF; CRP, TWEAK and IFNγ; CRP, TWEAK and IL-6; CRP, TWEAK and TNFα; CRP, TWEAK and ASCA-A; CRP, TWEAK and ASCA-G; CRP, TWEAK and CBir1; CRP, TWEAK and Fla2; CRP, TWEAK and FlaX; CRP, TWEAK and OmpC; CRP, TWEAK and ADA; CRP, ICAM and SAA; CRP, ICAM and VCAM; CRP, ICAM and IL-2; CRP, ICAM and IL-8; CRP, ICAM and IL-12p70; CRP, ICAM and IL-1β; CRP, ICAM and GMCSF; CRP, ICAM and IFNγ; CRP, ICAM and IL-6; CRP, ICAM and TNFα; CRP, ICAM and ASCA-A; CRP, ICAM and ASCA-G; CRP, ICAM and CBir1; CRP, ICAM and Fla2; CRP, ICAM and FlaX; CRP, ICAM and OmpC; CRP, ICAM and ADA; CRP, SAA and VCAM; CRP, SAA and IL-2; CRP, SAA and IL-8; CRP, SAA and IL-12p70; CRP, SAA and IL-1β; CRP, SAA and GMCSF; CRP, SAA and IFNγ; CRP, SAA and IL-6; CRP, SAA and TNFα; CRP, SAA and ASCA-A; CRP, SAA and ASCA-G; CRP, SAA and CBir1; CRP, SAA and Fla2; CRP, SAA and FlaX; CRP, SAA and OmpC; CRP, SAA and ADA; CRP, VCAM and IL-2; CRP, VCAM and IL-8; CRP, VCAM and IL-12p70; CRP, VCAM and IL-1β; CRP, VCAM and GMCSF; CRP, VCAM and IFNγ; CRP, VCAM and IL-6; CRP, VCAM and TNFα; CRP, VCAM and ASCA-A; CRP, VCAM and ASCA-G; CRP, VCAM and CBir1; CRP, VCAM and Fla2; CRP, VCAM and FlaX; CRP, VCAM and OmpC; CRP, VCAM and ADA; CRP, IL-2 and IL-8; CRP, IL-2 and IL-12p70; CRP, IL-2 and IL-1β; CRP, IL-2 and GMCSF; CRP, IL-2 and IFNγ; CRP, IL-2 and IL-6; CRP, IL-2 and TNFα; CRP, IL-2 and ASCA-A; CRP, IL-2 and ASCA-G; CRP, IL-2 and CBir1; CRP, IL-2 and Fla2; CRP, IL-2 and FlaX; CRP, IL-2 and OmpC; CRP, IL-2 and ADA; CRP, IL-8 and IL-12p70; CRP, IL-8 and IL-1β; CRP, IL-8 and GMCSF; CRP, IL-8 and IFNγ; CRP, IL-8 and IL-6; CRP, IL-8 and TNFα; CRP, IL-8 and ASCA-A; CRP, IL-8 and ASCA-G; CRP, IL-8 and CBir1; CRP, IL-8 and Fla2; CRP, IL-8 and FlaX; CRP, IL-8 and OmpC; CRP, IL-8 and ADA; CRP,IL-12p70 and IL-1β; CRP, IL-12p70 and GMCSF; CRP, IL-12p70 and IFNγ; CRP, IL-12p70 and IL-6; CRP, IL-12p70 and TNFα; CRP, IL-12p70 and ASCA-A; CRP, IL-12p70 and ASCA-G; CRP, IL-12p70 and CBir1; CRP, IL-12p70 and Fla2; CRP, IL-12p70 and FlaX; CRP, IL-12p70 and OmpC; CRP, IL-12p70 and ADA; CRP, IL-1 β and GMCSF; CRP, IL-1 β and IFNγ; CRP, IL-1 β and IL-6; CRP, IL-1 β and TNFα; CRP, IL-1β and ASCA-A; CRP, IL-1β and ASCA-G; CRP, IL-1β and CBir1; CRP, IL-1β and Fla2; CRP, IL-1β and FlaX; CRP, IL-1β and OmpC; CRP, IL-1β and ADA; CRP, GMCSF and IFNγ; CRP, GMCSF and IL-6; CRP, GMCSF and TNFα; CRP, GMCSF and ASCA-A; CRP, GMCSF and ASCA-G; CRP, GMCSF and CBir1; CRP, GMCSF and Fla2; CRP, GMCSF and FlaX; CRP, GMCSF and OmpC; CRP, GMCSF and ADA; CRP, IFNγ and IL-6; CRP, IFNγ and TNFα; CRP, IFNγ and ASCA-A; CRP, IFNγ and ASCA-G; CRP, IFNγ and CBir1; CRP, IFNγ and Fla2; CRP, IFNγ and FlaX; CRP, IFNγ and OmpC; CRP, IFNγ and ADA; CRP, IL-6 and TNFα; CRP, IL-6 and ASCA-A; CRP, IL-6 and ASCA-G; CRP, IL-6 and CBir1; CRP, IL-6 and Fla2; CRP, IL-6 and FlaX; CRP, IL-6 and OmpC; CRP, IL-6 and ADA; CRP, TNFα and ASCA-A; CRP, TNFα and ASCA-G; CRP, TNFα and CBir1; CRP, TNFα and Fla2; CRP, TNFα and FlaX; CRP, TNFα and OmpC; CRP, TNFα and ADA; CRP, ASCA-A and ASCA-G; CRP, ASCA-A and CBir1; CRP, ASCA-A and Fla2; CRP, ASCA-A and FlaX; CRP, ASCA-A and OmpC; CRP, ASCA-A and ADA; CRP, ASCA-G and CBir1; CRP, ASCA-G and Fla2; CRP, ASCA-G and FlaX; CRP, ASCA-G and OmpC; CRP, ASCA-G and ADA; CRP, CBirl and Fla2; CRP, CBirl and FlaX; CRP, CBirl and OmpC; CRP, CBirl and ADA; CRP, F1a2 and FlaX; CRP, F1a2 and OmpC; CRP, Fla2 and ADA; CRP, FlaX and OmpC; CRP, FlaX and ADA; CRP, OmpC and ADA; ICAM, TWEAK and CRP; ICAM, TWEAK and SAA; ICAM, TWEAK and VCAM; ICAM, TWEAK and IL-2; ICAM, TWEAK and IL-8; ICAM, TWEAK and IL-12p70; ICAM, TWEAK and IL-1β; ICAM, TWEAK and GMCSF; ICAM, TWEAK and IFNγ; ICAM, TWEAK and IL-6; ICAM, TWEAK and TNFα; ICAM, TWEAK and ASCA-A; ICAM, TWEAK and ASCA-G; ICAM, TWEAK and CBir1; ICAM, TWEAK and Fla2; ICAM, TWEAK and FlaX; ICAM, TWEAK and OmpC; ICAM, TWEAK and ADA; ICAM, CRP and SAA; ICAM, CRP and VCAM; ICAM, CRP and IL-2; ICAM, CRP and IL-8; ICAM, CRP and IL-12p70; ICAM, CRP and IL-1β; ICAM, CRP and GMCSF; ICAM, CRP and IFNγ; ICAM, CRP and IL-6; ICAM, CRP and TNFα; ICAM, CRP and ASCA-A; ICAM, CRP and ASCA-G; ICAM, CRP and CBir1; ICAM, CRP and Fla2; ICAM, CRP and FlaX; ICAM, CRP and OmpC; ICAM, CRP and ADA; ICAM, SAA and VCAM; ICAM, SAA and IL-2; ICAM, SAA and IL-8; ICAM, SAA and IL-12p70; ICAM, SAA and IL-1β; ICAM, SAA and GMCSF; ICAM, SAA and IFNγ; ICAM, SAA and IL-6; ICAM, SAA and TNFα; ICAM, SAA and ASCA-A; ICAM, SAA and ASCA-G; ICAM, SAA and CBir1; ICAM, SAA and Fla2; ICAM, SAA and FlaX; ICAM, SAA and OmpC; ICAM, SAA and ADA; ICAM, VCAM and IL-2; ICAM, VCAM and IL-8; ICAM, VCAM and IL-12p70; ICAM, VCAM and IL-1β; ICAM, VCAM and GMCSF; ICAM, VCAM and IFNγ; ICAM, VCAM and IL-6; ICAM, VCAM and TNFα; ICAM, VCAM and ASCA-A; ICAM, VCAM and ASCA-G; ICAM, VCAM and CBir1; ICAM, VCAM and Fla2; ICAM, VCAM and FlaX; ICAM, VCAM and OmpC; ICAM, VCAM and ADA; ICAM, IL-2 and IL-8; ICAM, IL-2 and IL-12p70; ICAM, IL-2 and IL-1β; ICAM, IL-2 and GMCSF; ICAM, IL-2 and IFNγ; ICAM, IL-2 and IL-6; ICAM, IL-2 and TNFα; ICAM, IL-2 and ASCA-A; ICAM, IL-2 and ASCA-G; ICAM, IL-2 and CBir1; ICAM, IL-2 and Fla2; ICAM, IL-2 and FlaX; ICAM, IL-2 and OmpC; ICAM, IL-2 and ADA; ICAM, IL-8 and IL-12p70; ICAM, IL-8 and IL-1β; ICAM, IL-8 and GMCSF; ICAM, IL-8 and IFNγ; ICAM, IL-8 and IL-6; ICAM, IL-8 and TNFα; ICAM, IL-8 and ASCA-A; ICAM, IL-8 and ASCA-G; ICAM, IL-8 and CBir1; ICAM, IL-8 and Fla2; ICAM, IL-8 and FlaX; ICAM, IL-8 and OmpC; ICAM, IL-8 and ADA; ICAM, IL-12p70 and IL-1β; ICAM, IL-12p70 and GMCSF; ICAM, IL-12p70 and IFNγ; ICAM, IL-12p70 and IL-6; ICAM, IL-12p70 and TNFα; ICAM, IL-12p70 and ASCA-A; ICAM, IL-12p70 and ASCA-G; ICAM, IL-12p70 and CBir1; ICAM, IL-12p70 and Fla2; ICAM, IL-12p70 and FlaX; ICAM, IL-12p70 and OmpC; ICAM, IL-12p70 and ADA; CAM, IL-1β and GMCSF; ICAM, IL-1β and IFNγ; ICAM, IL-1β and IL-6; ICAM, IL-1β and TNFα; ICAM, IL-1β and ASCA-A; ICAM, IL-Iβ and ASCA-G; ICAM, IL-Iβ and CBir1; ICAM, IL-Iβ and Fla2; ICAM, IL-1β and FlaX; ICAM, IL-Iβ and OmpC; ICAM, IL-Iβ and ADA; ICAM, GMCSF and IFNγ; ICAM, GMCSF and IL-6; ICAM, GMCSF and TNFα; ICAM, GMCSF and ASCA-A; ICAM, GMCSF and ASCA-G; ICAM, GMCSF and CBir1; ICAM, GMCSF and Fla2; ICAM, GMCSF and FlaX; ICAM, GMCSF and OmpC; ICAM, GMCSF and ADA; ICAM, IFNγ and IL-6; ICAM, IFNγ and TNFα; ICAM, IFNγ and ASCA-A; ICAM, IFNγ and ASCA-G; ICAM, IFNγ and CBir1; ICAM, IFNγ and Fla2; ICAM, IFNγ and FlaX; ICAM, IFNγ and OmpC; ICAM, IFNγ and ADA; ICAM, IL-6 and TNFα; ICAM, IL-6 and ASCA-A; ICAM, IL-6 and ASCA-G; ICAM, IL-6 and CBir1; ICAM, IL-6 and Fla2; ICAM, IL-6 and FlaX; ICAM, IL-6 and OmpC; ICAM, IL-6 and ADA; ICAM, TNFα and ASCA-A; ICAM, TNFα and ASCA-G; ICAM, TNFα and CBir1; ICAM, TNFα and Fla2; ICAM, TNFα and FlaX; ICAM, TNFα and OmpC; ICAM, TNFα and ADA; ICAM, ASCA-A and ASCA-G; ICAM, ASCA-A and CBir1; ICAM, ASCA-A and Fla2; ICAM, ASCA-A and FlaX; ICAM, ASCA-A and OmpC; ICAM, ASCA-A and ADA; ICAM, ASCA-G and CBir1; ICAM, ASCA-G and Fla2; ICAM, ASCA-G and FlaX; ICAM, ASCA-G and OmpC; ICAM, ASCA-G and ADA; ICAM, CBirl and Fla2; ICAM, CBirl and FlaX; ICAM, CBirl and OmpC; ICAM, CBirl and ADA; ICAM, F1a2 and FlaX; ICAM, F1a2 and OmpC; ICAM, F1a2 and ADA; ICAM, FlaX and OmpC; ICAM, FlaX and ADA; ICAM, OmpC and ADA; SAA, TWEAK and CRP; SAA, TWEAK and ICAM; SAA, TWEAK and SAA; SAA, TWEAK and VCAM; SAA, TWEAK and IL-2; SAA, TWEAK and IL-8; SAA, TWEAK and IL-12p70; SAA, TWEAK and IL-1 β; SAA, TWEAK and GMCSF; SAA, TWEAK and IFNγ; SAA, TWEAK and IL-6; SAA, TWEAK and TNFα; SAA, TWEAK and ASCA-A; SAA, TWEAK and ASCA-G; SAA, TWEAK and CBir1; SAA, TWEAK and Fla2; SAA, TWEAK and FlaX; SAA, TWEAK and OmpC; SAA, TWEAK and ADA; SAA, CRP and ICAM; SAA, CRP and VCAM; SAA, CRP and IL-2; SAA, CRP and IL-8; SAA, CRP and IL-12p70; SAA, CRP and IL-1β; SAA, CRP and GMCSF; SAA, CRP and IFNγ; SAA, CRP and IL-6; SAA, CRP and TNFα; SAA, CRP and ASCA-A; SAA, CRP and ASCA-G; SAA, CRP and CBir1; SAA, CRP and Fla2; SAA, CRP and FlaX; SAA, CRP and OmpC; SAA, CRP and ADA; SAA, ICAM and VCAM; SAA, ICAM and IL-2; SAA, ICAM and IL-8; SAA, ICAM and IL-12p70; SAA, ICAM and IL-1β; SAA, ICAM and GMCSF; SAA, ICAM and IFNγ; SAA, ICAM and IL-6; SAA, ICAM and TNFα; SAA, ICAM and ASCA-A; SAA, ICAM and ASCA-G; SAA, ICAM and CBir1; SAA, ICAM and Fla2; SAA, ICAM and FlaX; SAA, ICAM and OmpC; SAA, ICAM and ADA; SAA, VCAM and IL-2; SAA, VCAM and IL-8; SAA, VCAM and IL-12p70; SAA, VCAM and IL-1β; SAA, VCAM and GMCSF; SAA, VCAM and IFNγ; SAA, VCAM and IL-6; SAA, VCAM and TNFα; SAA, VCAM and ASCA-A; SAA, VCAM and ASCA-G; SAA, VCAM and CBir1; SAA, VCAM and Fla2; SAA, VCAM and FlaX; SAA, VCAM and OmpC; SAA, VCAM and ADA; SAA, IL-2 and IL-8; SAA, IL-2 and IL-12p70; SAA, IL-2 and IL-1β; SAA, IL-2 and GMCSF; SAA, IL-2 and IFNγ; SAA, IL-2 and IL-6; SAA, IL-2 and TNFα; SAA, IL-2 and ASCA-A; SAA, IL-2 and ASCA-G; SAA, IL-2 and CBir1; SAA, IL-2 and Fla2; SAA, IL-2 and FlaX; SAA, IL-2 and OmpC; SAA, IL-2 and ADA; SAA, IL-8 and IL-12p70; SAA, IL-8 and IL-1β; SAA, IL-8 and GMCSF; SAA, IL-8 and IFNγ; SAA, IL-8 and IL-6; SAA, IL-8 and TNFα; SAA, IL-8 and ASCA-A; SAA, IL-8 and ASCA-G; SAA, IL-8 and CBir1; SAA, IL-8 and Fla2; SAA, IL-8 and FlaX; SAA, IL-8 and OmpC; SAA, IL-8 and ADA; SAA, IL-12p70 and IL-1β; SAA, IL-12p70 and GMCSF; SAA, IL-12p70 and IFNγ; SAA, IL-12p70 and IL-6; SAA, IL-12p70 and TNFα; SAA, IL-12p70 and ASCA-A; SAA, IL-12p70 and ASCA-G; SAA, IL-12p70 and CBir1; SAA, IL-12p70 and Fla2; SAA, IL-12p70 and FlaX; SAA, IL-12p70 and OmpC; SAA, IL-12p70 and ADA; SAA, IL-1β and GMCSF; SAA, IL-1β and IFNγ; SAA, IL-1β and IL-6; SAA, IL-1β and TNFα; SAA, IL-1β and ASCA-A; SAA, IL-1β and ASCA-G; SAA, IL-1β and CBir1; SAA, IL-1β and Fla2; SAA, IL-1β and FlaX; SAA, IL-1β and OmpC; SAA, IL-1β and ADA; SAA, GMCSF and IFNγ; SAA, GMCSF and IL-6; SAA, GMCSF and TNFα; SAA, GMCSF and ASCA-A; SAA, GMCSF and ASCA-G; SAA, GMCSF and CBir1; SAA, GMCSF and Fla2; SAA, GMCSF and FlaX; SAA, GMCSF and OmpC; SAA, GMCSF and ADA; SAA, IFNγ and IL-6; SAA, IFNγ and TNFα; SAA, IFNγ and ASCA-A; SAA, IFNγ and ASCA-G; SAA, IFNγ and CBir1; SAA, IFNγ and Fla2; SAA, IFNγ and FlaX; SAA, IFNγ and OmpC; SAA, IFNγ and ADA; SAA, IL-6 and TNFα; SAA, IL-6 and ASCA-A; SAA, IL-6 and ASCA-G; SAA, IL-6 and CBir1; SAA, IL-6 and Fla2; SAA, IL-6 and FlaX; SAA, IL-6 and OmpC; SAA, IL-6 and ADA; SAA, TNFα and ASCA-A; SAA, TNFα and ASCA-G; SAA, TNFα and CBir1; SAA, TNFα and Fla2; SAA, TNFα and FlaX; SAA, TNFα and OmpC; SAA, TNFα and ADA; SAA, ASCA-A and ASCA-G; SAA, ASCA-A and CBir1; SAA, ASCA-A and Fla2; SAA, ASCA-A and FlaX; SAA, ASCA-A and OmpC; SAA, ASCA-A and ADA; SAA, ASCA-G and CBir1; SAA, ASCA-G and Fla2; SAA, ASCA-G and FlaX; SAA, ASCA-G and OmpC; SAA, ASCA-G and ADA; SAA, CBirl and Fla2; SAA, CBirl and FlaX; SAA, CBirl and OmpC; SAA, CBirl and ADA; SAA, Fla2 and FlaX; SAA, Fla2 and OmpC; SAA, Fla2 and ADA; SAA, FlaX and OmpC; SAA, FlaX and ADA; SAA, OmpC and ADA; VCAM, TWEAK and CRP; VCAM, TWEAK and ICAM; VCAM, TWEAK and SAA; VCAM, TWEAK and IL-2; VCAM, TWEAK and IL-8; VCAM, TWEAK and IL-12p70; VCAM, TWEAK and IL-1β; VCAM, TWEAK andGMCSF; VCAM, TWEAK and IFNγ; VCAM, TWEAK and IL-6; VCAM, TWEAK and TNFα; VCAM, TWEAK and ASCA-A; VCAM, TWEAK and ASCA-G; VCAM, TWEAK and CBir1; VCAM, TWEAK and Fla2; VCAM, TWEAK and FlaX; VCAM, TWEAK and OmpC; VCAM, TWEAK and ADA; VCAM, CRP and ICAM; VCAM, CRP and SAA; VCAM, CRP and IL-2; VCAM, CRP and IL-8; VCAM, CRP and IL-12p70; VCAM, CRP and IL-1β; VCAM, CRP and GMCSF; VCAM, CRP and IFNγ; VCAM, CRP and IL-6; VCAM, CRP and TNFα; VCAM, CRP and ASCA-A; VCAM, CRP and ASCA-G; VCAM, CRP and CBir1; VCAM, CRP and Fla2; VCAM, CRP and FlaX; VCAM, CRP and OmpC; VCAM, CRP and ADA; VCAM, ICAM and SAA; VCAM, ICAM and IL-2; VCAM, ICAM and IL-8; VCAM, ICAM and IL-12p70; VCAM, ICAM and IL-1β; VCAM, ICAM and GMCSF; VCAM, ICAM and IFNγ; VCAM, ICAM and IL-6; VCAM, ICAM and TNFα; VCAM, ICAM and ASCA-A; VCAM, ICAM and ASCA-G; VCAM, ICAM and CBir1; VCAM, ICAM and Fla2; VCAM, ICAM and FlaX; VCAM, ICAM and OmpC; VCAM, ICAM and ADA; VCAM, SAA and IL-2; VCAM, SAA and IL-8; VCAM, SAA and IL-12p70; VCAM, SAA and IL-1β; VCAM, SAA and GMCSF; VCAM, SAA and IFNγ; VCAM, SAA and IL-6; VCAM, SAA and TNFα; VCAM, SAA and ASCA-A; VCAM, SAA and ASCA-G; VCAM, SAA and CBir1; VCAM, SAA and Fla2; VCAM, SAA and FlaX; VCAM, SAA and OmpC; VCAM, SAA and ADA; VCAM, IL-2 and IL-8; VCAM, IL-2 and IL-12p70; VCAM, IL-2 and IL-1β; VCAM, IL-2 and GMCSF; VCAM, IL-2 and IFNγ; VCAM, IL-2 and IL-6; VCAM, IL-2 and TNFα; VCAM, IL-2 and ASCA-A; VCAM, IL-2 and ASCA-G; VCAM, IL-2 and CBir1; VCAM, IL-2 and Fla2; VCAM, IL-2 and FlaX; VCAM, IL-2 and OmpC; VCAM, IL-2 and ADA; VCAM, IL-8 and IL-12p70; VCAM, IL-8 and IL-1β; VCAM, IL-8 and GMCSF; VCAM, IL-8 and IFNγ; VCAM, IL-8 and IL-6; VCAM, IL-8 and TNFα; VCAM, IL-8 and ASCA-A; VCAM, IL-8 and ASCA-G; VCAM, IL-8 and CBir1; VCAM, IL-8 and Fla2; VCAM, IL-8 and FlaX; VCAM, IL-8 and OmpC; VCAM, IL-8 and ADA; VCAM, IL-12p70 and IL-1β; VCAM, IL-12p70 and GMCSF; VCAM, IL-12p70 and IFNγ; VCAM, IL-12p70 and IL-6; VCAM, IL-12p70 and TNFα; VCAM, IL-12p70 and ASCA-A; VCAM, IL-12p70 and ASCA-G; VCAM, IL-12p70 and CBir1; VCAM, IL-12p70 and Fla2; VCAM, IL-12p70 and FlaX; VCAM, IL-12p70 and OmpC; VCAM, IL-12p70 and ADA; VCAM, IL-1β and GMCSF; VCAM, IL-1β and IFNγ; VCAM, IL-1β and IL-6; VCAM, IL-1β and TNFα; VCAM, IL-Iβ and ASCA-A; VCAM, IL-Iβ and ASCA-G; VCAM, IL-Iβ and CBir1; VCAM, IL-Iβ and Fla2; VCAM, IL-Iβ and FlaX; VCAM, IL-Iβ and OmpC; VCAM, IL-1β and ADA; VCAM, GMCSF and IFNγ; VCAM, GMCSF and IL-6; VCAM, GMCSF and TNFα; VCAM, GMCSF and ASCA-A; VCAM, GMCSF and ASCA-G; VCAM, GMCSF and CBir1; VCAM, GMCSF and Fla2; VCAM, GMCSF and FlaX; VCAM, GMCSF and OmpC; VCAM, GMCSF and ADA; VCAM, IFNγ and IL-6; VCAM, IFNγ and TNFα; VCAM, IFNγ and ASCA-A; VCAM, IFNγ and ASCA-G; VCAM, IFNγ and CBir1; VCAM, IFNγ and Fla2; VCAM, IFNγ and FlaX; VCAM, IFNγ and OmpC; VCAM, IFNγ and ADA; VCAM, IL-6 and TNFα; VCAM, IL-6 and ASCA-A; VCAM, IL-6 and ASCA-G; VCAM, IL-6 and CBir1; VCAM, IL-6 and Fla2; VCAM, IL-6 and FlaX; VCAM, IL-6 and OmpC; VCAM, IL-6 and ADA; VCAM, TNFα and ASCA-A; VCAM, TNFα and ASCA-G; VCAM, TNFα and CBir1; VCAM, TNFα and Fla2; VCAM, TNFα and FlaX; VCAM, TNFα and OmpC; VCAM, TNFα and ADA; VCAM, ASCA-A and ASCA-G; VCAM, ASCA-A and CBir1; VCAM, ASCA-A and Fla2; VCAM, ASCA-A and FlaX; VCAM, ASCA-A and OmpC; VCAM, ASCA-A and ADA; VCAM, ASCA-G and CBir1; VCAM, ASCA-G and Fla2; VCAM, ASCA-G and FlaX; VCAM, ASCA-G and OmpC; VCAM, ASCA-G and ADA; VCAM, CBirl and Fla2; VCAM, CBirl and FlaX; VCAM, CBirl and OmpC; VCAM, CBirl and ADA; VCAM, F1a2 and FlaX; VCAM, F1a2 and OmpC; VCAM, F1a2 and ADA; VCAM, FlaX and OmpC; VCAM, FlaX and ADA; VCAM, OmpC and ADA; IL-2, TWEAK and CRP; IL-2, TWEAK and ICAM; IL-2, TWEAK and SAA; IL-2, TWEAK and VCAM; IL-2, TWEAK and IL-8; IL-2, TWEAK and IL-12p70; IL-2, TWEAK and IL-1β; IL-2, TWEAK and GMCSF; IL-2, TWEAK and IFNγ; IL-2, TWEAK and IL-6; IL-2, TWEAK and TNFα; IL-2, TWEAK and ASCA-A; IL-2, TWEAK and ASCA-G; IL-2, TWEAK and CBir1; IL-2, TWEAK and Fla2; IL-2, TWEAK and FlaX; IL-2, TWEAK and OmpC; IL-2, TWEAK and ADA; IL-2, CRP and ICAM; IL-2, CRP and SAA; IL-2, CRP and VCAM; IL-2, CRP and IL-8; IL-2, CRP and IL-12p70; IL-2, CRP and IL-1β; IL-2, CRP and GMCSF; IL-2, CRP and IFNγ; IL-2, CRP and IL-6; IL-2, CRP and TNFα; IL-2, CRP and ASCA-A; IL-2, CRP and ASCA-G; IL-2, CRP and CBir1; IL-2, CRP and Fla2; IL-2, CRP and FlaX; IL-2, CRP and OmpC; IL-2, CRP and ADA; IL-2, ICAM and SAA; IL-2, ICAM and VCAM; IL-2, ICAM and IL-8; IL-2, ICAM and IL-12p70; IL-2, ICAM and IL-1β; IL-2, ICAM and GMCSF; IL-2, ICAM and IFNγ; IL-2, ICAM and IL-6; IL-2, ICAM and TNFα; IL-2, ICAM and ASCA-A; IL-2, ICAM and ASCA-G; IL-2, ICAM and CBir1; IL-2, ICAM and Fla2; IL-2, ICAM and FlaX; IL-2, ICAM and OmpC; IL-2, ICAM and ADA; IL-2, SAA and VCAM; IL-2, SAA and IL-8; IL-2, SAA and IL-12p70; IL-2, SAA and IL-1β; IL-2, SAA and GMCSF; IL-2, SAA and IFNγ; IL-2, SAA and IL-6; IL-2, SAA and TNFα; IL-2, SAA and ASCA-A; IL-2, SAA and ASCA-G; IL-2, SAA and CBir1; IL-2, SAA and Fla2; IL-2, SAA and FlaX; IL-2, SAA and OmpC; IL-2, SAA and ADA; IL-2, VCAM and IL-8; IL-2, VCAM and IL-12p70; IL-2, VCAM and IL-1β; IL-2, VCAM and GMCSF; IL-2, VCAM and IFNγ; IL-2, VCAM and IL-6; IL-2, VCAM and TNFα; IL-2, VCAM and ASCA-A; IL-2, VCAM and ASCA-G; IL-2, VCAM and CBir1; IL-2, VCAM and Fla2; IL-2, VCAM and FlaX; IL-2, VCAM and OmpC; IL-2, VCAM and ADA; IL-2, IL-8 and IL-12p70; IL-2, IL-8 and IL-1β; IL-2, IL-8 and GMCSF; IL-2, IL-8 and IFNγ; IL-2, IL-8 and IL-6; IL-2, IL-8 and TNFα; IL-2, IL-8 and ASCA-A; IL-2, IL-8 and ASCA-G; IL-2, IL-8 and CBir1; IL-2, IL-8 and Fla2; IL-2, IL-8 and FlaX; IL-2, IL-8 and OmpC; IL-2, IL-8 and ADA; IL-2, IL-12p70 and IL-1β; IL-2, IL-12p70 and GMCSF; IL-2, IL-12p70 and IFNγ; IL-2, IL-12p70 and IL-6; IL-2, IL-12p70 and TNFα; IL-2, IL-12p70 and ASCA-A; IL-2, IL-12p70 and ASCA-G; IL-2, IL-12p70 and CBir1; IL-2, IL-12p70 and Fla2; IL-2, IL-12p70 and FlaX; IL-2, IL-12p70 and OmpC; IL-2, IL-12p70 and ADA; IL-2, IL-1β and GMCSF; IL-2, IL-1β and IFNγ; IL-2, IL-1β and IL-6; IL-2, IL-1β and TNFα; IL-2, IL-1β and ASCA-A; IL-2, IL-1β and ASCA-G; IL-2, IL-1β and CBir1; IL-2, IL-1β and Fla2; IL-2, IL-1β and FlaX; IL-2, IL-1β and OmpC; IL-2, IL-1β and ADA; IL-2, GMCSF and IFNγ; IL-2, GMCSF and IL-6; IL-2, GMCSF and TNFα; IL-2, GMCSF and ASCA-A; IL-2, GMCSF and ASCA-G; IL-2, GMCSF and CBir1; IL-2, GMCSF and Fla2; IL-2, GMCSF and FlaX; IL-2, GMCSF and OmpC; IL-2, GMCSF and ADA; IL-2, IFNγ and IL-6; IL-2, IFNγ and TNFα; IL-2, IFNγ and ASCA-A; IL-2, IFNγ and ASCA-G; IL-2, IFNγ and CBir1; IL-2, IFNγ and Fla2; IL-2, IFNγ and FlaX; IL-2, IFNγ and OmpC; IL-2, IFNγ and ADA; IL-2, IL-6 and TNFα; IL-2, IL-6 and ASCA-A; IL-2, IL-6 and ASCA-G; IL-2, IL-6 and CBir1; IL-2, IL-6 and Fla2; IL-2, IL-6 and FlaX; IL-2, IL-6 and OmpC; IL-2, IL-6 and ADA; IL-2, TNFα and ASCA-A; IL-2, TNFα and ASCA-G; IL-2, TNFα and CBir1; IL-2, TNFα and Fla2; IL-2, TNFα and FlaX; IL-2, TNFα and OmpC; IL-2, TNFα and ADA; IL-2, ASCA-A and ASCA-G; IL-2, ASCA-A and CBir1; IL-2, ASCA-A and Fla2; IL-2, ASCA-A and FlaX; IL-2, ASCA-A and OmpC; IL-2, ASCA-A and ADA; IL-2, ASCA-G and CBir1; IL-2, ASCA-G and Fla2; IL-2, ASCA-G and FlaX; IL-2, ASCA-G and OmpC; IL-2, ASCA-G and ADA; IL-2, CBirl and Fla2; IL-2, CBirl and FlaX; IL-2, CBirl and OmpC; IL-2, CBirl and ADA; IL-2, Fla2 and FlaX; IL-2, Fla2 and OmpC; IL-2, Fla2 and ADA; IL-2, FlaX and OmpC; IL-2, FlaX and ADA; IL-2, OmpC and ADA; IL-8, TWEAK and CRP; IL-8, TWEAK and ICAM; IL-8, TWEAK and SAA; IL-8, TWEAK and VCAM; IL-8, TWEAK and IL-2; IL-8, TWEAK and IL-12p70; IL-8, TWEAK and IL-1β; IL-8, TWEAK and GMCSF; IL-8, TWEAK and IFNγ; IL-8, TWEAK and IL-6; IL-8, TWEAK and TNFα; IL-8, TWEAK and ASCA-A; IL-8, TWEAK and ASCA-G; IL-8, TWEAK and CBir1; IL-8, TWEAK and Fla2; IL-8, TWEAK and FlaX; IL-8, TWEAK and OmpC; IL-8, TWEAK and ADA; IL-8, CRP and ICAM; IL-8, CRP and SAA; IL-8, CRP and VCAM; IL-8, CRP and IL-2; IL-8, CRP and IL-12p70; IL-8, CRP and IL-1β; IL-8, CRP and GMCSF; IL-8, CRP and IFNγ; IL-8, CRP and IL-6; IL-8, CRP and TNFα; IL-8, CRP and ASCA-A; IL-8, CRP and ASCA-G; IL-8, CRP and CBir1; IL-8, CRP and Fla2; IL-8, CRP and FlaX; IL-8, CRP and OmpC; IL-8, CRP and ADA; IL-8, ICAM and SAA; IL-8, ICAM and VCAM; IL-8, ICAM and IL-2; IL-8, ICAM and IL-12p70; IL-8, ICAM and IL-1β; IL-8, ICAM and GMCSF; IL-8, ICAM and IFNγ; IL-8, ICAM and IL-6; IL-8, ICAM and TNFα; IL-8, ICAM and ASCA-A; IL-8, ICAM and ASCA-G; IL-8, ICAM and CBir1; IL-8, ICAM and Fla2; IL-8, ICAM and FlaX; IL-8, ICAM and OmpC; IL-8, ICAM and ADA; IL-8, SAA and VCAM; IL-8, SAA and IL-2; IL-8, SAA and IL-12p70; IL-8, SAA and IL-1β; IL-8, SAA and GMCSF; IL-8, SAA and IFNγ; IL-8, SAA and IL-6; IL-8, SAA and TNFα; IL-8, SAA and ASCA-A; IL-8, SAA and ASCA-G; IL-8, SAA and CBir1; IL-8, SAA and Fla2; IL-8, SAA and FlaX; IL-8, SAA and OmpC; IL-8, SAA and ADA; IL-8, VCAM and IL-2; IL-8, VCAM and IL-12p70; IL-8, VCAM and IL-1β; IL-8, VCAM and GMCSF; IL-8, VCAM and IFNγ; IL-8, VCAM and IL-6; IL-8, VCAM and TNFα; IL-8, VCAM and ASCA-A; IL-8, VCAM and ASCA-G; IL-8, VCAM and CBir1; IL-8, VCAM and Fla2; IL-8, VCAM and FlaX; IL-8, VCAM and OmpC; IL-8, VCAM and ADA;IL-8, IL-2 and IL-12p70; IL-8, IL-2 and IL-1β; IL-8, IL-2 and GMCSF; IL-8, IL-2 and IFNγ; IL-8, IL-2 and IL-6; IL-8, IL-2 and TNFα; IL-8, IL-2 and ASCA-A; IL-8, IL-2 and ASCA-G; IL-8, IL-2 and CBir1; IL-8, IL-2 and Fla2; IL-8, IL-2 and FlaX; IL-8, IL-2 and OmpC; IL-8, IL-2 and ADA; IL-8, IL-12p70 and IL-1β; IL-8, IL-12p70 and GMCSF; IL-8, IL-12p70 and IFNγ; IL-8, IL-12p70 and IL-6; IL-8, IL-12p70 and TNFα; IL-8, IL-12p70 and ASCA-A; IL-8, IL-12p70 and ASCA-G; IL-8, IL-12p70 and CBir1; IL-8, IL-12p70 and Fla2; IL-8, IL-12p70 and FlaX; IL-8, IL-12p70 and OmpC; IL-8, IL-12p70 and ADA; IL-8, IL-Iβ and GMCSF; IL-8, IL-Iβ and IFNγ; IL-8, IL-Iβ and IL-6; IL-8, IL-Iβ and TNFα; IL-8, IL-1β and ASCA-A; IL-8, IL-Iβ and ASCA-G; IL-8, IL-Iβ and CBir1; IL-8, IL-Iβ and Fla2; IL-8, IL-1βand FlaX; IL-8, IL-Iβ and OmpC; IL-8, IL-1β and ADA; IL-8, GMCSF and IFNγ; IL-8, GMCSF and IL-6; IL-8, GMCSF and TNFα; IL-8, GMCSF and ASCA-A; IL-8, GMCSF and ASCA-G; IL-8, GMCSF and CBir1; IL-8, GMCSF and Fla2; IL-8, GMCSF and FlaX; IL-8, GMCSF and OmpC; IL-8, GMCSF and ADA; IL-8, IFNγ and IL-6; IL-8, IFNγ and TNFα; IL-8, IFNγ and ASCA-A; IL-8, IFNγ and ASCA-G; IL-8, IFNγ and CBir1; IL-8, IFNγ and Fla2; IL-8, IFNγ and FlaX; IL-8, IFNγ and OmpC; IL-8, IFNγ and ADA; IL-8, IL-6 and TNFα; IL-8, IL-6 and ASCA-A; IL-8, IL-6 and ASCA-G; IL-8, IL-6 and CBir1; IL-8, IL-6 and Fla2; IL-8, IL-6 and FlaX; IL-8, IL-6 and OmpC; IL-8, IL-6 and ADA;IL-8, TNFα and ASCA-A; IL-8, TNFα and ASCA-G; IL-8, TNFα and CBir1; IL-8, TNFα and Fla2; IL-8, TNFα and FlaX; IL-8, TNFα and OmpC; IL-8, TNFα and ADA; IL-8, ASCA-A and ASCA-G; IL-8, ASCA-A and CBir1; IL-8, ASCA-A and Fla2; IL-8, ASCA-A and FlaX; IL-8, ASCA-A and OmpC; IL-8, ASCA-A and ADA; IL-8, ASCA-G and CBir1; IL-8, ASCA-G and Fla2; IL-8, ASCA-G and FlaX; IL-8, ASCA-G and OmpC; IL-8, ASCA-G and ADA; IL-8, CBirl and Fla2; IL-8, CBirl and FlaX; IL-8, CBirl and OmpC; IL-8, CBirl and ADA; IL-8, Fla2 and FlaX; IL-8, Fla2 and OmpC; IL-8, Fla2 and ADA; IL-8, FlaX and OmpC; IL-8, FlaX and ADA; IL-8, OmpC and ADA; IL-12p70, TWEAK and CRP; IL-12p70, TWEAK and ICAM; IL-12p70, TWEAK and SAA; IL-12p70, TWEAK and VCAM; IL-12p70, TWEAK and IL-2; IL-12p70, TWEAK and IL-8; IL-12p70, TWEAK and IL-1β; IL-12p70, TWEAK and GMCSF; IL-12p70, TWEAK and IFNγ; IL-12p70, TWEAK and IL-6; IL-12p70, TWEAK and TNFα; IL-12p70, TWEAK and ASCA-A; IL-12p70, TWEAK and ASCA-G; IL-12p70, TWEAK and CBir1; IL-12p70, TWEAK and Fla2; IL-12p70, TWEAK and FlaX; IL-12p70, TWEAK and OmpC; IL-12p70, TWEAK and ADA; IL-12p70, CRP and ICAM; IL-12p70, CRP and SAA; IL-12p70, CRP and VCAM; IL-12p70, CRP and IL-2; IL-12p70, CRP and IL-8; IL-12p70, CRP and IL-1β; IL-12p70, CRP and GMCSF; IL-12p70, CRP and IFNγ; IL-12p70, CRP and IL-6; IL-12p70, CRP and TNFα; IL-12p70, CRP and ASCA-A; IL-12p70, CRP and ASCA-G; IL-12p70, CRP and CBir1; IL-12p70, CRP and Fla2; IL-12p70, CRP and FlaX; IL-12p70, CRP and OmpC; IL-12p70, CRP and ADA; IL-12p70, ICAM and SAA; IL-12p70, ICAM and VCAM; IL-12p70, ICAM and IL-2; IL-12p70, ICAM and IL-8; IL-12p70, ICAM and IL-1β; IL-12p70, ICAM and GMCSF; IL-12p70, ICAM and IFNγ; IL-12p70, ICAM and IL-6; IL-12p70, ICAM and TNFα; IL-12p70, ICAM and ASCA-A; IL-12p70, ICAM and ASCA-G; IL-12p70, ICAM and CBir1; IL-12p70, ICAM and Fla2; IL-12p70, ICAM and FlaX; IL-12p70, ICAM and OmpC; IL-12p70, ICAM and ADA; IL-12p70, SAA and VCAM; IL-12p70, SAA and IL-2; IL-12p70, SAA and IL-8; IL-12p70, SAA and IL-1β; IL-12p70, SAA and GMCSF; IL-12p70, SAA and IFNγ; IL-12p70, SAA and IL-6; IL-12p70, SAA and TNFα; IL-12p70, SAA and ASCA-A; IL-12p70, SAA and ASCA-G; IL-12p70, SAA and CBir1; IL-12p70, SAA and Fla2; IL-12p70, SAA and FlaX; IL-12p70, SAA and OmpC; IL-12p70, SAA and ADA; IL-12p70, VCAM and IL-2; IL-12p70, VCAM and IL-8; IL-12p70, VCAM and IL-1β; IL-12p70, VCAM and GMCSF; IL-12p70, VCAM and IFNγ; IL-12p70, VCAM and IL-6; IL-12p70, VCAM and TNFα; IL-12p70, VCAM and ASCA-A; IL-12p70, VCAM and ASCA-G; IL-12p70, VCAM and CBir1; IL-12p70, VCAM and Fla2; IL-12p70, VCAM and FlaX; IL-12p70, VCAM and OmpC; IL-12p70, VCAM and ADA; IL-12p70, IL-2 and IL-8; IL-12p70, IL-2 and IL-1β; IL-12p70, IL-2 and GMCSF; IL-12p70, IL-2 and IFNγ; IL-12p70, IL-2 and IL-6; IL-12p70, IL-2 and TNFα; IL-12p70, IL-2 and ASCA-A; IL-12p70, IL-2 and ASCA-G; IL-12p70, IL-2 and CBir1; IL-12p70, IL-2 and Fla2; IL-12p70, IL-2 and FlaX; IL-12p70, IL-2 and OmpC; IL-12p70, IL-2 and ADA; IL-12p70, IL-8 and IL-1β; IL-12p70, IL-8 and GMCSF; IL-12p70, IL-8 and IFNγ; IL-12p70, IL-8 and IL-6; IL-12p70, IL-8 and TNFα; IL-12p70, IL-8 and ASCA-A; IL-12p70, IL-8 and ASCA-G; IL-12p70, IL-8 and CBir1; IL-12p70, IL-8 and Fla2; IL-12p70, IL-8 and FlaX; IL-12p70, IL-8 and OmpC; IL-12p70, IL-8 and ADA; IL-12p70, IL-1β and GMCSF; IL-12p70, IL-1β and IFNγ; IL-12p70, IL-1β and IL-6; IL-12p70, IL-1β and TNFα; IL-12p70, IL-1β and ASCA-A; IL-12p70, IL-1β and ASCA-G; IL-12p70, IL-1β and CBir1; IL-12p70, IL-1β and Fla2; IL-12p70, IL-1β and FlaX; IL-12p70, IL-1β and OmpC; IL-12p70, IL-1β and ADA; IL-12p70, GMCSF and IFNγ; IL-12p70, GMCSF and IL-6; IL-12p70, GMCSF and TNFα; IL-12p70, GMCSF and ASCA-A; IL-12p70, GMCSF and ASCA-G; IL-12p70, GMCSF and CBir1; IL-12p70, GMCSF and Fla2; IL-12p70, GMCSF and FlaX; IL-12p70, GMCSF and OmpC; IL-12p70, GMCSF and ADA; IL-12p70, IFNγ and IL-6; IL-12p70, IFNγ and TNFα; IL-12p70, IFNγ and ASCA-A; IL-12p70, IFNγ and ASCA-G; IL-12p70, IFNγ and CBir1; IL-12p70, IFNγ and Fla2; IL-12p70, IFNγ and FlaX; IL-12p70, IFNγ and OmpC; IL-12p70, IFNγ and ADA; IL-12p70, IL-6 and TNFα; IL-12p70, IL-6 and ASCA-A; IL-12p70, IL-6 and ASCA-G; IL-12p70, IL-6 and CBir1; IL-12p70, IL-6 and Fla2; IL-12p70, IL-6 and FlaX; IL-12p70, IL-6 and OmpC; IL-12p70, IL-6 and ADA; IL-12p70, TNFα and ASCA-A; IL-12p70, TNFα and ASCA-G; IL-12p70, TNFα and CBir1; IL-12p70, TNFα and Fla2; IL-12p70, TNFα and FlaX; IL-12p70, TNFα and OmpC; IL-12p70, TNFα and ADA; IL-12p70, ASCA-A and ASCA-G; IL-12p70, ASCA-A and CBir1; IL-12p70, ASCA-A and Fla2; IL-12p70, ASCA-A and FlaX; IL-12p70, ASCA-A and OmpC; IL-12p70, ASCA-A and ADA; IL-12p70, ASCA-G and CBir1; IL-12p70, ASCA-G and Fla2; IL-12p70, ASCA-G and FlaX; IL-12p70, ASCA-G and OmpC; IL-12p70, ASCA-G and ADA; IL-12p70, CBirl and Fla2; IL-12p70, CBirl and FlaX; IL-12p70, CBirl and OmpC; IL-12p70, CBirl and ADA; IL-12p70, Fla2 and FlaX; IL-12p70, Fla2 and OmpC; IL-12p70, Fla2 and ADA; IL-12p70, FlaX and OmpC; IL-12p70, FlaX and ADA; IL-12p70, OmpC and ADA; IL-1β, TWEAK and CRP; IL-1β, TWEAK and ICAM; IL-1β, TWEAK and SAA; IL-1β, TWEAK and VCAM; IL-1β, TWEAK and IL-2; IL-1β, TWEAK and IL-8; IL-1β, TWEAK and IL-12p70; IL-11β b, TWEAK and GMCSF; IL-1β, TWEAK and IFNγ; IL-1β, TWEAK and IL-6; IL-1β, TWEAK and TNFα; IL-1b, TWEAK and ASCA-A; IL-1β, TWEAK and ASCA-G; IL-1β, TWEAK and CBir1; IL-1β, TWEAK and Fla2; IL-1β, TWEAK and FlaX; IL-1β, TWEAK and OmpC; IL-1β, TWEAK and ADA; IL-1β, CRP and ICAM; IL-1β, CRP and SAA; IL-1β, CRP and VCAM; IL-1β, CRP and IL-2; IL-1β, CRP and IL-8; IL-1β, CRP and IL-12p70; IL-1b, CRP and GMCSF; IL-1b, CRP and IFNγ; IL-1β, CRP and IL-6; IL-1β, CRP and TNFα; IL-1β, CRP and ASCA-A; IL-1b, CRP and ASCA-G; IL-1β, CRP and CBir1; IL-1β, CRP and Fla2; IL-1β, CRP and FlaX; IL-1β, CRP and OmpC; IL-1β, CRP and ADA; IL-1β, ICAM and SAA; IL-1β, ICAM and VCAM; IL-1β, ICAM and IL-2; IL-1β, ICAM and IL-8; IL-1β, ICAM and IL-12p70; IL-1β, ICAM and GMCSF; IL-1β, ICAM and IFNγ; IL-1β, ICAM and IL-6; IL-1β, ICAM and TNFα; IL-1β, ICAM and ASCA-A; IL-1β, ICAM and ASCA-G; IL-1β, ICAM and CBir1; IL-1β, ICAM and Fla2; IL-1β, ICAM and FlaX; IL-1β, ICAM and OmpC; IL-1β, ICAM and ADA; IL-1β, SAA and VCAM; IL-1β, SAA and IL-2; IL-1β, SAA, and IL-8;IL-1β, SAA and IL-12p70; IL-1β, SAA and GMCSF; IL-1β, SAA and IFNγ; IL-1β, SAA and IL-6; IL-1β, SAA and TNFα; IL-1β, SAA and ASCA-A; IL-1β, SAA and ASCA-G; IL-1β, SAA and CBir1; IL-1β, SAA and Fla2; IL-1β, SAA and FlaX; IL-1β, SAA and OmpC; IL-1β, SAA and ADA; IL-1β, VCAM and IL-2; IL-1β, VCAM and IL-8; IL-1β, VCAM and IL-12p70; IL-1β, VCAM and GMCSF; IL-1β, VCAM and IFNγ; IL-1β, VCAM and IL-6; IL-1β, VCAM and TNFα; IL-1β, VCAM and ASCA-A; IL-1β, VCAM and ASCA-G; IL-1β, VCAM and CBir1; IL-1β, VCAM and Fla2; IL-1β, VCAM and FlaX; IL-1β, VCAM and OmpC; IL-1β, VCAM and ADA; IL-1β, IL-2 and IL-8; IL-1β, IL-2 and IL-12p70; IL-1β, IL-2 and GMCSF; IL-1β, IL-2 and IFNγ; IL-1β, IL-2 and IL-6; IL-1β, IL-2 and TNFα; IL-1β, IL-2 and ASCA-A; IL-1β, IL-2 and ASCA-G; IL-1β, IL-2 and CBir1; IL-1β, IL-2 and Fla2; IL-1β, IL-2 and FlaX; IL-1β, IL-2 and OmpC; IL-1β, IL-2 and ADA; IL-1β, IL-8 and IL-12p70; IL-1β, IL-8 and GMCSF; IL-1β, IL-8 and IFNγ; IL-1β, IL-8 and IL-6; IL-1β, IL-8 and TNFα; IL-1β, IL-8 and ASCA-A; IL-1β, IL-8 and ASCA-G; IL-1β, IL-8 and CBir1; IL-1β, IL-8 and Fla2; IL-1β, IL-8 and FlaX; IL-1β, IL-8 and OmpC; IL-1β, IL-8 and ADA; IL-1β, IL-12p70 and GMCSF; IL-1β, IL-12p70 and IFNγ; IL-1β, IL-12p70 and IL-6; IL-1β, IL-12p70 and TNFα; IL-1β, IL-12p70 and ASCA-A; IL-1β, IL-12p70 and ASCA-G; IL-1β, IL-12p70 and CBir1; IL-1β, IL-12p70 and Fla2; IL-1β, IL-12p70 and FlaX; IL-1β, IL-12p70 and OmpC; IL-1β, IL-12p70 and ADA; IL-1β, GMCSF and IFNγ; IL-1β, GMCSF and IL-6; IL-1β, GMCSF and TNFα; IL-1β, GMCSF and ASCA-A; IL-1β, GMCSF and ASCA-G; IL-1β, GMCSF and CBir1; IL-1β, GMCSF and Fla2; IL-1β, GMCSF and FlaX; IL-1β, GMCSF and OmpC; IL-1β, GMCSF and ADA; IL-1β, IFNγ and IL-6; IL-1β, IFNγ and TNFα; IL-1β, IFNγ and ASCA-A; IL-1β, IFNγ and ASCA-G; IL-1β, IFNγ and CBir1; IL-1β, IFNγ and Fla2; IL-1β, IFNγ and FlaX; IL-1β, IFNγ and OmpC; IL-1β, IFNγ and ADA; IL-1β, IL-6 and TNFα; IL-1β, IL-6 and ASCA-A; IL-1β, IL-6 and ASCA-G; IL-1β, IL-6 and CBir1; IL-1β, IL-6 and Fla2; IL-1β, IL-6 and FlaX; IL-1β, IL-6 and OmpC; IL-1β, IL-6 and ADA; IL-1β, TNFα and ASCA-A; IL-1β, TNFα and ASCA-G; IL-1β, TNFα and CBir1; IL-1β, TNFα and Fla2; IL-1β, TNFα and FlaX; IL-1β, TNFα and OmpC; IL-1β, TNFα and ADA; IL-1β, ASCA-A and ASCA-G; IL-1β, ASCA-A and CBir1; IL-1β, ASCA-A and Fla2; IL-1β, ASCA-A and FlaX; IL-1β, ASCA-A and OmpC; IL-1β, ASCA-A and ADA; IL-1β, ASCA-G and CBirl; IL-1β, ASCA-G and Fla2; IL-1β, ASCA-G and FlaX; IL-1β, ASCA-G and OmpC; IL-1β, ASCA-G and ADA; IL-1β, CBirl and Fla2; IL-1β, CBirl and FlaX; IL-1β, CBirl and OmpC; IL-1β, CBirl and ADA; IL-1β, Fla2 and FlaX; IL-1β, Fla2 and OmpC; IL-1β, Fla2 and ADA; IL-1β, FlaX and OmpC; IL-1β, FlaX and ADA; IL-1β, OmpC and ADA; GMCSF, TWEAK and CRP; GMCSF, TWEAK and ICAM; GMCSF, TWEAK and SAA; GMCSF, TWEAK and VCAM; GMCSF, TWEAK and IL-2; GMCSF, TWEAK and IL-8; GMCSF, TWEAK and IL-12p70; GMCSF, TWEAK and IL-1β; GMCSF, TWEAK and IFNγ; GMCSF, TWEAK and IL-6; GMCSF, TWEAK and TNFα; GMCSF, TWEAK and ASCA-A; GMCSF, TWEAK and ASCA-G; GMCSF, TWEAK and CBir1; GMCSF, TWEAK and Fla2; GMCSF, TWEAK and FlaX; GMCSF, TWEAK and OmpC; GMCSF, TWEAK and ADA; GMCSF, CRP and ICAM; GMCSF, CRP and SAA; GMCSF, CRP and VCAM; GMCSF, CRP and IL-2; GMCSF, CRP and IL-8; GMCSF, CRP and IL-12p70; GMCSF, CRP and IL-1β; GMCSF, CRP and IFNγ; GMCSF, CRP and IL-6; GMCSF, CRP and TNFα; GMCSF, CRP and ASCA-A; GMCSF, CRP and ASCA-G; GMCSF, CRP and CBir1; GMCSF, CRP and Fla2; GMCSF, CRP and FlaX; GMCSF, CRP and OmpC; GMCSF, CRP and ADA; GMCSF, ICAM and SAA; GMCSF, ICAM and VCAM; GMCSF, ICAM and IL-2; GMCSF, ICAM and IL-8; GMCSF, ICAM and IL-12p70; GMCSF, ICAM and IL-1β; GMCSF, ICAM and IFNγ; GMCSF, ICAM and IL-6; GMCSF, ICAM and TNFα; GMCSF, ICAM and ASCA-A; GMCSF, ICAM and ASCA-G; GMCSF, ICAM and CBir1; GMCSF, ICAM and Fla2; GMCSF, ICAM and FlaX; GMCSF, ICAM and OmpC; GMCSF, ICAM and ADA; GMCSF, SAA and VCAM; GMCSF, SAA and IL-2; GMCSF, SAA and IL-8; GMCSF, SAA and IL-12p70; GMCSF, SAA and IL-1β; GMCSF, SAA and IFNγ; GMCSF, SAA and IL-6; GMCSF, SAA and TNFα; GMCSF, SAA and ASCA-A; GMCSF, SAA and ASCA-G; GMCSF, SAA and CBir1; GMCSF, SAA and Fla2; GMCSF, SAA and FlaX; GMCSF, SAA and OmpC; GMCSF, SAA and ADA; GMCSF, VCAM and IL-2; GMCSF, VCAM and IL-8; GMCSF, VCAM and IL-12p70; GMCSF, VCAM and IL-1β; GMCSF, VCAM and IFNγ; GMCSF, VCAM and IL-6; GMCSF, VCAM and TNFα; GMCSF, VCAM and ASCA-A; GMCSF, VCAM and ASCA-G; GMCSF, VCAM and CBir1; GMCSF, VCAM and Fla2; GMCSF, VCAM and FlaX; GMCSF, VCAM and OmpC; GMCSF, VCAM and ADA; GMCSF, IL-2 and IL-8; GMCSF, IL-2 and IL-12p70; GMCSF, IL-2 and IL-1β; GMCSF, IL-2 and IFNγ; GMCSF, IL-2 and IL-6; GMCSF, IL-2 and TNFα; GMCSF, IL-2 and ASCA-A; GMCSF, IL-2 and ASCA-G; GMCSF, IL-2 and CBir1; GMCSF, IL-2 and Fla2; GMCSF, IL-2 and FlaX; GMCSF, IL-2 and OmpC; GMCSF, IL-2 and ADA; GMCSF, IL-8 and IL-12p70; GMCSF, IL-8 and IL-1β; GMCSF, IL-8 and IFNγ; GMCSF, IL-8 and IL-6; GMCSF, IL-8 and TNFα; GMCSF, IL-8 and ASCA-A; GMCSF, IL-8 and ASCA-G; GMCSF, IL-8 and CBir1; GMCSF, IL-8 and Fla2; GMCSF, IL-8 and FlaX; GMCSF, IL-8 and OmpC; GMCSF, IL-8 and ADA; GMCSF, IL-12p70 and IL-1β; GMCSF, IL-12p70 and IFNγ; GMCSF, IL-12p70 and IL-6; GMCSF, IL-12p70 and TNFα; GMCSF, IL-12p70 and ASCA-A; GMCSF, IL-12p70 and ASCA-G; GMCSF, IL-12p70 and CBir1; GMCSF, IL-12p70 and Fla2; GMCSF, IL-12p70 and FlaX; GMCSF, IL-12p70 and OmpC; GMCSF, IL-12p70 and ADA; GMCSF, IL-1β and IFNγ; GMCSF, IL-1β and IL-6; GMCSF, IL-1β and TNFα; GMCSF, IL-1β and ASCA-A; GMCSF, IL-1β and ASCA-G; GMCSF, IL-1β and CBir1; GMCSF, IL-1β and Fla2; GMCSF, IL-1β and FlaX; GMCSF, IL-1β and OmpC; GMCSF, IL-1β and ADA; GMCSF, IFNγ and IL-6; GMCSF, IFNγ and TNFα; GMCSF, IFNγ and ASCA-A; GMCSF, IFNγ and ASCA-G; GMCSF, IFNγ and CBir1; GMCSF, IFNγ and Fla2; GMCSF, IFNγ and FlaX; GMCSF, IFNγ and OmpC; GMCSF, IFNγ and ADA; GMCSF, IL-6 and TNFα; GMCSF, IL-6 and ASCA-A; GMCSF, IL-6 and ASCA-G; GMCSF, IL-6 and CBir1; GMCSF, IL-6 and Fla2; GMCSF, IL-6 and FlaX; GMCSF, IL-6 and OmpC; GMCSF, IL-6 and ADA; GMCSF, TNFα and ASCA-A; GMCSF, TNFα and ASCA-G; GMCSF, TNFα and CBir1; GMCSF, TNFα and Fla2; GMCSF, TNFα and FlaX; GMCSF, TNFα and OmpC; GMCSF, TNFα and ADA; GMCSF, ASCA-A and ASCA-G; GMCSF, ASCA-A and CBir1; GMCSF, ASCA-A and Fla2; GMCSF, ASCA-A and FlaX; GMCSF, ASCA-A and OmpC; GMCSF, ASCA-A and ADA; GMCSF, ASCA-G and CBir1; GMCSF, ASCA-G and Fla2; GMCSF, ASCA-G and FlaX; GMCSF, ASCA-G and OmpC; GMCSF, ASCA-G and ADA; GMCSF, CBirl and Fla2; GMCSF, CBirl and FlaX; GMCSF, CBirl and OmpC; GMCSF, CBirl and ADA; GMCSF, Fla2 and FlaX; GMCSF, Fla2 and OmpC; GMCSF, Fla2 and ADA; GMCSF, FlaX and OmpC; GMCSF, FlaX and ADA; GMCSF, OmpC and ADA; IFNγ, TWEAK and CRP; IFNγ, TWEAK and ICAM; IFNγ, TWEAK and SAA; IFNγ, TWEAK and VCAM; IFNγ, TWEAK and IL-2; IFNγ, TWEAK and IL-8; IFNγ, TWEAK and IL-12p70; IFNγ, TWEAK and IL-1β; IFNγ, TWEAK and GMCSF; IFNγ, TWEAK and IL-6; IFNγ, TWEAK and TNFα; IFNγ, TWEAK and ASCA-A; IFNγ, TWEAK and ASCA-G; IFN, TWEAK and CBir1; IFNγ, TWEAK and Fla2; IFNγ, TWEAK and FlaX; IFNγ, TWEAK and OmpC; IFNγ, TWEAK and ADA; IFNγ, CRP and ICAM; IFNγ, CRP and SAA; IFNγ, CRP and VCAM; IFNγ, CRP and IL-2; IFNγ, CRP and IL-8; IFNγ, CRP and IL-12p70; IFNγ, CRP and IL-1β; IFNγ, CRP and GMCSF; IFNγ, CRP and IL-6; IFNγ, CRP and TNFα; IFNγ, CRP and ASCA-A; IFNγ, CRP and ASCA-G; IFNγ, CRP and CBir1; IFNγ, CRP and Fla2; IFNγ, CRP and FlaX; IFNγ, CRP and OmpC; IFNγ, CRP and ADA; IFNγ, ICAM and SAA; IFNγ, ICAM and VCAM; IFNγ, ICAM and IL-2; IFNγ, ICAM and IL-8; IFNγ, ICAM and IL-12p70; IFNγ, ICAM and IL-1β; IFNγ, ICAM and GMCSF; IFNγ, ICAM and IL-6; IFNγ, ICAM and TNFα; IFNγ, ICAM and ASCA-A; IFNγ, ICAM and ASCA-G; IFNγ, ICAM and CBir1; IFNγ, ICAM and Fla2; IFNγ, ICAM and FlaX; IFNγ, ICAM and OmpC; IFNγ, ICAM and ADA; IFNγ, SAA and VCAM; IFNγ, SAA and IL-2; IFNγ, SAA and IL-8; IFNγ, SAA and IL-12p70; IFNγ, SAA and IL-1β; IFNγ, SAA and GMCSF; IFNγ, SAA and IL-6; IFNγ, SAA and TNFα; IFNγ, SAA and ASCA-A; IFNγ, SAA and ASCA-G; IFNγ, SAA and CBir1; IFNγ, SAA and Fla2; IFNγ, SAA and FlaX; IFN, SAA and OmpC; IFNγ, SAA and ADA;IFNγ, VCAM and IL-2; IFNγ, VCAM and IL-8; IFNγ, VCAM and IL-12p70; IFNγ, VCAM and IL-1β; IFNγ, VCAM and GMCSF; IFNγ, VCAM and IL-6; IFNγ, VCAM and TNFα; IFNγ, VCAM and ASCA-A; IFNγ, VCAM and ASCA-G; IFNγ, VCAM and CBir1; IFNγ, VCAM and Fla2; IFNγ, VCAM and FlaX; IFNγ, VCAM and OmpC; IFNγ, VCAM and ADA; IFNγ, IL-2 and IL-8; IFNγ, IL-2 and IL-12p70; IFNγ, IL-2 and IL-1β; IFNγ, IL-2 and GMCSF; IFNγ, IL-2 and IL-6; IFNγ, IL-2 and TNFα; IFNγ, IL-2 and ASCA-A; IFNγ, IL-2 and ASCA-G; IFNγ, IL-2 and CBir1; IFNγ, IL-2 and Fla2; IFNγ, IL-2 and FlaX; IFNγ, IL-2 and OmpC; IFNγ, IL-2 and ADA; IFNγ, IL-8 and IL-12p70; IFNγ, IL-8 and IL-1β; IFNγ, IL-8 and GMCSF; IFNγ, IL-8 and IL-6; IFNγ, IL-8 and TNFα; IFNγ, IL-8 and ASCA-A; IFNγ, IL-8 and ASCA-G; IFNγ, IL-8 and CBir1; IFNγ, IL-8 and Fla2; IFNγ, IL-8 and FlaX; IFNγ, IL-8 and OmpC; IFNγ, IL-8 and ADA; IFNγ, IL-12p70 and IL-1β; IFNγ, IL-12p70 and GMCSF; IFNγ, IL-12p70 and IL-6; IFNγ, IL-12p70 and TNFα; IFNγ, IL-12p70 and ASCA-A; IFNγ, IL-12p70 and ASCA-G; IFNγ, IL-12p70 and CBir1; IFNγ, IL-12p70 and Fla2; IFNγ, IL-12p70 and FlaX; IFNγ, IL-12p70 and OmpC; IFNγ, IL-12p70 and ADA; IFNγ, IL-1β and GMCSF; IFNγ, IL-1β and IL-6; IFNγ, IL-1β and TNFα; IFNγ, IL-1β and ASCA-A; IFNγ, IL-1β and ASCA-G; IFNγ, IL-1β and CBir1; IFNγ, IL-1β and Fla2; IFNγ, IL-1β and FlaX; IFNγ, IL-1β and OmpC; IFNγ, IL-1β and ADA; IFNγ, GMCSF and IL-6; IFNγ, GMCSF and TNFα; IFNγ, GMCSF and ASCA-A; IFNγ, GMCSF and ASCA-G; IFNγ, GMCSF and CBir1; IFNγ, GMCSF and Fla2; IFNγ, GMCSF and FlaX; IFNγ, GMCSF and OmpC; IFNγ, GMCSF and ADA; IFNγ, IL-6 and TNFα; IFNγ, IL-6 and ASCA-A; IFNγ, IL-6 and ASCA-G; IFNγ, IL-6 and CBir1; IFNγ, IL-6 and Fla2; IFNγ, IL-6 and FlaX; IFNγ, IL-6 and OmpC; IFNγ, IL-6 and ADA; IFNγ, TNFα and ASCA-A; IFNγ, TNFα and ASCA-G; IFNγ, TNFα and CBir1; IFNγ, TNFα and Fla2; IFNγ, TNFα and FlaX; IFNγ, TNFα and OmpC; IFNγ, TNFα and ADA; IFNγ, ASCA-A and ASCA-G; IFNγ, ASCA-A and CBir1; IFNγ, ASCA-A and Fla2; IFNγ, ASCA-A and FlaX; IFNγ, ASCA-A and OmpC; IFNγ, ASCA-A and ADA; IFNγ, ASCA-G and CBir1; IFNγ, ASCA-G and Fla2; IFNγ, ASCA-G and FlaX; IFNγ, ASCA-G and OmpC; IFNγ, ASCA-G and ADA; IFNγ, CBirl and Fla2; IFNγ, CBirl and FlaX; IFNγ, CBirl and OmpC; IFNγ, CBirl and ADA; IFNγ, Fla2 and FlaX; IFNγ, Fla2 and OmpC; IFNγ, Fla2 and ADA; IFNγ, FlaX and OmpC; IFNγ, FlaX and ADA; IFNγ, OmpC and ADA; IL-6, TWEAK and CRP; IL-6, TWEAK and ICAM; IL-6, TWEAK and SAA; IL-6, TWEAK and VCAM; IL-6, TWEAK and IL-2; IL-6, TWEAK and IL-8; IL-6, TWEAK and IL-12p70; IL-6, TWEAK and IL-1β; IL-6, TWEAK and GMCSF; IL-6, TWEAK and IFNγ; TWEAK and TNFα; IL-6, TWEAK and ASCA-A; IL-6, TWEAK and ASCA-G; IL-6, TWEAK and CBir1; IL-6, TWEAK and Fla2; IL-6, TWEAK and FlaX; IL-6, TWEAK and OmpC; IL-6, TWEAK and ADA; IL-6, CRP and ICAM; IL-6, CRP and SAA; IL-6, CRP and VCAM; IL-6, CRP and IL-2; IL-6, CRP and IL-8; IL-6, CRP and IL-12p70; IL-6, CRP and IL-1β; IL-6, CRP and GMCSF; IL-6, CRP and IFNγ; IL-6, CRP and TNFα; IL-6, CRP and ASCA-A; IL-6, CRP and ASCA-G; IL-6, CRP and CBir1; IL-6, CRP and Fla2; IL-6, CRP and FlaX; IL-6, CRP and OmpC; IL-6, CRP and ADA; IL-6, ICAM and SAA; IL-6, ICAM and VCAM; IL-6, ICAM and IL-2; IL-6, ICAM and IL-8; IL-6, ICAM and IL-12p70; IL-6, ICAM and IL-1β; IL-6, ICAM and GMCSF; IL-6, ICAM and IFNγ; IL-6, ICAM and TNFα; IL-6, ICAM and ASCA-A; IL-6, ICAM and ASCA-G; IL-6, ICAM and CBir1; IL-6, ICAM and Fla2; IL-6, ICAM and FlaX; IL-6, ICAM and OmpC; IL-6, ICAM and ADA; IL-6, SAA and VCAM; IL-6, SAA and IL-2; IL-6, IL-8; IL-6, SAA and IL-12p70; IL-6, SAA and IL-1β; IL-6, SAA and GMCSF; IL-6, SAA and IFNγ; IL-6, SAA and TNFα; IL-6, SAA and ASCA-A; IL-6, SAA and ASCA-G; IL-6, SAA and CBir1; IL-6, SAA and Fla2; IL-6, SAA and FlaX; IL-6, SAA and OmpC; IL-6, SAA and ADA; IL-6, VCAM and IL-2; IL-6, VCAM and IL-8; IL-6, VCAM and IL-12p70; IL-6, VCAM and IL-1β; IL-6, VCAM and GMCSF; IL-6, VCAM and IFNγ; IL-6, VCAM and TNFα; IL-6, VCAM and ASCA-A; IL-6, VCAM and ASCA-G; IL-6, VCAM and CBir1; IL-6, VCAM and Fla2; IL-6, VCAM and FlaX; IL-6, VCAM and OmpC; IL-6, VCAM and ADA; IL-6, IL-2 and IL-8; IL-6, IL-2 and IL-12p70; IL-6, IL-2 and IL-1β; IL-6, IL-2 and GMCSF; IL-6, IL-2 and IFNγ; IL-6, IL-2 and TNFα; IL-6, IL-2 and ASCA-A; IL-6, IL-2 and ASCA-G; IL-6, IL-2 and CBir1; IL-6, IL-2 and Fla2; IL-6, IL-2 and FlaX; IL-6, IL-2 and OmpC; IL-6, IL-2 and ADA; IL-6, IL-8 and IL-12p70; IL-6, IL-8 and IL-1β; IL-6, IL-8 and GMCSF; IL-6, IL-8 and IFNγ; IL-6, IL-8 and TNFα; IL-6, IL-8 and ASCA-A; IL-6, IL-8 and ASCA-G; IL-6, IL-8 and CBir1; IL-6, IL-8 and Fla2; IL-6, IL-8 and FlaX; IL-6, IL-8 and OmpC; IL-6, IL-8 and ADA; IL-6, IL-12p70 and IL-1β; IL-6, IL-12p70 and GMCSF; IL-6, IL-12p70 and IFNγ; IL-6, IL-12p70 and TNFα; IL-6, IL-12p70 and ASCA-A; IL-6, IL-12p70 and ASCA-G; IL-6, IL-12p70 and CBir1; IL-6, IL-12p70 and Fla2; IL-6, IL-12p70 and FlaX; IL-6, IL-12p70 and OmpC; IL-6, IL-12p70 and ADA; IL-6, IL-1β and GMCSF; IL-6, IL-1β and IFNγ; IL-6, IL-1β and TNFα; IL-6, IL-1β and ASCA-A; IL-6, IL-1β and ASCA-G; IL-6, IL-1β and CBir1; IL-6, IL-1β and Fla2; IL-6, IL-1β and FlaX; IL-6, IL-1β and OmpC; IL-6, IL-1β and ADA; IL-6, GMCSF and IFNγ; IL-6, GMCSF and TNFα; IL-6, GMCSF and ASCA-A; IL-6, GMCSF and ASCA-G; IL-6, GMCSF and CBir1; IL-6, GMCSF and Fla2; IL-6, GMCSF and FlaX; IL-6, GMCSF and OmpC; IL-6, GMCSF and ADA; IL-6, IFNγ and TNFα; IL-6, IFNγ and ASCA-A; IL-6, IFNγ and ASCA-G; IL-6, IFNγ and CBir1; IL-6, IFNγ and Fla2; IL-6, IFNγ and FlaX; IL-6, IFNγ and OmpC; IL-6, IFNγ and ADA; IL-6, TNFα and ASCA-A; IL-6, TNFα and ASCA-G; IL-6, TNFα and CBir1; IL-6, TNFα and Fla2; IL-6, TNFα and FlaX; IL-6, TNFα and OmpC; IL-6, TNFα and ADA; IL-6, ASCA-A and ASCA-G; IL-6, ASCA-A and CBir1; IL-6, ASCA-A and Fla2; IL-6, ASCA-A and FlaX; IL-6, ASCA-A and OmpC; IL-6, ASCA-A and ADA; IL-6, ASCA-G and CBir1; IL-6, ASCA-G and Fla2; IL-6, ASCA-G and FlaX; IL-6, ASCA-G and OmpC; IL-6, ASCA-G and ADA; IL-6, CBirl and Fla2; IL-6, CBirl and FlaX; IL-6, CBirl and OmpC; IL-6, CBirl and ADA; IL-6, Fla2 and FlaX; IL-6, Fla2 and OmpC; IL-6, Fla2 and ADA; IL-6, FlaX and OmpC; IL-6, FlaX and ADA; IL-6, OmpC and ADA; TNFα, TWEAK and CRP; TNFα, TWEAK and ICAM; TNFα, TWEAK and SAA; TNFα, TWEAK and VCAM; TNFα, TWEAK and IL-2; TNFα, TWEAK and IL-8; TNFα, TWEAK and IL-12p70; TNFα, TWEAK and IL-1β; TNFα, TWEAK and GMCSF; TNFα, TWEAK and IFNγ; TNFα, TWEAK and IL-6; TNFα, TWEAK and ASCA-A; TNFα, TWEAK and ASCA-G; TNFα, TWEAK and CBir1; TNFα, TWEAK and Fla2; TNFα, TWEAK and FlaX; TNFα, TWEAK and OmpC; TNFα, TWEAK and ADA; TNFα, CRP and ICAM; TNFα, CRP and SAA; TNFα, CRP and VCAM; TNFα, CRP and IL-2; TNFα, CRP and IL-8; TNFα, CRP and IL-12p70; TNFα, CRP and IL-1β; TNFα, CRP and GMCSF; TNFα, CRP and IFNγ; TNFα, CRP and IL-6; TNFα, CRP and TNFα; TNFα, CRP and ASCA-A; TNFα, CRP and ASCA-G; TNFα, CRP and CBir1; TNFα, CRP and Fla2; TNFα, CRP and FlaX; TNFα, CRP and OmpC; TNFα, CRP and ADA; TNFα, ICAM and SAA; TNFα, ICAM and VCAM; TNFα, ICAM and IL-2; TNFα, ICAM and IL-8; TNFα, ICAM and IL-12p70; TNFα, ICAM and IL-1β; TNFα, ICAM and GMCSF; TNFα, ICAM and IFNγ; TNFα, ICAM and IL-6; TNFα, ICAM and ASCA-A; TNFα, ICAM and ASCA-G; TNFα, ICAM and CBir1; TNFα, ICAM and Fla2; TNFα, ICAM and FlaX; TNFα, ICAM and OmpC; TNFα, ICAM and ADA; TNFα, SAA and VCAM; TNFα, SAA and IL-2; TNFα, SAA and IL-8; TNFα, SAA and IL-12p70; TNFα, SAA and IL-1β; TNFα, SAA and GMCSF; TNFα, SAA and IFNγ; TNFα, SAA and IL-6; TNFα, SAA and ASCA-A; TNFα, SAA and ASCA-G; TNFα, SAA and CBir1; TNFα, SAA and Fla2; TNFα, SAA and FlaX; TNFα, SAA and OmpC; TNFα, SAA and ADA; TNFα, VCAM and IL-2; TNFα, VCAM and IL-8; TNFα, VCAM and IL-12p70; TNFα, VCAM and IL-1β; TNFα, VCAM and GMCSF; TNFα, VCAM and IFNγ; TNFα, VCAM and IL-6; TNFα, VCAM and ASCA-A; TNFα, VCAM and ASCA-G; TNFα, VCAM and CBir1; TNFα, VCAM and Fla2; TNFα, VCAM and FlaX; TNFα, VCAM and OmpC; TNFα, VCAM and ADA; TNFα, IL-2 and IL-8; TNFα, IL-2 and IL-12p70; TNFα, IL-2 and IL-1β; TNFα, IL-2 and GMCSF; TNFα, IL-2 and IFNγ; TNFα, IL-2 and IL-6; TNFα, IL-2 and ASCA-A; TNFα, IL-2 and ASCA-G; TNFα, IL-2 and CBir1; TNFα, IL-2 and Fla2; TNFα, IL-2 and FlaX; TNFα, IL-2 and OmpC; TNFα, IL-2 and ADA; TNFα, IL-8 and IL-12p70; TNFα, IL-8 and IL-1β; TNFα, IL-8 and GMCSF; TNFα, IL-8 and IFNγ; TNFα, IL-8 and IL-6; TNFα, IL-8 and ASCA-A; TNFα, IL-8 and ASCA-G; TNFα, IL-8 and CBir1; TNFα, IL-8 and Fla2; TNFα, IL-8 and FlaX; TNFα, IL-8 and OmpC; TNFα, IL-8 and ADA; TNFα, IL-12p70 and IL-1β; TNFα, IL-12p70 and GMCSF; TNFα, IL-12p70 and IFNγ; TNFα, IL-12p70 and IL-6; TNFα, IL-12p70 and ASCA-A; TNFα, IL-12p70 and ASCA-G; TNFα, IL-12p70 and CBir1; TNFα, IL-12p70 and Fla2; TNFα, IL-12p70 and FlaX; TNFα, IL-12p70 and OmpC; TNFα, IL-12p70 and ADA; TNFα, IL-1β and GMCSF; TNFα, IL-1β and IFNγ; TNFα, IL-1β and IL-6; TNFα, IL-1β and ASCA-A; TNFα, IL-1β and ASCA-G; TNFα, IL-1β and CBir1; TNFα, IL-1β and Fla2; TNFα, IL-1β and FlaX; TNFα, IL-1β and OmpC; TNFα, IL-1β and ADA; TNFα, GMCSF and IFNγ; TNFα, GMCSF and IL-6; TNFα, GMCSF and TNFα; TNFα, GMCSF and ASCA-A; TNFα, GMCSF and ASCA-G; TNFα, GMCSF and CBir1; TNFα, GMCSF and Fla2; TNFα, GMCSF and FlaX; TNFα, GMCSF and OmpC; TNFα, GMCSF and ADA; TNFα, IFNγ and IL-6; TNFα, IFNγ and ASCA-A; TNFα, IFNγ and ASCA-G; TNFα, IFNγ and CBir1; TNFα, IFNγ and Fla2; TNFα, IFNγ and FlaX; TNFα, IFNγ and OmpC; TNFα, IFNγ and ADA; TNFα, IL-6 and ASCA-A; TNFα, IL-6 and ASCA-G; TNFα, IL-6 and CBir1; TNFα, IL-6 and Fla2; TNFα, IL-6 and FlaX; TNFα, IL-6 and OmpC; TNFα, IL-6 and ADA; TNFα, ASCA-A and ASCA-G; TNFα, ASCA-A and CBir1; TNFα, ASCA-A and Fla2; TNFα, ASCA-A and FlaX; TNFα, ASCA-A and OmpC; TNFα, ASCA-A and ADA; TNFα, ASCA-G and CBir1; TNFα, ASCA-G and Fla2; TNFα, ASCA-G and FlaX; TNFα, ASCA-G and OmpC; TNFα, ASCA-G and ADA; TNFα, CBirl and Fla2; TNFα, CBir1 and FlaX; TNFα, CBirl and OmpC; TNFα, CBirl and ADA; TNFα, Fla2 and FlaX; TNFα, Fla2 and OmpC; TNFα, Fla2 and ADA; TNFα, FlaX and OmpC; TNFα, FlaX and ADA; TNFα, OmpC and ADA; ASCA-A, TWEAK and CRP; ASCA-A, TWEAK and ICAM; ASCA-A, TWEAK and SAA; ASCA-A, TWEAK and VCAM; ASCA-A, TWEAK and IL-2; ASCA-A, TWEAK and IL-8; ASCA-A, TWEAK and IL-12p70; ASCA-A, TWEAK and IL-1β; ASCA-A, TWEAK and GMCSF; ASCA-A, TWEAK and IFNγ; ASCA-A, TWEAK and IL-6; ASCA-A, TWEAK and TNFα; ASCA-A, TWEAK and ASCA-G; ASCA-A, TWEAK and CBir1; ASCA-A, TWEAK and Fla2; ASCA-A, TWEAK and FlaX; ASCA-A, TWEAK and OmpC; ASCA-A, TWEAK and ADA; ASCA-A, CRP and ICAM; ASCA-A, CRP and SAA; ASCA-A, CRP and VCAM; ASCA-A, CRP and IL-2; ASCA-A, CRP and IL-8; ASCA-A, CRP and IL-12p70; ASCA-A, CRP and IL-1β; ASCA-A, CRP and GMCSF; ASCA-A, CRP and IFNγ; ASCA-A, CRP and IL-6; ASCA-A, CRP and TNFα; ASCA-A, CRP and ASCA-G; ASCA-A, CRP and CBir1; ASCA-A, CRP and Fla2; ASCA-A, CRP and FlaX; ASCA-A, CRP and OmpC; ASCA-A, CRP and ADA; ASCA-A, ICAM and SAA; ASCA-A, ICAM and VCAM; ASCA-A, ICAM and IL-2; ASCA-A, ICAM and IL-8; ASCA-A, ICAM and IL-12p70; ASCA-A, ICAM and IL-1β; ASCA-A, ICAM and GMCSF; ASCA-A, ICAM and IFNγ; ASCA-A, ICAM and IL-6; ASCA-A, ICAM and TNFα; ASCA-A, ICAM and ASCA-G; ASCA-A, ICAM and CBirl; ASCA-A, ICAM and Fla2; ASCA-A, ICAM and FlaX; ASCA-A, ICAM and OmpC; ASCA-A, ICAM and ADA; ASCA-A, SAA and VCAM; ASCA-A, SAA and IL-2; ASCA-A, SAA and IL-8; ASCA-A, SAA and IL-12p70; ASCA-A, SAA and IL-1β; ASCA-A, SAA and GMCSF; ASCA-A, SAA and IFNγ; ASCA-A, SAA and IL-6; ASCA-A, SAA and TNFα; ASCA-A, SAA and ASCA-G; ASCA-A, SAA and CBir1; ASCA-A, SAA and Fla2; ASCA-A, SAA and FlaX; ASCA-A, SAA and OmpC; ASCA-A, SAA and ADA; ASCA-A, VCAM and IL-2; ASCA-A, VCAM and IL-8; ASCA-A, VCAM and IL-12p70; ASCA-A, VCAM and IL-1β; ASCA-A, VCAM and GMCSF; ASCA-A, VCAM and IFNγ; ASCA-A, VCAM and IL-6; ASCA-A, VCAM and TNFα; ASCA-A, VCAM and ASCA-A; ASCA-A, VCAM and CBir1; ASCA-A, VCAM and Fla2; ASCA-A, VCAM and FlaX; ASCA-A, VCAM and OmpC; ASCA-A, VCAM and ADA; ASCA-A, IL-2 and IL-8; ASCA-A, IL-2 and IL-12p70; ASCA-A, IL-2 and IL-1β; ASCA-A, IL-2 and GMCSF; ASCA-A, IL-2 and IFNγ; ASCA-A, IL-2 and IL-6; ASCA-A, IL-2 and TNFα; ASCA-A, IL-2 and ASCA-G; ASCA-A, IL-2 and CBir1; ASCA-A, IL-2 and Fla2; ASCA-A, IL-2 and FlaX; ASCA-A, IL-2 and OmpC; ASCA-A, IL-2 and ADA; ASCA-A, IL-8 and IL-12p70; ASCA-A, IL-8 and IL-1β; ASCA-A, IL-8 and GMCSF; ASCA-A, IL-8 and IFNγ; ASCA-A, IL-8 and IL-6; ASCA-A, IL-8 and TNFα; ASCA-A, IL-8 and ASCA-G; ASCA-A, IL-8 and CBir1; ASCA-A, IL-8 and Fla2; ASCA-A, IL-8 and FlaX; ASCA-A, IL-8 and OmpC; ASCA-A, IL-8 and ADA; ASCA-A, IL-12p70 and IL-1β; ASCA-A, IL-12p70 and GMCSF; ASCA-A, IL-12p70 and IFNγ; ASCA-A, IL-12p70 and IL-6; ASCA-A, IL-12p70 and TNFα; ASCA-A, IL-12p70 and ASCA-G; ASCA-A, IL-12p70 and CBir1; ASCA-A, IL-12p70 and Fla2; ASCA-A, IL-12p70 and FlaX; ASCA-A, IL-12p70 and OmpC; ASCA-A, IL-12p70 and ADA; ASCA-A, IL-1β and GMCSF; ASCA-A, IL-1β and IFNγ; ASCA-A, IL-1β and IL-6; ASCA-A, IL-1β and TNFα; ASCA-A, IL-1β and ASCA-G; ASCA-A, IL-1β and CBir1; ASCA-A, IL-1β and Fla2; ASCA-A, IL-1β and FlaX; ASCA-A, IL-1β and OmpC; ASCA-A, IL-1β and ADA; ASCA-A, GMCSF and IFNγ; ASCA-A, GMCSF and IL-6; ASCA-A, GMCSF and TNFα; ASCA-A, GMCSF and ASCA-G; ASCA-A, GMCSF and CBir1; ASCA-A, GMCSF and Fla2; ASCA-A, GMCSF and FlaX; ASCA-A, GMCSF and OmpC; ASCA-A, GMCSF and ADA; ASCA-A, IFNγ and IL-6; ASCA-A, IFNγ and TNFα; ASCA-A, IFNγ and ASCA-G; ASCA-A, IFNγ and CBir1; ASCA-A, IFNγ and Fla2; ASCA-A, IFNγ and FlaX; ASCA-A, IFNγ and OmpC; ASCA-A, IFNγ and ADA; ASCA-A, IL-6 and TNFα; ASCA-A, IL-6 and ASCA-G; ASCA-A, IL-6 and CBir1; ASCA-A, IL-6 and Fla2; ASCA-A, IL-6 and FlaX; ASCA-A, IL-6 and OmpC; ASCA-A, IL-6 and ADA; ASCA-A, ASCA-G and CBir1; ASCA-A, ASCA-G and Fla2; ASCA-A, ASCA-G and FlaX; ASCA-A, ASCA-G and OmpC; ASCA-A, ASCA-G and ADA; ASCA-A, CBir1 and Fla2; ASCA-A, CBirl and FlaX; ASCA-A, CBirl and OmpC; ASCA-A, CBirl and ADA; ASCA-A, Fla2 and FlaX; ASCA-A, Fla2 and OmpC; ASCA-A, Fla2 and ADA; ASCA-A, FlaX and OmpC; ASCA-A, FlaX and ADA; ASCA-A, OmpC and ADA; ASCA-G, TWEAK and CRP; ASCA-G, TWEAK and ICAM; ASCA-G, TWEAK and SAA; ASCA-G, TWEAK and VCAM; ASCA-G, TWEAK and IL-2; ASCA-G, TWEAK and IL-8; ASCA-G, TWEAK and IL-12p70; ASCA-G, TWEAK and IL-1β; ASCA-G, TWEAK and GMCSF; ASCA-G, TWEAK and IFNγ; ASCA-G, TWEAK and IL-6; ASCA-G, TWEAK and TNFα; ASCA-G, TWEAK and ASCA-A; ASCA-G, TWEAK and CBir1; ASCA-G, TWEAK and Fla2; ASCA-G, TWEAK and FlaX; ASCA-G, TWEAK and OmpC; ASCA-G, TWEAK and ADA; ASCA-G, CRP and ICAM; ASCA-G, CRP and SAA; ASCA-G, CRP and VCAM; ASCA-G, CRP and IL-2; ASCA-G, CRP and IL-8; ASCA-G, CRP and IL-12p70; ASCA-G, CRP and IL-1β; ASCA-G, CRP and GMCSF; ASCA-G, CRP and IFNγ; ASCA-G, CRP and IL-6; ASCA-G, CRP and TNFα; ASCA-G, CRP and ASCA-A; ASCA-G, CRP and CBir1; ASCA-G, CRP and Fla2; ASCA-G, CRP and FlaX; ASCA-G, CRP and OmpC; ASCA-G, CRP and ADA; ASCA-G, ICAM and SAA; ASCA-G, ICAM and VCAM; ASCA-G, ICAM and IL-2; ASCA-G, ICAM and IL-8; ASCA-G, ICAM and IL-12p70; ASCA-G, ICAM and IL-1β; ASCA-G, ICAM and GMCSF; ASCA-G, ICAM and IFNγ; ASCA-G, ICAM and IL-6; ASCA-G, ICAM and TNFα; ASCA-G, ICAM and ASCA-A; ASCA-G, ICAM and CBir1; ASCA-G, ICAM and Fla2; ASCA-G, ICAM and FlaX; ASCA-G, ICAM and OmpC; ASCA-G, ICAM and ADA; ASCA-G, SAA and VCAM; ASCA-G, SAA and IL-2; ASCA-G, SAA and IL-8; ASCA-G, SAA and IL-12p70; ASCA-G, SAA and IL-1β; ASCA-G, SAA and GMCSF; ASCA-G, SAA and IFNγ; ASCA-G, SAA and IL-6; ASCA-G, SAA and TNFα; ASCA-G, SAA and ASCA-A; ASCA-G, SAA and CBir1; ASCA-G, SAA and Fla2; ASCA-G, SAA and FlaX; ASCA-G, SAA and OmpC; ASCA-G, SAA and ADA; ASCA-G, VCAM and IL-2; ASCA-G, VCAM and IL-8; ASCA-G, VCAM and IL-12p70; ASCA-G, VCAM and IL-1β; ASCA-G, VCAM and GMCSF; ASCA-G, VCAM and IFNγ; ASCA-G, VCAM and IL-6; ASCA-G, VCAM and TNFα; ASCA-G, VCAM and ASCA-A; ASCA-G, VCAM and CBir1; ASCA-G, VCAM and Fla2; ASCA-G, VCAM and FlaX; ASCA-G, VCAM and OmpC; ASCA-G, VCAM and ADA; ASCA-G, IL-2 and IL-8; ASCA-G, IL-2 and IL-12p70; ASCA-G, IL-2 and IL-1β; ASCA-G, IL-2 and GMCSF; ASCA-G, IL-2 and IFNγ; ASCA-G, IL-2 and IL-6; ASCA-G, IL-2 and TNFα; ASCA-G, IL-2 and ASCA-A; ASCA-G, IL-2 and CBir1; ASCA-G, IL-2 and Fla2; ASCA-G, IL-2 and FlaX; ASCA-G, IL-2 and OmpC; ASCA-G, IL-2 and ADA; ASCA-G, IL-8 and IL-12p70; ASCA-G, IL-8 and IL-1β; ASCA-G, IL-8 and GMCSF; ASCA-G, IL-8 and IFNγ; ASCA-G, IL-8 and IL-6; ASCA-G, IL-8 and TNFα; ASCA-G, IL-8 and ASCA-A; ASCA-G, IL-8 and CBir1; ASCA-G, IL-8 and Fla2; ASCA-G, IL-8 and FlaX; ASCA-G, IL-8 and OmpC; ASCA-G, IL-8 and ADA; ASCA-G, IL-12p70 and IL-1β; ASCA-G, IL-12p70 and GMCSF; ASCA-G, IL-12p70 and IFNγ; ASCA-G, IL-12p70 and IL-6; ASCA-G, IL-12p70 and TNFα; ASCA-G, IL-12p70 and ASCA-A; ASCA-G, IL-12p70 and CBir1; ASCA-G, IL-12p70 and Fla2; ASCA-G, IL-12p70 and FlaX; ASCA-G, IL-12p70 and OmpC; ASCA-G, IL-12p70 and ADA; ASCA-G,IL-1β and GMCSF; ASCA-G, IL-1β and IFNγ; ASCA-G, IL-1β and IL-6; ASCA-G, IL-1β and TNFα; ASCA-G, IL-1β and ASCA-A; ASCA-G, IL-1β and ASCA-G; ASCA-G, IL-1β and CBir1; ASCA-G, IL-1β and Fla2; ASCA-G, IL-1β and FlaX; ASCA-G, IL-1β and OmpC; ASCA-G, IL-1β and ADA; ASCA-G, GMCSF and IFNγ; ASCA-G, GMCSF and IL-6; ASCA-G, GMCSF and TNFα; ASCA-G, GMCSF and ASCA-A; ASCA-G, GMCSF and ASCA-G; ASCA-G, GMCSF and CBir1; ASCA-G, GMCSF and Fla2; ASCA-G, GMCSF and FlaX; ASCA-G, GMCSF and OmpC; ASCA-G, GMCSF and ADA; ASCA-G, IFNγ and IL-6; ASCA-G, IFNγ and TNFα; ASCA-G, IFNγ and ASCA-A; ASCA-G, IFNγ and CBir1; ASCA-G, IFNγ and Fla2; ASCA-G, IFNγ and FlaX; ASCA-G, IFNγ and OmpC; ASCA-G, IFNγ and ADA; ASCA-G, IL-6 and TNFα; ASCA-G, IL-6 and ASCA-A; ASCA-G, IL-6 and CBir1; ASCA-G, IL-6 and Fla2; ASCA-G, IL-6 and FlaX; ASCA-G, IL-6 and OmpC; ASCA-G, IL-6 and ADA; ASCA-G, TNFα and ASCA-A; ASCA-G, TNFα and CBir1; ASCA-G, TNFα and Fla2; ASCA-G, TNFα and FlaX; ASCA-G, TNFα and OmpC; ASCA-G, TNFα and ADA; ASCA-G, ASCA-A and CBir1; ASCA-G, ASCA-A and Fla2; ASCA-G, ASCA-A and FlaX; ASCA-G, ASCA-A and OmpC; ASCA-G, ASCA-A and ADA; ASCA-G, CBirl and Fla2; ASCA-G, CBirl and FlaX; ASCA-G, CBirl and OmpC; ASCA-G, CBirl and ADA; ASCA-G, Fla2 and FlaX; ASCA-G, Fla2 and OmpC; ASCA-G, Fla2 and ADA; ASCA-G, FlaX and OmpC; ASCA-G, FlaX and ADA; ASCA-G, OmpC and ADA; CBir1, TWEAK and CRP; CBir1, TWEAK and ICAM; CBirl, TWEAK and SAA; CBirl, TWEAK and VCAM; CBirl, TWEAK and IL-2; CBir1, TWEAK and IL-8; CBir1, TWEAK and IL-12p70; CBir1, TWEAK and IL-1β; CBir1, TWEAK and GMCSF; CBir1, TWEAK and IFNγ; CBir1, TWEAK and IL-6; CBir1, TWEAK and TNFα; CBir1, TWEAK and ASCA-A; CBir1, TWEAK and ASCA-G; CBir1, TWEAK and Fla2; CBirl, TWEAK and FlaX; CBir1, TWEAK and OmpC; CBir1, TWEAK and ADA; CBir1, CRP and ICAM; CBir1, CRP and SAA; CBir1, CRP and VCAM; CBir1, CRP and IL-2; CBir1, CRP and IL-8; CBir1, CRP and IL-12p70; CBir1, CRP and IL-1β; CBir1, CRP and GMCSF; CBir1, CRP and IFNγ; CBir1, CRP and IL-6; CBir1, CRP and TNFα; CBir1, CRP and ASCA-A; CBir1, CRP and ASCA-G; CBir1, CRP and Fla2; CBir1, CRP and FlaX; CBir1, CRP and OmpC; CBir1, CRP and ADA; CBir1, ICAM and SAA; CBir1, ICAM and VCAM; CBir1, ICAM and IL-2; CBir1, ICAM and IL-8; CBir1, ICAM and IL-12p70; CBir1, ICAM and IL-1β; CBir1, ICAM and GMCSF; CBir1, ICAM and IFNγ; CBir1, ICAM and IL-6; CBir1, ICAM and TNFα; CBir1, ICAM and ASCA-A; CBir1, ICAM and ASCA-G; CBir1, ICAM and Fla2; CBir1, ICAM and FlaX; CBir1, ICAM and OmpC; CBir1, ICAM and ADA; CBir1, SAA and VCAM; CBir1, SAA and IL-2; CBir1, IL-8; CBir1, SAA and IL-12p70; CBir1, SAA and IL-1β; CBir1, SAA and GMCSF; CBir1, SAA and IFNγ; CBir1, SAA and IL-6; CBir1, SAA and TNFα; CBir1, SAA and ASCA-A; CBir1, SAA and ASCA-G; CBir1, SAA and Fla2; CBir1, SAA and FlaX; CBir1, SAA and OmpC; CBir1, SAA and ADA; CBir1, VCAM and IL-2; CBir1, VCAM and IL-8; CBir1, VCAM and IL-12p70; CBir1, VCAM and IL-1β; CBir1, VCAM and GMCSF; CBir1, VCAM and IFNγ; CBir1, VCAM and IL-6; CBir1, VCAM and TNFα; CBir1, VCAM and ASCA-A; CBir1, VCAM and ASCA-G; CBir1, VCAM and Fla2; CBir1, VCAM and FlaX; CBir1, VCAM and OmpC; CBir1, VCAM and ADA; CBir1, IL-2 and IL-8; CBir1, IL-2 and IL-12p70; CBir1, IL-2 and IL-1β; CBir1, IL-2 and GMCSF; CBir1, IL-2 and IFNγ; CBir1, IL-2 and IL-6; CBir1, IL-2 and TNFα; CBir1, IL-2 and ASCA-A; CBir1, IL-2 and ASCA-G; CBir1, IL-2 and Fla2; CBir1, IL-2 and FlaX; CBir1, IL-2 and OmpC; CBir1, IL-2 and ADA; CBir1, IL-8 and IL-12p70; CBir1, IL-8 and IL-1β; CBir1, IL-8 and GMCSF; CBir1, IL-8 and IFNγ; CBir1, IL-8 and IL-6; CBir1, IL-8 and TNFα; CBir1, IL-8 and ASCA-A; CBir1, IL-8 and ASCA-G; CBir1, IL-8 and Fla2; CBir1, IL-8 and FlaX; CBirl, IL-8 and OmpC; CBirl, IL-8 and ADA; CBirl, IL-12p70 and IL-1β; CBir1, IL-12p70 and GMCSF; CBir1, IL-12p70 and IFNγ; CBir1, IL-12p70 and IL-6; CBir1, IL-12p70 and TNFα; CBir1, IL-12p70 and ASCA-A; CBir1, IL-12p70 and ASCA-G; CBir1, IL-12p70 and Fla2; CBir1, IL-12p70 and FlaX; CBir1, IL-12p70 and OmpC; CBir1, IL-12p70 and ADA; CBir1, IL-1β and GMCSF; CBir1, IL-1β and IFNγ; CBir1, IL-1β and IL-6; CBir1, IL-1β and TNFα; CBir1, IL-1β and ASCA-A; CBir1, IL-1β and ASCA-G; CBir1, IL-1β and Fla2; CBir1, IL-1β and FlaX; CBir1, IL-1β and OmpC; CBir1, IL-1β and ADA; CBir1, GMCSF and IFNγ; CBir1, GMCSF and IL-6; CBir1, GMCSF and TNFα; CBir1, GMCSF and ASCA-A; CBir1, GMCSF and ASCA-G; CBir1, GMCSF and Fla2; CBir1, GMCSF and FlaX; CBir1, GMCSF and OmpC; CBir1, GMCSF and ADA; CBir1, IFNγ and IL-6; CBir1, IFNγ and TNFα; CBir1, IFNγ and ASCA-A; CBir1, IFNγ and ASCA-G; CBir1, IFNγ and Fla2; CBir1, IFNγ and FlaX; CBir1, IFNγ and OmpC; CBir1, IFNγ and ADA; CBir1, IL-6 and TNFα; CBir1, IL-6 and ASCA-A; CBir1, IL-6 and ASCA-G; CBir1, IL-6 and Fla2; CBir1, IL-6 and FlaX; CBir1, IL-6 and OmpC; CBir1, IL-6 and ADA; CBir1, TNFα and ASCA-A; CBir1, TNFα and ASCA-G; CBir1, TNFα and CBirl; CBirl, TNFα and Fla2; CBirl, TNFα and FlaX; CBir1, TNFα and OmpC; CBir1, TNFα and ADA; CBir1, ASCA-A and ASCA-G; CBir1, ASCA-A and Fla2; CBir1, ASCA-A and FlaX; CBir1, ASCA-A and OmpC; CBir1, ASCA-A and ADA; CBir1, ASCA-G and Fla2; CBir1, ASCA-G and FlaX; CBir1, ASCA-G and OmpC; CBir1, ASCA-G and ADA; CBirl, Fla2 and FlaX; CBir1, Fla2 and OmpC; CBir1, Fla2 and ADA; CBir1, FlaX and OmpC; CBir1, FlaX and ADA; CBir1, OmpC and ADA; Fla2, TWEAK and CRP; Fla2, TWEAK and ICAM; Fla2, TWEAK and SAA; Fla2, TWEAK and VCAM; Fla2, TWEAK and IL-2; Fla2, TWEAK and IL-8; Fla2, TWEAK and IL-12p70; Fla2, TWEAK and IL-1β; Fla2, TWEAK and GMCSF; Fla2, TWEAK and IFNγ; Fla2, TWEAK and IL-6; Fla2, TWEAK and TNFα; Fla2, TWEAK and ASCA-A; Fla2, TWEAK and ASCA-G; Fla2, TWEAK and CBir1; Fla2, TWEAK and FlaX; Fla2, TWEAK and OmpC; Fla2, TWEAK and ADA;Fla2, CRP and ICAM; Fla2, CRP and SAA; Fla2, CRP and VCAM; Fla2, CRP and IL-2; Fla2, CRP and IL-8; Fla2, CRP and IL-12p70; Fla2, CRP and IL-1β; Fla2, CRP and GMCSF; Fla2, CRP and IFNγ; Fla2, CRP and IL-6; Fla2, CRP and TNFα; Fla2, CRP and ASCA-A; Fla2, CRP and ASCA-G; Fla2, CRP and CBir1; Fla2, CRP and FlaX; Fla2, CRP and OmpC; Fla2, CRP and ADA; Fla2, ICAM and SAA; Fla2, ICAM and VCAM; Fla2, ICAM and IL-2; Fla2, ICAM and IL-8; Fla2, ICAM and IL-12p70; Fla2, ICAM and IL-1β; Fla2, ICAM and GMCSF; Fla2, ICAM and IFNγ; Fla2, ICAM and IL-6; Fla2, ICAM and TNFα; Fla2, ICAM and ASCA-A; Fla2, ICAM and ASCA-G; Fla2, ICAM and CBir1; Fla2, ICAM and FlaX; Fla2, ICAM and OmpC; Fla2, ICAM and ADA; Fla2, SAA and VCAM; Fla2, SAA and IL-2; Fla2, SAA, and IL-8; Fla2, SAA and IL-12p70; Fla2, SAA and IL-1β; Fla2, SAA and GMCSF; Fla2, SAA and IFNγ; Fla2, SAA and IL-6; Fla2, SAA and TNFα; Fla2, SAA and ASCA-A; Fla2, SAA and ASCA-G; Fla2, SAA and CBir1; Fla2, SAA and FlaX; Fla2, SAA and OmpC; Fla2, SAA and ADA; Fla2, VCAM and IL-2; Fla2, VCAM and IL-8; Fla2, VCAM and IL-12p70; Fla2, VCAM and IL-1β; Fla2, VCAM and GMCSF; Fla2, VCAM and IFNγ; Fla2, VCAM and IL-6; Fla2, VCAM and TNFα; Fla2, VCAM and ASCA-A; Fla2, VCAM and ASCA-G; Fla2, VCAM and CBir1; Fla2, VCAM and FlaX; Fla2, VCAM and OmpC; Fla2, VCAM and ADA; Fla2, IL-2 and IL-8; Fla2, IL-2 and IL-12p70; Fla2, IL-2 and IL-1β; Fla2, IL-2 and GMCSF; Fla2, IL-2 and IFNγ; Fla2, IL-2 and IL-6; Fla2, IL-2 and TNFα; Fla2, IL-2 and ASCA-A; Fla2, IL-2 and ASCA-G; Fla2, IL-2 and CBir1; Fla2, IL-2 and FlaX; Fla2, IL-2 and OmpC; Fla2, IL-2 and ADA; Fla2, IL-8 and IL-12p70; Fla2, IL-8 and IL-1β; Fla2, IL-8 and GMCSF; Fla2, IL-8 and IFNγ; Fla2, IL-8 and IL-6; Fla2, IL-8 and TNFα; Fla2, IL-8 and ASCA-A; Fla2, IL-8 and ASCA-G; Fla2, IL-8 and CBir1; Fla2, IL-8 and FlaX; Fla2, IL-8 and OmpC; Fla2, IL-8 and ADA; Fla2, IL-12p70 and IL-1β; Fla2, IL-12p70 and GMCSF; Fla2, IL-12p70 and IFNγ; Fla2, IL-12p70 and IL-6; Fla2, IL-12p70 and TNFα; Fla2, IL-12p70 and ASCA-A; Fla2, IL-12p70 and ASCA-G; Fla2, IL-12p70 and CBir1; Fla2, IL-12p70 and FlaX; Fla2, IL-12p70 and OmpC; Fla2, IL-12p70 and ADA; Fla2, IL-1β and GMCSF; Fla2, IL-1β and IFNγ; Fla2, IL-1β and IL-6; Fla2, IL-1β and TNFα; Fla2, IL-1β and ASCA-A; Fla2, IL-1β and ASCA-G; Fla2, IL-1β and CBir1; Fla2, IL-1β and FlaX; Fla2, IL-1β and OmpC; Fla2, IL-1β and ADA; Fla2, GMCSF and IFNγ; Fla2, GMCSF and IL-6; Fla2, GMCSF and TNFα; Fla2, GMCSF and ASCA-A; Fla2, GMCSF and ASCA-G; Fla2, GMCSF and CBir1; Fla2, GMCSF and FlaX; Fla2, GMCSF and OmpC; Fla2, GMCSF and ADA; Fla2, IFNγ and IL-6; Fla2, IFNγ and TNFα; Fla2, IFNγ and ASCA-A; Fla2, IFNγ and ASCA-G; Fla2, IFNγ and CBir1; Fla2, IFNγ and FlaX; Fla2, IFNγ and OmpC; Fla2, IFNγ and ADA; Fla2, IL-6 and TNFα; Fla2, IL-6 and ASCA-A; Fla2, IL-6 and ASCA-G; Fla2, IL-6 and CBir1; Fla2, IL-6 and FlaX; Fla2, IL-6 and OmpC; Fla2, IL-6 and ADA; Fla2, TNFα and ASCA-A; Fla2, TNFα and ASCA-G; Fla2, TNFα and CBir1; Fla2, TNFα and FlaX; Fla2, TNFα and OmpC; Fla2, TNFα and ADA; Fla2, ASCA-A and ASCA-G; Fla2, ASCA-A and CBir1; Fla2, ASCA-A and FlaX; Fla2, ASCA-A and OmpC; Fla2, ASCA-A and ADA; Fla2, ASCA-G and CBir1; Fla2, ASCA-G and FlaX; Fla2, ASCA-G and OmpC; Fla2, ASCA-G and ADA; Fla2, CBirl and FlaX; Fla2, CBirl and OmpC; Fla2, CBirl and ADA; Fla2, FlaX and OmpC; Fla2, FlaX and ADA; Fla2, OmpC and ADA; FlaX, TWEAK and CRP; FlaX, TWEAK and ICAM; FlaX, TWEAK and SAA; FlaX, TWEAK and VCAM; FlaX, TWEAK and IL-2; FlaX, TWEAK and IL-8; FlaX, TWEAK and IL-12p70; FlaX, TWEAK and IL-1β; FlaX, TWEAK and GMCSF; FlaX, TWEAK and IFNγ; FlaX, TWEAK and IL-6; FlaX, TWEAK and TNFα; FlaX, TWEAK and ASCA-A; FlaX, TWEAK and ASCA-G; FlaX, TWEAK and CBir1; FlaX, TWEAK and Fla2; FlaX, TWEAK and OmpC; FlaX, TWEAK and ADA; FlaX, CRP and ICAM; FlaX, CRP and SAA; FlaX, CRP and VCAM; FlaX, CRP and IL-2; FlaX, CRP and IL-8; FlaX, CRP and IL-12p70; FlaX, CRP and IL-1β; FlaX, CRP and GMCSF; FlaX, CRP and IFNγ; FlaX, CRP and IL-6; FlaX, CRP and TNFα; FlaX, CRP and ASCA-A; FlaX, CRP and ASCA-G; FlaX, CRP and CBir1; FlaX, CRP and Fla2; FlaX, CRP and OmpC; FlaX, CRP and ADA; FlaX, ICAM and SAA; FlaX, ICAM and VCAM; FlaX, ICAM and IL-2; FlaX, ICAM and IL-8; FlaX, ICAM and IL-12p70; FlaX, ICAM and IL-1β; FlaX, ICAM and GMCSF; FlaX, ICAM and IFNγ; FlaX, ICAM and IL-6; FlaX, ICAM and TNFα; FlaX, ICAM and ASCA-A; FlaX, ICAM and ASCA-G; FlaX, ICAM and CBir1; FlaX, ICAM and Fla2; FlaX, ICAM and OmpC; FlaX, ICAM and ADA; FlaX, SAA and VCAM; FlaX, SAA and IL-2; FlaX, SAA, and IL-8; FlaX, SAA and IL-12p70; FlaX, SAA and IL-1β; FlaX, SAA and GMCSF; FlaX, SAA and IFNγ; FlaX, SAA and IL-6; FlaX, SAA and TNFα; FlaX, SAA and ASCA-A; FlaX, SAA and ASCA-G; FlaX, SAA and CBir1; FlaX, SAA and Fla2; FlaX, SAA and OmpC; FlaX, SAA and ADA;FlaX, VCAM and IL-2; FlaX, VCAM and IL-8; FlaX, VCAM and IL-12p70; FlaX, VCAM and IL-1β; FlaX, VCAM and GMCSF; FlaX, VCAM and IFNγ; FlaX, VCAM and IL-6; FlaX, VCAM and TNFα; FlaX, VCAM and ASCA-A; FlaX, VCAM and ASCA-G; FlaX, VCAM and CBir1; FlaX, VCAM and Fla2; FlaX, VCAM and OmpC; FlaX, VCAM and ADA; FlaX, IL-2 and IL-8; FlaX, IL-2 and IL-12p70; FlaX, IL-2 and IL-1β; FlaX, IL-2 and GMCSF; FlaX, IL-2 and IFNγ; FlaX, IL-2 and IL-6; FlaX, IL-2 and TNFα; FlaX, IL-2 and ASCA-A; FlaX, IL-2 and ASCA-G; FlaX, IL-2 and CBir1; FlaX, IL-2 and Fla2; FlaX, IL-2 and OmpC; FlaX, IL-2 and ADA; FlaX, IL-8 and IL-12p70; FlaX, IL-8 and IL-1β; FlaX, IL-8 and GMCSF; FlaX, IL-8 and IFNγ; FlaX, IL-8 and IL-6; FlaX, IL-8 and TNFα; FlaX, IL-8 and ASCA-A; FlaX, IL-8 and ASCA-G; FlaX, IL-8 and CBir1; FlaX, IL-8 and Fla2; FlaX, IL-8 and OmpC; FlaX, IL-8 and ADA; FlaX, IL-12p70 and IL-1β; FlaX, IL-12p70 and GMCSF; FlaX, IL-12p70 and IFNγ; FlaX, IL-12p70 and IL-6; FlaX, IL-12p70 and TNFα; FlaX, IL-12p70 and ASCA-A; FlaX, IL-12p70 and ASCA-G; FlaX, IL-12p70 and CBir1; FlaX, IL-12p70 and Fla2; FlaX, IL-12p70 and OmpC; FlaX, IL-12p70 and ADA; FlaX, IL-1β and GMCSF; FlaX, IL-1β and IFNγ; FlaX, IL-1β and IL-6; FlaX, IL-1β and TNFα; FlaX, IL-1β and ASCA-A; FlaX, IL-1β and ASCA-G; FlaX, IL-1β and CBir1; FlaX, IL-1β and Fla2; FlaX, IL-1β and OmpC; FlaX, IL-1β and ADA;FlaX, GMCSF and IFNγ; FlaX, GMCSF and IL-6; FlaX, GMCSF and TNFα; FlaX, GMCSF and ASCA-A; FlaX, GMCSF and ASCA-G; FlaX, GMCSF and CBir1; FlaX, GMCSF and Fla2; FlaX, GMCSF and OmpC; FlaX, GMCSF and ADA;FlaX, IFNγ and IL-6; FlaX, IFNγ and TNFα; FlaX, IFNγ and ASCA-A; FlaX, IFNγ and ASCA-G; FlaX, IFNγ and CBir1; FlaX, IFNγ and Fla2; FlaX, IFNγ and FlaX; FlaX, IFNγ and OmpC; FlaX, IFNγ and ADA; FlaX, IL-6 and TNFα; FlaX, IL-6 and ASCA-A; FlaX, IL-6 and ASCA-G; FlaX, IL-6 and CBir1; FlaX, IL-6 and Fla2; FlaX, IL-6 and OmpC; FlaX, IL-6 and ADA; FlaX, TNFα and ASCA-A; FlaX, TNFα and ASCA-G; FlaX, TNFα and CBir1; FlaX, TNFα and Fla2; FlaX, TNFα and OmpC; FlaX, TNFα and ADA; FlaX, ASCA-A and ASCA-G; FlaX, ASCA-A and CBir1; FlaX, ASCA-A and Fla2; FlaX, ASCA-A and OmpC; FlaX, ASCA-A and ADA; FlaX, ASCA-G and CBir1; FlaX, ASCA-G and Fla2; FlaX, ASCA-G and FlaX; FlaX, ASCA-G and OmpC; FlaX, ASCA-G and ADA; FlaX, CBirl and Fla2; FlaX, CBirl and FlaX; FlaX, CBirl and OmpC; FlaX, CBirl and ADA; FlaX, Fla2 and FlaX; FlaX, Fla2 and OmpC; FlaX, Fla2 and ADA; FlaX, OmpC and ADA; OmpC, TWEAK and CRP; OmpC, TWEAK and ICAM; OmpC, TWEAK and SAA; OmpC, TWEAK and VCAM; OmpC, TWEAK and IL-2; OmpC, TWEAK and IL-8; OmpC, TWEAK and IL-12p70; OmpC, TWEAK and IL-1β; OmpC, TWEAK and GMCSF; OmpC, TWEAK and IFNγ; OmpC, TWEAK and IL-6; OmpC, TWEAK and TNFα; OmpC, TWEAK and ASCA-A; OmpC, TWEAK and ASCA-G; OmpC, TWEAK and CBir1; OmpC, TWEAK and Fla2; OmpC, TWEAK and FlaX; OmpC, TWEAK and ADA; OmpC, CRP and ICAM; OmpC, CRP and SAA; OmpC, CRP and VCAM; OmpC, CRP and IL-2; OmpC, CRP and IL-8; OmpC, CRP and IL-12p70; OmpC, CRP and IL-1β; OmpC, CRP and GMCSF; OmpC, CRP and IFNγ; OmpC, CRP and IL-6; OmpC, CRP and TNFα; OmpC, CRP and ASCA-A; OmpC, CRP and ASCA-G; OmpC, CRP and CBir1; OmpC, CRP and Fla2; OmpC, CRP and FlaX; OmpC, CRP and ADA; OmpC, ICAM and SAA; OmpC, ICAM and VCAM; OmpC, ICAM and IL-2; OmpC, ICAM and IL-8; OmpC, ICAM and IL-12p70; OmpC, ICAM and IL-1β; OmpC, ICAM and GMCSF; OmpC, ICAM and IFNγ; OmpC, ICAM and IL-6; OmpC, ICAM and TNFα; OmpC, ICAM and ASCA-A; OmpC, ICAM and ASCA-G; OmpC, ICAM and CBir1; OmpC, ICAM and Fla2; OmpC, ICAM and FlaX; OmpC, ICAM and ADA; OmpC, SAA and VCAM; OmpC, SAA and IL-2; OmpC, SAA, and IL-8; OmpC, SAA and IL-12p70; OmpC, SAA and IL-1β; OmpC, SAA and GMCSF; OmpC, SAA and IFNγ; OmpC, SAA and IL-6; OmpC, SAA and TNFα; OmpC, SAA and ASCA-A; OmpC, SAA and ASCA-G; OmpC, SAA and CBir1; OmpC, SAA and Fla2; OmpC, SAA and FlaX; OmpC, SAA and ADA; OmpC, VCAM and IL-2; OmpC, VCAM and IL-8; OmpC, VCAM and IL-12p70; OmpC, VCAM and IL-1β; OmpC, VCAM and GMCSF; OmpC, VCAM and IFNγ; OmpC, VCAM and IL-6; OmpC, VCAM and TNFα; OmpC, VCAM and ASCA-A; OmpC, VCAM and ASCA-G; OmpC, VCAM and CBir1; OmpC, VCAM and Fla2; OmpC, VCAM and FlaX; OmpC, VCAM and ADA; OmpC, IL-2 and IL-8; OmpC, IL-2 and IL-12p70; OmpC, IL-2 and IL-1β; OmpC, IL-2 and GMCSF; OmpC, IL-2 and IFNγ; OmpC, IL-2 and IL-6; OmpC, IL-2 and TNFα; OmpC, IL-2 and ASCA-A; OmpC, IL-2 and ASCA-G; OmpC, IL-2 and CBir1; OmpC, IL-2 and Fla2; OmpC, IL-2 and FlaX; OmpC, IL-2 and ADA; OmpC, IL-8 and IL-12p70; OmpC, IL-8 and IL-1β; OmpC, IL-8 and GMCSF; OmpC, IL-8 and IFNγ; OmpC, IL-8 and IL-6; OmpC, IL-8 and TNFα; OmpC, IL-8 and ASCA-A; OmpC, IL-8 and ASCA-G; OmpC, IL-8 and CBir1; OmpC, IL-8 and Fla2; OmpC, IL-8 and FlaX; OmpC, IL-8 and ADA; OmpC, IL-12p70 and IL-1β; OmpC, IL-12p70 and GMCSF; OmpC, IL-12p70 and IFNγ; OmpC, IL-12p70 and IL-6; OmpC, IL-12p70 and TNFα; OmpC, IL-12p70 and ASCA-A; OmpC, IL-12p70 and ASCA-G; OmpC, IL-12p70 and CBir1; OmpC, IL-12p70 and Fla2; OmpC, IL-12p70 and FlaX; OmpC, IL-12p70 and ADA; OmpC, IL-1β and GMCSF; OmpC, IL-1β and IFNγ; OmpC, IL-1β and IL-6; OmpC, IL-1β and TNFα; OmpC, IL-1β and ASCA-A; OmpC, IL-1β and ASCA-G; OmpC, IL-1β and CBir1; OmpC, IL-1β and Fla2; OmpC, IL-1β and FlaX; OmpC, IL-1β and ADA; OmpC, GMCSF and IFNγ; OmpC, GMCSF and IL-6; OmpC, GMCSF and TNFα; OmpC, GMCSF and ASCA-A; OmpC, GMCSF and ASCA-G; OmpC, GMCSF and CBir1; OmpC, GMCSF and Fla2; OmpC, GMCSF and FlaX; OmpC, GMCSF and ADA; OmpC, IFNγ and IL-6; OmpC, IFNγ and TNFα; OmpC, IFNγ and ASCA-A; OmpC, IFNγ and ASCA-G; OmpC, IFNγ and CBir1; OmpC, IFNγ and Fla2; OmpC, IFNγ and FlaX; OmpC, IFNγ and ADA; OmpC, IL-6 and TNFα; OmpC, IL-6 and ASCA-A; OmpC, IL-6 and ASCA-G; OmpC, IL-6 and CBir1; OmpC, IL-6 and Fla2; OmpC, IL-6 and FlaX; OmpC, IL-6 and ADA; OmpC, TNFα and ASCA-A; OmpC, TNFα and ASCA-G; OmpC, TNFα and CBir1; OmpC, TNFα and Fla2; OmpC, TNFα and FlaX; OmpC, TNFα and ADA; OmpC, ASCA-A and ASCA-G; OmpC, ASCA-A and CBir1; OmpC, ASCA-A and Fla2; OmpC, ASCA-A and FlaX; OmpC, ASCA-A and ADA; OmpC, ASCA-G and CBir1; OmpC, ASCA-G and Fla2; OmpC, ASCA-G and FlaX; OmpC, ASCA-G and ADA; OmpC, CBirl and Fla2; OmpC, CBirl and FlaX; OmpC, CBirl and ADA; OmpC, Fla2 and FlaX; OmpC, Fla2 and ADA; OmpC, FlaX and ADA; ADA, TWEAK and CRP; ADA, TWEAK and ICAM; ADA, TWEAK and SAA; ADA, TWEAK and VCAM; ADA, TWEAK and IL-2; ADA, TWEAK and IL-8; ADA, TWEAK and IL-12p70; ADA, TWEAK and IL-1β; ADA, TWEAK and GMCSF; ADA, TWEAK and IFNγ; ADA, TWEAK and IL-6; ADA, TWEAK and TNFα; ADA, TWEAK and ASCA-A; ADA, TWEAK and ASCA-G; ADA, TWEAK and CBir1; ADA, TWEAK and Fla2; ADA, TWEAK and FlaX; ADA, TWEAK and OmpC; ADA, CRP and ICAM; ADA, CRP and SAA; ADA, CRP and VCAM; ADA, CRP and IL-2; ADA, CRP and IL-8; ADA, CRP and IL-12p70; ADA, CRP and IL-1β; ADA, CRP and GMCSF; ADA, CRP and IPNγ; ADA, CRP and IL-6; ADA, CRP and TNFα; ADA, CRP and ASCA-A; ADA, CRP and ASCA-G; ADA, CRP and CBir1; ADA, CRP and Fla2; ADA, CRP and FlaX; ADA, CRP and OmpC; ADA, ICAM and SAA; ADA, ICAM and VCAM; ADA, ICAM and IL-2; ADA, ICAM and IL-8; ADA, ICAM and IL-12p70; ADA, ICAM and IL-1β; ADA, ICAM and GMCSF; ADA, ICAM and IFNγ; ADA, ICAM and IL-6; ADA, ICAM and TNFα; ADA, ICAM and ASCA-A; ADA, ICAM and ASCA-G; ADA, ICAM and CBir1; ADA, ICAM and Fla2; ADA, ICAM and FlaX; ADA, ICAM and OmpC; ADA, SAA and VCAM; ADA, SAA and IL-2; ADA, SAA and IL-8; ADA, SAA and IL-12p70; ADA, SAA and IL-1β; ADA, SAA and GMCSF; ADA, SAA and IFNγ; ADA, SAA and IL-6; ADA, SAA and TNFα; ADA, SAA and ASCA-A; ADA, SAA and ASCA-G; ADA, SAA and CBirl; ADA, SAA and Fla2; ADA, SAA and FlaX; ADA, SAA and OmpC; ADA, VCAM and IL-2; ADA, VCAM and IL-8; ADA, VCAM and IL-12p70; ADA, VCAM and IL-1β; ADA, VCAM and GMCSF; ADA, VCAM and IFNγ; ADA, VCAM and IL-6; ADA, VCAM and TNFα; ADA, VCAM and ASCA-A; ADA, VCAM and ASCA-G; ADA, VCAM and CBir1; ADA, VCAM and Fla2; ADA, VCAM and FlaX; ADA, VCAM and OmpC; ADA, IL-2 and IL-8; ADA, IL-2 and IL-12p70; ADA, IL-2 and IL-1β; ADA, IL-2 and GMCSF; ADA, IL-2 and IFNγ; ADA, IL-2 and IL-6; ADA, IL-2 and TNFα; ADA, IL-2 and ASCA-A; ADA, IL-2 and ASCA-G; ADA, IL-2 and CBir1; ADA, IL-2 and Fla2; ADA, IL-2 and FlaX; ADA, IL-2 and OmpC; ADA, IL-8 and IL-12p70; ADA, IL-8 and IL-1β; ADA, IL-8 and GMCSF; ADA, IL-8 and IFNγ; ADA, IL-8 and IL-6; ADA, IL-8 and TNFα; ADA, IL-8 and ASCA-A; ADA, IL-8 and ASCA-G; ADA, IL-8 and CBir1; ADA, IL-8 and Fla2; ADA, IL-8 and FlaX; ADA, IL-8 and OmpC; ADA, IL-12p70 and IL-1β; ADA, IL-12p70 and GMCSF; ADA, IL-12p70 and IFNγ; ADA, IL-12p70 and IL-6; ADA, IL-12p70 and TNFα; ADA, IL-12p70 and ASCA-A; ADA, IL-12p70 and ASCA-G; ADA, IL-12p70 and CBir1; ADA, IL-12p70 and Fla2; ADA, IL-12p70 and FlaX; ADA, IL-12p70 and OmpC; ADA, IL-Iβ and GMCSF; ADA, IL-Iβ and IFNγ; ADA, IL-Iβ and IL-6; ADA, IL-Iβ and TNFα; ADA, IL-Iβ and ASCA-A; ADA, IL-Iβ and ASCA-G; ADA, IL-Iβ and CBir1; ADA, IL-1β and Fla2; ADA, IL-Iβ and FlaX; ADA, IL-Iβ and OmpC; ADA, GMCSF and IFNγ; ADA, GMCSF and IL-6; ADA, GMCSF and TNFα; ADA, GMCSF and ASCA-A; ADA, GMCSF and ASCA-G; ADA, GMCSF and CBir1; ADA, GMCSF and Fla2; ADA, GMCSF and FlaX; ADA, GMCSF and OmpC; ADA, IFNγ and IL-6; ADA, IFNγ and TNFα; ADA, IFNγ and ASCA-A; ADA, IFNγ and ASCA-G; ADA, IFNγ and CBir1; ADA, IFNγ and Fla2; ADA, IFNγ and FlaX; ADA, IFNγ and OmpC; ADA, IFNγ and ADA; ADA, IL-6 and TNFα; ADA, IL-6 and ASCA-A; ADA, IL-6 and ASCA-G; ADA, IL-6 and CBir1; ADA, IL-6 and Fla2; ADA, IL-6 and FlaX; ADA, IL-6 and OmpC; ADA, TNFα and ASCA-A; ADA, TNFα and ASCA-G; ADA, TNFα and CBir1; ADA, TNFα and Fla2; ADA, TNFα and FlaX; ADA, TNFα and OmpC; ADA, ASCA-A and ASCA-G; ADA, ASCA-A and CBir1; ADA, ASCA-A and Fla2; ADA, ASCA-A and FlaX; ADA, ASCA-A and OmpC; ADA, ASCA-G and CBir1; ADA, ASCA-G and Fla2; ADA, ASCA-G and FlaX; ADA, ASCA-G and OmpC; ADA, CBirl and Fla2; ADA, CBirl and FlaX; ADA, CBirl and OmpC; ADA, Fla2 and FlaX; ADA, Fla2 and OmpC; ADA, FlaX and OmpC, and the like. In some instances, the combination of at least three markers can further include at least 1, 2, 3, 4, 5, 6, 7, 8, 9, 10, 11, 12, 13, 14, 15, 16, or 17 of the other markers selected from the first set of markers to form an inflammatory phase marker score.

As described herein, the methods for assessing mucosal healing in a subject include measuring the concentration or level of a second set of markers used to form the proliferation phase marker score, wherein at least one or a plurality (*e.g*., at least 2, 3, 4, 5, 6, 7, 8, 9, 10, 11, 12, 13, 14,15,16, 17, 18, 19, 20, 21, 22, 23, 24, or 25) of the markers are selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, an anti-TNFa antibody, and combinations thereof. In some embodiments, the methods include measuring a combination of at least two markers selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody, *e.g*., AREG and EREG, AREG and HBEGF, AREG and HGF, AREG and HRGB, AREG and BTC, AREG and EGF, AREG and TGFA, AREG and FGF1, AREG and FGF2, AREG and FGF4, AREG and FGF7, AREG and FGF9, AREG and FGF19, AREG and SCF, AREG and PDGFA, AREG and PDGFB, AREG and PDGFC, AREG and VEGFA, AREG and VEGFB, AREG and VEGFC, AREG and VEGFD, AREG and TGFB1, AREG and IL-10, AREG and an anti-TNFα antibody, EREG and HBEGF, EREG and HGF, EREG and HRGB, EREG and BTC, EREG and EGF, EREG and TGFA, EREG and FGF1, EREG and FGF2, EREG and FGF4, EREG and FGF7, EREG and FGF9, EREG and FGF19, EREG and SCF, EREG and PDGFA, EREG and PDGFB, EREG and PDGFC, EREG and VEGFA, EREG and VEGFB, EREG and VEGFC, EREG and VEGFD, EREG and TGFB1, EREG and IL-10, EREG and an anti-TNF antibody, HBEGF and HGF, HBEGF and HRGB, HBEGF and BTC, HBEGF and EGF, HBEGF and TGFA, HBEGF and FGF1, HBEGF and FGF2, HBEGF and FGF4, HBEGF and FGF7, HBEGF and FGF9, HBEGF and FGF19, HBEGF and SCF, HBEGF and PDGFA, HBEGF and PDGFB, HBEGF and PDGFC, HBEGF and VEGFA, HBEGF and VEGFB, HBEGF and VEGFC, HBEGF and VEGFD, HBEGF and TGFB1, HBEGF and IL-10, HBEGF and an anti-TNFa antibody, HGF and HRGB, HGF and BTC, HGF and EGF, HGF and TGFA, HGF and FGF1, HGF and FGF2, HGF and FGF4, HGF and FGF7, HGF and FGF9, HGF and FGF19, HGF and SCF, HGF and PDGFA, HGF and PDGFB, HGF and PDGFC, HGF and VEGFA, HGF and VEGFB, HGF and VEGFC, HGF and VEGFD, HGF and TGFB1, HGF and IL-10, HGF and an anti-TNFa antibody, HRGB and BTC, HRGB and EGF, HRGB and TGFA, HRGB and FGF1, HRGB and FGF2, HRGB and FGF4, HRGB and FGF7, HRGB and FGF9, HRGB and FGF19, HRGB and SCF, HRGB and PDGFA, HRGB and PDGFB, HRGB and PDGFC, HRGB and VEGFA, HRGB and VEGFB, HRGB and VEGFC, HRGB and VEGFD, HRGB and TGFB1, HRGB and IL-10, HRGB and an anti-TNF antibody, BTC and EGF, BTC and TGFA, BTC and FGF1, BTC and FGF2, BTC and FGF4, BTC and FGF7, BTC and FGF9, BTC and FGF19, BTC and SCF, BTC and PDGFA, BTC and PDGFB, BTC and PDGFC, BTC and VEGFA, BTC and VEGFB, BTC and VEGFC, BTC and VEGFD, BTC and TGFB1, BTC and IL-10, BTC and an anti-TNFα antibody, EGF and TGFA, EGF and FGF1, EGF and FGF2, EGF and FGF4, EGF and FGF7, EGF and FGF9, EGF and FGF19, EGF and SCF, EGF and PDGFA, EGF and PDGFB, EGF and PDGFC, EGF and VEGFA, EGF and VEGFB, EGF and VEGFC, EGF and VEGFD, EGF and TGFB1, EGF and IL-10, EGF and an anti-TNFα antibody, TGFA and FGF1, TGFA and FGF2, TGFA and FGF4, TGFA and FGF7, TGFA and FGF9, TGFA and FGF19, TGFA and SCF, TGFA and PDGFA, TGFA and PDGFB, TGFA and PDGFC, TGFA and VEGFA, TGFA and VEGFB, TGFA and VEGFC, TGFA and VEGFD, TGFA and TGFB1, TGFA and IL-10, TGFA and an anti-TNFα antibody, FGF1 and FGF2, FGF1 and FGF4, FGF1 and FGF7, FGF1 and FGF9, FGF1 and FGF19, FGF1 and SCF, FGF1 and PDGFA, FGF1 and PDGFB, FGF1 and PDGFC, FGF1 and VEGFA, FGF1 and VEGFB, FGF1 and VEGFC, FGF1 and VEGFD, FGF1 and TGFB1, FGF1 and IL-10, FGF1 and an anti-TNFα antibody, FGF2 and FGF4, FGF2 and FGF7, FGF2 and FGF9, FGF2 and FGF19, FGF2 and SCF, FGF2 and PDGFA, FGF2 and PDGFB, FGF2 and PDGFC, FGF2 and VEGFA, FGF2 and VEGFB, FGF2 and VEGFC, FGF2 and VEGFD, FGF2 and TGFB1, FGF2 and IL-10, FGF2 and an anti-TNFα antibody, FGF4 and FGF7, FGF4 and FGF9, FGF4 and FGF19, FGF4 and SCF, FGF4 and PDGFA, FGF4 and PDGFB, FGF4 and PDGFC, FGF4 and VEGFA, FGF4 and VEGFB, FGF4 and VEGFC, FGF4 and VEGFD, FGF4 and TGFB1, FGF4 and IL-10, FGF4 and an anti-TNFα antibody, FGF7 and FGF9, FGF7 and FGF19, FGF7 and SCF, FGF7 and PDGFA, FGF7 and PDGFB, FGF7 and PDGFC, FGF7 and VEGFA, FGF7 and VEGFB, FGF7 and VEGFC, FGF7 and VEGFD, FGF7 and TGFB1, FGF7 and IL-10, FGF7 and an anti-TNFα antibody, FGF9 and FGF19, FGF9 and SCF, FGF9 and PDGFA, FGF9 and PDGFB, FGF9 and PDGFC, FGF9 and VEGFA, FGF9 and VEGFB, FGF9 and VEGFC, FGF9 and VEGFD, FGF9 and TGFB1, FGF9 and IL-10, FGF9 and an anti-TNFα antibody, FGF19 and SCF, FGF19 and PDGFA, FGF19 and PDGFB, FGF19 and PDGFC, FGF19 and VEGFA, FGF19 and VEGFB, FGF19 and VEGFC, FGF19 and VEGFD, FGF19 and TGFB1, FGF19 and IL-10, FGF19 and an anti-TNFα antibody, SCF and PDGFA, SCF and PDGFB, SCF and PDGFC, SCF and VEGFA, SCF and VEGFB, SCF and VEGFC, SCF and VEGFD, SCF and TGFB1, SCF and IL-10, SCF and an anti-TNFα antibody, PDGFA and PDGFB, PDGFA and PDGFC, PDGFA and VEGFA, PDGFA and VEGFB, PDGFA and VEGFC, PDGFA and VEGFD, PDGFA and TGFB1, PDGFA and IL-10, PDGFA and an anti-TNFa antibody, PDGFB and PDGFC, PDGFB and VEGFA, PDGFB and VEGFB, PDGFB and VEGFC, PDGFB and VEGFD, PDGFB and TGFB1, PDGFB and IL-10, PDGFB and an anti-TNFα antibody, PDGFC and VEGFA, PDGFC and VEGFB, PDGFC and VEGFC, PDGFC and VEGFD, PDGFC and TGFB1, PDGFC and IL-10, PDGFC and an anti-TNFα antibody, VEGFA and VEGFB, VEGFA and VEGFC, VEGFA and VEGFD, VEGFA and TGFB1, VEGFA and IL-10, VEGFA and an anti-TNFα antibody, VEGFB and VEGFC, VEGFB and VEGFD, VEGFB and TGFB1, VEGFB and IL-10, VEGFB and an anti-TNFα antibody, VEGFC and VEGFD, VEGFC and TGFB1, VEGFC and IL-10, VEGFC and an anti-TNFα antibody, VEGFD and TGFB1, VEGFD and IL-10, VEGFD and an anti-TNFα antibody, TGFB1 and IL-10, TGFB1 and an anti-TNFα antibody, IL-10 and an anti-TNFα antibody, and the like. In some instances, the combination of at least two markers can further include at least 1, 2, 3, 4, 5, 6, 7, 8, 9, 10, 11, 12, 13, 14,15, 16, 17, 18, 19, 20, 21, 22, or 23 of the other markers selected from the second set of markers to form a proliferation phase marker score.

In some embodiments, the methods include measuring a combination of at least three markers selected from the group consisting of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody, *e.g*., AREG, EREG and HBEGF; AREG, EREG and HGF; AREG, EREG and HRGB; AREG, EREG and BTC; AREG, EREG and EGF; AREG, EREG and TGFA; AREG, EREG and FGF1; AREG, EREG and FGF2; AREG, EREG and FGF4; AREG, EREG and FGF7; AREG, EREG and FGF9; AREG, EREG and FGF19; AREG, EREG and SCF; AREaG, EREG and PDGFA; AREG, EREG and PDGFB; AREG, EREG and PDGFC; AREG, EREG and VEGFA; AREG, EREG and VEGFB; AREG, EREG and VEGFC; AREG, EREG and VEGFD; AREG, EREG and TGFB1; AREG, EREG and IL-10; AREG, EREG and an anti-TNFα antibody; AREG, HBEGF and HGF; AREG, HBEGF and HRGB; AREG, HBEGF and BTC; AREG, HBEGF and EGF; AREG, HBEGF and TGFA; AREG, HBEGF and FGF1; AREG, HBEGF and FGF2; AREG, HBEGF and FGF4; AREG, HBEGF and FGF7; AREG, HBEGF and FGF9; AREG, HBEGF and FGF19; AREG, HBEGF and SCF; AREG, HBEGF and PDGFA; AREG, HBEGF and PDGFB; AREG, HBEGF and PDGFC; AREG, HBEGF and VEGFA; AREG, HBEGF and VEGFB; AREG, HBEGF and VEGFC; AREG, HBEGF and VEGFD; AREG, HBEGF and TGFB1; AREG, HBEGF and IL-10; AREG, HBEGF and an anti-TNFα antibody; AREG, HGF and HRGB; AREG, HGF and BTC; AREG, HGF and EGF; AREG, HGF and TGFA; AREG, HGF and FGF1; AREG, HGF and FGF2; AREG, HGF and FGF4; AREG, HGF and FGF7; AREG, HGF and FGF9; AREG, HGF and FGF19; AREG, HGF and SCF; AREG, HGF and PDGFA; AREG, HGF and PDGFB; AREG, HGF and PDGFC; AREG, HGF and VEGFA; AREG, HGF and VEGFB; AREG, HGF and VEGFC; AREG, HGF and VEGFD; AREG, HGF and TGFB1; AREG, HGF and IL-10; AREG, HGF and an anti-TNFα antibody; AREG, HRGB and BTC; AREG, HRGB and EGF; AREG, HRGB and TGFA; AREG, HRGB and FGF1; AREG, HRGB and FGF2; AREG, HRGB and FGF4; AREG, HRGB and FGF7; AREG, HRGB and FGF9; AREG, HRGB and FGF19; AREG, HRGB and SCF; AREG, HRGB and PDGFA; AREG, HRGB and PDGFB; AREG, HRGB and PDGFC; AREG, HRGB and VEGFA; AREG, HRGB and VEGFB; AREG, HRGB and VEGFC; AREG, HRGB and VEGFD; AREG, HRGB and TGFB1; AREG, HRGB and IL-10; AREG, HRGB and an anti-TNFa antibody; AREG, BTC and EGF; AREG, BTC and TGFA; AREG, BTC and FGF1; AREG, BTC and FGF2; AREG, BTC and FGF4; AREG, BTC and FGF7; AREG, BTC and FGF9; AREG, BTC and FGF19; AREG, BTC and SCF; AREG, BTC and PDGFA; AREG, BTC and PDGFB; AREG, BTC and PDGFC; AREG, BTC and VEGFA; AREG, BTC and VEGFB; AREG, BTC and VEGFC; AREG, BTC and VEGFD; AREG, BTC and TGFB1; AREG, BTC and IL-10; AREG, BTC and an anti-TNFα antibody; AREG, EGF and TGFA; AREG, EGF and FGF1; AREG, EGF and FGF2; AREG, EGF and FGF4; AREG, EGF and FGF7; AREG, EGF and FGF9; AREG, EGF and FGF19; AREG, EGF and SCF; AREG, EGF and PDGFA; AREG, EGF and PDGFB; AREG, EGF and PDGFC; AREG, EGF and VEGFA; AREG, EGF and VEGFB; AREG, EGF and VEGFC; AREG, EGF and VEGFD; AREG, EGF and TGFB1; AREG, EGF and IL-10; AREG, EGF and an anti-TNFα antibody; AREG, TGFA and FGF1; AREG, TGFA and FGF2; AREG, TGFA and FGF4; AREG, TGFA and FGF7; AREG, TGFA and FGF9; AREG, TGFA and FGF19; AREG, TGFA and SCF; AREG, TGFA and PDGFA; AREG, TGFA and PDGFB; AREG, TGFA and PDGFC; AREG, TGFA and VEGFA; AREG, TGFA and VEGFB; AREG, TGFA and VEGFC; AREG, TGFA and VEGFD; AREG, TGFA and TGFB1; AREG, TGFA and IL-10; AREG, TGFA and an anti-TNFα antibody; AREG, FGF1 and FGF2; AREG, FGF1 and FGF4; AREG, FGF1 and FGF7; AREG, FGF1 and FGF9; AREG, FGF1 and FGF19; AREG, FGF1 and SCF; AREG, FGF1 and PDGFA; AREG, FGF1 and PDGFB; AREG, FGF1 and PDGFC; AREG, FGF1 and VEGFA; AREG, FGF1 and VEGFB; AREG, FGF1 and VEGFC; AREG, FGF1 and VEGFD; AREG, FGF1 and TGFB1; AREG, FGF1 and IL-10; AREG, FGF1 and an anti-TNFα antibody; AREG, FGF2 and FGF4; AREG, FGF2 and FGF7; AREG, FGF2 and FGF9; AREG, FGF2 and FGF19; AREG, FGF2 and SCF; AREG, FGF2 and PDGFA; AREG, FGF2 and PDGFB; AREG, FGF2 and PDGFC; AREG, FGF2 and VEGFA; AREG, FGF2 and VEGFB; AREG, FGF2 and VEGFC; AREG, FGF2 and VEGFD; AREG, FGF2 and TGFB1; AREG, FGF2 and IL-10; AREG, FGF2 and an anti-TNFα antibody; AREG, FGF4 and FGF7; AREG, FGF4 and FGF9; AREG, FGF4 and FGF19; AREG, FGF4 and SCF; AREG, FGF4 and PDGFA; AREG, FGF4 and PDGFB; AREG, FGF4 and PDGFC; AREG, FGF4 and VEGFA; AREG, FGF4 and VEGFB; AREG, FGF4 and VEGFC; AREG, FGF4 and VEGFD; AREG, FGF4 and TGFB1; AREG, FGF4 and IL-10; AREG, FGF4 and an anti-TNFα antibody; AREG, FGF7 and FGF9; AREG, FGF7 and FGF19; AREG, FGF7 and SCF; AREG, FGF7 and PDGFA; AREG, FGF7 and PDGFB; AREG, FGF7 and PDGFC; AREG, FGF7 and VEGFA; AREG, FGF7 and VEGFB; AREG, FGF7 and VEGFC; AREG, FGF7 and VEGFD; AREG, FGF7 and TGFB1; AREG, FGF7 and IL-10; AREG, FGF7 and an anti-TNFα antibody; AREG, FGF9 and FGF19; AREG, FGF9 and SCF; AREG, FGF9 and PDGFA; AREG, FGF9 and PDGFB; AREG, FGF9 and PDGFC; AREG, FGF9 and VEGFA; AREG, FGF9 and VEGFB; AREG, FGF9 and VEGFC; AREG, FGF9 and VEGFD; AREG, FGF9 and TGFB1; AREG, FGF9 and IL-10; AREG, FGF9 and an anti-TNFα antibody; AREG, FGF19 and SCF; AREG, FGF19 and PDGFA; AREG, FGF19 and PDGFB; AREG, FGF19 and PDGFC; AREG, FGF19 and VEGFA; AREG, FGF19 and VEGFB; AREG, FGF19 and VEGFC; AREG, FGF19 and VEGFD; AREG, FGF19 and TGFB1; AREG, FGF19 and IL-10; AREG, FGF19 and an anti-TNFa antibody; AREG, SCF and PDGFA; AREG, SCF and PDGFB; AREG, SCF and PDGFC; AREG, SCF and VEGFA; AREG, SCF and VEGFB; AREG, SCF and VEGFC; AREG, SCF and VEGFD; AREG, SCF and TGFB1; AREG, SCF and IL-10; AREG, SCF and an anti-TNFα antibody; AREG, PDGFA and PDGFB; AREG, PDGFA and PDGFC; AREG, PDGFA and VEGFA; AREG, PDGFA and VEGFB; AREG, PDGFA and VEGFC; AREG, PDGFA and VEGFD; AREG, PDGFA and TGFB1; AREG, PDGFA and IL-10; AREG, PDGFA and an anti-TNFα antibody; AREG, PDGFB and PDGFC; AREG, PDGFB and VEGFA; AREG, PDGFB and VEGFB; AREG, PDGFB and VEGFC; AREG, PDGFB and VEGFD; AREG, PDGFB and TGFB1; AREG, PDGFB and IL-10; AREG, PDGFB and an anti-TNFα antibody; AREG, PDGFC and VEGFA; AREG, PDGFC and VEGFB; AREG, PDGFC and VEGFC; AREG, PDGFC and VEGFD; AREG, PDGFC and TGFB1; AREG, PDGFC and IL-10; AREG, PDGFC and an anti-TNFα antibody; AREG, VEGFA and VEGFB; AREG, VEGFA and VEGFC; AREG, VEGFA and VEGFD; AREG, VEGFA and TGFB1; AREG, VEGFA and IL-10; AREG, VEGFA and an anti-TNFα antibody; AREG, VEGFB and VEGFC; AREG, VEGFB and VEGFD; AREG, VEGFB and TGFB1; AREG, VEGFB and IL-10; AREG, VEGFB and an anti-TNFa antibody; AREG, VEGFC and VEGFD; AREG, VEGFC and TGFB1; AREG, VEGFC and IL-10; AREG, VEGFC and an anti-TNFα antibody; AREG, VEGFD and TGFB1; AREG, VEGFD and IL-10; AREG, VEGFD and an anti-TNFa antibody; AREG, TGFB1 and IL-10; AREG, TGFB1 and an anti-TNFa antibody; AREG, IL-10 and an anti-TNFa antibody; EREG, AREG and HBEGF; EREG, AREG and HGF; EREG, AREG and HRGB; EREG, AREG and BTC; EREG, AREG and EGF; EREG, AREG and TGFA; EREG, AREG and FGF1; EREG, AREG and FGF2; EREG, AREG and FGF4; EREG, AREG and FGF7; EREG, AREG and FGF9; EREG, AREG and FGF19; EREG, AREG and SCF; EREG, AREG and PDGFA; EREG, AREG and PDGFB; EREG, AREG and PDGFC; EREG, AREG and VEGFA; EREG, AREG and VEGFB; EREG, AREG and VEGFC; EREG, AREG and VEGFD; EREG, AREG and TGFB1; EREG, AREG and IL-10; EREG, AREG and an anti-TNFa antibody; EREG, HBEGF and HGF; EREG, HBEGF and HRGB; EREG, HBEGF and BTC; EREG, HBEGF and EGF; EREG, HBEGF and TGFA; EREG, HBEGF and FGF1; EREG, HBEGF and FGF2; EREG, HBEGF and FGF4; EREG, HBEGF and FGF7; EREG, HBEGF and FGF9; EREG, HBEGF and FGF19; EREG, HBEGF and SCF; EREG, HBEGF and PDGFA; EREG, HBEGF and PDGFB; EREG, HBEGF and PDGFC; EREG, HBEGF and VEGFA; EREG, HBEGF and VEGFB; EREG, HBEGF and VEGFC; EREG, HBEGF and VEGFD; EREG, HBEGF and TGFB1; EREG, HBEGF and IL-10; EREG, HBEGF and an anti-TNFa antibody; EREG, HGF and HRGB; EREG, HGF and BTC; EREG, HGF and EGF; EREG, HGF and TGFA; EREG, HGF and FGF1; EREG, HGF and FGF2; EREG, HGF and FGF4; EREG, HGF and FGF7; EREG, HGF and FGF9; EREG, HGF and FGF19; EREG, HGF and SCF; EREG, HGF and PDGFA; EREG, HGF and PDGFB; EREG, HGF and PDGFC; EREG, HGF and VEGFA; EREG, HGF and VEGFB; EREG, HGF and VEGFC; EREG, HGF and VEGFD; EREG, HGF and TGFB1; EREG, HGF and IL-10; EREG, HGF and an anti-TNFa antibody; EREG, HRGB and BTC; EREG, HRGB and EGF; EREG, HRGB and TGFA; EREG, HRGB and FGF1; EREG, HRGB and FGF2; EREG, HRGB and FGF4; EREG, HRGB and FGF7; EREG, HRGB and FGF9; EREG, HRGB and FGF19; EREG, HRGB and SCF; EREG, HRGB and PDGFA; EREG, HRGB and PDGFB; EREG, HRGB and PDGFC; EREG, HRGB and VEGFA; EREG, HRGB and VEGFB; EREG, HRGB and VEGFC; EREG, HRGB and VEGFD; EREG, HRGB and TGFB1; EREG, HRGB and IL-10; EREG, HRGB and an anti-TNFa antibody; EREG, BTC and EGF; EREG, BTC and TGFA; EREG, BTC and FGF1; EREG, BTC and FGF2; EREG, BTC and FGF4; EREG, BTC and FGF7; EREG, BTC and FGF9; EREG, BTC and FGF19; EREG, BTC and SCF; EREG, BTC and PDGFA; EREG, BTC and PDGFB; EREG, BTC and PDGFC; EREG, BTC and VEGFA; EREG, BTC and VEGFB; EREG, BTC and VEGFC; EREG, BTC and VEGFD; EREG, BTC and TGFB1; EREG, BTC and IL-10; EREG, BTC and an anti-TNFa antibody; EREG, EGF and TGFA; EREG, EGF and FGF1; EREG, EGF and FGF2; EREG, EGF and FGF4; EREG, EGF and FGF7; EREG, EGF and FGF9; EREG, EGF and FGF19; EREG, EGF and SCF; EREG, EGF and PDGFA; EREG, EGF and PDGFB; EREG, EGF and PDGFC; EREG, EGF and VEGFA; EREG, EGF and VEGFB; EREG, EGF and VEGFC; EREG, EGF and VEGFD; EREG, EGF and TGFB1; EREG, EGF and IL-10; EREG, EGF and an anti-TNFa antibody; EREG, TGFA and FGF1; EREG, TGFA and FGF2; EREG, TGFA and FGF4; EREG, TGFA and FGF7; EREG, TGFA and FGF9; EREG, TGFA and FGF19; EREG, TGFA and SCF; EREG, TGFA and PDGFA; EREG, TGFA and PDGFB; EREG, TGFA and PDGFC; EREG, TGFA and VEGFA; EREG, TGFA and VEGFB; EREG, TGFA and VEGFC; EREG, TGFA and VEGFD; EREG, TGFA and TGFB1; EREG, TGFA and IL-10; EREG, TGFA and an anti-TNFα antibody; EREG, FGF1 and FGF2; EREG, FGF1 and FGF4; EREG, FGF1 and FGF7; EREG, FGF1 and FGF9; EREG, FGF1 and FGF19; EREG, FGF1 and SCF; EREG, FGF1 and PDGFA; EREG, FGF1 and PDGFB; EREG, FGF1 and PDGFC; EREG, FGF1 and VEGFA; EREG, FGF1 and VEGFB; EREG, FGF1 and VEGFC; EREG, FGF1 and VEGFD; EREG, FGF1 and TGFB1; EREG, FGF1 and IL-10; EREG, FGF1 and an anti-TNFa antibody; EREG, FGF2 and FGF4; EREG, FGF2 and FGF7; EREG, FGF2 and FGF9; EREG, FGF2 and FGF19; EREG, FGF2 and SCF; EREG, FGF2 and PDGFA; EREG, FGF2 and PDGFB; EREG, FGF2 and PDGFC; EREG, FGF2 and VEGFA; EREG, FGF2 and VEGFB; EREG, FGF2 and VEGFC; EREG, FGF2 and VEGFD; EREG, FGF2 and TGFB1; EREG, FGF2 and IL-10; EREG, FGF2 and an anti-TNFa antibody; EREG, FGF4 and FGF7; EREG, FGF4 and FGF9; EREG, FGF4 and FGF19; EREG, FGF4 and SCF; EREG, FGF4 and PDGFA; EREG, FGF4 and PDGFB; EREG, FGF4 and PDGFC; EREG, FGF4 and VEGFA; EREG, FGF4 and VEGFB; EREG, FGF4 and VEGFC; EREG, FGF4 and VEGFD; EREG, FGF4 and TGFB1; EREG, FGF4 and IL-10; EREG, FGF4 and an anti-TNFa antibody; EREG, FGF7 and FGF9; EREG, FGF7 and FGF19; EREG, FGF7 and SCF; EREG, FGF7 and PDGFA; EREG, FGF7 and PDGFB; EREG, FGF7 and PDGFC; EREG, FGF7 and VEGFA; EREG, FGF7 and VEGFB; EREG, FGF7 and VEGFC; EREG, FGF7 and VEGFD; EREG, FGF7 and TGFB1; EREG, FGF7 and IL-10; EREG, FGF7 and an anti-TNFa antibody; EREG, FGF9 and FGF19; EREG, FGF9 and SCF; EREG, FGF9 and PDGFA; EREG, FGF9 and PDGFB; EREG, FGF9 and PDGFC; EREG, FGF9 and VEGFA; EREG, FGF9 and VEGFB; EREG, FGF9 and VEGFC; EREG, FGF9 and VEGFD; EREG, FGF9 and TGFB1; EREG, FGF9 and IL-10; EREG, FGF9 and an anti-TNFa antibody; EREG, FGF19 and SCF; EREG, FGF19 and PDGFA; EREG, FGF19 and PDGFB; EREG, FGF19 and PDGFC; EREG, FGF19 and VEGFA; EREG, FGF19 and VEGFB; EREG, FGF19 and VEGFC; EREG, FGF19 and VEGFD; EREG, FGF19 and TGFB1; EREG, FGF19 and IL-10; EREG, FGF19 and an anti-TNFα antibody; EREG, SCF and PDGFA; EREG, SCF and PDGFB; EREG, SCF and PDGFC; EREG, SCF and VEGFA; EREG, SCF and VEGFB; EREG, SCF and VEGFC; EREG, SCF and VEGFD; EREG, SCF and TGFB1; EREG, SCF and IL-10; EREG, SCF and an anti-TNFa antibody; EREG, PDGFA and PDGFB; EREG, PDGFA and PDGFC; EREG, PDGFA and VEGFA; EREG, PDGFA and VEGFB; EREG, PDGFA and VEGFC; EREG, PDGFA and VEGFD; EREG, PDGFA and TGFB1; EREG, PDGFA and IL-10; EREG, PDGFA and an anti-TNFα antibody; EREG, PDGFB and PDGFC; EREG, PDGFB and VEGFA; EREG, PDGFB and VEGFB; EREG, PDGFB and VEGFC; EREG, PDGFB and VEGFD; EREG, PDGFB and TGFB1; EREG, PDGFB and IL-10; EREG, PDGFB and an anti-TNFa antibody; EREG, PDGFC and VEGFA; EREG, PDGFC and VEGFB; EREG, PDGFC and VEGFC; EREG, PDGFC and VEGFD; EREG, PDGFC and TGFB1; EREG, PDGFC and IL-10; EREG, PDGFC and an anti-TNFa antibody; EREG, VEGFA and VEGFB; EREG, VEGFA and VEGFC; EREG, VEGFA and VEGFD; EREG, VEGFA and TGFB1; EREG, VEGFA and IL-10; EREG, VEGFA and an anti-TNFa antibody; EREG, VEGFB and VEGFC; EREG, VEGFB and VEGFD; EREG, VEGFB and TGFB1; EREG, VEGFB and IL-10; EREG, VEGFB and an anti-TNFα antibody; EREG, VEGFC and VEGFD; EREG, VEGFC and TGFB1; EREG, VEGFC and IL-10; EREG, VEGFC and an anti-TNFα antibody; EREG, VEGFD and TGFB1; EREG, VEGFD and IL-10; EREG, VEGFD and an anti-TNFa antibody; EREG, TGFB1 and IL-10; EREG, TGFB1 and an anti-TNFα antibody; EREG, IL-10 and an anti-TNFα antibody; HBEGF, AREG and EREG; HBEGF, AREG and HGF; HBEGF, AREG and HRGB; HBEGF, AREG and BTC; HBEGF, AREG and EGF; HBEGF, AREG and TGFA; HBEGF, AREG and FGF1; HBEGF, AREG and FGF2; HBEGF, AREG and FGF4; HBEGF, AREG and FGF7; HBEGF, AREG and FGF9; HBEGF, AREG and FGF19; HBEGF, AREG and SCF; HBEGF, AREG and PDGFA; HBEGF, AREG and PDGFB; HBEGF, AREG and PDGFC; HBEGF, AREG and VEGFA; HBEGF, AREG and VEGFB; HBEGF, AREG and VEGFC; HBEGF, AREG and VEGFD; HBEGF, AREG and TGFB1; HBEGF, AREG and IL-10; HBEGF, AREG and an anti-TNFα antibody; HBEGF, EREG and HBEGF; HBEGF, EREG and HGF; HBEGF, EREG and HRGB; HBEGF, EREG and BTC; HBEGF, EREG and EGF; HBEGF, EREG and TGFA; HBEGF, EREG and FGF1; HBEGF, EREG and FGF2; HBEGF, EREG and FGF4; HBEGF, EREG and FGF7; HBEGF, EREG and FGF9; HBEGF, EREG and FGF19; HBEGF, EREG and SCF; HBEGF, EREG and PDGFA; HBEGF, EREG and PDGFB; HBEGF, EREG and PDGFC; HBEGF, EREG and VEGFA; HBEGF, EREG and VEGFB; HBEGF, EREG and VEGFC; HBEGF, EREG and VEGFD; HBEGF, EREG and TGFB1; HBEGF, EREG and IL-10; HBEGF, EREG and an anti-TNFa antibody; HBEGF, HGF and HRGB; HBEGF, HGF and BTC; HBEGF, HGF and EGF; HBEGF, HGF and TGFA; HBEGF, HGF and FGF1; HBEGF, HGF and FGF2; HBEGF, HGF and FGF4; HBEGF, HGF and FGF7; HBEGF, HGF and FGF9; HBEGF, HGF and FGF19; HBEGF, HGF and SCF; HBEGF, HGF and PDGFA; HBEGF, HGF and PDGFB; HBEGF, HGF and PDGFC; HBEGF, HGF and VEGFA; HBEGF, HGF and VEGFB; HBEGF, HGF and VEGFC; HBEGF, HGF and VEGFD; HBEGF, HGF and TGFB1; HBEGF, HGF and IL-10; HBEGF, HGF and an anti-TNFα antibody; HBEGF, HRGB and BTC; HBEGF, HRGB and EGF; HBEGF, HRGB and TGFA; HBEGF, HRGB and FGF1; HBEGF, HRGB and FGF2; HBEGF, HRGB and FGF4; HBEGF, HRGB and FGF7; HBEGF, HRGB and FGF9; HBEGF, HRGB and FGF19; HBEGF, HRGB and SCF; HBEGF, HRGB and PDGFA; HBEGF, HRGB and PDGFB; HBEGF, HRGB and PDGFC; HBEGF, HRGB and VEGFA; HBEGF, HRGB and VEGFB; HBEGF, HRGB and VEGFC; HBEGF, HRGB and VEGFD; HBEGF, HRGB and TGFB1; HBEGF, HRGB and IL-10; HBEGF, HRGB and an anti-TNFα antibody; HBEGF, BTC and EGF; HBEGF, BTC and TGFA; HBEGF, BTC and FGF1; HBEGF, BTC and FGF2; HBEGF, BTC and FGF4; HBEGF, BTC and FGF7; HBEGF, BTC and FGF9; HBEGF, BTC and FGF19; HBEGF, BTC and SCF; HBEGF, BTC and PDGFA; HBEGF, BTC and PDGFB; HBEGF, BTC and PDGFC; HBEGF, BTC and VEGFA; HBEGF, BTC and VEGFB; HBEGF, BTC and VEGFC; HBEGF, BTC and VEGFD; HBEGF, BTC and TGFB1; HBEGF, BTC and IL-10; HBEGF, BTC and an anti-TNFa antibody; HBEGF, EGF and TGFA; HBEGF, EGF and FGF1; HBEGF, EGF and FGF2; HBEGF, EGF and FGF4; HBEGF, EGF and FGF7; HBEGF, EGF and FGF9; HBEGF, EGF and FGF19; HBEGF, EGF and SCF; HBEGF, EGF and PDGFA; HBEGF, EGF and PDGFB; HBEGF, EGF and PDGFC; HBEGF, EGF and VEGFA; HBEGF, EGF and VEGFB; HBEGF, EGF and VEGFC; HBEGF, EGF and VEGFD; HBEGF, EGF and TGFB1; HBEGF, EGF and IL-10; HBEGF, EGF and an anti-TNFa antibody; HBEGF, TGFA and FGF1; HBEGF, TGFA and FGF2; HBEGF, TGFA and FGF4; HBEGF, TGFA and FGF7; HBEGF, TGFA and FGF9; HBEGF, TGFA and FGF19; HBEGF, TGFA and SCF; HBEGF, TGFA and PDGFA; HBEGF, TGFA and PDGFB; HBEGF, TGFA and PDGFC; HBEGF, TGFA and VEGFA; HBEGF, TGFA and VEGFB; HBEGF, TGFA and VEGFC; HBEGF, TGFA and VEGFD; HBEGF, TGFA and TGFB1; HBEGF, TGFA and IL-10; HBEGF, TGFA and an anti-TNFa antibody; HBEGF, FGF1 and FGF2; HBEGF, FGF1 and FGF4; HBEGF, FGF1 and FGF7; HBEGF, FGF1 and FGF9; HBEGF, FGF1 and FGF19; HBEGF, FGF1 and SCF; HBEGF, FGF1 and PDGFA; HBEGF, FGF1 and PDGFB; HBEGF, FGF1 and PDGFC; HBEGF, FGF1 and VEGFA; HBEGF, FGF1 and VEGFB; HBEGF, FGF1 and VEGFC; HBEGF, FGF1 and VEGFD; HBEGF, FGF1 and TGFB1; HBEGF, FGF1 and IL-10; HBEGF, FGF1 and an anti-TNFα antibody; HBEGF, FGF2 and FGF4; HBEGF, FGF2 and FGF7; HBEGF, FGF2 and FGF9; HBEGF, FGF2 and FGF19; HBEGF, FGF2 and SCF; HBEGF, FGF2 and PDGFA; HBEGF, FGF2 and PDGFB; HBEGF, FGF2 and PDGFC; HBEGF, FGF2 and VEGFA; HBEGF, FGF2 and VEGFB; HBEGF, FGF2 and VEGFC; HBEGF, FGF2 and VEGFD; HBEGF, FGF2 and TGFB1; HBEGF, FGF2 and IL-10; HBEGF, FGF2 and an anti-TNFa antibody; HBEGF, FGF4 and FGF7; HBEGF, FGF4 and FGF9; HBEGF, FGF4 and FGF19; HBEGF, FGF4 and SCF; HBEGF, FGF4 and PDGFA; HBEGF, FGF4 and PDGFB; HBEGF, FGF4 and PDGFC; HBEGF, FGF4 and VEGFA; HBEGF, FGF4 and VEGFB; HBEGF, FGF4 and VEGFC; HBEGF, FGF4 and VEGFD; HBEGF, FGF4 and TGFB1; HBEGF, FGF4 and IL-10; HBEGF, FGF4 and an anti-TNFα antibody; HBEGF, FGF7 and FGF9; HBEGF, FGF7 and FGF19; HBEGF, FGF7 and SCF; HBEGF, FGF7 and PDGFA; HBEGF, FGF7 and PDGFB; HBEGF, FGF7 and PDGFC; HBEGF, FGF7 and VEGFA; HBEGF, FGF7 and VEGFB; HBEGF, FGF7 and VEGFC; HBEGF, FGF7 and VEGFD; HBEGF, FGF7 and TGFB1; HBEGF, FGF7 and IL-10; HBEGF, FGF7 and an anti-TNFα antibody; HBEGF, FGF9 and FGF19; HBEGF, FGF9 and SCF; HBEGF, FGF9 and PDGFA; HBEGF, FGF9 and PDGFB; HBEGF, FGF9 and PDGFC; HBEGF, FGF9 and VEGFA; HBEGF, FGF9 and VEGFB; HBEGF, FGF9 and VEGFC; HBEGF, FGF9 and VEGFD; HBEGF, FGF9 and TGFB1; HBEGF, FGF9 and IL-10; HBEGF, FGF9 and an anti-TNFa antibody; HBEGF, FGF19 and SCF; HBEGF, FGF19 and PDGFA; HBEGF, FGF19 and PDGFB; HBEGF, FGF19 and PDGFC; HBEGF, FGF19 and VEGFA; HBEGF, FGF19 and VEGFB; HBEGF, FGF19 and VEGFC; HBEGF, FGF19 and VEGFD; HBEGF, FGF19 and TGFB1; HBEGF, FGF19 and IL-10; HBEGF, FGF19 and an anti-TNFα antibody; HBEGF, SCF and PDGFA; HBEGF, SCF and PDGFB; HBEGF, SCF and PDGFC; HBEGF, SCF and VEGFA; HBEGF, SCF and VEGFB; HBEGF, SCF and VEGFC; HBEGF, SCF and VEGFD; HBEGF, SCF and TGFB1; HBEGF, SCF and IL-10; HBEGF, SCF and an anti-TNFα antibody; HBEGF, PDGFA and PDGFB; HBEGF, PDGFA and PDGFC; HBEGF, PDGFA and VEGFA; HBEGF, PDGFA and VEGFB; HBEGF, PDGFA and VEGFC; HBEGF, PDGFA and VEGFD; HBEGF, PDGFA and TGFB1; HBEGF, PDGFA and IL-10; HBEGF, PDGFA and an anti-TNFa antibody; HBEGF, PDGFB and PDGFC; HBEGF, PDGFB and VEGFA; HBEGF, PDGFB and VEGFB; HBEGF, PDGFB and VEGFC; HBEGF, PDGFB and VEGFD; HBEGF, PDGFB and TGFB1; HBEGF, PDGFB and IL-10; HBEGF, PDGFB and an anti-TNFα antibody; HBEGF, PDGFC and VEGFA; HBEGF, PDGFC and VEGFB; HBEGF, PDGFC and VEGFC; HBEGF, PDGFC and VEGFD; HBEGF, PDGFC and TGFB1; HBEGF, PDGFC and IL-10; HBEGF, PDGFC and an anti-TNFα antibody; HBEGF, VEGFA and VEGFB; HBEGF, VEGFA and VEGFC; HBEGF, VEGFA and VEGFD; HBEGF, VEGFA and TGFB1; HBEGF, VEGFA and IL-10; HBEGF, VEGFA and an anti-TNFα antibody; HBEGF, VEGFB and VEGFC; HBEGF, VEGFB and VEGFD; HBEGF, VEGFB and TGFB1; HBEGF, VEGFB and IL-10; HBEGF, VEGFB and an anti-TNFa antibody; HBEGF, VEGFC and VEGFD; HBEGF, VEGFC and TGFB1; HBEGF, VEGFC and IL-10; HBEGF, VEGFC and an anti-TNFa antibody; HBEGF, VEGFD and TGFB1; HBEGF, VEGFD and IL-10; HBEGF, VEGFD and an anti-TNFα antibody; HBEGF, TGFB1 and IL-10; HBEGF, TGFB1 and an anti-TNFa antibody; HBEGF, IL-10 and an anti-TNFa antibody; HGF, AREG and EREG; HGF, AREG and HBEGF; HGF, AREG and HRGB; HGF, AREG and BTC; HGF, AREG and EGF; HGF, AREG and TGFA; HGF, AREG and FGF1; HGF, AREG and FGF2; HGF, AREG and FGF4; HGF, AREG and FGF7; HGF, AREG and FGF9; HGF, AREG and FGF19; HGF, AREG and SCF; HGF, AREG and PDGFA; HGF, AREG and PDGFB; HGF, AREG and PDGFC; HGF, AREG and VEGFA; HGF, AREG and VEGFB; HGF, AREG and VEGFC; HGF, AREG and VEGFD; HGF, AREG and TGFB1; HGF, AREG and IL-10; HGF, AREG and an anti-TNFa antibody; HGF, EREG and HBEGF; HGF, EREG and HRGB; HGF, EREG and BTC; HGF, EREG and EGF; HGF, EREG and TGFA; HGF, EREG and FGF1; HGF, EREG and FGF2; HGF, EREG and FGF4; HGF, EREG and FGF7; HGF, EREG and FGF9; HGF, EREG and FGF19; HGF, EREG and SCF; HGF, EREG and PDGFA; HGF, EREG and PDGFB; HGF, EREG and PDGFC; HGF, EREG and VEGFA; HGF, EREG and VEGFB; HGF, EREG and VEGFC; HGF, EREG and VEGFD; HGF, EREG and TGFB1; HGF, EREG and IL-10; HGF, EREG and an anti-TNFα antibody; HGF, HBEGF and HRGB; HGF, HBEGF and BTC; 00HGF, HBEGF and EGF; HGF, HBEGF and TGFA; HGF, HBEGF and FGF1; HGF, HBEGF and FGF2; HGF, HBEGF and FGF4; HGF, HBEGF and FGF7; HGF, HBEGF and FGF9; HGF, HBEGF and FGF19; HGF, HBEGF and SCF; HGF, HBEGF and PDGFA; HGF, HBEGF and PDGFB; HGF, HBEGF and PDGFC; HGF, HBEGF and VEGFA; HGF, HBEGF and VEGFB; HGF, HBEGF and VEGFC; HGF, HBEGF and VEGFD; HGF, HBEGF and TGFB1; HGF, HBEGF and IL-10; HGF, HBEGF and an anti-TNFa antibody; HGF, HRGB and BTC; HGF, HRGB and EGF; HGF, HRGB and TGFA; HGF, HRGB and FGF1; HGF, HRGB and FGF2; HGF, HRGB and FGF4; HGF, HRGB and FGF7; HGF, HRGB and FGF9; HGF, HRGB and FGF19; HGF, HRGB and SCF; HGF, HRGB and PDGFA; HGF, HRGB and PDGFB; HGF, HRGB and PDGFC; HGF, HRGB and VEGFA; HGF, HRGB and VEGFB; HGF, HRGB and VEGFC; HGF, HRGB and VEGFD; HGF, HRGB and TGFB1; HGF, HRGB and IL-10; HGF, HRGB and an anti-TNFa antibody; HGF, BTC and EGF; HGF, BTC and TGFA; HGF, BTC and FGF1; HGF, BTC and FGF2; HGF, BTC and FGF4; HGF, BTC and FGF7; HGF, BTC and FGF9; HGF, BTC and FGF19; HGF, BTC and SCF; HGF, BTC and PDGFA; HGF, BTC and PDGFB; HGF, BTC and PDGFC; HGF, BTC and VEGFA; HGF, BTC and VEGFB; HGF, BTC and VEGFC; HGF, BTC and VEGFD; HGF, BTC and TGFB1; HGF, BTC and IL-10; HGF, BTC and an anti-TNFα antibody; HGF, EGF and TGFA; HGF, EGF and FGF1; HGF, EGF and FGF2; HGF, EGF and FGF4; HGF, EGF and FGF7; HGF, EGF and FGF9; HGF, EGF and FGF19; HGF, EGF and SCF; HGF, EGF and PDGFA; HGF, EGF and PDGFB; HGF, EGF and PDGFC; HGF, EGF and VEGFA; HGF, EGF and VEGFB; HGF, EGF and VEGFC; HGF, EGF and VEGFD; HGF, EGF and TGFB1; HGF, EGF and IL-10; HGF, EGF and an anti-TNFα antibody; HGF, TGFA and FGF1; HGF, TGFA and FGF2; HGF, TGFA and FGF4; HGF, TGFA and FGF7; HGF, TGFA and FGF9; HGF, TGFA and FGF19; HGF, TGFA and SCF; HGF, TGFA and PDGFA; HGF, TGFA and PDGFB; HGF, TGFA and PDGFC; HGF, TGFA and VEGFA; HGF, TGFA and VEGFB; HGF, TGFA and VEGFC; HGF, TGFA and VEGFD; HGF, TGFA and TGFB1; HGF, TGFA and IL-10; HGF, TGFA and an anti-TNFa antibody; HGF, FGF1 and FGF2; HGF, FGF1 and FGF4; HGF, FGF1 and FGF7; HGF, FGF1 and FGF9; HGF, FGF1 and FGF19; HGF, FGF1 and SCF; HGF, FGF1 and PDGFA; HGF, FGF1 and PDGFB; HGF, FGF1 and PDGFC; HGF, FGF1 and VEGFA; HGF, FGF1 and VEGFB; HGF, FGF1 and VEGFC; HGF, FGF1 and VEGFD; HGF, FGF1 and TGFB1; HGF, FGF1 and IL-10; HGF, FGF1 and an anti-TNFα antibody; HGF, FGF2 and FGF4; HGF, FGF2 and FGF7; HGF, FGF2 and FGF9; HGF, FGF2 and FGF19; HGF, FGF2 and SCF; HGF, FGF2 and PDGFA; HGF, FGF2 and PDGFB; HGF, FGF2 and PDGFC; HGF, FGF2 and VEGFA; HGF, FGF2 and VEGFB; HGF, FGF2 and VEGFC; HGF, FGF2 and VEGFD; HGF, FGF2 and TGFB1; HGF, FGF2 and IL-10; HGF, FGF2 and an anti-TNFa antibody; HGF, FGF4 and FGF7; HGF, FGF4 and FGF9; HGF, FGF4 and FGF19; HGF, FGF4 and SCF; HGF, FGF4 and PDGFA; HGF, FGF4 and PDGFB; HGF, FGF4 and PDGFC; HGF, FGF4 and VEGFA; HGF, FGF4 and VEGFB; HGF, FGF4 and VEGFC; HGF, FGF4 and VEGFD; HGF, FGF4 and TGFB1; HGF, FGF4 and IL-10; HGF, FGF4 and an anti-TNFα antibody; HGF, FGF7 and FGF9; HGF, FGF7 and FGF19; HGF, FGF7 and SCF; HGF, FGF7 and PDGFA; HGF, FGF7 and PDGFB; HGF, FGF7 and PDGFC; HGF, FGF7 and VEGFA; HGF, FGF7 and VEGFB; HGF, FGF7 and VEGFC; HGF, FGF7 and VEGFD; HGF, FGF7 and TGFB1; HGF, FGF7 and IL-10; HGF, FGF7 and an anti-TNFa antibody; HGF, FGF9 and FGF19; HGF, FGF9 and SCF; HGF, FGF9 and PDGFA; HGF, FGF9 and PDGFB; HGF, FGF9 and PDGFC; HGF, FGF9 and VEGFA; HGF, FGF9 and VEGFB; HGF, FGF9 and VEGFC; HGF, FGF9 and VEGFD; HGF, FGF9 and TGFB1; HGF, FGF9 and IL-10; HGF, FGF9 and an anti-TNFα antibody; HGF, FGF19 and SCF; HGF, FGF19 and PDGFA; HGF, FGF19 and PDGFB; HGF, FGF19 and PDGFC; HGF, FGF19 and VEGFA; HGF, FGF19 and VEGFB; HGF, FGF19 and VEGFC; HGF, FGF19 and VEGFD; HGF, FGF19 and TGFB1; HGF, FGF19 and IL-10; HGF, FGF19 and an anti-TNFα antibody; HGF, SCF and PDGFA; HGF, SCF and PDGFB; HGF, SCF and PDGFC; HGF, SCF and VEGFA; HGF, SCF and VEGFB; HGF, SCF and VEGFC; HGF, SCF and VEGFD; HGF, SCF and TGFB1; HGF, SCF and IL-10; HGF, SCF and an anti-TNFα antibody; HGF, PDGFA and PDGFB; HGF, PDGFA and PDGFC; HGF, PDGFA and VEGFA; HGF, PDGFA and VEGFB; HGF, PDGFA and VEGFC; HGF, PDGFA and VEGFD; HGF, PDGFA and TGFB1; HGF, PDGFA and IL-10; HGF, PDGFA and an anti-TNFα antibody; HGF, PDGFB and PDGFC; HGF, PDGFB and VEGFA; HGF, PDGFB and VEGFB; HGF, PDGFB and VEGFC; HGF, PDGFB and VEGFD; HGF, PDGFB and TGFB1; HGF, PDGFB and IL-10; HGF, PDGFB and an anti-TNFα antibody; HGF, PDGFC and VEGFA; HGF, PDGFC and VEGFB; HGF, PDGFC and VEGFC; HGF, PDGFC and VEGFD; HGF, PDGFC and TGFB1; HGF, PDGFC and IL-10; HGF, PDGFC and an anti-TNFa antibody; HGF, VEGFA and VEGFB; HGF, VEGFA and VEGFC; HGF, VEGFA and VEGFD; HGF, VEGFA and TGFB1; HGF, VEGFA and IL-10; HGF, VEGFA and an anti-TNFa antibody; HGF, VEGFB and VEGFC; HGF, VEGFB and VEGFD; HGF, VEGFB and TGFB1; HGF, VEGFB and IL-10; HGF, VEGFB and an anti-TNFα antibody; HGF, VEGFC and VEGFD; HGF, VEGFC and TGFB1; HGF, VEGFC and IL-10; HGF, VEGFC and an anti-TNFa antibody; HGF, VEGFD and TGFB1; HGF, VEGFD and IL-10; HGF, VEGFD and an anti-TNFα antibody; HGF, TGFB1 and IL-10; HGF, TGFB1 and an anti-TNFα antibody; HGF, IL-10 and an anti-TNFα antibody; HRGB, AREG and EREG; HRGB, AREG and HBEGF; HRGB, AREG and HGF; HRGB, AREG and BTC; HRGB, AREG and EGF; HRGB, AREG and TGFA; HRGB, AREG and FGF1; HRGB, AREG and FGF2; HRGB, AREG and FGF4; HRGB, AREG and FGF7; HRGB, AREG and FGF9; HRGB, AREG and FGF19; HRGB, AREG and SCF; HRGB, AREG and PDGFA; HRGB, AREG and PDGFB; HRGB, AREG and PDGFC; HRGB, AREG and VEGFA; HRGB, AREG and VEGFB; HRGB, AREG and VEGFC; HRGB, AREG and VEGFD; HRGB, AREG and TGFB1; HRGB, AREG and IL-10; HRGB, AREG and an anti-TNFα antibody; HRGB, EREG and HBEGF; HRGB, EREG and HGF; HRGB, EREG and BTC; HRGB, EREG and EGF; HRGB, EREG and TGFA; HRGB, EREG and FGF1; HRGB, EREG and FGF2; HRGB, EREG and FGF4; HRGB, EREG and FGF7; HRGB, EREG and FGF9; HRGB, EREG and FGF19; HRGB, EREG and SCF; HRGB, EREG and PDGFA; HRGB, EREG and PDGFB; HRGB, EREG and PDGFC; HRGB, EREG and VEGFA; HRGB, EREG and VEGFB; HRGB, EREG and VEGFC; HRGB, EREG and VEGFD; HRGB, EREG and TGFB1; HRGB, EREG and IL-10; HRGB, EREG and an anti-TNFa antibody; HRGB, HBEGF and HGF; HRGB, HBEGF and BTC; HRGB, HBEGF and EGF; HRGB, HBEGF and TGFA; HRGB, HBEGF and FGF1; HRGB, HBEGF and FGF2; HRGB, HBEGF and FGF4; HRGB, HBEGF and FGF7; HRGB, HBEGF and FGF9; HRGB, HBEGF and FGF19; HRGB, HBEGF and SCF; HRGB, HBEGF and PDGFA; HRGB, HBEGF and PDGFB; HRGB, HBEGF and PDGFC; HRGB, HBEGF and VEGFA; HRGB, HBEGF and VEGFB; HRGB, HBEGF and VEGFC; HRGB, HBEGF and VEGFD; HRGB, HBEGF and TGFB1; HRGB, HBEGF and IL-10; HRGB, HBEGF and an anti-TNFα antibody; HRGB, HGF and BTC; HRGB, HGF and EGF; HRGB, HGF and TGFA; HRGB, HGF and FGF1; HRGB, HGF and FGF2; HRGB, HGF and FGF4; HRGB, HGF and FGF7; HRGB, HGF and FGF9; HRGB, HGF and FGF19; HRGB, HGF and SCF; HRGB, HGF and PDGFA; HRGB, HGF and PDGFB; HRGB, HGF and PDGFC; HRGB, HGF and VEGFA; HRGB, HGF and VEGFB; HRGB, HGF and VEGFC; HRGB, HGF and VEGFD; HRGB, HGF and TGFB1; HRGB, HGF and IL-10; HRGB, HGF and an anti-TNFa antibody; HRGB, and an anti-TNFα antibody; HRGB, BTC and EGF; HRGB, BTC and TGFA; HRGB, BTC and FGF1; HRGB, BTC and FGF2; HRGB, BTC and FGF4; HRGB, BTC and FGF7; HRGB, BTC and FGF9; HRGB, BTC and FGF19; HRGB, BTC and SCF; HRGB, BTC and PDGFA; HRGB, BTC and PDGFB; HRGB, BTC and PDGFC; HRGB, BTC and VEGFA; HRGB, BTC and VEGFB; HRGB, BTC and VEGFC; HRGB, BTC and VEGFD; HRGB, BTC and TGFB1; HRGB, BTC and IL-10; HRGB, BTC and an anti-TNFα antibody; HRGB, EGF and TGFA; HRGB, EGF and FGF1; HRGB, EGF and FGF2; HRGB, EGF and FGF4; HRGB, EGF and FGF7; HRGB, EGF and FGF9; HRGB, EGF and FGF19; HRGB, EGF and SCF; HRGB, EGF and PDGFA; HRGB, EGF and PDGFB; HRGB, EGF and PDGFC; HRGB, EGF and VEGFA; HRGB, EGF and VEGFB; HRGB, EGF and VEGFC; HRGB, EGF and VEGFD; HRGB, EGF and TGFB1; HRGB, EGF and IL-10; HRGB, EGF and an anti-TNFα antibody; HRGB, TGFA and FGF1; HRGB, TGFA and FGF2; HRGB, TGFA and FGF4; HRGB, TGFA and FGF7; HRGB, TGFA and FGF9; HRGB, TGFA and FGF19; HRGB, TGFA and SCF; HRGB, TGFA and PDGFA; HRGB, TGFA and PDGFB; HRGB, TGFA and PDGFC; HRGB, TGFA and VEGFA; HRGB, TGFA and VEGFB; HRGB, TGFA and VEGFC; HRGB, TGFA and VEGFD; HRGB, TGFA and TGFB1; HRGB, TGFA and IL-10; HRGB, TGFA and an anti-TNFa antibody; HRGB, FGF1 and FGF2; HRGB, FGF1 and FGF4; HRGB, FGF1 and FGF7; HRGB, FGF1 and FGF9; HRGB, FGF1 and FGF19; HRGB, FGF1 and SCF; HRGB, FGF1 and PDGFA; HRGB, FGF1 and PDGFB; HRGB, FGF1 and PDGFC; HRGB, FGF1 and VEGFA; HRGB, FGF1 and VEGFB; HRGB, FGF1 and VEGFC; HRGB, FGF1 and VEGFD; HRGB, FGF1 and TGFB1; HRGB, FGF1 and IL-10; HRGB, FGF1 and an anti-TNFα antibody; HRGB, FGF2 and FGF4; HRGB, FGF2 and FGF7; HRGB, FGF2 and FGF9; HRGB, FGF2 and FGF19; HRGB, FGF2 and SCF; HRGB, FGF2 and PDGFA; HRGB, FGF2 and PDGFB; HRGB, FGF2 and PDGFC; HRGB, FGF2 and VEGFA; HRGB, FGF2 and VEGFB; HRGB, FGF2 and VEGFC; HRGB, FGF2 and VEGFD; HRGB, FGF2 and TGFB1; HRGB, FGF2 and IL-10; HRGB, FGF2 and an anti-TNFa antibody; HRGB, FGF4 and FGF7; HRGB, FGF4 and FGF9; HRGB, FGF4 and FGF19; HRGB, FGF4 and SCF; HRGB, FGF4 and PDGFA; HRGB, FGF4 and PDGFB; HRGB, FGF4 and PDGFC; HRGB, FGF4 and VEGFA; HRGB, FGF4 and VEGFB; HRGB, FGF4 and VEGFC; HRGB, FGF4 and VEGFD; HRGB, FGF4 and TGFB1; HRGB, FGF4 and IL-10; HRGB, FGF4 and an anti-TNFα antibody; HRGB, FGF7 and FGF9; HRGB, FGF7 and FGF19; HRGB, FGF7 and SCF; HRGB, FGF7 and PDGFA; HRGB, FGF7 and PDGFB; HRGB, FGF7 and PDGFC; HRGB, FGF7 and VEGFA; HRGB, FGF7 and VEGFB; HRGB, FGF7 and VEGFC; HRGB, FGF7 and VEGFD; HRGB, FGF7 and TGFB1; HRGB, FGF7 and IL-10; HRGB, FGF7 and an anti-TNFα antibody; HRGB, FGF9 and FGF19; HRGB, FGF9 and SCF; HRGB, FGF9 and PDGFA; HRGB, FGF9 and PDGFB; HRGB, FGF9 and PDGFC; HRGB, FGF9 and VEGFA; HRGB, FGF9 and VEGFB; HRGB, FGF9 and VEGFC; HRGB, FGF9 and VEGFD; HRGB, FGF9 and TGFB1; HRGB, FGF9 and IL-10; HRGB, FGF9 and an anti-TNFα antibody; HRGB, FGF19 and SCF; HRGB, FGF19 and PDGFA; HRGB, FGF19 and PDGFB; HRGB, FGF19 and PDGFC; HRGB, FGF19 and VEGFA; HRGB, FGF19 and VEGFB; HRGB, FGF19 and VEGFC; HRGB, FGF19 and VEGFD; HRGB, FGF19 and TGFB1; HRGB, FGF19 and IL-10; HRGB, FGF19 and an anti-TNFα antibody; HRGB, SCF and PDGFA; HRGB, SCF and PDGFB; HRGB, SCF and PDGFC; HRGB, SCF and VEGFA; HRGB, SCF and VEGFB; HRGB, SCF and VEGFC; HRGB, SCF and VEGFD; HRGB, SCF and TGFB1; HRGB, SCF and IL-10; HRGB, SCF and an anti-TNFα antibody; HRGB, PDGFA and PDGFB; HRGB, PDGFA and PDGFC; HRGB, PDGFA and VEGFA; HRGB, PDGFA and VEGFB; HRGB, PDGFA and VEGFC; HRGB, PDGFA and VEGFD; HRGB, PDGFA and TGFB1; HRGB, PDGFA and IL-10; HRGB, PDGFA and an anti-TNFα antibody; HRGB, PDGFB and PDGFC; HRGB, PDGFB and VEGFA; HRGB, PDGFB and VEGFB; HRGB, PDGFB and VEGFC; HRGB, PDGFB and VEGFD; HRGB, PDGFB and TGFB1; HRGB, PDGFB and IL-10; HRGB, PDGFB and an anti-TNFa antibody; HRGB, PDGFC and VEGFA; HRGB, PDGFC and VEGFB; HRGB, PDGFC and VEGFC; HRGB, PDGFC and VEGFD; HRGB, PDGFC and TGFB1; HRGB, PDGFC and IL-10; HRGB, PDGFC and an anti-TNFα antibody; HRGB, VEGFA and VEGFB; HRGB, VEGFA and VEGFC; HRGB, VEGFA and VEGFD; HRGB, VEGFA and TGFB1; HRGB, VEGFA and IL-10; HRGB, VEGFA and an anti-TNFa antibody; HRGB, VEGFB and VEGFC; HRGB, VEGFB and VEGFD; HRGB, VEGFB and TGFB1; HRGB, VEGFB and IL-10; HRGB, VEGFB and an anti-TNFa antibody; HRGB, VEGFC and VEGFD; HRGB, VEGFC and TGFB1; HRGB, VEGFC and IL-10; HRGB, VEGFC and an anti-TNFa antibody; HRGB, VEGFD and TGFB1; HRGB, VEGFD and IL-10; HRGB, VEGFD and an anti-TNFα antibody; HRGB, TGFB1 and IL-10; HRGB, TGFB1 and an anti-TNFa antibody; HRGB, IL-10 and an anti-TNFα antibody; BTC, AREG and EREG; BTC, AREG and HBEGF; BTC, AREG and HGF; BTC, AREG and HRGB; BTC, AREG and EGF; BTC, AREG and TGFA; BTC, AREG and FGF1; BTC, AREG and FGF2; BTC, AREG and FGF4; BTC, AREG and FGF7; BTC, AREG and FGF9; BTC, AREG and FGF19; BTC, AREG and SCF; BTC, AREG and PDGFA; BTC, AREG and PDGFB; BTC, AREG and PDGFC; BTC, AREG and VEGFA; BTC, AREG and VEGFB; BTC, AREG and VEGFC; BTC, AREG and VEGFD; BTC, AREG and TGFB1; BTC, AREG and IL-10; BTC, AREG and an anti-TNFα antibody; BTC, EREG and HBEGF; BTC, EREG and HGF; BTC, EREG and HRGB; BTC, EREG and EGF; BTC, EREG and TGFA; BTC, EREG and FGF1; BTC, EREG and FGF2; BTC, EREG and FGF4; BTC, EREG and FGF7; BTC, EREG and FGF9; BTC, EREG and FGF19; BTC, EREG and SCF; BTC, EREG and PDGFA; BTC, EREG and PDGFB; BTC, EREG and PDGFC; BTC, EREG and VEGFA; BTC, EREG and VEGFB; BTC, EREG and VEGFC; BTC, EREG and VEGFD; BTC, EREG and TGFB1; BTC, EREG and IL-10; BTC, EREG and an anti-TNFα antibody; BTC, HBEGF and HGF; BTC, HBEGF and HRGB; BTC, HBEGF and EGF; BTC, HBEGF and TGFA; BTC, HBEGF and FGF1; BTC, HBEGF and FGF2; BTC, HBEGF and FGF4; BTC, HBEGF and FGF7; BTC, HBEGF and FGF9; BTC, HBEGF and FGF19; BTC, HBEGF and SCF; BTC, HBEGF and PDGFA; BTC, HBEGF and PDGFB; BTC, HBEGF and PDGFC; BTC, HBEGF and VEGFA; BTC, HBEGF and VEGFB; BTC, HBEGF and VEGFC; BTC, HBEGF and VEGFD; BTC, HBEGF and TGFB1; BTC, HBEGF and IL-10; BTC, HBEGF and an anti-TNFα antibody; BTC, HGF and HRGB; BTC, HGF and EGF; BTC, HGF and TGFA; BTC, HGF and FGF1; BTC, HGF and FGF2; BTC, HGF and FGF4; BTC, HGF and FGF7; BTC, HGF and FGF9; BTC, HGF and FGF19; BTC, HGF and SCF; BTC, HGF and PDGFA; BTC, HGF and PDGFB; BTC, HGF and PDGFC; BTC, HGF and VEGFA; BTC, HGF and VEGFB; BTC, HGF and VEGFC; BTC, HGF and VEGFD; BTC, HGF and TGFB1; BTC, HGF and IL-10; BTC, HGF and an anti-TNFα antibody; BTC, HRGB and EGF; BTC, HRGB and TGFA; BTC, HRGB and FGF1; BTC, HRGB and FGF2; BTC, HRGB and FGF4; BTC, HRGB and FGF7; BTC, HRGB and FGF9; BTC, HRGB and FGF19; BTC, HRGB and SCF; BTC, HRGB and PDGFA; BTC, HRGB and PDGFB; BTC, HRGB and PDGFC; BTC, HRGB and VEGFA; BTC, HRGB and VEGFB; BTC, HRGB and VEGFC; BTC, HRGB and VEGFD; BTC, HRGB and TGFB1; BTC, HRGB and IL-10; BTC, HRGB and an anti-TNFα antibody; BTC, EGF and TGFA; BTC, EGF and FGF1; BTC, EGF and FGF2; BTC, EGF and FGF4; BTC, EGF and FGF7; BTC, EGF and FGF9; BTC, EGF and FGF19; BTC, EGF and SCF; BTC, EGF and PDGFA; BTC, EGF and PDGFB; BTC, EGF and PDGFC; BTC, EGF and VEGFA; BTC, EGF and VEGFB; BTC, EGF and VEGFC; BTC, EGF and VEGFD; BTC, EGF and TGFB1; BTC, EGF and IL-10; BTC, EGF and an anti-TNFα antibody; BTC, TGFA and FGF1; BTC, TGFA and FGF2; BTC, TGFA and FGF4; BTC, TGFA and FGF7; BTC, TGFA and FGF9; BTC, TGFA and FGF19; BTC, TGFA and SCF; BTC, TGFA and PDGFA; BTC, TGFA and PDGFB; BTC, TGFA and PDGFC; BTC, TGFA and VEGFA; BTC, TGFA and VEGFB; BTC, TGFA and VEGFC; BTC, TGFA and VEGFD; BTC, TGFA and TGFB1; BTC, TGFA and IL-10; BTC, TGFA and an anti-TNFα antibody; BTC, FGF1 and FGF2; BTC, FGF1 and FGF4; BTC, FGF1 and FGF7; BTC, FGF1 and FGF9; BTC, FGF1 and FGF19; BTC, FGF1 and SCF; BTC, FGF1 and PDGFA; BTC, FGF1 and PDGFB; BTC, FGF1 and PDGFC; BTC, FGF1 and VEGFA; BTC, FGF1 and VEGFB; BTC, FGF1 and VEGFC; BTC, FGF1 and VEGFD; BTC, FGF1 and TGFB1; BTC, FGF1 and IL-10; BTC, FGF1 and an anti-TNFα antibody; BTC, FGF2 and FGF4; BTC, FGF2 and FGF7; BTC, FGF2 and FGF9; BTC, FGF2 and FGF19; BTC, FGF2 and SCF; BTC, FGF2 and PDGFA; BTC, FGF2 and PDGFB; BTC, FGF2 and PDGFC; BTC, FGF2 and VEGFA; BTC, FGF2 and VEGFB; BTC, FGF2 and VEGFC; BTC, FGF2 and VEGFD; BTC, FGF2 and TGFB1; BTC, FGF2 and IL-10; BTC, FGF2 and an anti-TNFα antibody; BTC, FGF4 and FGF7; BTC, FGF4 and FGF9; BTC, FGF4 and FGF19; BTC, FGF4 and SCF; BTC, FGF4 and PDGFA; BTC, FGF4 and PDGFB; BTC, FGF4 and PDGFC; BTC, FGF4 and VEGFA; BTC, FGF4 and VEGFB; BTC, FGF4 and VEGFC; BTC, FGF4 and VEGFD; BTC, FGF4 and TGFB1; BTC, FGF4 and IL-10; BTC, FGF4 and an anti-TNFα antibody; BTC, FGF7 and FGF9; BTC, FGF7 and FGF19; BTC, FGF7 and SCF; BTC, FGF7 and PDGFA; BTC, FGF7 and PDGFB; BTC, FGF7 and PDGFC; BTC, FGF7 and VEGFA; BTC, FGF7 and VEGFB; BTC, FGF7 and VEGFC; BTC, FGF7 and VEGFD; BTC, FGF7 and TGFB1; BTC, FGF7 and IL-10; BTC, FGF7 and an anti-TNFα antibody; BTC, FGF9 and FGF19; BTC, FGF9 and SCF; BTC, FGF9 and PDGFA; BTC, FGF9 and PDGFB; BTC, FGF9 and PDGFC; BTC, FGF9 and VEGFA; BTC, FGF9 and VEGFB; BTC, FGF9 and VEGFC; BTC, FGF9 and VEGFD; BTC, FGF9 and TGFB1; BTC, FGF9 and IL-10; BTC, FGF9 and an anti-TNFα antibody; BTC, FGF19 and SCF; BTC, FGF19 and PDGFA; BTC, FGF19 and PDGFB; BTC, FGF19 and PDGFC; BTC, FGF19 and VEGFA; BTC, FGF19 and VEGFB; BTC, FGF19 and VEGFC; BTC, FGF19 and VEGFD; BTC, FGF19 and TGFB1; BTC, FGF19 and IL-10; BTC, FGF19 and an anti-TNFα antibody; BTC, SCF and PDGFA; BTC, SCF and PDGFB; BTC, SCF and PDGFC; BTC, SCF and VEGFA; BTC, SCF and VEGFB; BTC, SCF and VEGFC; BTC, SCF and VEGFD; BTC, SCF and TGFB1; BTC, SCF and IL-10; BTC, SCF and an anti-TNFα antibody; BTC, PDGFA and PDGFB; BTC, PDGFA and PDGFC; BTC, PDGFA and VEGFA; BTC, PDGFA and VEGFB; BTC, PDGFA and VEGFC; BTC, PDGFA and VEGFD; BTC, PDGFA and TGFB1; BTC, PDGFA and IL-10; BTC, PDGFA and an anti-TNFa antibody; BTC, PDGFB and PDGFC; BTC, PDGFB and VEGFA; BTC, PDGFB and VEGFB; BTC, PDGFB and VEGFC; BTC, PDGFB and VEGFD; BTC, PDGFB and TGFB1; BTC, PDGFB and IL-10; BTC, PDGFB and an anti-TNFα antibody; BTC, PDGFC and VEGFA; BTC, PDGFC and VEGFB; BTC, PDGFC and VEGFC; BTC, PDGFC and VEGFD; BTC, PDGFC and TGFB1; BTC, PDGFC and IL-10; BTC, PDGFC and an anti-TNFa antibody; BTC, VEGFA and VEGFB; BTC, VEGFA and VEGFC; BTC, VEGFA and VEGFD; BTC, VEGFA and TGFB1; BTC, VEGFA and IL-10; BTC, VEGFA and an anti-TNFα antibody; BTC, VEGFB and VEGFC; BTC, VEGFB and VEGFD; BTC, VEGFB and TGFB1; BTC, VEGFB and IL-10; BTC, VEGFB and an anti-TNFa antibody; BTC, VEGFC and VEGFD; BTC, VEGFC and TGFB1; BTC, VEGFC and IL-10; BTC, VEGFC and an anti-TNFa antibody; BTC, VEGFD and TGFB1; BTC, VEGFD and IL-10; BTC, VEGFD and an anti-TNFa antibody; BTC, TGFB1 and IL-10; BTC, TGFB1 and an anti-TNFα antibody; BTC, IL-10 and an anti-TNFα antibody; EGF, AREG and EREG; EGF, AREG and HBEGF; EGF, AREG and HGF; EGF, AREG and HRGB; EGF, AREG and BTC; EGF, AREG and TGFA; EGF, AREG and FGF1; EGF, AREG and FGF2; EGF, AREG and FGF4; EGF, AREG and FGF7; EGF, AREG and FGF9; EGF, AREG and FGF19; EGF, AREG and SCF; EGF, AREG and PDGFA; EGF, AREG and PDGFB; EGF, AREG and PDGFC; EGF, AREG and VEGFA; EGF, AREG and VEGFB; EGF, AREG and VEGFC; EGF, AREG and VEGFD; EGF, AREG and TGFB1; EGF, AREG and IL-10; EGF, AREG and an anti-TNFα antibody; EGF, EREG and HBEGF; EGF, EREG and HGF; EGF, EREG and HRGB; EGF, EREG and BTC; EGF, EREG and TGFA; EGF, EREG and FGF1; EGF, EREG and FGF2; EGF, EREG and FGF4; EGF, EREG and FGF7; EGF, EREG and FGF9; EGF, EREG and FGF19; EGF, EREG and SCF; EGF, EREG and PDGFA; EGF, EREG and PDGFB; EGF, EREG and PDGFC; EGF, EREG and VEGFA; EGF, EREG and VEGFB; EGF, EREG and VEGFC; EGF, EREG and VEGFD; EGF, EREG and TGFB1; EGF, EREG and IL-10; EGF, EREG and an anti-TNFa antibody; EGF, HBEGF and HGF; EGF, HBEGF and HRGB; EGF, HBEGF and BTC; EGF, HBEGF and TGFA; EGF, HBEGF and FGF1; EGF, HBEGF and FGF2; EGF, HBEGF and FGF4; EGF, HBEGF and FGF7; EGF, HBEGF and FGF9; EGF, HBEGF and FGF19; EGF, HBEGF and SCF; EGF, HBEGF and PDGFA; EGF, HBEGF and PDGFB; EGF, HBEGF and PDGFC; EGF, HBEGF and VEGFA; EGF, HBEGF and VEGFB; EGF, HBEGF and VEGFC; EGF, HBEGF and VEGFD; EGF, HBEGF and TGFB1; EGF, HBEGF and IL-10; EGF, HBEGF and an anti-TNFα antibody; EGF, HGF and HRGB; EGF, HGF and BTC; EGF, HGF and TGFA; EGF, HGF and FGF1; EGF, HGF and FGF2; EGF, HGF and FGF4; EGF, HGF and FGF7; EGF, HGF and FGF9; EGF, HGF and FGF19; EGF, HGF and SCF; EGF, HGF and PDGFA; EGF, HGF and PDGFB; EGF, HGF and PDGFC; EGF, HGF and VEGFA; EGF, HGF and VEGFB; EGF, HGF and VEGFC; EGF, HGF and VEGFD; EGF, HGF and TGFB1; EGF, HGF and IL-10; EGF, HGF and an anti-TNFα antibody; EGF, HRGB and BTC; EGF, HRGB and TGFA; EGF, HRGB and FGF1; EGF, HRGB and FGF2; EGF, HRGB and FGF4; EGF, HRGB and FGF7; EGF, HRGB and FGF9; EGF, HRGB and FGF19; EGF, HRGB and SCF; EGF, HRGB and PDGFA; EGF, HRGB and PDGFB; EGF, HRGB and PDGFC; EGF, HRGB and VEGFA; EGF, HRGB and VEGFB; EGF, HRGB and VEGFC; EGF, HRGB and VEGFD; EGF, HRGB and TGFB1; EGF, HRGB and IL-10; EGF, HRGB and an anti-TNFα antibody; EGF, BTC and TGFA; EGF, BTC and FGF1; EGF, BTC and FGF2; EGF, BTC and FGF4; EGF, BTC and FGF7; EGF, BTC and FGF9; EGF, BTC and FGF19; EGF, BTC and SCF; EGF, BTC and PDGFA; EGF, BTC and PDGFB; EGF, BTC and PDGFC; EGF, BTC and VEGFA; EGF, BTC and VEGFB; EGF, BTC and VEGFC; EGF, BTC and VEGFD; EGF, BTC and TGFB1; EGF, BTC and IL-10; EGF, BTC and an anti-TNFα antibody; EGF, TGFA and FGF1; EGF, TGFA and FGF2; EGF, TGFA and FGF4; EGF, TGFA and FGF7; EGF, TGFA and FGF9; EGF, TGFA and FGF19; EGF, TGFA and SCF; EGF, TGFA and PDGFA; EGF, TGFA and PDGFB; EGF, TGFA and PDGFC; EGF, TGFA and VEGFA; EGF, TGFA and VEGFB; EGF, TGFA and VEGFC; EGF, TGFA and VEGFD; EGF, TGFA and TGFB1; EGF, TGFA and IL-10; EGF, TGFA and an anti-TNFa antibody; EGF, FGF1 and FGF2; EGF, FGF1 andFGF4; EGF, FGF1 and FGF7; EGF, FGF1 and FGF9; EGF, FGF1 and FGF19; EGF, FGF1 and SCF; EGF, FGF1 and PDGFA; EGF, FGF1 and PDGFB; EGF, FGF1 and PDGFC; EGF, FGF1 and VEGFA; EGF, FGF1 and VEGFB; EGF, FGF1 and VEGFC; EGF, FGF1 and VEGFD; EGF, FGF1 and TGFB1; EGF, FGF1 and IL-10; EGF, FGF1 and an anti-TNFα antibody; EGF, FGF2 and FGF4; EGF, FGF2 and FGF7; EGF, FGF2 and FGF9; EGF, FGF2 and FGF19; EGF, FGF2 and SCF; EGF, FGF2 and PDGFA; EGF, FGF2 and PDGFB; EGF, FGF2 and PDGFC; EGF, FGF2 and VEGFA; EGF, FGF2 and VEGFB; EGF, FGF2 and VEGFC; EGF, FGF2 and VEGFD; EGF, FGF2 and TGFB1; EGF, FGF2 and IL-10; EGF, FGF2 and an anti-TNFα antibody; EGF, FGF4 and FGF7; EGF, FGF4 and FGF9; EGF, FGF4 and FGF19; EGF, FGF4 and SCF; EGF, FGF4 and PDGFA; EGF, FGF4 and PDGFB; EGF, FGF4 and PDGFC; EGF, FGF4 and VEGFA; EGF, FGF4 and VEGFB; EGF, FGF4 and VEGFC; EGF, FGF4 and VEGFD; EGF, FGF4 and TGFB1; EGF, FGF4 and IL-10; EGF, FGF4 and an anti-TNFα antibody; EGF, FGF7 and FGF9; EGF, FGF7 and FGF19; EGF, FGF7 and SCF; EGF, FGF7 and PDGFA; EGF, FGF7 and PDGFB; EGF, FGF7 and PDGFC; EGF, FGF7 and VEGFA; EGF, FGF7 and VEGFB; EGF, FGF7 and VEGFC; EGF, FGF7 and VEGFD; EGF, FGF7 and TGFB1; EGF, FGF7 and IL-10; EGF, FGF7 and an anti-TNFa antibody; EGF, FGF9 and FGF19; EGF, FGF9 and SCF; EGF, FGF9 and PDGFA; EGF, FGF9 and PDGFB; EGF, FGF9 and PDGFC; EGF, FGF9 and VEGFA; EGF, FGF9 and VEGFB; EGF, FGF9 and VEGFC; EGF, FGF9 and VEGFD; EGF, FGF9 and TGFB1; EGF, FGF9 and IL-10; EGF, FGF9 and an anti-TNFα antibody; EGF, FGF19 and SCF; EGF, FGF19 and PDGFA; EGF, FGF19 and PDGFB; EGF, FGF19 and PDGFC; EGF, FGF19 and VEGFA; EGF, FGF19 and VEGFB; EGF, FGF19 and VEGFC; EGF, FGF19 and VEGFD; EGF, FGF19 and TGFB1; EGF, FGF19 and IL-10; EGF, FGF19 and an anti-TNFα antibody; EGF, SCF and PDGFA; EGF, SCF and PDGFB; EGF, SCF and PDGFC; EGF, SCF and VEGFA; EGF, SCF and VEGFB; EGF, SCF and VEGFC; EGF, SCF and VEGFD; EGF, SCF and TGFB1; EGF, SCF and IL-10; EGF, SCF and an anti-TNFα antibody; EGF, PDGFA and PDGFB; EGF, PDGFA and PDGFC; EGF, PDGFA and VEGFA; EGF, PDGFA and VEGFB; EGF, PDGFA and VEGFC; EGF, PDGFA and VEGFD; EGF, PDGFA and TGFB1; EGF, PDGFA and IL-10; EGF, PDGFA and an anti-TNFa antibody; EGF, PDGFB and PDGFC; EGF, PDGFB and VEGFA; EGF, PDGFB and VEGFB; EGF, PDGFB and VEGFC; EGF, PDGFB and VEGFD; EGF, PDGFB and TGFB1; EGF, PDGFB and IL-10; EGF, PDGFB and an anti-TNFα antibody; EGF, PDGFC and VEGFA; EGF, PDGFC and VEGFB; EGF, PDGFC and VEGFC; EGF, PDGFC and VEGFD; EGF, PDGFC and TGFB1; EGF, PDGFC and IL-10; EGF, PDGFC and an anti-TNFα antibody; EGF, VEGFA and VEGFB; EGF, VEGFA and VEGFC; EGF, VEGFA and VEGFD; EGF, VEGFA and TGFB1; EGF, VEGFA and IL-10; EGF, VEGFA and an anti-TNFa antibody; EGF, VEGFB and VEGFC; EGF, VEGFB and VEGFD; EGF, VEGFB and TGFB1; EGF, VEGFB and IL-10; EGF, VEGFB and an anti-TNFα antibody; EGF, VEGFC and VEGFD; EGF, VEGFC and TGFB1; EGF, VEGFC and IL-10; EGF, VEGFC and an anti-TNFα antibody; EGF, VEGFD and TGFB1; EGF, VEGFD and IL-10; EGF, VEGFD and an anti-TNFα antibody; EGF, TGFB1 and IL-10; EGF, TGFB1 and an anti-TNFα antibody; EGF, IL-10 and an anti-TNFa antibody; TGFA, AREG and EREG; TGFA, AREG and HBEGF; TGFA, AREG and HGF; TGFA, AREG and HRGB; TGFA, AREG and BTC; TGFA, AREG and EGF; TGFA, AREG and FGF1; TGFA, AREG and FGF2; TGFA, AREG and FGF4; TGFA, AREG and FGF7; TGFA, AREG and FGF9; TGFA, AREG and FGF19; TGFA, AREG and SCF; TGFA, AREG and PDGFA; TGFA, AREG and PDGFB; TGFA, AREG and PDGFC; TGFA, AREG and VEGFA; TGFA, AREG and VEGFB; TGFA, AREG and VEGFC; TGFA, AREG and VEGFD; TGFA, AREG and TGFB1; TGFA, AREG and IL-10; TGFA, AREG and an anti-TNFα antibody; TGFA, EREG and HBEGF; TGFA, EREG and HGF; TGFA, EREG and HRGB; TGFA, EREG and BTC; TGFA, EREG and EGF; TGFA, EREG and FGF1; TGFA, EREG and FGF2; TGFA, EREG and FGF4; TGFA, EREG and FGF7; TGFA, EREG and FGF9; TGFA, EREG and FGF19; TGFA, EREG and SCF; TGFA, EREG and PDGFA; TGFA, EREG and PDGFB; TGFA, EREG and PDGFC; TGFA, EREG and VEGFA; TGFA, EREG and VEGFB; TGFA, EREG and VEGFC; TGFA, EREG and VEGFD; TGFA, EREG and TGFB1; TGFA, EREG and IL-10; TGFA, EREG and an anti-TNFα antibody; TGFA, HBEGF and HGF; TGFA, HBEGF and HRGB; TGFA, HBEGF and BTC; TGFA, HBEGF and EGF; TGFA, HBEGF and FGF1; TGFA, HBEGF and FGF2; TGFA, HBEGF and FGF4; TGFA, HBEGF and FGF7; TGFA, HBEGF and FGF9; TGFA, HBEGF and FGF19; TGFA, HBEGF and SCF; TGFA, HBEGF and PDGFA; TGFA, HBEGF and PDGFB; TGFA, HBEGF and PDGFC; TGFA, HBEGF and VEGFA; TGFA, HBEGF and VEGFB; TGFA, HBEGF and VEGFC; TGFA, HBEGF and VEGFD; TGFA, HBEGF and TGFB1; TGFA, HBEGF and IL-10; TGFA, HBEGF and an anti-TNFα antibody; TGFA, HGF and HRGB; TGFA, HGF and BTC; TGFA, HGF and EGF; TGFA, HGF and FGF1; TGFA, HGF and FGF2; TGFA, HGF and FGF4; TGFA, HGF and FGF7; TGFA, HGF and FGF9; TGFA, HGF and FGF19; TGFA, HGF and SCF; TGFA, HGF and PDGFA; TGFA, HGF and PDGFB; TGFA, HGF and PDGFC; TGFA, HGF and VEGFA; TGFA, HGF and VEGFB; TGFA, HGF and VEGFC; TGFA, HGF and VEGFD; TGFA, HGF and TGFB1; TGFA, HGF and IL-10; TGFA, HGF and an anti-TNFa antibody; TGFA, HRGB and BTC; TGFA, HRGB and EGF; TGFA, HRGB and FGF1; TGFA, HRGB and FGF2; TGFA, HRGB and FGF4; TGFA, HRGB and FGF7; TGFA, HRGB and FGF9; TGFA, HRGB and FGF19; TGFA, HRGB and SCF; TGFA, HRGB and PDGFA; TGFA, HRGB and PDGFB; TGFA, HRGB and PDGFC; TGFA, HRGB and VEGFA; TGFA, HRGB and VEGFB; TGFA, HRGB and VEGFC; TGFA, HRGB and VEGFD; TGFA, HRGB and TGFB1; TGFA, HRGB and IL-10; TGFA, HRGB and an anti-TNFα antibody; TGFA, BTC and EGF; TGFA, BTC and FGF1; TGFA, BTC and FGF2; TGFA, BTC and FGF4; TGFA, BTC and FGF7; TGFA, BTC and FGF9; TGFA, BTC and FGF19; TGFA, BTC and SCF; TGFA, BTC and PDGFA; TGFA, BTC and PDGFB; TGFA, BTC and PDGFC; TGFA, BTC and VEGFA; TGFA, BTC and VEGFB; TGFA, BTC and VEGFC; TGFA, BTC and VEGFD; TGFA, BTC and TGFB1; TGFA, BTC and IL-10; TGFA, BTC and an anti-TNFa antibody; TGFA, EGF and FGF1; TGFA, EGF and FGF2; TGFA, EGF and FGF4; TGFA, EGF and FGF7; TGFA, EGF and FGF9; TGFA, EGF and FGF19; TGFA, EGF and SCF; TGFA, EGF and PDGFA; TGFA, EGF and PDGFB; TGFA, EGF and PDGFC; TGFA, EGF and VEGFA; TGFA, EGF and VEGFB; TGFA, EGF and VEGFC; TGFA, EGF and VEGFD; TGFA, EGF and TGFB1; TGFA, EGF and IL-10; TGFA, EGF and an anti-TNFa antibody; TGFA, FGF1 and FGF2; TGFA, FGF1 and FGF4; TGFA, FGF1 and FGF7; TGFA, FGF1 and FGF9; TGFA, FGF1 and FGF19; TGFA, FGF1 and SCF; TGFA, FGF1 and PDGFA; TGFA, FGF1 and PDGFB; TGFA, FGF1 and PDGFC; TGFA, FGF1 and VEGFA; TGFA, FGF1 and VEGFB; TGFA, FGF1 and VEGFC; TGFA, FGF1 and VEGFD; TGFA, FGF1 and TGFB1; TGFA, FGF1 and IL-10; TGFA, FGF1 and an anti-TNFa antibody; TGFA, FGF2 and FGF4; TGFA, FGF2 and FGF7; TGFA, FGF2 and FGF9; TGFA, FGF2 and FGF19; TGFA, FGF2 and SCF; TGFA, FGF2 and PDGFA; TGFA, FGF2 and PDGFB; TGFA, FGF2 and PDGFC; TGFA, FGF2 and VEGFA; TGFA, FGF2 and VEGFB; TGFA, FGF2 and VEGFC; TGFA, FGF2 and VEGFD; TGFA, FGF2 and TGFB1; TGFA, FGF2 and IL-10; TGFA, FGF2 and an anti-TNFa antibody; TGFA, FGF4 and FGF7; TGFA, FGF4 and FGF9; TGFA, FGF4 and FGF19; TGFA, FGF4 and SCF; TGFA, FGF4 and PDGFA; TGFA, FGF4 and PDGFB; TGFA, FGF4 and PDGFC; TGFA, FGF4 and VEGFA; TGFA, FGF4 and VEGFB; TGFA, FGF4 and VEGFC; TGFA, FGF4 and VEGFD; TGFA, FGF4 and TGFB1; TGFA, FGF4 and IL-10; TGFA, FGF4 and an anti-TNFα antibody; TGFA, FGF7 and FGF9; TGFA, FGF7 and FGF19; TGFA, FGF7 and SCF; TGFA, FGF7 and PDGFA; TGFA, FGF7 and PDGFB; TGFA, FGF7 and PDGFC; TGFA, FGF7 and VEGFA; TGFA, FGF7 and VEGFB; TGFA, FGF7 and VEGFC; TGFA, FGF7 and VEGFD; TGFA, FGF7 and TGFB1; TGFA, FGF7 and IL-10; TGFA, FGF7 and an anti-TNFa antibody; TGFA, FGF9 and FGF19; TGFA, FGF9 and SCF; TGFA, FGF9 and PDGFA; TGFA, FGF9 and PDGFB; TGFA, FGF9 and PDGFC; TGFA, FGF9 and VEGFA; TGFA, FGF9 and VEGFB; TGFA, FGF9 and VEGFC; TGFA, FGF9 and VEGFD; TGFA, FGF9 and TGFB1; TGFA, FGF9 and IL-10; TGFA, FGF9 and an anti-TNFa antibody; TGFA, FGF19 and SCF; TGFA, FGF19 and PDGFA; TGFA, FGF19 and PDGFB; TGFA, FGF19 and PDGFC; TGFA, FGF19 and VEGFA; TGFA, FGF19 and VEGFB; TGFA, FGF19 and VEGFC; TGFA, FGF19 and VEGFD; TGFA, FGF19 and TGFB1; TGFA, FGF19 and IL-10; TGFA, FGF19 and an anti-TNFa antibody; TGFA, SCF and PDGFA; TGFA, SCF and PDGFB; TGFA, SCF and PDGFC; TGFA, SCF and VEGFA; TGFA, SCF and VEGFB; TGFA, SCF and VEGFC; TGFA, SCF and VEGFD; TGFA, SCF and TGFB1; TGFA, SCF and IL-10; TGFA, SCF and an anti-TNFα antibody; TGFA, PDGFA and PDGFB; TGFA, PDGFA and PDGFC; TGFA, PDGFA and VEGFA; TGFA, PDGFA and VEGFB; TGFA, PDGFA and VEGFC; TGFA, PDGFA and VEGFD; TGFA, PDGFA and TGFB1; TGFA, PDGFA and IL-10; TGFA, PDGFA and an anti-TNFα antibody; TGFA, PDGFB and PDGFC; TGFA, PDGFB and VEGFA; TGFA, PDGFB and VEGFB; TGFA, PDGFB and VEGFC; TGFA, PDGFB and VEGFD; TGFA, PDGFB and TGFB1; TGFA, PDGFB and IL-10; TGFA, PDGFB and an anti-TNFα antibody; TGFA, PDGFC and VEGFA; TGFA, PDGFC and VEGFB; TGFA, PDGFC and VEGFC; TGFA, PDGFC and VEGFD; TGFA, PDGFC and TGFB1; TGFA, PDGFC and IL-10; TGFA, PDGFC and an anti-TNFα antibody; TGFA, VEGFA and VEGFB; TGFA, VEGFA and VEGFC; TGFA, VEGFA and VEGFD; TGFA, VEGFA and TGFB1; TGFA, VEGFA and IL-10; TGFA, VEGFA and an anti-TNFα antibody; TGFA, VEGFB and VEGFC; TGFA, VEGFB and VEGFD; TGFA, VEGFB and TGFB1; TGFA, VEGFB and IL-10; TGFA, VEGFB and an anti-TNFα antibody; TGFA, VEGFC and VEGFD; TGFA, VEGFC and TGFB1; TGFA, VEGFC and IL-10; TGFA, VEGFC and an anti-TNFα antibody; TGFA, VEGFD and TGFB1; TGFA, VEGFD and IL-10; TGFA, VEGFD and an anti-TNFα antibody; TGFA, TGFB1 and IL-10; TGFA, TGFB1 and an anti-TNFα antibody; TGFA, IL-10 and an anti-TNFα antibody; FGF1, AREG and EREG; FGF1, AREG and HBEGF; FGF1, AREG and HGF; FGF1, AREG and HRGB; FGF1, AREG and BTC; FGF1, AREG and EGF; FGF1, AREG and TGFA; FGF1, AREG and FGF1; FGF1, AREG and FGF2; FGF1, AREG and FGF4; FGF1, AREG and FGF7; FGF1, AREG and FGF9; FGF1, AREG and FGF19; FGF1, AREG and SCF; FGF1, AREG and PDGFA; FGF1, AREG and PDGFB; FGF1, AREG and PDGFC; FGF1, AREG and VEGFA; FGF1, AREG and VEGFB; FGF1, AREG and VEGFC; FGF1, AREG and VEGFD; FGF1, AREG and TGFB1; FGF1, AREG and IL-10; FGF1, AREG and an anti-TNFα antibody; FGF1, EREG and HBEGF; FGF1, EREG and HGF; FGF1, EREG and HRGB; FGF1, EREG and BTC; FGF1, EREG and EGF; FGF1, EREG and TGFA; FGF1, EREG and FGF1; FGF1, EREG and FGF2; FGF1, EREG and FGF4; FGF1, EREG and FGF7; FGF1, EREG and FGF9; FGF1, EREG and FGF19; FGF1, EREG and SCF; FGF1, EREG and PDGFA; FGF1, EREG and PDGFB; FGF1, EREG and PDGFC; FGF1, EREG and VEGFA; FGF1, EREG and VEGFB; FGF1, EREG and VEGFC; FGF1, EREG and VEGFD; FGF1, EREG and TGFB1; FGF1, EREG and IL-10; FGF1, EREG and an anti-TNFα antibody; FGF1, HBEGF and HGF; FGF1, HBEGF and HRGB; FGF1, HBEGF and BTC; FGF1, HBEGF and EGF; FGF1, HBEGF and TGFA; FGF1, HBEGF and FGF1; FGF1, HBEGF and FGF2; FGF1, HBEGF and FGF4; FGF1, HBEGF and FGF7; FGF1, HBEGF and FGF9; FGF1, HBEGF and FGF19; FGF1, HBEGF and SCF; FGF1, HBEGF and PDGFA; FGF1, HBEGF and PDGFB; FGF1, HBEGF and PDGFC; FGF1, HBEGF and VEGFA; FGF1, HBEGF and VEGFB; FGF1, HBEGF and VEGFC; FGF1, HBEGF and VEGFD; FGF1, HBEGF and TGFB1; FGF1, HBEGF and IL-10; FGF1, HBEGF and an anti-TNFα antibody; FGF1, HGF and HRGB; FGF1, HGF and BTC; FGF1, HGF and EGF; FGF1, HGF and TGFA; FGF1, HGF and FGF2; FGF1, HGF and FGF4; FGF1, HGF and FGF7; FGF1, HGF and FGF9; FGF1, HGF and FGF19; FGF1, HGF and SCF; FGF1, HGF and PDGFA; FGF 1, HGF and PDGFB; FGF 1, HGF and PDGFC; FGF 1, HGF and VEGFA; FGF 1, HGF and VEGFB; FGF1, HGF and VEGFC; FGF1, HGF and VEGFD; FGF1, HGF and TGFB1; FGF1, HGF and IL-10; FGF1, HGF and an anti-TNFα antibody; FGF1, HRGB and BTC; FGF1, HRGB and EGF; FGF1, HRGB and TGFA; FGF1, HRGB and FGF2; FGF1, HRGB and FGF4; FGF1, HRGB and FGF7; FGF1, HRGB and FGF9; FGF1, HRGB and FGF19; FGF1, HRGB and SCF; FGF1, HRGB and PDGFA; FGF1, HRGB and PDGFB; FGF1, HRGB and PDGFC; FGF1, HRGB and VEGFA; FGF1, HRGB and VEGFB; FGF1, HRGB and VEGFC; FGF1, HRGB and VEGFD; FGF1, HRGB and TGFB1; FGF1, HRGB and IL-10; FGF1, HRGB and an anti-TNFα antibody; FGF1, BTC and EGF; FGF1, BTC and TGFA; FGF1, BTC and FGF2; FGF1, BTC and FGF4; FGF1, BTC and FGF7; FGF1, BTC and FGF9; FGF1, BTC and FGF19; FGF1, BTC and SCF; FGF1, BTC and PDGFA; FGF1, BTC and PDGFB; FGF1, BTC and PDGFC; FGF1, BTC and VEGFA; FGF1, BTC and VEGFB; FGF1, BTC and VEGFC; FGF1, BTC and VEGFD; FGF1, BTC and TGFB1; FGF1, BTC and IL-10; FGF1, BTC and an anti-TNFa antibody; FGF1, EGF and TGFA; FGF1, EGF and FGF2; FGF1, EGF and FGF4; FGF1, EGF and FGF7; FGF1, EGF and FGF9; FGF1, EGF and FGF19; FGF1, EGF and SCF; FGF1, EGF and PDGFA; FGF1, EGF and PDGFB; FGF1, EGF and PDGFC; FGF1, EGF and VEGFA; FGF1, EGF and VEGFB; FGF1, EGF and VEGFC; FGF1, EGF and VEGFD; FGF1, EGF and TGFB1; FGF1, EGF and IL-10; FGF1, EGF and an anti-TNFα antibody; FGF1, TGFA and FGF2; FGF1, TGFA and FGF4; FGF1, TGFA and FGF7; FGF1, TGFA and FGF9; FGF1, TGFA and FGF19; FGF1, TGFA and SCF; FGF1, TGFA and PDGFA; FGF1, TGFA and PDGFB; FGF1, TGFA and PDGFC; FGF1, TGFA and VEGFA; FGF1, TGFA and VEGFB; FGF1, TGFA and VEGFC; FGF1, TGFA and VEGFD; FGF1, TGFA and TGFB1; FGF1, TGFA and IL-10; FGF1, TGFA and an anti-TNFα antibody; FGF1, FGF2 and FGF4; FGF1, FGF2 and FGF7; FGF1, FGF2 and FGF9; FGF1, FGF2 and FGF19; FGF1, FGF2 and SCF; FGF1, FGF2 and PDGFA; FGF1, FGF2 and PDGFB; FGF1, FGF2 and PDGFC; FGF1, FGF2 and VEGFA; FGF1, FGF2 and VEGFB; FGF1, FGF2 and VEGFC; FGF1, FGF2 and VEGFD; FGF1, FGF2 and TGFB1; FGF1, FGF2 and IL-10; FGF1, FGF2 and an anti-TNFα antibody; FGF1, FGF4 and FGF7; FGF1, FGF4 and FGF9; FGF1, FGF4 and FGF19; FGF1, FGF4 and SCF; FGF1, FGF4 and PDGFA; FGF1, FGF4 and PDGFB; FGF1, FGF4 and PDGFC; FGF1, FGF4 and VEGFA; FGF1, FGF4 and VEGFB; FGF1, FGF4 and VEGFC; FGF1, FGF4 and VEGFD; FGF1, FGF4 and TGFB1; FGF1, FGF4 and IL-10; FGF1, FGF4 and an anti-TNFα antibody; FGF1, FGF7 and FGF9; FGF1, FGF7 and FGF19; FGF1, FGF7 and SCF; FGF1, FGF7 and PDGFA; FGF1, FGF7 and PDGFB; FGF1, FGF7 and PDGFC; FGF1, FGF7 and VEGFA; FGF1, FGF7 and VEGFB; FGF1, FGF7 and VEGFC; FGF1, FGF7 and VEGFD; FGF1, FGF7 and TGFB1; FGF1, FGF7 and IL-10; FGF1, FGF7 and an anti-TNFα antibody; FGF1, FGF9 and FGF19; FGF1, FGF9 and SCF; FGF1, FGF9 and PDGFA; FGF1, FGF9 and PDGFB; FGF1, FGF9 and PDGFC; FGF1, FGF9 and VEGFA; FGF1, FGF9 and VEGFB; FGF1, FGF9 and VEGFC; FGF1, FGF9 and VEGFD; FGF1, FGF9 and TGFB1; FGF1, FGF9 and IL-10; FGF1, FGF9 and an anti-TNFα antibody; FGF1, FGF19 and SCF; FGF1, FGF19 and PDGFA; FGF1, FGF19 and PDGFB; FGF1, FGF19 and PDGFC; FGF1, FGF19 and VEGFA; FGF1, FGF19 and VEGFB; FGF1, FGF19 and VEGFC; FGF1, FGF19 and VEGFD; FGF1, FGF19 and TGFB1; FGF1, FGF19 and IL-10; FGF1, FGF19 and an anti-TNFa antibody; FGF1, SCF and PDGFA; FGF1, SCF and PDGFB; FGF1, SCF and PDGFC; FGF1, SCF and VEGFA; FGF1, SCF and VEGFB; FGF1, SCF and VEGFC; FGF1, SCF and VEGFD; FGF1, SCF and TGFB1; FGF1, SCF and IL-10; FGF1, SCF and an anti-TNFα antibody; FGF1, PDGFA and PDGFB; FGF1, PDGFA and PDGFC; FGF1, PDGFA and VEGFA; FGF1, PDGFA and VEGFB; FGF1, PDGFA and VEGFC; FGF1, PDGFA and VEGFD; FGF1, PDGFA and TGFB1; FGF1, PDGFA and IL-10; FGF1, PDGFA and an anti-TNFα antibody; FGF1, PDGFB and PDGFC; FGF1, PDGFB and VEGFA; FGF1, PDGFB and VEGFB; FGF1, PDGFB and VEGFC; FGF1, PDGFB and VEGFD; FGF1, PDGFB and TGFB1; FGF1, PDGFB and IL-10; FGF1, PDGFB and an anti-TNFα antibody; FGF1, PDGFC and VEGFA; FGF1, PDGFC and VEGFB; FGF1, PDGFC and VEGFC; FGF1, PDGFC and VEGFD; FGF1, PDGFC and TGFB1; FGF1, PDGFC and IL-10; FGF1, PDGFC and an anti-TNFα antibody; FGF1, VEGFA and VEGFB; FGF1, VEGFA and VEGFC; FGF1, VEGFA and VEGFD; FGF1, VEGFA and TGFB1; FGF1, VEGFA and IL-10; FGF1, VEGFA and an anti-TNFα antibody; FGF1, VEGFB and VEGFC; FGF1, VEGFB and VEGFD; FGF1, VEGFB and TGFB1; FGF1, VEGFB and IL-10; FGF1, VEGFB and an anti-TNFα antibody; FGF1, VEGFC and VEGFD; FGF1, VEGFC and TGFB1; FGF1, VEGFC and IL-10; FGF1, VEGFC and an anti-TNFα antibody; FGF1, VEGFD and TGFB1; FGF1, VEGFD and IL-10; FGF1, VEGFD and an anti-TNFα antibody; FGF1, TGFB1 and IL-10; FGF1, TGFB1 and an anti-TNFα antibody; FGF1, IL-10 and an anti-TNFα antibody; FGF2, AREG and EREG; FGF2, AREG and HBEGF; FGF2, AREG and HGF; FGF2, AREG and HRGB; FGF2, AREG and BTC; FGF2, AREG and EGF; FGF2, AREG and TGFA; FGF2, AREG and FGF1; FGF2, AREG and FGF4; FGF2, AREG and FGF7; FGF2, AREG and FGF9; FGF2, AREG and FGF19; FGF2, AREG and SCF; FGF2, AREG and PDGFA; FGF2, AREG and PDGFB; FGF2, AREG and PDGFC; FGF2, AREG and VEGFA; FGF2, AREG and VEGFB; FGF2, AREG and VEGFC; FGF2, AREG and VEGFD; FGF2, AREG and TGFB1; FGF2, AREG and IL-10; FGF2, AREG and an anti-TNFα antibody; FGF2, EREG and HBEGF; FGF2, EREG and HGF; FGF2, EREG and HRGB; FGF2, EREG and BTC; FGF2, EREG and EGF; FGF2, EREG and TGFA; FGF2, EREG and FGF1; FGF2, EREG and FGF4; FGF2, EREG and FGF7; FGF2, EREG and FGF9; FGF2, EREG and FGF19; FGF2, EREG and SCF; FGF2, EREG and PDGFA; FGF2, EREG and PDGFB; FGF2, EREG and PDGFC; FGF2, EREG and VEGFA; FGF2, EREG and VEGFB; FGF2, EREG and VEGFC; FGF2, EREG and VEGFD; FGF2, EREG and TGFB1; FGF2, EREG and IL-10; FGF2, EREG and an anti-TNFa antibody; FGF2, HBEGF and HGF; FGF2, HBEGF and HRGB; FGF2, HBEGF and BTC; FGF2, HBEGF and EGF; FGF2, HBEGF and TGFA; FGF2, HBEGF and FGF1; FGF2, HBEGF and FGF4; FGF2, HBEGF and FGF7; FGF2, HBEGF and FGF9; FGF2, HBEGF and FGF19; FGF2, HBEGF and SCF; FGF2, HBEGF and PDGFA; FGF2, HBEGF and PDGFB; FGF2, HBEGF and PDGFC; FGF2, HBEGF and VEGFA; FGF2, HBEGF and VEGFB; FGF2, HBEGF and VEGFC; FGF2, HBEGF and VEGFD; FGF2, HBEGF and TGFB1; FGF2, HBEGF and IL-10; FGF2, HBEGF and an anti-TNFα antibody; FGF2, HGF and HRGB; FGF2, HGF and BTC; FGF2, HGF and EGF; FGF2, HGF and TGFA; FGF2, HGF and FGF1; FGF2, HGF and FGF4; FGF2, HGF and FGF7; FGF2, HGF and FGF9; FGF2, HGF and FGF19; FGF2, HGF and SCF; FGF2, HGF and PDGFA; FGF2, HGF and PDGFB; FGF2, HGF and PDGFC; FGF2, HGF and VEGFA; FGF2, HGF and VEGFB; FGF2, HGF and VEGFC; FGF2, HGF and VEGFD; FGF2, HGF and TGFB1; FGF2, HGF and IL-10; FGF2, HGF and an anti-TNFα antibody; FGF2, HRGB and BTC; FGF2, HRGB and EGF; FGF2, HRGB and TGFA; FGF2, HRGB and FGF1; FGF2, HRGB and FGF4; FGF2, HRGB and FGF7; FGF2, HRGB and FGF9; FGF2, HRGB and FGF19; FGF2, HRGB and SCF; FGF2, HRGB and PDGFA; FGF2, HRGB and PDGFB; FGF2, HRGB and PDGFC; FGF2, HRGB and VEGFA; FGF2, HRGB and VEGFB; FGF2, HRGB and VEGFC; FGF2, HRGB and VEGFD; FGF2, HRGB and TGFB1; FGF2, HRGB and IL-10; FGF2, HRGB and an anti-TNFα antibody; FGF2, BTC and EGF; FGF2, BTC and TGFA; FGF2, BTC and FGF1; FGF2, BTC and FGF4; FGF2, BTC and FGF7; FGF2, BTC and FGF9; FGF2, BTC and FGF19; FGF2, BTC and SCF; FGF2, BTC and PDGFA; FGF2, BTC and PDGFB; FGF2, BTC and PDGFC; FGF2, BTC and VEGFA; FGF2, BTC and VEGFB; FGF2, BTC and VEGFC; FGF2, BTC and VEGFD; FGF2, BTC and TGFB1; FGF2, BTC and IL-10; FGF2, BTC and an anti-TNFα antibody; FGF2, EGF and TGFA; FGF2, EGF and FGF1; FGF2, EGF and FGF4; FGF2, EGF and FGF7; FGF2, EGF and FGF9; FGF2, EGF and FGF19; FGF2, EGF and SCF; FGF2, EGF and PDGFA; FGF2, EGF and PDGFB; FGF2, EGF and PDGFC; FGF2, EGF and VEGFA; FGF2, EGF and VEGFB; FGF2, EGF and VEGFC; FGF2, EGF and VEGFD; FGF2, EGF and TGFB1; FGF2, EGF and IL-10; FGF2, EGF and an anti-TNFa antibody; FGF2, TGFA and FGF1; FGF2, TGFA and FGF4; FGF2, TGFA and FGF7; FGF2, TGFA and FGF9; FGF2, TGFA and FGF19; FGF2, TGFA and SCF; FGF2, TGFA and PDGFA; FGF2, TGFA and PDGFB; FGF2, TGFA and PDGFC; FGF2, TGFA and VEGFA; FGF2, TGFA and VEGFB; FGF2, TGFA and VEGFC; FGF2, TGFA and VEGFD; FGF2, TGFA and TGFB1; FGF2, TGF A and IL-10; FGF2, TGFA and an anti-TNFα antibody; FGF2, FGF1 and FGF4; FGF2, FGF1 and FGF7; FGF2, FGF1 and FGF9; FGF2, FGF1 and FGF19; FGF2, FGF1 and SCF; FGF2, FGF1 and PDGFA; FGF2, FGF1 and PDGFB; FGF2, FGF1 and PDGFC; FGF2, FGF1 and VEGFA; FGF2, FGF1 and VEGFB; FGF2, FGF1 and VEGFC; FGF2, FGF1 and VEGFD; FGF2, FGF1 and TGFB1; FGF2, FGF1 and IL-10; FGF2, FGF1 and an anti-TNFα antibody; FGF2, FGF4 and FGF7; FGF2, FGF4 and FGF9; FGF2, FGF4 and FGF19; FGF2, FGF4 and SCF; FGF2, FGF4 and PDGFA; FGF2, FGF4 and PDGFB; FGF2, FGF4 and PDGFC; FGF2, FGF4 and VEGFA; FGF2, FGF4 and VEGFB; FGF2, FGF4 and VEGFC; FGF2, FGF4 and VEGFD; FGF2, FGF4 and TGFB1; FGF2, FGF4 and IL-10; FGF2, FGF4 and an anti-TNFα antibody; FGF2, FGF7 and FGF9; FGF2, FGF7 and FGF19; FGF2, FGF7 and SCF; FGF2, FGF7 and PDGFA; FGF2, FGF7 and PDGFB; FGF2, FGF7 and PDGFC; FGF2, FGF7 and VEGFA; FGF2, FGF7 and VEGFB; FGF2, FGF7 and VEGFC; FGF2, FGF7 and VEGFD; FGF2, FGF7 and TGFB1; FGF2, FGF7 and IL-10; FGF2, FGF7 and an anti-TNFα antibody; FGF2, FGF9 and FGF19; FGF2, FGF9 and SCF; FGF2, FGF9 and PDGFA; FGF2, FGF9 and PDGFB; FGF2, FGF9 and PDGFC; FGF2, FGF9 and VEGFA; FGF2, FGF9 and VEGFB; FGF2, FGF9 and VEGFC; FGF2, FGF9 and VEGFD; FGF2, FGF9 and TGFB1; FGF2, FGF9 and IL-10; FGF2, FGF9 and an anti-TNFα antibody; FGF2, FGF19 and SCF; FGF2, FGF19 and PDGFA; FGF2, FGF19 and PDGFB; FGF2, FGF19 and PDGFC; FGF2, FGF19 and VEGFA; FGF2, FGF19 and VEGFB; FGF2, FGF19 and VEGFC; FGF2, FGF19 and VEGFD; FGF2, FGF19 and TGFB1; FGF2, FGF19 and IL-10; FGF2, FGF19 and an anti-TNFa antibody; FGF2, SCF and PDGFA; FGF2, SCF and PDGFB; FGF2, SCF and PDGFC; FGF2, SCF and VEGFA; FGF2, SCF and VEGFB; FGF2, SCF and VEGFC; FGF2, SCF and VEGFD; FGF2, SCF and TGFB1; FGF2, SCF and IL-10; FGF2, SCF and an anti-TNFa antibody; FGF2, PDGFA and PDGFB; FGF2, PDGFA and PDGFC; FGF2, PDGFA and VEGFA; FGF2, PDGFA and VEGFB; FGF2, PDGFA and VEGFC; FGF2, PDGFA and VEGFD; FGF2, PDGFA and TGFB1; FGF2, PDGFA and IL-10; FGF2, PDGFA and an anti-TNFα antibody; FGF2, PDGFB and PDGFC; FGF2, PDGFB and VEGFA; FGF2, PDGFB and VEGFB; FGF2, PDGFB and VEGFC; FGF2, PDGFB and VEGFD; FGF2, PDGFB and TGFB1; FGF2, PDGFB and IL-10; FGF2, PDGFB and an anti-TNFα antibody; FGF2, PDGFC and VEGFA; FGF2, PDGFC and VEGFB; FGF2, PDGFC and VEGFC; FGF2, PDGFC and VEGFD; FGF2, PDGFC and TGFB1; FGF2, PDGFC and IL-10; FGF2, PDGFC and an anti-TNFα antibody; FGF2, VEGFA and VEGFB; FGF2, VEGFA and VEGFC; FGF2, VEGFA and VEGFD; FGF2, VEGFA and TGFB1; FGF2, VEGFA and IL-10; FGF2, VEGFA and an anti-TNFa antibody; FGF2, VEGFB and VEGFC; FGF2, VEGFB and VEGFD; FGF2, VEGFB and TGFB1; FGF2, VEGFB and IL-10; FGF2, VEGFB and an anti-TNFα antibody; FGF2, VEGFC and VEGFD; FGF2, VEGFC and TGFB1; FGF2, VEGFC and IL-10; FGF2, VEGFC and an anti-TNFα antibody; FGF2, VEGFD and TGFB1; FGF2, VEGFD and IL-10; FGF2, VEGFD and an anti-TNFα antibody; FGF2, TGFB1 and IL-10; FGF2, TGFB1 and an anti-TNFa antibody; FGF2, IL-10 and an anti-TNFa antibody; FGF4, AREG and EREG; FGF4, AREG and HBEGF; FGF4, AREG and HGF; FGF4, AREG and HRGB; FGF4, AREG and BTC; FGF4, AREG and EGF; FGF4, AREG and TGFA; FGF4, AREG and FGF1; FGF4, AREG and FGF2; FGF4, AREG and FGF4; FGF4, AREG and FGF7; FGF4, AREG and FGF9; FGF4, AREG and FGF19; FGF4, AREG and SCF; FGF4, AREG and PDGFA; FGF4, AREG and PDGFB; FGF4, AREG and PDGFC; FGF4, AREG and VEGFA; FGF4, AREG and VEGFB; FGF4, AREG and VEGFC; FGF4, AREG and VEGFD; FGF4, AREG and TGFB1; FGF4, AREG and IL-10; FGF4, AREG and an anti-TNFα antibody; FGF4, EREG and HBEGF; FGF4, EREG and HGF; FGF4, EREG and HRGB; FGF4, EREG and BTC; FGF4, EREG and EGF; FGF4, EREG and TGFA; FGF4, EREG and FGF1; FGF4, EREG and FGF2; FGF4, EREG and FGF7; FGF4, EREG and FGF9; FGF4, EREG and FGF19; FGF4, EREG and SCF; FGF4, EREG and PDGFA; FGF4, EREG and PDGFB; FGF4, EREG and PDGFC; FGF4, EREG and VEGFA; FGF4, EREG and VEGFB; FGF4, EREG and VEGFC; FGF4, EREG and VEGFD; FGF4, EREG and TGFB1; FGF4, EREG and IL-10; FGF4, EREG and an anti-TNFα antibody; FGF4, HBEGF and HGF; FGF4, HBEGF and HRGB; FGF4, HBEGF and BTC; FGF4, HBEGF and EGF; FGF4, HBEGF and TGFA; FGF4, HBEGF and FGF1; FGF4, HBEGF and FGF2; FGF4, HBEGF and FGF7; FGF4, HBEGF and FGF9; FGF4, HBEGF and FGF19; FGF4, HBEGF and SCF; FGF4, HBEGF and PDGFA; FGF4, HBEGF and PDGFB; FGF4, HBEGF and PDGFC; FGF4, HBEGF and VEGFA; FGF4, HBEGF and VEGFB; FGF4, HBEGF and VEGFC; FGF4, HBEGF and VEGFD; FGF4, HBEGF and TGFB1; FGF4, HBEGF and IL-10; FGF4, HBEGF and an anti-TNFα antibody; FGF4, HGF and HRGB; FGF4, HGF and BTC; FGF4, HGF and EGF; FGF4, HGF and TGFA; FGF4, HGF and FGF1; FGF4, HGF and FGF2; FGF4, HGF and FGF7; FGF4, HGF and FGF9; FGF4, HGF and FGF19; FGF4, HGF and SCF; FGF4, HGF and PDGFA; FGF4, HGF and PDGFB; FGF4, HGF and PDGFC; FGF4, HGF and VEGFA; FGF4, HGF and VEGFB; FGF4, HGF and VEGFC; FGF4, HGF and VEGFD; FGF4, HGF and TGFB1; FGF4, HGF and IL-10; FGF4, HGF and an anti-TNFα antibody; FGF4, HRGB and BTC; FGF4, HRGB and EGF; FGF4, HRGB and TGFA; FGF4, HRGB and FGF1; FGF4, HRGB and FGF2; FGF4, HRGB and FGF7; FGF4, HRGB and FGF9; FGF4, HRGB and FGF19; FGF4, HRGB and SCF; FGF4, HRGB and PDGFA; FGF4, HRGB and PDGFB; FGF4, HRGB and PDGFC; FGF4, HRGB and VEGFA; FGF4, HRGB and VEGFB; FGF4, HRGB and VEGFC; FGF4, HRGB and VEGFD; FGF4, HRGB and TGFB1; FGF4, HRGB and IL-10; FGF4, HRGB and an anti-TNFα antibody; FGF4, BTC and EGF; FGF4, BTC and TGFA; FGF4, BTC and FGF1; FGF4, BTC and FGF2; FGF4, BTC and FGF7; FGF4, BTC and FGF9; FGF4, BTC and FGF19; FGF4, BTC and SCF; FGF4, BTC and PDGFA; FGF4, BTC and PDGFB; FGF4, BTC and PDGFC; FGF4, BTC and VEGFA; FGF4, BTC and VEGFB; FGF4, BTC and VEGFC; FGF4, BTC and VEGFD; FGF4, BTC and TGFB1; FGF4, BTC and IL-10; FGF4, BTC and an anti-TNFa antibody; FGF4, EGF and TGFA; FGF4, EGF and FGF1; FGF4, EGF and FGF2; FGF4, EGF and FGF7; FGF4, EGF and FGF9; FGF4, EGF and FGF19; FGF4, EGF and SCF; FGF4, EGF and PDGFA; FGF4, EGF and PDGFB; FGF4, EGF and PDGFC; FGF4, EGF and VEGFA; FGF4, EGF and VEGFB; FGF4, EGF and VEGFC; FGF4, EGF and VEGFD; FGF4, EGF and TGFB1; FGF4, EGF and IL-10; FGF4, EGF and an anti-TNFα antibody; FGF4, TGFA and FGF1; FGF4, TGFA and FGF2; FGF4, TGFA and FGF7; FGF4, TGFA and FGF9; FGF4, TGFA andFGF19; FGF4, TGFA and SCF; FGF4, TGFA and PDGFA; FGF4, TGFA and PDGFB; FGF4, TGFA and PDGFC; FGF4, TGFA and VEGFA; FGF4, TGFA and VEGFB; FGF4, TGFA and VEGFC; FGF4, TGFA and VEGFD; FGF4, TGFA and TGFB1; FGF4, TGFA and IL-10; FGF4, TGFA and an anti-TNFα antibody; FGF4, FGF1 and FGF2; FGF4, FGF1 and FGF7; FGF4, FGF1 and FGF9; FGF4, FGF1 and FGF19; FGF4, FGF1 and SCF; FGF4, FGF1 and PDGFA; FGF4, FGF1 and PDGFB; FGF4, FGF1 and PDGFC; FGF4, FGF1 and VEGFA; FGF4, FGF1 and VEGFB; FGF4, FGF1 and VEGFC; FGF4, FGF1 and VEGFD; FGF4, FGF1 and TGFB1; FGF4, FGF1 and IL-10; FGF4, FGF1 and an anti-TNFα antibody; FGF4, FGF2 and FGF7; FGF4, FGF2 and FGF9; FGF4, FGF2 and FGF19; FGF4, FGF2 and SCF; FGF4, FGF2 and PDGFA; FGF4, FGF2 and PDGFB; FGF4, FGF2 and PDGFC; FGF4, FGF2 and VEGFA; FGF4, FGF2 and VEGFB; FGF4, FGF2 and VEGFC; FGF4, FGF2 and VEGFD; FGF4, FGF2 and TGFB1; FGF4, FGF2 and IL-10; FGF4, FGF2 and an anti-TNFα antibody; FGF4, FGF7 and FGF9; FGF4, FGF7 and FGF19; FGF4, FGF7 and SCF; FGF4, FGF7 and PDGFA; FGF4, FGF7 and PDGFB; FGF4, FGF7 and PDGFC; FGF4, FGF7 and VEGFA; FGF4, FGF7 and VEGFB; FGF4, FGF7 and VEGFC; FGF4, FGF7 and VEGFD; FGF4, FGF7 and TGFB1; FGF4, FGF7 and IL-10; FGF4, FGF7 and an anti-TNFα antibody; FGF4, FGF9 and FGF19; FGF4, FGF9 and SCF; FGF4, FGF9 and PDGFA; FGF4, FGF9 and PDGFB; FGF4, FGF9 and PDGFC; FGF4, FGF9 and VEGFA; FGF4, FGF9 and VEGFB; FGF4, FGF9 and VEGFC; FGF4, FGF9 and VEGFD; FGF4, FGF9 and TGFB1; FGF4, FGF9 and IL-10; FGF4, FGF9 and an anti-TNFα antibody; FGF4, FGF19 and SCF; FGF4, FGF19 and PDGFA; FGF4, FGF19 and PDGFB; FGF4, FGF19 and PDGFC; FGF4, FGF19 and VEGFA; FGF4, FGF19 and VEGFB; FGF4, FGF19 and VEGFC; FGF4, FGF19 and VEGFD; FGF4, FGF19 and TGFB1; FGF4, FGF19 and IL-10; FGF4, FGF19 and an anti-TNFa antibody; FGF4, SCF and PDGFA; FGF4, SCF and PDGFB; FGF4, SCF and PDGFC; FGF4, SCF and VEGFA; FGF4, SCF and VEGFB; FGF4, SCF and VEGFC; FGF4, SCF and VEGFD; FGF4, SCF and TGFB1; FGF4, SCF and IL-10; FGF4, SCF and an anti-TNFα antibody; FGF4, PDGFA and PDGFB; FGF4, PDGFA and PDGFC; FGF4, PDGFA and VEGFA; FGF4, PDGFA and VEGFB; FGF4, PDGFA and VEGFC; FGF4, PDGFA and VEGFD; FGF4, PDGFA and TGFB1; FGF4, PDGFA and IL-10; FGF4, PDGFA and an anti-TNFa antibody; FGF4, PDGFB and PDGFC; FGF4, PDGFB and VEGFA; FGF4, PDGFB and VEGFB; FGF4, PDGFB and VEGFC; FGF4, PDGFB and VEGFD; FGF4, PDGFB and TGFB1; FGF4, PDGFB and IL-10; FGF4, PDGFB and an anti-TNFα antibody; FGF4, PDGFC and VEGFA; FGF4, PDGFC and VEGFB; FGF4, PDGFC and VEGFC; FGF4, PDGFC and VEGFD; FGF4, PDGFC and TGFB1; FGF4, PDGFC and IL-10; FGF4, PDGFC and an anti-TNFα antibody; FGF4, VEGFA and VEGFB; FGF4, VEGFA and VEGFC; FGF4, VEGFA and VEGFD; FGF4, VEGFA and TGFB1; FGF4, VEGFA and IL-10; FGF4, VEGFA and an anti-TNFα antibody; FGF4, VEGFB and VEGFC; FGF4, VEGFB and VEGFD; FGF4, VEGFB and TGFB1; FGF4, VEGFB and IL-10; FGF4, VEGFB and an anti-TNFα antibody; FGF4, VEGFC and VEGFD; FGF4, VEGFC and TGFB1; FGF4, VEGFC and IL-10; FGF4, VEGFC and an anti-TNFα antibody; FGF4, VEGFD and TGFB1; FGF4, VEGFD and IL-10; FGF4, VEGFD and an anti-TNFα antibody; FGF4, TGFB1 and IL-10; FGF4, TGFB1 and an anti-TNFα antibody; FGF4, IL-10 and an anti-TNFα antibody; FGF7, AREG and EREG; FGF7, AREG and HBEGF; FGF7, AREG and HGF; FGF7, AREG and HRGB; FGF7, AREG and BTC; FGF7, AREG and EGF; FGF7, AREG and TGFA; FGF7, AREG and FGF1; FGF7, AREG and FGF2; FGF7, AREG and FGF4; FGF7, AREG and FGF9; FGF7, AREG and FGF19; FGF7, AREG and SCF; FGF7, AREG and PDGFA; FGF7, AREG and PDGFB; FGF7, AREG and PDGFC; FGF7, AREG and VEGFA; FGF7, AREG and VEGFB; FGF7, AREG and VEGFC; FGF7, AREG and VEGFD; FGF7, AREG and TGFB1; FGF7, AREG and IL-10; FGF7, AREG and an anti-TNFα antibody; FGF7, EREG and HBEGF; FGF7, EREG and HGF; FGF7, EREG and HRGB; FGF7, EREG and BTC; FGF7, EREG and EGF; FGF7, EREG and TGFA; FGF7, EREG and FGF1; FGF7, EREG and FGF2; FGF7, EREG and FGF4; FGF7, EREG and FGF9; FGF7, EREG and FGF19; FGF7, EREG and SCF; FGF7, EREG and PDGFA; FGF7, EREG and PDGFB; FGF7, EREG and PDGFC; FGF7, EREG and VEGFA; FGF7, EREG and VEGFB; FGF7, EREG and VEGFC; FGF7, EREG and VEGFD; FGF7, EREG and TGFB1; FGF7, EREG and IL-10; FGF7, EREG and an anti-TNFα antibody; FGF7, HBEGF and HGF; FGF7, HBEGF and HRGB; FGF7, HBEGF and BTC; FGF7, HBEGF and EGF; FGF7, HBEGF and TGFA; FGF7, HBEGF and FGF1; FGF7, HBEGF and FGF2; FGF7, HBEGF and FGF4; FGF7, HBEGF and FGF9; FGF7, HBEGF and FGF19; FGF7, HBEGF and SCF; FGF7, HBEGF and PDGFA; FGF7, HBEGF and PDGFB; FGF7, HBEGF and PDGFC; FGF7, HBEGF and VEGFA; FGF7, HBEGF and VEGFB; FGF7, HBEGF and VEGFC; FGF7, HBEGF and VEGFD; FGF7, HBEGF and TGFB1; FGF7, HBEGF and IL-10; FGF7, HBEGF and an anti-TNFα antibody; FGF7, HGF and HRGB; FGF7, HGF and BTC; FGF7, HGF and EGF; FGF7, HGF and TGFA; FGF7, HGF and FGF1; FGF7, HGF and FGF2; FGF7, HGF and FGF4; FGF7, HGF and FGF9; FGF7, HGF and FGF19; FGF7, HGF and SCF; FGF7, HGF and PDGFA; FGF7, HGF and PDGFB; FGF7, HGF and PDGFC; FGF7, HGF and VEGFA; FGF7, HGF and VEGFB; FGF7, HGF and VEGFC; FGF7, HGF and VEGFD; FGF7, HGF and TGFB1; FGF7, HGF and IL-10; FGF7, HGF and an anti-TNFα antibody; FGF7, HRGB and BTC; FGF7, HRGB and EGF; FGF7, HRGB and TGFA; FGF7, HRGB and FGF1; FGF7, HRGB and FGF2; FGF7, HRGB and FGF4; FGF7, HRGB and FGF9; FGF7, HRGB and FGF19; FGF7, HRGB and SCF; FGF7, HRGB and PDGFA; FGF7, HRGB and PDGFB; FGF7, HRGB and PDGFC; FGF7, HRGB and VEGFA; FGF7, HRGB and VEGFB; FGF7, HRGB and VEGFC; FGF7, HRGB and VEGFD; FGF7, HRGB and TGFB1; FGF7, HRGB and IL-10; FGF7, HRGB and an anti-TNFa antibody; FGF7, BTC and EGF; FGF7, BTC and TGFA; FGF7, BTC and FGF1; FGF7, BTC and FGF2; FGF7, BTC and FGF4; FGF7, BTC and FGF9; FGF7, BTC and FGF19; FGF7, BTC and SCF; FGF7, BTC and PDGFA; FGF7, BTC and PDGFB; FGF7, BTC and PDGFC; FGF7, BTC and VEGFA; FGF7, BTC and VEGFB; FGF7, BTC and VEGFC; FGF7, BTC and VEGFD; FGF7, BTC and TGFB1; FGF7, BTC and IL-10; FGF7, BTC and an anti-TNFα antibody; FGF7, EGF and TGFA; FGF7, EGF and FGF1; FGF7, EGF and FGF2; FGF7, EGF and FGF4; FGF7, EGF and FGF9; FGF7, EGF and FGF19; FGF7, EGF and SCF; FGF7, EGF and PDGFA; FGF7, EGF and PDGFB; FGF7, EGF and PDGFC; FGF7, EGF and VEGFA; FGF7, EGF and VEGFB; FGF7, EGF and VEGFC; FGF7, EGF and VEGFD; FGF7, EGF and TGFB1; FGF7, EGF and IL-10; FGF7, EGF and an anti-TNFα antibody; FGF7, TGFA and FGF1; FGF7, TGFA and FGF2; FGF7, TGFA and FGF4; FGF7, TGFA and FGF9; FGF7, TGFA and FGF19; FGF7, TGFA and SCF; FGF7, TGFA and PDGFA; FGF7, TGFA and PDGFB; FGF7, TGFA and PDGFC; FGF7, TGFA and VEGFA; FGF7, TGFA and VEGFB; FGF7, TGFA and VEGFC; FGF7, TGFA and VEGFD; FGF7, TGFA and TGFB1; FGF7, TGFA and IL-10; FGF7, TGFA and an anti-TNFα antibody; FGF7, FGF1 and FGF2; FGF7, FGF1 and FGF4; FGF7, FGF1 and FGF9; FGF7, FGF1 and FGF19; FGF7, FGF1 and SCF; FGF7, FGF1 and PDGFA; FGF7, FGF1 and PDGFB; FGF7, FGF1 and PDGFC; FGF7, FGF1 and VEGFA; FGF7, FGF1 and VEGFB; FGF7, FGF1 and VEGFC; FGF7, FGF1 and VEGFD; FGF7, FGF1 and TGFB1; FGF7, FGF1 and IL-10; FGF7, FGF1 and an anti-TNFa antibody; FGF7, FGF2 and FGF4; FGF7, FGF2 and FGF9; FGF7, FGF2 and FGF19; FGF7, FGF2 and SCF; FGF7, FGF2 and PDGFA; FGF7, FGF2 and PDGFB; FGF7, FGF2 and PDGFC; FGF7, FGF2 and VEGFA; FGF7, FGF2 and VEGFB; FGF7, FGF2 and VEGFC; FGF7, FGF2 and VEGFD; FGF7, FGF2 and TGFB1; FGF7, FGF2 and IL-10; FGF7, FGF2 and an anti-TNFa antibody; FGF7, FGF4 and FGF9; FGF7, FGF4 and FGF19; FGF7, FGF4 and SCF; FGF7, FGF4 and PDGFA; FGF7, FGF4 and PDGFB; FGF7, FGF4 and PDGFC; FGF7, FGF4 and VEGFA; FGF7, FGF4 and VEGFB; FGF7, FGF4 and VEGFC; FGF7, FGF4 and VEGFD; FGF7, FGF4 and TGFB1; FGF7, FGF4 and IL-10; FGF7, FGF4 and an anti-TNFα antibody; FGF7, FGF9 and FGF19; FGF7, FGF9 and SCF; FGF7, FGF9 and PDGFA; FGF7, FGF9 and PDGFB; FGF7, FGF9 and PDGFC; FGF7, FGF9 and VEGFA; FGF7, FGF9 and VEGFB; FGF7, FGF9 and VEGFC; FGF7, FGF9 and VEGFD; FGF7, FGF9 and TGFB1; FGF7, FGF9 and IL-10; FGF7, FGF9 and an anti-TNFa antibody; FGF7, FGF19 and SCF; FGF7, FGF19 and PDGFA; FGF7, FGF19 and PDGFB; FGF7, FGF19 and PDGFC; FGF7, FGF19 and VEGFA; FGF7, FGF19 and VEGFB; FGF7, FGF19 and VEGFC; FGF7, FGF19 and VEGFD; FGF7, FGF19 and TGFB1; FGF7, FGF19 and IL-10; FGF7, FGF19 and an anti-TNFa antibody; FGF7, SCF and PDGFA; FGF7, SCF and PDGFB; FGF7, SCF and PDGFC; FGF7, SCF and VEGFA; FGF7, SCF and VEGFB; FGF7, SCF and VEGFC; FGF7, SCF and VEGFD; FGF7, SCF and TGFB1; FGF7, SCF and IL-10; FGF7, SCF and an anti-TNFa antibody; FGF7, PDGFA and PDGFB; FGF7, PDGFA and PDGFC; FGF7, PDGFA and VEGFA; FGF7, PDGFA and VEGFB; FGF7, PDGFA and VEGFC; FGF7, PDGFA and VEGFD; FGF7, PDGFA and TGFB1; FGF7, PDGFA and IL-10; FGF7, PDGFA and an anti-TNFα antibody; FGF7, PDGFB and PDGFC; FGF7, PDGFB and VEGFA; FGF7, PDGFB and VEGFB; FGF7, PDGFB and VEGFC; FGF7, PDGFB and VEGFD; FGF7, PDGFB and TGFB1; FGF7, PDGFB and IL-10; FGF7, PDGFB and an anti-TNFα antibody; FGF7, PDGFC and VEGFA; FGF7, PDGFC and VEGFB; FGF7, PDGFC and VEGFC; FGF7, PDGFC and VEGFD; FGF7, PDGFC and TGFB1; FGF7, PDGFC and IL-10; FGF7, PDGFC and an anti-TNFα antibody; FGF7, VEGFA and VEGFB; FGF7, VEGFA and VEGFC; FGF7, VEGFA and VEGFD; FGF7, VEGFA and TGFB1; FGF7, VEGFA and IL-10; FGF7, VEGFA and an anti-TNFα antibody; FGF7, VEGFB and VEGFC; FGF7, VEGFB and VEGFD; FGF7, VEGFB and TGFB1; FGF7, VEGFB and IL-10; FGF7, VEGFB and an anti-TNFα antibody; FGF7, VEGFC and VEGFD; FGF7, VEGFC and TGFB1; FGF7, VEGFC and IL-10; FGF7, VEGFC and an anti-TNFα antibody; FGF7, VEGFD and TGFB1; FGF7, VEGFD and IL-10; FGF7, VEGFD and an anti-TNFα antibody; FGF7, TGFB1 and IL-10; FGF7, TGFB1 and an anti-TNFα antibody; FGF7, IL-10 and an anti-TNFa antibody; FGF9, AREG and EREG; FGF9, AREG and HBEGF; FGF9, AREG and HGF; FGF9, AREG and HRGB; FGF9, AREG and BTC; FGF9, AREG and EGF; FGF9, AREG and TGFA; FGF9, AREG and FGF1; FGF9, AREG and FGF2; FGF9, AREG and FGF4; FGF9, AREG and FGF7; FGF9, AREG and FGF19; FGF9, AREG and SCF; FGF9, AREG and PDGFA; FGF9, AREG and PDGFB; FGF9, AREG and PDGFC; FGF9, AREG and VEGFA; FGF9, AREG and VEGFB; FGF9, AREG and VEGFC; FGF9, AREG and VEGFD; FGF9, AREG and TGFB1; FGF9, AREG and IL-10; FGF9, AREG and an anti-TNFα antibody; FGF9, EREG and HBEGF; FGF9, EREG and HGF; FGF9, EREG and HRGB; FGF9, EREG and BTC; FGF9, EREG and EGF; FGF9, EREG and TGFA; FGF9, EREG and FGF1; FGF9, EREG and FGF2; FGF9, EREG and FGF4; FGF9, EREG and FGF7; FGF9, EREG and FGF19; FGF9, EREG and SCF; FGF9, EREG and PDGFA; FGF9, EREG and PDGFB; FGF9, EREG and PDGFC; FGF9, EREG and VEGFA; FGF9, EREG and VEGFB; FGF9, EREG and VEGFC; FGF9, EREG and VEGFD; FGF9, EREG and TGFB1; FGF9, EREG and IL-10; FGF9, EREG and an anti-TNFa antibody; FGF9, HBEGF and HGF; FGF9, HBEGF and HRGB; FGF9, HBEGF and BTC; FGF9, HBEGF and EGF; FGF9, HBEGF and TGFA; FGF9, HBEGF and FGF1; FGF9, HBEGF and FGF2; FGF9, HBEGF and FGF4; FGF9, HBEGF and FGF7; FGF9, HBEGF and FGF19; FGF9, HBEGF and SCF; FGF9, HBEGF and PDGFA; FGF9, HBEGF and PDGFB; FGF9, HBEGF and PDGFC; FGF9, HBEGF and VEGFA; FGF9, HBEGF and VEGFB; FGF9, HBEGF and VEGFC; FGF9, HBEGF and VEGFD; FGF9, HBEGF and TGFB1; FGF9, HBEGF and IL-10; FGF9, HBEGF and an anti-TNFα antibody; FGF9, HGF and HRGB; FGF9, HGF and BTC; FGF9, HGF and EGF; FGF9, HGF and TGFA; FGF9, HGF and FGF1; FGF9, HGF and FGF2; FGF9, HGF and FGF4; FGF9, HGF and FGF7; FGF9, HGF and FGF19; FGF9, HGF and SCF; FGF9, HGF and PDGFA; FGF9, HGF and PDGFB; FGF9, HGF and PDGFC; FGF9, HGF and VEGFA; FGF9, HGF and VEGFB; FGF9, HGF and VEGFC; FGF9, HGF and VEGFD; FGF9, HGF and TGFB1; FGF9, HGF and IL-10; FGF9, HGF and an anti-TNFα antibody; FGF9, HRGB and BTC; FGF9, HRGB and EGF; FGF9, HRGB and TGFA; FGF9, HRGB and FGF1; FGF9, HRGB and FGF2; FGF9, HRGB and FGF4; FGF9, HRGB and FGF7; FGF9, HRGB and FGF19; FGF9, HRGB and SCF; FGF9, HRGB and PDGFA; FGF9, HRGB and PDGFB; FGF9, HRGB and PDGFC; FGF9, HRGB and VEGFA; FGF9, HRGB and VEGFB; FGF9, HRGB and VEGFC; FGF9, HRGB and VEGFD; FGF9, HRGB and TGFB1; FGF9, HRGB and IL-10; FGF9, HRGB and an anti-TNFα antibody; FGF9, BTC and EGF; FGF9, BTC and TGFA; FGF9, BTC and FGF1; FGF9, BTC and FGF2; FGF9, BTC and FGF4; FGF9, BTC and FGF7; FGF9, BTC and FGF19; FGF9, BTC and SCF; FGF9, BTC and PDGFA; FGF9, BTC and PDGFB; FGF9, BTC and PDGFC; FGF9, BTC and VEGFA; FGF9, BTC and VEGFB; FGF9, BTC and VEGFC; FGF9, BTC and VEGFD; FGF9, BTC and TGFB1; FGF9, BTC and IL-10; FGF9, BTC and an anti-TNFα antibody; FGF9, EGF and TGFA; FGF9, EGF and FGF1; FGF9, EGF and FGF2; FGF9, EGF and FGF4; FGF9, EGF and FGF7; FGF9, EGF and FGF19; FGF9, EGF and SCF; FGF9, EGF and PDGFA; FGF9, EGF and PDGFB; FGF9, EGF and PDGFC; FGF9, EGF and VEGFA; FGF9, EGF and VEGFB; FGF9, EGF and VEGFC; FGF9, EGF and VEGFD; FGF9, EGF and TGFB1; FGF9, EGF and IL-10; FGF9, EGF and an anti-TNFα antibody; FGF9, TGFA and FGF1; FGF9, TGFA and FGF2; FGF9, TGFA and FGF4; FGF9, TGFA and FGF7; FGF9, TGFA and FGF19; FGF9, TGFA and SCF; FGF9, TGFA and PDGFA; FGF9, TGFA and PDGFB; FGF9, TGFA and PDGFC; FGF9, TGFA and VEGFA; FGF9, TGFA and VEGFB; FGF9, TGFA and VEGFC; FGF9, TGFA and VEGFD; FGF9, TGFA and TGFB1; FGF9, TGFA and IL-10; FGF9, TGFA and an anti-TNFα antibody; FGF9, FGF1 and FGF2; FGF9, FGF1 and FGF4; FGF9, FGF1 and FGF7; FGF9, FGF1 and FGF19; FGF9, FGF1 and SCF; FGF9, FGF1 and PDGFA; FGF9, FGF1 and PDGFB; FGF9, FGF1 and PDGFC; FGF9, FGF1 and VEGFA; FGF9, FGF1 and VEGFB; FGF9, FGF1 and VEGFC; FGF9, FGF1 and VEGFD; FGF9, FGF1 and TGFB1; FGF9, FGF1 and IL-10; FGF9, FGF1 and an anti-TNFα antibody; FGF9, FGF2 and FGF4; FGF9, FGF2 and FGF7; FGF9, FGF2 and FGF19; FGF9, FGF2 and SCF; FGF9, FGF2 and PDGFA; FGF9, FGF2 and PDGFB; FGF9, FGF2 and PDGFC; FGF9, FGF2 and VEGFA; FGF9, FGF2 and VEGFB; FGF9, FGF2 and VEGFC; FGF9, FGF2 and VEGFD; FGF9, FGF2 and TGFB1; FGF9, FGF2 and IL-10; FGF9, FGF2 and an anti-TNFα antibody; FGF9, FGF4 and FGF7; FGF9, FGF4 and FGF19; FGF9, FGF4 and SCF; FGF9, FGF4 and PDGFA; FGF9, FGF4 and PDGFB; FGF9, FGF4 and PDGFC; FGF9, FGF4 and VEGFA; FGF9, FGF4 and VEGFB; FGF9, FGF4 and VEGFC; FGF9, FGF4 and VEGFD; FGF9, FGF4 and TGFB1; FGF9, FGF4 and IL-10; FGF9, FGF4 and an anti-TNFα antibody; FGF9, FGF7 and FGF19; FGF9, FGF7 and SCF; FGF9, FGF7 and PDGFA; FGF9, FGF7 and PDGFB; FGF9, FGF7 and PDGFC; FGF9, FGF7 and VEGFA; FGF9, FGF7 and VEGFB; FGF9, FGF7 and VEGFC; FGF9, FGF7 and VEGFD; FGF9, FGF7 and TGFB1; FGF9, FGF7 and IL-10; FGF9, FGF7 and an anti-TNFα antibody; FGF9, FGF19 and SCF; FGF9, FGF19 and PDGFA; FGF9, FGF19 and PDGFB; FGF9, FGF19 and PDGFC; FGF9, FGF19 and VEGFA; FGF9, FGF19 and VEGFB; FGF9, FGF19 and VEGFC; FGF9, FGF19 and VEGFD; FGF9, FGF19 and TGFB1; FGF9, FGF19 and IL-10; FGF9, FGF19 and an anti-TNFα antibody; FGF9, SCF and PDGFA; FGF9, SCF and PDGFB; FGF9, SCF and PDGFC; FGF9, SCF and VEGFA; FGF9, SCF and VEGFB; FGF9, SCF and VEGFC; FGF9, SCF and VEGFD; FGF9, SCF and TGFB1; FGF9, SCF and IL-10; FGF9, SCF and an anti-TNFα antibody; FGF9, PDGFA and PDGFB; FGF9, PDGFA and PDGFC; FGF9, PDGFA and VEGFA; FGF9, PDGFA and VEGFB; FGF9, PDGFA and VEGFC; FGF9, PDGFA and VEGFD; FGF9, PDGFA and TGFB1; FGF9, PDGFA and IL-10; FGF9, PDGFA and an anti-TNFα antibody; FGF9, PDGFB and PDGFC; FGF9, PDGFB and VEGFA; FGF9, PDGFB and VEGFB; FGF9, PDGFB and VEGFC; FGF9, PDGFB and VEGFD; FGF9, PDGFB and TGFB1; FGF9, PDGFB and IL-10; FGF9, PDGFB and an anti-TNFα antibody; FGF9, PDGFC and VEGFA; FGF9, PDGFC and VEGFB; FGF9, PDGFC and VEGFC; FGF9, PDGFC and VEGFD; FGF9, PDGFC and TGFB1; FGF9, PDGFC and IL-10; FGF9, PDGFC and an anti-TNFα antibody; FGF9, VEGFA and VEGFB; FGF9, VEGFA and VEGFC; FGF9, VEGFA and VEGFD; FGF9, VEGFA and TGFB1; FGF9, VEGFA and IL-10; FGF9, VEGFA and an anti-TNFα antibody; FGF9, VEGFB and VEGFC; FGF9, VEGFB and VEGFD; FGF9, VEGFB and TGFB1; FGF9, VEGFB and IL-10; FGF9, VEGFB and an anti-TNFα antibody; FGF9, VEGFC and VEGFD; FGF9, VEGFC and TGFB1; FGF9, VEGFC and IL-10; FGF9, VEGFC and an anti-TNFa antibody; FGF9, VEGFD and TGFB1; FGF9, VEGFD and IL-10; FGF9, VEGFD and an anti-TNFα antibody; FGF9, TGFB1 and IL-10; FGF9, TGFB1 and an anti-TNFα antibody; FGF9, IL-10 and an anti-TNFα antibody; FGF19, AREG and EREG; FGF19, AREG and HBEGF; FGF19, AREG and HGF; FGF19, AREG and HRGB; FGF19, AREG and BTC; FGF19, AREG and EGF; FGF19, AREG and TGFA; FGF19, AREG and FGF1; FGF19, AREG and FGF2; FGF19, AREG and FGF4; FGF19, AREG and FGF7; FGF19, AREG and FGF9; FGF19, AREG and SCF; FGF19, AREG and PDGFA; FGF19, AREG and PDGFB; FGF19, AREG and PDGFC; FGF19, AREG and VEGFA; FGF19, AREG and VEGFB; FGF19, AREG and VEGFC; FGF19, AREG and VEGFD; FGF19, AREG and TGFB1; FGF19, AREG and IL-10; FGF19, AREG and an anti-TNFα antibody; FGF19, EREG and HBEGF; FGF19, EREG and HGF; FGF19, EREG and HRGB; FGF19, EREG and BTC; FGF19, EREG and EGF; FGF19, EREG and TGFA; FGF19, EREG and FGF1; FGF19, EREG and FGF2; FGF19, EREG and FGF4; FGF19, EREG and FGF7; FGF19, EREG and FGF9; FGF19, EREG and SCF; FGF19, EREG and PDGFA; FGF19, EREG and PDGFB; FGF19, EREG and PDGFC; FGF19, EREG and VEGFA; FGF19, EREG and VEGFB; FGF19, EREG and VEGFC; FGF19, EREG and VEGFD; FGF19, EREG and TGFB1; FGF19, EREG and IL-10; FGF19, EREG and an anti-TNFα antibody; FGF19, HBEGF and HGF; FGF19, HBEGF and HRGB; FGF19, HBEGF and BTC; FGF19, HBEGF and EGF; FGF19, HBEGF and TGFA; FGF19, HBEGF and FGF1; FGF19, HBEGF and FGF2; FGF19, HBEGF and FGF4; FGF19, HBEGF and FGF7; FGF19, HBEGF and FGF9; FGF19, HBEGF and SCF; FGF19, HBEGF and PDGFA; FGF19, HBEGF and PDGFB; FGF19, HBEGF and PDGFC; FGF19, HBEGF and VEGFA; FGF19, HBEGF and VEGFB; FGF19, HBEGF and VEGFC; FGF19, HBEGF and VEGFD; FGF19, HBEGF and TGFB1; FGF19, HBEGF and IL-10; FGF19, HBEGF and an anti-TNFα antibody; FGF19, HGF and HRGB; FGF19, HGF and BTC; FGF19, HGF and EGF; FGF19, HGF and TGFA; FGF19, HGF and FGF1; FGF19, HGF and FGF2; FGF19, HGF and FGF4; FGF19, HGF and FGF7; FGF19, HGF and FGF9; FGF19, HGF and SCF; FGF19, HGF and PDGFA; FGF19, HGF and PDGFB; FGF19, HGF and PDGFC; FGF19, HGF and VEGFA; FGF19, HGF and VEGFB; FGF19, HGF and VEGFC; FGF19, HGF and VEGFD; FGF19, HGF and TGFB1; FGF19, HGF and IL-10; FGF19, HGF and an anti-TNFα antibody; FGF19, HRGB and BTC; FGF19, HRGB and EGF; FGF19, HRGB and TGFA; FGF19, HRGB and FGF1; FGF19, HRGB and FGF2; FGF19, HRGB and FGF4; FGF19, HRGB and FGF7; FGF19, HRGB and FGF9; FGF19, HRGB and SCF; FGF19, HRGB and PDGFA; FGF19, HRGB and PDGFB; FGF19, HRGB and PDGFC; FGF19, HRGB and VEGFA; FGF19, HRGB and VEGFB; FGF19, HRGB and VEGFC; FGF19, HRGB and VEGFD; FGF19, HRGB and TGFB1; FGF19, HRGB and IL-10; FGF19, HRGB and an anti-TNFα antibody; FGF19, BTC and EGF; FGF19, BTC and TGFA; FGF19, BTC and FGF1; FGF19, BTC and FGF2; FGF19, BTC and FGF4; FGF19, BTC and FGF7; FGF19, BTC and FGF9; FGF19, BTC and SCF; FGF19, BTC and PDGFA; FGF19, BTC and PDGFB; FGF19, BTC and PDGFC; FGF19, BTC and VEGFA; FGF19, BTC and VEGFB; FGF19, BTC and VEGFC; FGF19, BTC and VEGFD; FGF19, BTC and TGFB1; FGF19, BTC and IL-10; FGF19, BTC and an anti-TNFα antibody; FGF19, EGF and TGFA; FGF19, EGF and FGF1; FGF19, EGF and FGF2; FGF19, EGF and FGF4; FGF19, EGF and FGF7; FGF19, EGF and FGF9; FGF19, EGF and SCF; FGF19, EGF and PDGFA; FGF19, EGF and PDGFB; FGF19, EGF and PDGFC; FGF19, EGF and VEGFA; FGF19, EGF and VEGFB; FGF19, EGF and VEGFC; FGF19, EGF and VEGFD; FGF19, EGF and TGFB1; FGF19, EGF and IL-10; FGF19, EGF and an anti-TNFα antibody; FGF19, TGFA and FGF1; FGF19, TGFA and FGF2; FGF19, TGFA and FGF4; FGF19, TGFA and FGF7; FGF19, TGFA and FGF9; FGF19, TGFA and SCF; FGF19, TGFA and PDGFA; FGF19, TGFA and PDGFB; FGF19, TGFA and PDGFC; FGF19, TGFA and VEGFA; FGF19, TGFA and VEGFB; FGF19, TGFA and VEGFC; FGF19, TGFA and VEGFD; FGF19, TGFA and TGFB1; FGF19, TGFA and IL-10; FGF19, TGFA and an anti-TNFα antibody; FGF19, FGF1 and FGF2; FGF19, FGF1 and FGF4; FGF19, FGF1 and FGF7; FGF19, FGF1 and FGF9; FGF19, FGF1 and SCF; FGF19, FGF1 and PDGFA; FGF19, FGF1 and PDGFB; FGF19, FGF1 and PDGFC; FGF19, FGF1 and VEGFA; FGF19, FGF1 and VEGFB; FGF19, FGF1 and VEGFC; FGF19, FGF1 and VEGFD; FGF19, FGF1 and TGFB1; FGF19, FGF1 and IL-10; FGF19, FGF1 and an anti-TNFα antibody; FGF19, FGF2 and FGF4; FGF19, FGF2 and FGF7; FGF19, FGF2 and FGF9; FGF19, FGF2 and SCF; FGF19, FGF2 and PDGFA; FGF19, FGF2 and PDGFB; FGF19, FGF2 and PDGFC; FGF19, FGF2 and VEGFA; FGF19, FGF2 and VEGFB; FGF19, FGF2 and VEGFC; FGF19, FGF2 and VEGFD; FGF19, FGF2 and TGFB1; FGF19, FGF2 and IL-10; FGF19, FGF2 and an anti-TNFa antibody; FGF19, FGF4 and FGF7; FGF19, FGF4 and FGF9; FGF19, FGF4 and SCF; FGF19, FGF4 and PDGFA; FGF19, FGF4 and PDGFB; FGF19, FGF4 and PDGFC; FGF19, FGF4 and VEGFA; FGF19, FGF4 and VEGFB; FGF19, FGF4 and VEGFC; FGF19, FGF4 and VEGFD; FGF19, FGF4 and TGFB1; FGF19, FGF4 and IL-10; FGF19, FGF4 and an anti-TNFα antibody; FGF19, FGF7 and FGF9; FGF19, FGF7 and SCF; FGF19, FGF7 and PDGFA; FGF19, FGF7 and PDGFB; FGF19, FGF7 and PDGFC; FGF19, FGF7 and VEGFA; FGF19, FGF7 and VEGFB; FGF19, FGF7 and VEGFC; FGF19, FGF7 and VEGFD; FGF19, FGF7 and TGFB1; FGF19, FGF7 and IL-10; FGF19, FGF7 and an anti-TNFα antibody; FGF19, FGF9 and SCF; FGF19, FGF9 and PDGFA; FGF19, FGF9 and PDGFB; FGF19, FGF9 and PDGFC; FGF19, FGF9 and VEGFA; FGF19, FGF9 and VEGFB; FGF19, FGF9 and VEGFC; FGF19, FGF9 and VEGFD; FGF19, FGF9 and TGFB1; FGF19, FGF9 and IL-10; FGF19, FGF9 and an anti-TNFα antibody; FGF19, SCF and PDGFA; FGF19, SCF and PDGFB; FGF19, SCF and PDGFC; FGF19, SCF and VEGFA; FGF19, SCF and VEGFB; FGF19, SCF and VEGFC; FGF19, SCF and VEGFD; FGF19, SCF and TGFB1; FGF19, SCF and IL-10; FGF19, SCF and an anti-TNFa antibody; FGF19, PDGFA and PDGFB; FGF19, PDGFA and PDGFC; FGF19, PDGFA and VEGFA; FGF19, PDGFA and VEGFB; FGF19, PDGFA and VEGFC; FGF19, PDGFA and VEGFD; FGF19, PDGFA and TGFB1; FGF19, PDGFA and IL-10; FGF19, PDGFA and an anti-TNFα antibody; FGF19, PDGFB and PDGFC; FGF19, PDGFB and VEGFA; FGF19, PDGFB and VEGFB; FGF19, PDGFB and VEGFC; FGF19, PDGFB and VEGFD; FGF19, PDGFB and TGFB1; FGF19, PDGFB and IL-10; FGF19, PDGFB and an anti-TNFa antibody; FGF19, PDGFC and VEGFA; FGF19, PDGFC and VEGFB; FGF19, PDGFC and VEGFC; FGF19, PDGFC and VEGFD; FGF19, PDGFC and TGFB1; FGF19, PDGFC and IL-10; FGF19, PDGFC and an anti-TNFα antibody; FGF19, VEGFA and VEGFB; FGF19, VEGFA and VEGFC; FGF19, VEGFA and VEGFD; FGF19, VEGFA and TGFB1; FGF19, VEGFA and IL-10; FGF19, VEGFA and an anti-TNFα antibody; FGF19, VEGFB and VEGFC; FGF19, VEGFB and VEGFD; FGF19, VEGFB and TGFB1; FGF19, VEGFB and IL-10; FGF19, VEGFB and an anti-TNFα antibody; FGF19, VEGFC and VEGFD; FGF19, VEGFC and TGFB1; FGF19, VEGFC and IL-10; FGF19, VEGFC and an anti-TNFα antibody; FGF19, VEGFD and TGFB1; FGF19, VEGFD and IL-10; FGF19, VEGFD and an anti-TNFα antibody; FGF19, TGFB1 and IL-10; FGF19, TGFB1 and an anti-TNFα antibody; FGF19, IL-10 and an anti-TNFα antibody; SCF, AREG and EREG; SCF, AREG and HBEGF; SCF, AREG and HGF; SCF, AREG and HRGB; SCF, AREG and BTC; SCF, AREG and EGF; SCF, AREG and TGFA; SCF, AREG and FGF1; SCF, AREG and FGF2; SCF, AREG and FGF4; SCF, AREG and FGF7; SCF, AREG and FGF9; SCF, AREG and FGF19; SCF, AREG and SCF; SCF, AREG and PDGFA; SCF, AREG and PDGFB; SCF, AREG and PDGFC; SCF, AREG and VEGFA; SCF, AREG and VEGFB; SCF, AREG and VEGFC; SCF, AREG and VEGFD; SCF, AREG and TGFB1; SCF, AREG and IL-10; SCF, AREG and an anti-TNFα antibody; SCF, EREG and HBEGF; SCF, EREG and HGF; SCF, EREG and HRGB; SCF, EREG and BTC; SCF, EREG and EGF; SCF, EREG and TGFA; SCF, EREG and FGF1; SCF, EREG and FGF2; SCF, EREG and FGF4; SCF, EREG and FGF7; SCF, EREG and FGF9; SCF, EREG and FGF19; SCF, EREG and PDGFA; SCF, EREG and PDGFB; SCF, EREG and PDGFC; SCF, EREG and VEGFA; SCF, EREG and VEGFB; SCF, EREG and VEGFC; SCF, EREG and VEGFD; SCF, EREG and TGFB1; SCF, EREG and IL-10; SCF, EREG and an anti-TNFα antibody; SCF, HBEGF and HGF; SCF, HBEGF and HRGB; SCF, HBEGF and BTC; SCF, HBEGF and EGF; SCF, HBEGF and TGFA; SCF, HBEGF and FGF1; SCF, HBEGF and FGF2; SCF, HBEGF and FGF4; SCF, HBEGF and FGF7; SCF, HBEGF and FGF9; SCF, HBEGF and FGF19; SCF, HBEGF and PDGFA; SCF, HBEGF and PDGFB; SCF, HBEGF and PDGFC; SCF, HBEGF and VEGFA; SCF, HBEGF and VEGFB; SCF, HBEGF and VEGFC; SCF, HBEGF and VEGFD; SCF, HBEGF and TGFB1; SCF, HBEGF and IL-10; SCF, HBEGF and an anti-TNFα antibody; SCF, HGF and HRGB; SCF, HGF and BTC; SCF, HGF and EGF; SCF, HGF and TGFA; SCF, HGF and FGF1; SCF, HGF and FGF2; SCF, HGF and FGF4; SCF, HGF and FGF7; SCF, HGF and FGF9; SCF, HGF and FGF19; SCF, HGF and PDGFA; SCF, HGF and PDGFB; SCF, HGF and PDGFC; SCF, HGF and VEGFA; SCF, HGF and VEGFB; SCF, HGF and VEGFC; SCF, HGF and VEGFD; SCF, HGF and TGFB1; SCF, HGF and IL-10; SCF, HGF and an anti-TNFα antibody; SCF, HRGB and BTC; SCF, HRGB and EGF; SCF, HRGB and TGFA; SCF, HRGB and FGF1; SCF, HRGB and FGF2; SCF, HRGB and FGF4; SCF, HRGB and FGF7; SCF, HRGB and FGF9; SCF, HRGB and FGF19; SCF, HRGB and PDGFA; SCF, HRGB and PDGFB; SCF, HRGB and PDGFC; SCF, HRGB and VEGFA; SCF, HRGB and VEGFB; SCF, HRGB and VEGFC; SCF, HRGB and VEGFD; SCF, HRGB and TGFB1; SCF, HRGB and IL-10; SCF, HRGB and an anti-TNFa antibody; SCF, BTC and EGF; SCF, BTC and TGFA; SCF, BTC and FGF1; SCF, BTC and FGF2; SCF, BTC and FGF4; SCF, BTC and FGF7; SCF, BTC and FGF9; SCF, BTC and FGF19; SCF, BTC and PDGFA; SCF, BTC and PDGFB; SCF, BTC and PDGFC; SCF, BTC and VEGFA; SCF, BTC and VEGFB; SCF, BTC and VEGFC; SCF, BTC and VEGFD; SCF, BTC and TGFB1; SCF, BTC and IL-10; SCF, BTC and an anti-TNFα antibody; SCF, EGF and TGFA; SCF, EGF and FGF1; SCF, EGF and FGF2; SCF, EGF and FGF4; SCF, EGF and FGF7; SCF, EGF and FGF9; SCF, EGF and FGF19; SCF, EGF and PDGFA; SCF, EGF and PDGFB; SCF, EGF and PDGFC; SCF, EGF and VEGFA; SCF, EGF and VEGFB; SCF, EGF and VEGFC; SCF, EGF and VEGFD; SCF, EGF and TGFB1; SCF, EGF and IL-10; SCF, EGF and an anti-TNFα antibody; SCF, TGFA and FGF1; SCF, TGFA and FGF2; SCF, TGFA and FGF4; SCF, TGFA and FGF7; SCF, TGFA and FGF9; SCF, TGFA and FGF19; SCF, TGFA and PDGFA; SCF, TGFA and PDGFB; SCF, TGFA and PDGFC; SCF, TGFA and VEGFA; SCF, TGF A and VEGFB; SCF, TGFA and VEGFC; SCF, TGFA and VEGFD; SCF, TGFA and TGFB1; SCF, TGFA and IL-10; SCF, TGFA and an anti-TNFα antibody; SCF, FGF1 and FGF2; SCF, FGF1 and FGF4; SCF, FGF1 and FGF7; SCF, FGF1 and FGF9; SCF, FGF1 and FGF19; SCF, FGF1 and PDGFA; SCF, FGF1 and PDGFB; SCF, FGF1 and PDGFC; SCF, FGF1 and VEGFA; SCF, FGF1 and VEGFB; SCF, FGF1 and VEGFC; SCF, FGF1 and VEGFD; SCF, FGF1 and TGFB1; SCF, FGF1 and IL-10; SCF, FGF1 and an anti-TNFα antibody; SCF, FGF2 and FGF4; SCF, FGF2 and FGF7; SCF, FGF2 and FGF9; SCF, FGF2 and FGF19; SCF, FGF2 and PDGFA; SCF, FGF2 and PDGFB; SCF, FGF2 and PDGFC; SCF, FGF2 and VEGFA; SCF, FGF2 and VEGFB; SCF, FGF2 and VEGFC; SCF, FGF2 and VEGFD; SCF, FGF2 and TGFB1; SCF, FGF2 and IL-10; SCF, FGF2 and an anti-TNFα antibody; SCF, FGF4 and FGF7; SCF, FGF4 and FGF9; SCF, FGF4 and FGF19; SCF, FGF4 and PDGFA; SCF, FGF4 and PDGFB; SCF, FGF4 and PDGFC; SCF, FGF4 and VEGFA; SCF, FGF4 and VEGFB; SCF, FGF4 and VEGFC; SCF, FGF4 and VEGFD; SCF, FGF4 and TGFB1; SCF, FGF4 and IL-10; SCF, FGF4 and an anti-TNFα antibody; SCF, FGF7 and FGF9; SCF, FGF7 and FGF19; SCF, FGF7 and PDGFA; SCF, FGF7 and PDGFB; SCF, FGF7 and PDGFC; SCF, FGF7 and VEGFA; SCF, FGF7 and VEGFB; SCF, FGF7 and VEGFC; SCF, FGF7 and VEGFD; SCF, FGF7 and TGFB1; SCF, FGF7 and IL-10; SCF, FGF7 and an anti-TNFα antibody; SCF, FGF9 and FGF19; SCF, FGF9 and PDGFA; SCF, FGF9 and PDGFB; SCF, FGF9 and PDGFC; SCF, FGF9 and VEGFA; SCF, FGF9 and VEGFB; SCF, FGF9 and VEGFC; SCF, FGF9 and VEGFD; SCF, FGF9 and TGFB1; SCF, FGF9 and IL-10; SCF, FGF9 and an anti-TNFα antibody; SCF, FGF19 and PDGFA; SCF, FGF19 and PDGFB; SCF, FGF19 and PDGFC; SCF, FGF19 and VEGFA; SCF, FGF19 and VEGFB; SCF, FGF19 and VEGFC; SCF, FGF19 and VEGFD; SCF, FGF19 and TGFB1; SCF, FGF19 and IL-10; SCF, FGF19 and an anti-TNFα antibody; SCF, PDGFA and PDGFB; SCF, PDGFA and PDGFC; SCF, PDGFA and VEGFA; SCF, PDGFA and VEGFB; SCF, PDGFA and VEGFC; SCF, PDGFA and VEGFD; SCF, PDGFA and TGFB1; SCF, PDGFA and IL-10; SCF, PDGFA and an anti-TNFa antibody; SCF, PDGFB and PDGFC; SCF, PDGFB and VEGFA; SCF, PDGFB and VEGFB; SCF, PDGFB and VEGFC; SCF, PDGFB and VEGFD; SCF, PDGFB and TGFB1; SCF, PDGFB and IL-10; SCF, PDGFB and an anti-TNFα antibody; SCF, PDGFC and VEGFA; SCF, PDGFC and VEGFB; SCF, PDGFC and VEGFC; SCF, PDGFC and VEGFD; SCF, PDGFC and TGFB1; SCF, PDGFC and IL-10; SCF, PDGFC and an anti-TNFa antibody; SCF, VEGFA and VEGFB; SCF, VEGFA and VEGFC; SCF, VEGFA and VEGFD; SCF, VEGFA and TGFB1; SCF, VEGFA and IL-10; SCF, VEGFA and an anti-TNFa antibody; SCF, VEGFB and VEGFC; SCF, VEGFB and VEGFD; SCF, VEGFB and TGFB1; SCF, VEGFB and IL-10; SCF, VEGFB and an anti-TNFα antibody; SCF, VEGFC and VEGFD; SCF, VEGFC and TGFBl; SCF, VEGFC and IL-10; SCF, VEGFC and an anti-TNFα antibody; SCF, VEGFD and TGFB1; SCF, VEGFD and IL-10; SCF, VEGFD and an anti-TNFα antibody; SCF, TGFB1 and IL-10; SCF, TGFB1 and an anti-TNFα antibody; SCF, IL-10 and an anti-TNFα antibody; PDGFA, AREG and EREG; PDGFA, AREG and HBEGF; PDGFA, AREG and HGF; PDGFA, AREG and HRGB; PDGFA, AREG and BTC; PDGFA, AREG and EGF; PDGFA, AREG and TGFA; PDGFA, AREG and FGF1; PDGFA, AREG and FGF2; PDGFA, AREG and FGF4; PDGFA, AREG and FGF7; PDGFA, AREG and FGF9; PDGFA, AREG and FGF19; PDGFA, AREG and SCF; PDGFA, AREG and PDGFA; PDGFA, AREG and PDGFB; PDGFA, AREG and PDGFC; PDGFA, AREG and VEGFA; PDGFA, AREG and VEGFB; PDGFA, AREG and VEGFC; PDGFA, AREG and VEGFD; PDGFA, AREG and TGFB1; PDGFA, AREG and IL-10; PDGFA, AREG and an anti-TNFα antibody; PDGFA, EREG and HBEGF; PDGFA, EREG and HGF; PDGFA, EREG and HRGB; PDGFA, EREG and BTC; PDGFA, EREG and EGF; PDGFA, EREG and TGFA; PDGFA, EREG and FGF1; PDGFA, EREG and FGF2; PDGFA, EREG and FGF4; PDGFA, EREG and FGF7; PDGFA, EREG and FGF9; PDGFA, EREG and FGF19; PDGFA, EREG and SCF; PDGFA, EREG and PDGFA; PDGFA, EREG and PDGFB; PDGFA, EREG and PDGFC; PDGFA, EREG and VEGFA; PDGFA, EREG and VEGFB; PDGFA, EREG and VEGFC; PDGFA, EREG and VEGFD; PDGFA, EREG and TGFB1; PDGFA, EREG and IL-10; PDGFA, EREG and an anti-TNFα antibody; PDGFA, HBEGF and HGF; PDGFA, HBEGF and HRGB; PDGFA, HBEGF and BTC; PDGFA, HBEGF and EGF; PDGFA, HBEGF and TGFA; PDGFA, HBEGF and FGF1; PDGFA, HBEGF and FGF2; PDGFA, HBEGF and FGF4; PDGFA, HBEGF and FGF7; PDGFA, HBEGF and FGF9; PDGFA, HBEGF and FGF19; PDGFA, HBEGF and SCF; PDGFA, HBEGF and PDGFB; PDGFA, HBEGF and PDGFC; PDGFA, HBEGF and VEGFA; PDGFA, HBEGF and VEGFB; PDGFA, HBEGF and VEGFC; PDGFA, HBEGF and VEGFD; PDGFA, HBEGF and TGFB1; PDGFA, HBEGF and IL-10; PDGFA, HBEGF and an anti-TNFα antibody; PDGFA, HGF and HRGB; PDGFA, HGF and BTC; PDGFA, HGF and EGF; PDGFA, HGF and TGFA; PDGFA, HGF and FGF1; PDGFA, HGF and FGF2; PDGFA, HGF and FGF4; PDGFA, HGF and FGF7; PDGFA, HGF and FGF9; PDGFA, HGF and FGF19; PDGFA, HGF and SCF; PDGFA, HGF and PDGFB; PDGFA, HGF and PDGFC; PDGFA, HGF and VEGFA; PDGFA, HGF and VEGFB; PDGFA, HGF and VEGFC; PDGFA, HGF and VEGFD; PDGFA, HGF and TGFB1; PDGFA, HGF and IL-10; PDGFA, HGF and an anti-TNFα antibody; PDGFA, HRGB and BTC; PDGFA, HRGB and EGF; PDGFA, HRGB and TGFA; PDGFA, HRGB and FGF1; PDGFA, HRGB and FGF2; PDGFA, HRGB and FGF4; PDGFA, HRGB and FGF7; PDGFA, HRGB and FGF9; PDGFA, HRGB and FGF19; PDGFA, HRGB and SCF; PDGFA, HRGB and PDGFB; PDGFA, HRGB and PDGFC; PDGFA, HRGB and VEGFA; PDGFA, HRGB and VEGFB; PDGFA, HRGB and VEGFC; PDGFA, HRGB and VEGFD; PDGFA, HRGB and TGFB1; PDGFA, HRGB and IL-10; PDGFA, HRGB and an anti-TNFα antibody; PDGFA, BTC and EGF; PDGFA, BTC and TGFA; PDGFA, BTC and FGF1; PDGFA, BTC and FGF2; PDGFA, BTC and FGF4; PDGFA, BTC and FGF7; PDGFA, BTC and FGF9; PDGFA, BTC and FGF19; PDGFA, BTC and SCF; PDGFA, BTC and PDGFB; PDGFA, BTC and PDGFC; PDGFA, BTC and VEGFA; PDGFA, BTC and VEGFB; PDGFA, BTC and VEGFC; PDGFA, BTC and VEGFD; PDGFA, BTC and TGFB1; PDGFA, BTC and IL-10; PDGFA, BTC and an anti-TNFα antibody; PDGFA, EGF and TGFA; PDGFA, EGF and FGF1; PDGFA, EGF and FGF2; PDGFA, EGF and FGF4; PDGFA, EGF and FGF7; PDGFA, EGF and FGF9; PDGFA, EGF and FGF19; PDGFA, EGF and SCF; PDGFA, EGF and PDGFB; PDGFA, EGF and PDGFC; PDGFA, EGF and VEGFA; PDGFA, EGF and VEGFB; PDGFA, EGF and VEGFC; PDGFA, EGF and VEGFD; PDGFA, EGF and TGFB1; PDGFA, EGF and IL-10; PDGFA, EGF and an anti-TNFα antibody; PDGFA, TGFA and FGF1; PDGFA, TGFA and FGF2; PDGFA, TGFA and FGF4; PDGFA, TGFA and FGF7; PDGFA, TGFA and FGF9; PDGFA, TGFA and FGF19; PDGFA, TGFA and SCF; PDGFA, TGFA and PDGFB; PDGFA, TGFA and PDGFC; PDGFA, TGFA and VEGFA; PDGFA, TGFA and VEGFB; PDGFA, TGFA and VEGFC; PDGFA, TGFA and VEGFD; PDGFA, TGFA and TGFB1; PDGFA, TGFA and IL-10; PDGFA, TGFA and an anti-TNFα antibody; PDGFA, FGF1 and FGF2; PDGFA, FGF1 and FGF4; PDGFA, FGF1 and FGF7; PDGFA, FGF1 and FGF9; PDGFA, FGF1 and FGF19; PDGFA, FGF1 and SCF; PDGFA, FGF1 and PDGFB; PDGFA, FGF1 and PDGFC; PDGFA, FGF1 and VEGFA; PDGFA, FGF1 and VEGFB; PDGFA, FGF1 and VEGFC; PDGFA, FGF1 and VEGFD; PDGFA, FGF1 and TGFB1; PDGFA, FGF1 and IL-10; PDGFA, FGF1 and an anti-TNFα antibody; PDGFA, FGF2 and FGF4; PDGFA, FGF2 and FGF7; PDGFA, FGF2 and FGF9; PDGFA, FGF2 and FGF19; PDGFA, FGF2 and SCF; PDGFA, FGF2 and PDGFB; PDGFA, FGF2 and PDGFC; PDGFA, FGF2 and VEGFA; PDGFA, FGF2 and VEGFB; PDGFA, FGF2 and VEGFC; PDGFA, FGF2 and VEGFD; PDGFA, FGF2 and TGFB1; PDGFA, FGF2 and IL-10; PDGFA, FGF2 and an anti-TNFα antibody; PDGFA, FGF4 and FGF7; PDGFA, FGF4 and FGF9; PDGFA, FGF4 and FGF19; PDGFA, FGF4 and SCF; PDGFA, FGF4 and PDGFB; PDGFA, FGF4 and PDGFC; PDGFA, FGF4 and VEGFA; PDGFA, FGF4 and VEGFB; PDGFA, FGF4 and VEGFC; PDGFA, FGF4 and VEGFD; PDGFA, FGF4 and TGFB1; PDGFA, FGF4 and IL-10; PDGFA, FGF4 and an anti-TNFα antibody; PDGFA, FGF7 and FGF9; PDGFA, FGF7 and FGF19; PDGFA, FGF7 and SCF; PDGFA, FGF7 and PDGFB; PDGFA, FGF7 and PDGFC; PDGFA, FGF7 and VEGFA; PDGFA, FGF7 and VEGFB; PDGFA, FGF7 and VEGFC; PDGFA, FGF7 and VEGFD; PDGFA, FGF7 and TGFB1; PDGFA, FGF7 and IL-10; PDGFA, FGF7 and an anti-TNFα antibody; PDGFA, FGF9 and FGF19; PDGFA, FGF9 and SCF; PDGFA, FGF9 and PDGFB; PDGFA, FGF9 and PDGFC; PDGFA, FGF9 and VEGFA; PDGFA, FGF9 and VEGFB; PDGFA, FGF9 and VEGFC; PDGFA, FGF9 and VEGFD; PDGFA, FGF9 and TGFB1; PDGFA, FGF9 and IL-10; PDGFA, FGF9 and an anti-TNFα antibody; PDGFA, FGF19 and SCF; PDGFA, FGF19 and PDGFB; PDGFA, FGF19 and PDGFC; PDGFA, FGF19 and VEGFA; PDGFA, FGF19 and VEGFB; PDGFA, FGF19 and VEGFC; PDGFA, FGF19 and VEGFD; PDGFA, FGF19 and TGFB1; PDGFA, FGF19 and IL-10; PDGFA, FGF19 and an anti-TNFα antibody; PDGFA, SCF and PDGFB; PDGFA, SCF and PDGFC; PDGFA, SCF and VEGFA; PDGFA, SCF and VEGFB; PDGFA, SCF and VEGFC; PDGFA, SCF and VEGFD; PDGFA, SCF and TGFB1; PDGFA, SCF and IL-10; PDGFA, SCF and an anti-TNFα antibody; PDGFA, PDGFB and PDGFC; PDGFA, PDGFB and VEGFA; PDGFA, PDGFB and VEGFB; PDGFA, PDGFB and VEGFC; PDGFA, PDGFB and VEGFD; PDGFA, PDGFB and TGFB1; PDGFA, PDGFB and IL-10; PDGFA, PDGFB and an anti-TNFα antibody; PDGFA, PDGFC and VEGFA; PDGFA, PDGFC and VEGFB; PDGFA, PDGFC and VEGFC; PDGFA, PDGFC and VEGFD; PDGFA, PDGFC and TGFB1; PDGFA, PDGFC and IL-10; PDGFA, PDGFC and an anti-TNFα antibody; PDGFA, VEGFA and VEGFB; PDGFA, VEGFA and VEGFC; PDGFA, VEGFA and VEGFD; PDGFA, VEGFA and TGFB1; PDGFA, VEGFA and IL-10; PDGFA, VEGFA and an anti-TNFα antibody; PDGFA, VEGFB and VEGFC; PDGFA, VEGFB and VEGFD; PDGFA, VEGFB and TGFB1; PDGFA, VEGFB and IL-10; PDGFA, VEGFB and an anti-TNFα antibody; PDGFA, VEGFC and VEGFD; PDGFA, VEGFC and TGFB1; PDGFA, VEGFC and IL-10; PDGFA, VEGFC and an anti-TNFα antibody; PDGFA, VEGFD and TGFB1; PDGFA, VEGFD and IL-10; PDGFA, VEGFD and an anti-TNFα antibody; PDGFA, TGFB1 and IL-10; PDGFA, TGFB1 and an anti-TNFα antibody; PDGFA, IL-10 and an anti-TNFα antibody; PDGFB, AREG and EREG; PDGFB, AREG and HBEGF; PDGFB, AREG and HGF; PDGFB, AREG and HRGB; PDGFB, AREG and BTC; PDGFB, AREG and EGF; PDGFB, AREG and TGFA; PDGFB, AREG and FGF1; PDGFB, AREG and FGF2; PDGFB, AREG and FGF4; PDGFB, AREG and FGF7; PDGFB, AREG and FGF9; PDGFB, AREG and FGF19; PDGFB, AREG and SCF; PDGFB, AREG and PDGFA; PDGFB, AREG and PDGFC; PDGFB, AREG and VEGFA; PDGFB, AREG and VEGFB; PDGFB, AREG and VEGFC; PDGFB, AREG and VEGFD; PDGFB, AREG and TGFB1; PDGFB, AREG and IL-10; PDGFB, AREG and an anti-TNFα antibody; PDGFB, EREG and HBEGF; PDGFB, EREG and HGF; PDGFB, EREG and HRGB; PDGFB, EREG and BTC; PDGFB, EREG and EGF; PDGFB, EREG and TGFA; PDGFB, EREG and FGF1; PDGFB, EREG and FGF2; PDGFB, EREG and FGF4; PDGFB, EREG and FGF7; PDGFB, EREG and FGF9; PDGFB, EREG and FGF19; PDGFB, EREG and SCF; PDGFB, EREG and PDGFA; PDGFB, EREG and PDGFC; PDGFB, EREG and VEGFA; PDGFB, EREG and VEGFB; PDGFB, EREG and VEGFC; PDGFB, EREG and VEGFD; PDGFB, EREG and TGFB1; PDGFB, EREG and IL-10; PDGFB, EREG and an anti-TNFα antibody; PDGFB, HBEGF and HGF; PDGFB, HBEGF and HRGB; PDGFB, HBEGF and BTC; PDGFB, HBEGF and EGF; PDGFB, HBEGF and TGFA; PDGFB, HBEGF and FGF1; PDGFB, HBEGF and FGF2; PDGFB, HBEGF and FGF4; PDGFB, HBEGF and FGF7; PDGFB, HBEGF and FGF9; PDGFB, HBEGF and FGF19; PDGFB, HBEGF and SCF; PDGFB, HBEGF and PDGFA; PDGFB, HBEGF and PDGFC; PDGFB, HBEGF and VEGFA; PDGFB, HBEGF and VEGFB; PDGFB, HBEGF and VEGFC; PDGFB, HBEGF and VEGFD; PDGFB, HBEGF and TGFB1; PDGFB, HBEGF and IL-10; PDGFB, HBEGF and an anti-TNFα antibody; PDGFB, HGF and HRGB; PDGFB, HGF and BTC; PDGFB, HGF and EGF; PDGFB, HGF and TGFA; PDGFB, HGF and FGF1; PDGFB, HGF and FGF2; PDGFB, HGF and FGF4; PDGFB, HGF and FGF7; PDGFB, HGF and FGF9; PDGFB, HGF and FGF19; PDGFB, HGF and SCF; PDGFB, HGF and PDGFA; PDGFB, HGF and PDGFC; PDGFB, HGF and VEGFA; PDGFB, HGF and VEGFB; PDGFB, HGF and VEGFC; PDGFB, HGF and VEGFD; PDGFB, HGF and TGFB1; PDGFB, HGF and IL-10; PDGFB, HGF and an anti-TNFα antibody; PDGFB, HRGB and BTC; PDGFB, HRGB and EGF; PDGFB, HRGB and TGFA; PDGFB, HRGB and FGF1; PDGFB, HRGB and FGF2; PDGFB, HRGB and FGF4; PDGFB, HRGB and FGF7; PDGFB, HRGB and FGF9; PDGFB, HRGB and FGF19; PDGFB, HRGB and SCF; PDGFB, HRGB and PDGFA; PDGFB, HRGB and PDGFC; PDGFB, HRGB and VEGFA; PDGFB, HRGB and VEGFB; PDGFB, HRGB and VEGFC; PDGFB, HRGB and VEGFD; PDGFB, HRGB and TGFB1; PDGFB, HRGB and IL-10; PDGFB, HRGB and an anti-TNFα antibody; PDGFB, BTC and EGF; PDGFB, BTC and TGFA; PDGFB, BTC and FGF1; PDGFB, BTC and FGF2; PDGFB, BTC and FGF4; PDGFB, BTC and FGF7; PDGFB, BTC and FGF9; PDGFB, BTC and FGF19; PDGFB, BTC and SCF; PDGFB, BTC and PDGFA; PDGFB, BTC and PDGFC; PDGFB, BTC and VEGFA; PDGFB, BTC and VEGFB; PDGFB, BTC and VEGFC; PDGFB, BTC and VEGFD; PDGFB, BTC and TGFB1; PDGFB, BTC and IL-10; PDGFB, BTC and an anti-TNFα antibody; PDGFB, EGF and TGFA; PDGFB, EGF and FGF1; PDGFB, EGF and FGF2; PDGFB, EGF and FGF4; PDGFB, EGF and FGF7; PDGFB, EGF and FGF9; PDGFB, EGF and FGF19; PDGFB, EGF and SCF; PDGFB, EGF and PDGFA; PDGFB, EGF and PDGFB; PDGFB, EGF and PDGFC; PDGFB, EGF and VEGFA; PDGFB, EGF and VEGFB; PDGFB, EGF and VEGFC; PDGFB, EGF and VEGFD; PDGFB, EGF and TGFB1; PDGFB, EGF and IL-10; PDGFB, EGF and an anti-TNFα antibody; PDGFB, TGFA and FGF1; PDGFB, TGFA and FGF2; PDGFB, TGFA and FGF4; PDGFB, TGFA and FGF7; PDGFB, TGFA and FGF9; PDGFB, TGFA and FGF19; PDGFB, TGFA and SCF; PDGFB, TGFA and PDGFA; PDGFB, TGFA and PDGFC; PDGFB, TGFA and VEGFA; PDGFB, TGFA and VEGFB; PDGFB, TGFA and VEGFC; PDGFB, TGFA and VEGFD; PDGFB, TGFA and TGFB1; PDGFB, TGFA and IL-10; PDGFB, TGFA and an anti-TNFα antibody; PDGFB, FGF1 and FGF2; PDGFB, FGF1 and FGF4; PDGFB, FGF1 and FGF7; PDGFB, FGF1 and FGF9; PDGFB, FGF1 and FGF19; PDGFB, FGF1 and SCF; PDGFB, FGF1 and PDGFA; PDGFB, FGF1 and PDGFC; PDGFB, FGF1 and VEGFA; PDGFB, FGF1 and VEGFB; PDGFB, FGF1 and VEGFC; PDGFB, FGF1 and VEGFD; PDGFB, FGF1 and TGFB1; PDGFB, FGF1 and IL-10; PDGFB, FGF1 and an anti-TNFα antibody; PDGFB, FGF2 and FGF4; PDGFB, FGF2 and FGF7; PDGFB, FGF2 and FGF9; PDGFB, FGF2 and FGF19; PDGFB, FGF2 and SCF; PDGFB, FGF2 and PDGFA; PDGFB, FGF2 and PDGFC; PDGFB, FGF2 and VEGFA; PDGFB, FGF2 and VEGFB; PDGFB, FGF2 and VEGFC; PDGFB, FGF2 and VEGFD; PDGFB, FGF2 and TGFB1; PDGFB, FGF2 and IL-10; PDGFB, FGF2 and an anti-TNFα antibody; PDGFB, FGF4 and FGF7; PDGFB, FGF4 and FGF9; PDGFB, FGF4 and FGF19; PDGFB, FGF4 and SCF; PDGFB, FGF4 and PDGFA; PDGFB, FGF4 and PDGFB; PDGFB, FGF4 and PDGFC; PDGFB, FGF4 and VEGFA; PDGFB, FGF4 and VEGFB; PDGFB, FGF4 and VEGFC; PDGFB, FGF4 and VEGFD; PDGFB, FGF4 and TGFB1; PDGFB, FGF4 and IL-10; PDGFB, FGF4 and an anti-TNFα antibody; PDGFB, FGF7 and FGF9; PDGFB, FGF7 and FGF19; PDGFB, FGF7 and SCF; PDGFB, FGF7 and PDGFA; PDGFB, FGF7 and PDGFB; PDGFB, FGF7 and PDGFC; PDGFB, FGF7 and VEGFA; PDGFB, FGF7 and VEGFB; PDGFB, FGF7 and VEGFC; PDGFB, FGF7 and VEGFD; PDGFB, FGF7 and TGFB1; PDGFB, FGF7 and IL-10; PDGFB, FGF7 and an anti-TNFα antibody; PDGFB, FGF9 and FGF19; PDGFB, FGF9 and SCF; PDGFB, FGF9 and PDGFA; PDGFB, FGF9 and PDGFC; PDGFB, FGF9 and VEGFA; PDGFB, FGF9 and VEGFB; PDGFB, FGF9 and VEGFC; PDGFB, FGF9 and VEGFD; PDGFB, FGF9 and TGFB1; PDGFB, FGF9 and IL-10; PDGFB, FGF9 and an anti-TNFα antibody; PDGFB, FGF19 and SCF; PDGFB, FGF19 and PDGFA; PDGFB, FGF19 and PDGFB; PDGFB, FGF19 and PDGFC; PDGFB, FGF19 and VEGFA; PDGFB, FGF19 and VEGFB; PDGFB, FGF19 and VEGFC; PDGFB, FGF19 and VEGFD; PDGFB, FGF19 and TGFB1; PDGFB, FGF19 and IL-10; PDGFB, FGF19 and an anti-TNFα antibody; PDGFB, SCF and PDGFA, SCF and PDGFC; PDGFB, SCF and VEGFA; PDGFB, SCF and VEGFB; PDGFB, SCF and VEGFC; PDGFB, SCF and VEGFD; PDGFB, SCF and TGFB1; PDGFB, SCF and IL-10; PDGFB, SCF and an anti-TNFα antibody; PDGFB, PDGFA and PDGFC; PDGFB, PDGFA and VEGFA; PDGFB, PDGFA and VEGFB; PDGFB, PDGFA and VEGFC; PDGFB, PDGFA and VEGFD; PDGFB, PDGFA and TGFB1; PDGFB, PDGFA and IL-10; PDGFB, PDGFA and an anti-TNFα antibody; PDGFB, PDGFC and VEGFA; PDGFB, PDGFC and VEGFB; PDGFB, PDGFC and VEGFC; PDGFB, PDGFC and VEGFD; PDGFB, PDGFC and TGFB1; PDGFB, PDGFC and IL-10; PDGFB, PDGFC and an anti-TNFα antibody; PDGFB, VEGFA and VEGFB; PDGFB, VEGFA and VEGFC; PDGFB, VEGFA and VEGFD; PDGFB, VEGFA and TGFB1; PDGFB, VEGFA and IL-10; PDGFB, VEGFA and an anti-TNFα antibody; PDGFB, VEGFB and VEGFC; PDGFB, VEGFB and VEGFD; PDGFB, VEGFB and TGFB1; PDGFB, VEGFB and IL-10; PDGFB, VEGFB and an anti-TNFα antibody; PDGFB, VEGFC and VEGFD; PDGFB, VEGFC and TGFB1; PDGFB, VEGFC and IL-10; PDGFB, VEGFC and an anti-TNFα antibody; PDGFB, VEGFD and TGFB1; PDGFB, VEGFD and IL-10; PDGFB, VEGFD and an anti-TNFα antibody; PDGFB, TGFB1 and IL-10; PDGFB, TGFB1 and an anti-TNFα antibody; PDGFB, IL-10 and an anti-TNFα antibody; PDGFC, AREG and EREG; PDGFC, AREG and HBEGF; PDGFC, AREG and HGF; PDGFC, AREG and HRGB; PDGFC, AREG and BTC; PDGFC, AREG and EGF; PDGFC, AREG and TGFA; PDGFC, AREG and FGF1; PDGFC, AREG and FGF2; PDGFC, AREG and FGF4; PDGFC, AREG and FGF7; PDGFC, AREG and FGF9; PDGFC, AREG and FGF19; PDGFC, AREG and SCF; PDGFC, AREG and PDGFA; PDGFC, AREG and PDGFB; PDGFC, AREG and PDGFC; PDGFC, AREG and VEGFA; PDGFC, AREG and VEGFB; PDGFC, AREG and VEGFC; PDGFC, AREG and VEGFD; PDGFC, AREG and TGFB1; PDGFC, AREG and IL-10; PDGFC, AREG and an anti-TNFα antibody; PDGFC, EREG and HBEGF; PDGFC, EREG and HGF; PDGFC, EREG and HRGB; PDGFC, EREG and BTC; PDGFC, EREG and EGF; PDGFC, EREG and TGFA; PDGFC, EREG and FGF1; PDGFC, EREG and FGF2; PDGFC, EREG and FGF4; PDGFC, EREG and FGF7; PDGFC, EREG and FGF9; PDGFC, EREG and FGF19; PDGFC, EREG and SCF; PDGFC, EREG and PDGFA; PDGFC, EREG and PDGFB; PDGFC, EREG and VEGFA; PDGFC, EREG and VEGFB; PDGFC, EREG and VEGFC; PDGFC, EREG and VEGFD; PDGFC, EREG and TGFB1; PDGFC, EREG and IL-10; PDGFC, EREG and an anti-TNFα antibody; PDGFC, HBEGF and HGF; PDGFC, HBEGF and HRGB; PDGFC, HBEGF and BTC; PDGFC, HBEGF and EGF; PDGFC, HBEGF and TGFA; PDGFC, HBEGF and FGF1; PDGFC, HBEGF and FGF2; PDGFC, HBEGF and FGF4; PDGFC, HBEGF and FGF7; PDGFC, HBEGF and FGF9; PDGFC, HBEGF and FGF19; PDGFC, HBEGF and SCF; PDGFC, HBEGF and PDGFA; PDGFC, HBEGF and PDGFB; PDGFC, HBEGF and VEGFA; PDGFC, HBEGF and VEGFB; PDGFC, HBEGF and VEGFC; PDGFC, HBEGF and VEGFD; PDGFC, HBEGF and TGFB1; PDGFC, HBEGF and IL-10; PDGFC, HBEGF and an anti-TNFα antibody; PDGFC, HGF and HRGB; PDGFC, HGF and BTC; PDGFC, HGF and EGF; PDGFC, HGF and TGFA; PDGFC, HGF and FGF1; PDGFC, HGF and FGF2; PDGFC, HGF and FGF4; PDGFC, HGF and FGF7; PDGFC, HGF and FGF9; PDGFC, HGF and FGF19; PDGFC, HGF and SCF; PDGFC, HGF and PDGFA; PDGFC, HGF and PDGFB; PDGFC, HGF and VEGFA; PDGFC, HGF and VEGFB; PDGFC, HGF and VEGFC; PDGFC, HGF and VEGFD; PDGFC, HGF and TGFB1; PDGFC, HGF and IL-10; PDGFC, HGF and an anti-TNFα antibody; PDGFC, HRGB and BTC; PDGFC, HRGB and EGF; PDGFC, HRGB and TGFA; PDGFC, HRGB and FGF1; PDGFC, HRGB and FGF2; PDGFC, HRGB and FGF4; PDGFC, HRGB and FGF7; PDGFC, HRGB and FGF9; PDGFC, HRGB and FGF19; PDGFC, HRGB and SCF; PDGFC, HRGB and PDGFA; PDGFC, HRGB and PDGFB; PDGFC, HRGB and VEGFA; PDGFC, HRGB and VEGFB; PDGFC, HRGB and VEGFC; PDGFC, HRGB and VEGFD; PDGFC, HRGB and TGFB1; PDGFC, HRGB and IL-10; PDGFC, HRGB and an anti-TNFα antibody; PDGFC, BTC and EGF; PDGFC, BTC and TGFA; PDGFC, BTC and FGF1; PDGFC, BTC and FGF2; PDGFC, BTC and FGF4; PDGFC, BTC and FGF7; PDGFC, BTC and FGF9; PDGFC, BTC and FGF19; PDGFC, BTC and SCF; PDGFC, BTC and PDGFA; PDGFC, BTC and PDGFB; PDGFC, BTC and VEGFA; PDGFC, BTC and VEGFB; PDGFC, BTC and VEGFC; PDGFC, BTC and VEGFD; PDGFC, BTC and TGFB1; PDGFC, BTC and IL-10; PDGFC, BTC and an anti-TNFα antibody; PDGFC, EGF and TGFA; PDGFC, EGF and FGF1; PDGFC, EGF and FGF2; PDGFC, EGF and FGF4; PDGFC, EGF and FGF7; PDGFC, EGF and FGF9; PDGFC, EGF and FGF19; PDGFC, EGF and SCF; PDGFC, EGF and PDGFA; PDGFC, EGF and PDGFB; PDGFC, EGF and VEGFA; PDGFC, EGF and VEGFB; PDGFC, EGF and VEGFC; PDGFC, EGF and VEGFD; PDGFC, EGF and TGFB1; PDGFC, EGF and IL-10; PDGFC, EGF and an anti-TNFα antibody; PDGFC, TGFA and FGF1; PDGFC, TGFA and FGF2; PDGFC, TGFA and FGF4; PDGFC, TGFA and FGF7; PDGFC, TGFA and FGF9; PDGFC, TGFA and FGF19; PDGFC, TGFA and SCF; PDGFC, TGFA and PDGFA; PDGFC, TGFA and PDGFB; PDGFC, TGFA and VEGFA; PDGFC, TGFA and VEGFB; PDGFC, TGFA and VEGFC; PDGFC, TGFA and VEGFD; PDGFC, TGFA and TGFB1; PDGFC, TGFA and IL-10; PDGFC, TGFA and an anti-TNFα antibody; PDGFC, FGF1 and FGF2; PDGFC, FGF1 and FGF4; PDGFC, FGF1 and FGF7; PDGFC, FGF1 and FGF9; PDGFC, FGF1 and FGF19; PDGFC, FGF1 and SCF; PDGFC, FGF1 and PDGFA; PDGFC, FGF1 and PDGFB; PDGFC, FGF1 and VEGFA; PDGFC, FGF1 and VEGFB; PDGFC, FGF1 and VEGFC; PDGFC, FGF1 and VEGFD; PDGFC, FGF1 and TGFB1; PDGFC, FGF1 and IL-10; PDGFC, FGF1 and an anti-TNFα antibody; PDGFC, FGF2 and FGF4; PDGFC, FGF2 and FGF7; PDGFC, FGF2 and FGF9; PDGFC, FGF2 and FGF19; PDGFC, FGF2 and SCF; PDGFC, FGF2 and PDGFA; PDGFC, FGF2 and PDGFB; PDGFC, FGF2 and VEGFA; PDGFC, FGF2 and VEGFB; PDGFC, FGF2 and VEGFC; PDGFC, FGF2 and VEGFD; PDGFC, FGF2 and TGFB1; PDGFC, FGF2 and IL-10; PDGFC, FGF2 and an anti-TNFα antibody; PDGFC, FGF4 and FGF7; PDGFC, FGF4 and FGF9; PDGFC, FGF4 and FGF19; PDGFC, FGF4 and SCF; PDGFC, FGF4 and PDGFA; PDGFC, FGF4 and PDGFB; PDGFC, FGF4 and VEGFA; PDGFC, FGF4 and VEGFB; PDGFC, FGF4 and VEGFC; PDGFC, FGF4 and VEGFD; PDGFC, FGF4 and TGFB1; PDGFC, FGF4 and IL-10; PDGFC, FGF4 and an anti-TNFα antibody; PDGFC, FGF7 and FGF9; PDGFC, FGF7 and FGF19; PDGFC, FGF7 and SCF; PDGFC, FGF7 and PDGFA; PDGFC, FGF7 and PDGFB; PDGFC, FGF7 and VEGFA; PDGFC, FGF7 and VEGFB; PDGFC, FGF7 and VEGFC; PDGFC, FGF7 and VEGFD; PDGFC, FGF7 and TGFB1; PDGFC, FGF7 and IL-10; PDGFC, FGF7 and an anti-TNFα antibody; PDGFC, FGF9 and FGF19; PDGFC, FGF9 and SCF; PDGFC, FGF9 and PDGFA; PDGFC, FGF9 and PDGFB; PDGFC, FGF9 and VEGFA; PDGFC, FGF9 and VEGFB; PDGFC, FGF9 and VEGFC; PDGFC, FGF9 and VEGFD; PDGFC, FGF9 and TGFB1; PDGFC, FGF9 and IL-10; PDGFC, FGF9 and an anti-TNFα antibody; PDGFC, FGF19 and SCF; PDGFC, FGF19 and PDGFA; PDGFC, FGF19 and PDGFB; PDGFC, FGF19 and VEGFA; PDGFC, FGF19 and VEGFB; PDGFC, FGF19 and VEGFC; PDGFC, FGF19 and VEGFD; PDGFC, FGF19 and TGFB1; PDGFC, FGF19 and IL-10; PDGFC, FGF19 and an anti-TNFα antibody; PDGFC, SCF and PDGFA; PDGFC, SCF and PDGFB; PDGFC, SCF and VEGFA; PDGFC, SCF and VEGFB; PDGFC, SCF and VEGFC; PDGFC, SCF and VEGFD; PDGFC, SCF and TGFB1; PDGFC, SCF and IL-10; PDGFC, SCF and an anti-TNFα antibody; PDGFC, PDGFA and PDGFB; PDGFC, PDGFA and VEGFA; PDGFC, PDGFA and VEGFB; PDGFC, PDGFA and VEGFC; PDGFC, PDGFA and VEGFD; PDGFC, PDGFA and TGFB1; PDGFC, PDGFA and IL-10; PDGFC, PDGFA and an anti-TNFα antibody; PDGFC, PDGFB and VEGFA; PDGFC, PDGFB and VEGFB; PDGFC, PDGFB and VEGFC; PDGFC, PDGFB and VEGFD; PDGFC, PDGFB andTGFB1; PDGFC, PDGFB and IL-10; PDGFC, PDGFB and an anti-TNFα antibody; PDGFC, VEGFA and VEGFB; PDGFC, VEGFA and VEGFC; PDGFC, VEGFA and VEGFD; PDGFC, VEGFA and TGFB1; PDGFC, VEGFA and IL-10; PDGFC, VEGFA and an anti-TNFα antibody; PDGFC, VEGFB and VEGFC; PDGFC, VEGFB and VEGFD; PDGFC, VEGFB and TGFB1; PDGFC, VEGFB and IL-10; PDGFC, VEGFB and an anti-TNFα antibody; PDGFC, VEGFC and VEGFD; PDGFC, VEGFC and TGFB1; PDGFC, VEGFC and IL-10; PDGFC, VEGFC and an anti-TNFα antibody; PDGFC, VEGFD and TGFB1; PDGFC, VEGFD and IL-10; PDGFC, VEGFD and an anti-TNFα antibody; PDGFC, TGFB1 and IL-10; PDGFC, TGFB1 and an anti-TNFα antibody; PDGFC, IL-10 and an anti-TNFα antibody; VEGFA, AREG and EREG; VEGFA, AREG and HBEGF; VEGFA, AREG and HGF; VEGFA, AREG and HRGB; VEGFA, AREG and BTC; VEGFA, AREG and EGF; VEGFA, AREG and TGFA; VEGFA, AREG and FGF1; VEGFA, AREG and FGF2; VEGFA, AREG and FGF4; VEGFA, AREG and FGF7; VEGFA, AREG and FGF9; VEGFA, AREG and FGF19; VEGFA, AREG and SCF; VEGFA, AREG and PDGFA; VEGFA, AREG and PDGFB; VEGFA, AREG and PDGFC; VEGFA, AREG and VEGFA; VEGFA, AREG and VEGFB; VEGFA, AREG and VEGFC; VEGFA, AREG and VEGFD; VEGFA, AREG and TGFB1; VEGFA, AREG and IL-10; VEGFA, AREG and an anti-TNFα antibody; VEGFA, EREG and HBEGF; VEGFA, EREG and HGF; VEGFA, EREG and HRGB; VEGFA, EREG and BTC; VEGFA, EREG and EGF; VEGFA, EREG and TGFA; VEGFA, EREG and FGF1; VEGFA, EREG and FGF2; VEGFA, EREG and FGF4; VEGFA, EREG and FGF7; VEGFA, EREG and FGF9; VEGFA, EREG and FGF19; VEGFA, EREG and SCF; VEGFA, EREG and PDGFA; VEGFA, EREG and PDGFB; VEGFA, EREG and PDGFC; VEGFA, EREG and VEGFB; VEGFA, EREG and VEGFC; VEGFA, EREG and VEGFD; VEGFA, EREG and TGFB1; VEGFA, EREG and IL-10; VEGFA, EREG and an anti-TNFa antibody; VEGFA, HBEGF and HGF; VEGFA, HBEGF and HRGB; VEGFA, HBEGF and BTC; VEGFA, HBEGF and EGF; VEGFA, HBEGF and TGFA; VEGFA, HBEGF and FGF1; VEGFA, HBEGF and FGF2; VEGFA, HBEGF and FGF4; VEGFA, HBEGF and FGF7; VEGFA, HBEGF and FGF9; VEGFA, HBEGF and FGF19; VEGFA, HBEGF and SCF; VEGFA, HBEGF and PDGFA; VEGFA, HBEGF and PDGFB; VEGFA, HBEGF and PDGFC; VEGFA, HBEGF and VEGFB; VEGFA, HBEGF and VEGFC; VEGFA, HBEGF and VEGFD; VEGFA, HBEGF and TGFB1; VEGFA, HBEGF and IL-10; VEGFA, HBEGF and an anti-TNFα antibody; VEGFA, HGF and HRGB; VEGFA, HGF and BTC; VEGFA, HGF and EGF; VEGFA, HGF and TGFA; VEGFA, HGF and FGF1; VEGFA, HGF and FGF2; VEGFA, HGF and FGF4; VEGFA, HGF and FGF7; VEGFA, HGF and FGF9; VEGFA, HGF and FGF19; VEGFA, HGF and SCF; VEGFA, HGF and PDGFA; VEGFA, HGF and PDGFB; VEGFA, HGF and PDGFC; VEGFA, HGF and VEGFB; VEGFA, HGF and VEGFC; VEGFA, HGF and VEGFD; VEGFA, HGF and TGFB1; VEGFA, HGF and IL-10; VEGFA, HGF and an anti-TNFα antibody; VEGFA, HRGB and BTC; VEGFA, HRGB and EGF; VEGFA, HRGB and TGFA; VEGFA, HRGB and FGF1; VEGFA, HRGB and FGF2; VEGFA, HRGB and FGF4; VEGFA, HRGB and FGF7; VEGFA, HRGB and FGF9; VEGFA, HRGB and FGF19; VEGFA, HRGB and SCF; VEGFA, HRGB and PDGFA; VEGFA, HRGB and PDGFB; VEGFA, HRGB and PDGFC; VEGFA, HRGB and VEGFB; VEGFA, HRGB and VEGFC; VEGFA, HRGB and VEGFD; VEGFA, HRGB and TGFB1; VEGFA, HRGB and IL-10; VEGFA, HRGB and an anti-TNFα antibody; VEGFA, BTC and EGF; VEGFA, BTC and TGFA; VEGFA, BTC and FGF1; VEGFA, BTC and FGF2; VEGFA, BTC and FGF4; VEGFA, BTC and FGF7; VEGFA, BTC and FGF9; VEGFA, BTC and FGF19; VEGFA, BTC and SCF; VEGFA, BTC and PDGFA; VEGFA, BTC and PDGFB; VEGFA, BTC and PDGFC; VEGFA, BTC and VEGFB; VEGFA, BTC and VEGFC; VEGFA, BTC and VEGFD; VEGFA, BTC and TGFB1; VEGFA, BTC and IL-10; VEGFA, BTC and an anti-TNFα antibody; VEGFA, EGF and TGFA; VEGFA, EGF and FGF1; VEGFA, EGF and FGF2; VEGFA, EGF and FGF4; VEGFA, EGF and FGF7; VEGFA, EGF and FGF9; VEGFA, EGF and FGF19; VEGFA, EGF and SCF; VEGFA, EGF and PDGFA; VEGFA, EGF and PDGFB; VEGFA, EGF and PDGFC; VEGFA, EGF and VEGFB; VEGFA, EGF and VEGFC; VEGFA, EGF and VEGFD; VEGFA, EGF and TGFB1; VEGFA, EGF and IL-10; VEGFA, EGF and an anti-TNFα antibody; VEGFA, TGFA and FGF1; VEGFA, TGFA and FGF2; VEGFA, TGFA and FGF4; VEGFA, TGFA and FGF7; VEGFA, TGFA and FGF9; VEGFA, TGFA and FGF19; VEGFA, TGFA and SCF; VEGFA, TGFA and PDGFA; VEGFA, TGFA and PDGFB; VEGFA, TGFA and PDGFC; VEGFA, TGFA and VEGFB; VEGFA, TGFA and VEGFC; VEGFA, TGFA and VEGFD; VEGFA, TGFA and TGFB1; VEGFA, TGFA and IL-10; VEGFA, TGFA and an anti-TNFα antibody; VEGFA, FGF1 and FGF2; VEGFA, FGF1 and FGF4; VEGFA, FGF1 and FGF7; VEGFA, FGF1 and FGF9; VEGFA, FGF1 and FGF19; VEGFA, FGF1 and SCF; VEGFA, FGF1 and PDGFA; VEGFA, FGF1 and PDGFB; VEGFA, FGF1 and PDGFC; VEGFA, FGF1 and VEGFB; VEGFA, FGF1 and VEGFC; VEGFA, FGF1 and VEGFD; VEGFA, FGF1 and TGFB1; VEGFA, FGF1 and IL-10; VEGFA, FGF1 and an anti-TNFα antibody; VEGFA, FGF2 and FGF4; VEGFA, FGF2 and FGF7; VEGFA, FGF2 and FGF9; VEGFA, FGF2 and FGF19; VEGFA, FGF2 and SCF; VEGFA, FGF2 and PDGFA; VEGFA, FGF2 and PDGFB; VEGFA, FGF2 and PDGFC; VEGFA, FGF2 and VEGFB; VEGFA, FGF2 and VEGFC; VEGFA, FGF2 and VEGFD; VEGFA, FGF2 and TGFB1; VEGFA, FGF2 and IL-10; VEGFA, FGF2 and an anti-TNFa antibody; VEGFA, FGF4 and FGF7; VEGFA, FGF4 and FGF9; VEGFA, FGF4 and FGF19; VEGFA, FGF4 and SCF; VEGFA, FGF4 and PDGFA; VEGFA, FGF4 and PDGFB; VEGFA, FGF4 and PDGFC; VEGFA, FGF4 and VEGFB; VEGFA, FGF4 and VEGFC; VEGFA, FGF4 and VEGFD; VEGFA, FGF4 and TGFB1; VEGFA, FGF4 and IL-10; VEGFA, FGF4 and an anti-TNFα antibody; VEGFA, FGF7 and FGF9; VEGFA, FGF7 and FGF19; VEGFA, FGF7 and SCF; VEGFA, FGF7 and PDGFA; VEGFA, FGF7 and PDGFB; VEGFA, FGF7 and PDGFC; VEGFA, FGF7 and VEGFB; VEGFA, FGF7 and VEGFC; VEGFA, FGF7 and VEGFD; VEGFA, FGF7 and TGFB1; VEGFA, FGF7 and IL-10; VEGFA, FGF7 and an anti-TNFα antibody; VEGFA, FGF9 and FGF19; VEGFA, FGF9 and SCF; VEGFA, FGF9 and PDGFA; VEGFA, FGF9 and PDGFB; VEGFA, FGF9 and PDGFC VEGFA, FGF9 and VEGFB; VEGFA, FGF9 and VEGFC; VEGFA, FGF9 and VEGFD; VEGFA, FGF9 and TGFB1; VEGFA, FGF9 and IL-10; VEGFA, FGF9 and an anti-TNFα antibody; VEGFA, FGF19 and SCF; VEGFA, FGF19 and PDGFA; VEGFA, FGF19 and PDGFB; VEGFA, FGF19 and PDGFC; VEGFA, FGF19 and VEGFB; VEGFA, FGF19 and VEGFC; VEGFA, FGF19 and VEGFD; VEGFA, FGF19 and TGFB1; VEGFA, FGF19 and IL-10; VEGFA, FGF19 and an anti-TNFα antibody; VEGFA, SCF and PDGFA; VEGFA, SCF and PDGFB; VEGFA, SCF and PDGFC; VEGFA, SCF and VEGFB; VEGFA, SCF and VEGFC; VEGFA, SCF and VEGFD; VEGFA, SCF and TGFB1; VEGFA, SCF and IL-10; VEGFA, SCF and an anti-TNFα antibody; VEGFA, PDGFA and PDGFB; VEGFA, PDGFA and PDGFC; VEGFA, PDGFA and VEGFB; VEGFA, PDGFA and VEGFC; VEGFA, PDGFA and VEGFD; VEGFA, PDGFA and TGFB1; VEGFA, PDGFA and IL-10; VEGFA, PDGFA and an anti-TNFα antibody; VEGFA, PDGFB and PDGFC; VEGFA, PDGFB and VEGFB; VEGFA, PDGFB and VEGFC; VEGFA, PDGFB and VEGFD; VEGFA, PDGFB and TGFB1; VEGFA, PDGFB and IL-10; VEGFA, PDGFB and an anti-TNFα antibody; VEGFA, PDGFC and VEGFB; VEGFA, PDGFC and VEGFC; VEGFA, PDGFC and VEGFD; VEGFA, PDGFC and TGFB1; VEGFA, PDGFC and IL-10; VEGFA, PDGFC and an anti-TNFα antibody; VEGFA, VEGFB and VEGFC; VEGFA, VEGFB and VEGFD; VEGFA, VEGFB and TGFB1; VEGFA, VEGFB and IL-10; VEGFA, VEGFB and an anti-TNFα antibody; VEGFA, VEGFC and VEGFD; VEGFA, VEGFC and TGFB1; VEGFA, VEGFC and IL-10; VEGFA, VEGFC and an anti-TNFα antibody; VEGFA, VEGFD and TGFB1; VEGFA, VEGFD and IL-10; VEGFA, VEGFD and an anti-TNFα antibody; VEGFA, TGFB1 and IL-10; VEGFA, TGFB1 and an anti-TNFα antibody; VEGFA, IL-10 and an anti-TNFα antibody; VEGFB, AREG and EREG; VEGFB, AREG and HBEGF; VEGFB, AREG and HGF; VEGFB, AREG and HRGB; VEGFB, AREG and BTC; VEGFB, AREG and EGF; VEGFB, AREG and TGFA; VEGFB, AREG and FGF1; VEGFB, AREG and FGF2; VEGFB, AREG and FGF4; VEGFB, AREG and FGF7; VEGFB, AREG and FGF9; VEGFB, AREG and FGF19; VEGFB, AREG and SCF; VEGFB, AREG and PDGFA; VEGFB, AREG and PDGFB; VEGFB, AREG and PDGFC; VEGFB, AREG and VEGFA; VEGFB, AREG and VEGFB; VEGFB, AREG and VEGFC; VEGFB, AREG and VEGFD; VEGFB, AREG and TGFB1; VEGFB, AREG and IL-10; VEGFB, AREG and an anti-TNFα antibody; VEGFB, EREG and HBEGF; VEGFB, EREG and HGF; VEGFB, EREG and HRGB; VEGFB, EREG and BTC; VEGFB, EREG and EGF; VEGFB, EREG and TGFA; VEGFB, EREG and FGF1; VEGFB, EREG and FGF2; VEGFB, EREG and FGF4; VEGFB, EREG and FGF7; VEGFB, EREG and FGF9; VEGFB, EREG and FGF19; VEGFB, EREG and SCF; VEGFB, EREG and PDGFA; VEGFB, EREG and PDGFB; VEGFB, EREG and PDGFC; VEGFB, EREG and VEGFA; VEGFB, EREG and VEGFC; VEGFB, EREG and VEGFD; VEGFB, EREG and TGFB1; VEGFB, EREG and IL-10; VEGFB, EREG and an anti-TNFα antibody; VEGFB, HBEGF and HGF; VEGFB, HBEGF and HRGB; VEGFB, HBEGF and BTC; VEGFB, HBEGF and EGF; VEGFB, HBEGF and TGFA; VEGFB, HBEGF and FGF1; VEGFB, HBEGF and FGF2; VEGFB, HBEGF and FGF4; VEGFB, HBEGF and FGF7; VEGFB, HBEGF and FGF9; VEGFB, HBEGF and FGF19; VEGFB, HBEGF and SCF; VEGFB, HBEGF and PDGFA; VEGFB, HBEGF and PDGFB; VEGFB, HBEGF and PDGFC; VEGFB, HBEGF and VEGFA; VEGFB, HBEGF and VEGFC; VEGFB, HBEGF and VEGFD; VEGFB, HBEGF and TGFB1; VEGFB, HBEGF and IL-10; VEGFB, HBEGF and an anti-TNFα antibody; VEGFB, HGF and HRGB; VEGFB, HGF and BTC; VEGFB, HGF and EGF; VEGFB, HGF and TGFA; VEGFB, HGF and FGF1; VEGFB, HGF and FGF2; VEGFB, HGF and FGF4; VEGFB, HGF and FGF7; VEGFB, HGF and FGF9; VEGFB, HGF and FGF19; VEGFB, HGF and SCF; VEGFB, HGF and PDGFA; VEGFB, HGF and PDGFB; VEGFB, HGF and PDGFC; VEGFB, HGF and VEGFA; VEGFB, HGF and VEGFC; VEGFB, HGF and VEGFD; VEGFB, HGF and TGFB1; VEGFB, HGF and IL-10; VEGFB, HGF and an anti-TNFα antibody; VEGFB, HRGB and BTC; VEGFB, HRGB and EGF; VEGFB, HRGB and TGFA; VEGFB, HRGB and FGF1; VEGFB, HRGB and FGF2; VEGFB, HRGB and FGF4; VEGFB, HRGB and FGF7; VEGFB, HRGB and FGF9; VEGFB, HRGB and FGF19; VEGFB, HRGB and SCF; VEGFB, HRGB and PDGFA; VEGFB, HRGB and PDGFB; VEGFB, HRGB and PDGFC; VEGFB, HRGB and VEGFA; VEGFB, HRGB and VEGFC; VEGFB, HRGB and VEGFD; VEGFB, HRGB and TGFB1; VEGFB, HRGB and IL-10; VEGFB, HRGB and an anti-TNFα antibody; VEGFB, BTC and EGF; VEGFB, BTC and TGFA; VEGFB, BTC and FGF1; VEGFB, BTC and FGF2; VEGFB, BTC and FGF4; VEGFB, BTC and FGF7; VEGFB, BTC and FGF9; VEGFB, BTC and FGF19; VEGFB, BTC and SCF; VEGFB, BTC and PDGFA; VEGFB, BTC and PDGFB; VEGFB, BTC and PDGFC; VEGFB, BTC and VEGFA; VEGFB, BTC and VEGFC; VEGFB, BTC and VEGFD; VEGFB, BTC and TGFB1; VEGFB, BTC and IL-10; VEGFB, BTC and an anti-TNFα antibody; VEGFB, EGF and TGFA; VEGFB, EGF and FGF1; VEGFB, EGF and FGF2; VEGFB, EGF and FGF4; VEGFB, EGF and FGF7; VEGFB, EGF and FGF9; VEGFB, EGF and FGF19; VEGFB, EGF and SCF; VEGFB, EGF and PDGFA; VEGFB, EGF and PDGFB; VEGFB, EGF and PDGFC; VEGFB, EGF and VEGFA; VEGFB, EGF and VEGFC; VEGFB, EGF and VEGFD; VEGFB, EGF and TGFB1; VEGFB, EGF and IL-10; VEGFB, EGF and an anti-TNFα antibody; VEGFB, TGFA and FGF1; VEGFB, TGFA and FGF2; VEGFB, TGFA and FGF4; VEGFB, TGFA and FGF7; VEGFB, TGFA and FGF9; VEGFB, TGFA and FGF19; VEGFB, TGFA and SCF; VEGFB, TGFA and PDGFA; VEGFB, TGFA and PDGFB; VEGFB, TGFA and PDGFC; VEGFB, TGFA and VEGFA; VEGFB, TGFA and VEGFC; VEGFB, TGFA and VEGFD; VEGFB, TGFA and TGFB1; VEGFB, TGFA and IL-10; VEGFB, TGFA and an anti-TNFα antibody; VEGFB, FGF1 and FGF2; VEGFB, FGF1 and FGF4; VEGFB, FGF1 and FGF7; VEGFB, FGF1 and FGF9; VEGFB, FGF1 and FGF19; VEGFB, FGF1 and SCF; VEGFB, FGF1 and PDGFA; VEGFB, FGF1 and PDGFB; VEGFB, FGF1 and PDGFC; VEGFB, FGF1 and VEGFA; VEGFB, FGF1 and VEGFC; VEGFB, FGF1 and VEGFD; VEGFB, FGF1 and TGFB1; VEGFB, FGF1 and IL-10; VEGFB, FGF1 and an anti-TNFα antibody; VEGFB, FGF2 and FGF4; VEGFB, FGF2 and FGF7; VEGFB, FGF2 and FGF9; VEGFB, FGF2 and FGF19; VEGFB, FGF2 and SCF; VEGFB, FGF2 and PDGFA; VEGFB, FGF2 and PDGFB; VEGFB, FGF2 and PDGFC; VEGFB, FGF2 and VEGFA; VEGFB, FGF2 and VEGFC; VEGFB, FGF2 and VEGFD; VEGFB, FGF2 and TGFB1; VEGFB, FGF2 and IL-10; VEGFB, FGF2 and an anti-TNFα antibody; VEGFB, FGF4 and FGF7; VEGFB, FGF4 and FGF9; VEGFB, FGF4 and FGF19; VEGFB, FGF4 and SCF; VEGFB, FGF4 and PDGFA; VEGFB, FGF4 and PDGFB; VEGFB, FGF4 and PDGFC; VEGFB, FGF4 and VEGFA; VEGFB, FGF4 and VEGFC; VEGFB, FGF4 and VEGFD; VEGFB, FGF4 and TGFB1; VEGFB, FGF4 and IL-10; VEGFB, FGF4 and an anti-TNFα antibody; VEGFB, FGF7 and FGF9; VEGFB, FGF7 and FGF19; VEGFB, FGF7 and SCF; VEGFB, FGF7 and PDGFA; VEGFB, FGF7 and PDGFB; VEGFB, FGF7 and PDGFC; VEGFB, FGF7 and VEGFA; VEGFB, FGF7 and VEGFC; VEGFB, FGF7 and VEGFD; VEGFB, FGF7 and TGFB1; VEGFB, FGF7 and IL-10; VEGFB, FGF7 and an anti-TNFα antibody; VEGFB, FGF9 and FGF19; VEGFB, FGF9 and SCF; VEGFB, FGF9 and PDGFA; VEGFB, FGF9 and PDGFB; VEGFB, FGF9 and PDGFC; VEGFB, FGF9 and VEGFA; VEGFB, FGF9 and VEGFC; VEGFB, FGF9 and VEGFD; VEGFB, FGF9 and TGFB1; VEGFB, FGF9 and IL-10; VEGFB, FGF9 and an anti-TNFα antibody; VEGFB, FGF19 and SCF; VEGFB, FGF19 and PDGFA; VEGFB, FGF19 and PDGFB; VEGFB, FGF19 and PDGFC; VEGFB, FGF19 and VEGFA; VEGFB, FGF19 and VEGFC; VEGFB, FGF19 and VEGFD; VEGFB, FGF19 and TGFB1; VEGFB, FGF19 and IL-10; VEGFB, FGF19 and an anti-TNFα antibody; VEGFB, SCF and PDGFA; VEGFB, SCF and PDGFB; VEGFB, SCF and PDGFC; VEGFB, SCF and VEGFA; VEGFB, SCF and VEGFC; VEGFB, SCF and VEGFD; VEGFB, SCF and TGFB1; VEGFB, SCF and IL-10; VEGFB, SCF and an anti-TNFα antibody; VEGFB, PDGFA and PDGFB; VEGFB, PDGFA and PDGFC; VEGFB, PDGFA and VEGFA; VEGFB, PDGFA and VEGFC; VEGFB, PDGFA and VEGFD; VEGFB, PDGFA and TGFB1; VEGFB, PDGFA and IL-10; VEGFB, PDGFA and an anti-TNFα antibody; VEGFB, PDGFB and PDGFC; VEGFB, PDGFB and VEGFA; VEGFB, PDGFB and VEGFC; VEGFB, PDGFB and VEGFD; VEGFB, PDGFB and TGFB1; VEGFB, PDGFB and IL-10; VEGFB, PDGFB and an anti-TNFα antibody; VEGFB, PDGFC and VEGFA; VEGFB, PDGFC and VEGFC; VEGFB, PDGFC and VEGFD; VEGFB, PDGFC and TGFB1; VEGFB, PDGFC and IL-10; VEGFB, PDGFC and an anti-TNFa antibody; VEGFB, VEGFA and VEGFC; VEGFB, VEGFA and VEGFD; VEGFB, VEGFA and TGFB1; VEGFB, VEGFA and IL-10; VEGFB, VEGFA and an anti-TNFa antibody; VEGFB, VEGFC and VEGFD; VEGFB, VEGFC and TGFB1; VEGFB, VEGFC and IL-10; VEGFB, VEGFC and an anti-TNFα antibody; VEGFB, VEGFD and TGFB1; VEGFB, VEGFD and IL-10; VEGFB, VEGFD and an anti-TNFa antibody; VEGFB, TGFB1 and IL-10; VEGFB, TGFB1 and an anti-TNFα antibody; VEGFB, IL-10 and an anti-TNFα antibody; and the like.

VEGFC, AREG and EREG; VEGFC, AREG and HBEGF; VEGFC, AREG and HGF; VEGFC, AREG and HRGB; VEGFC, AREG and BTC; VEGFC, AREG and EGF; VEGFC, AREG and TGFA; VEGFC, AREG and FGF1; VEGFC, AREG and FGF2; VEGFC, AREG and FGF4; VEGFC, AREG and FGF7; VEGFC, AREG and FGF9; VEGFC, AREG and FGF19; VEGFC, AREG and SCF; VEGFC, AREG and PDGFA; VEGFC, AREG and PDGFB; VEGFC, AREG and PDGFC; VEGFC, AREG and VEGFA; VEGFC, AREG and VEGFB; VEGFC, AREG and VEGFC; VEGFC, AREG and VEGFD; VEGFC, AREG and TGFB1; VEGFC, AREG and IL-10; VEGFC, AREG and an anti-TNFα antibody; VEGFC, EREG and HBEGF; VEGFC, EREG and HGF; VEGFC, EREG and HRGB; VEGFC, EREG and BTC; VEGFC, EREG and EGF; VEGFC, EREG and TGFA; VEGFC, EREG and FGF1; VEGFC, EREG and FGF2; VEGFC, EREG and FGF4; VEGFC, EREG and FGF7; VEGFC, EREG and FGF9; VEGFC, EREG and FGF19; VEGFC, EREG and SCF; VEGFC, EREG and PDGFA; VEGFC, EREG and PDGFB; VEGFC, EREG and PDGFC; VEGFC, EREG and VEGFA; VEGFC, EREG and VEGFB; VEGFC, EREG and VEGFD; VEGFC, EREG and TGFB1; VEGFC, EREG and IL-10; VEGFC, EREG and an anti-TNFα antibody; VEGFC, HBEGF and HGF; VEGFC, HBEGF and HRGB; VEGFC, HBEGF and BTC; VEGFC, HBEGF and EGF; VEGFC, HBEGF and TGFA; VEGFC, HBEGF and FGF1; VEGFC, HBEGF and FGF2; VEGFC, HBEGF and FGF4; VEGFC, HBEGF and FGF7; VEGFC, HBEGF and FGF9; VEGFC, HBEGF and FGF19; VEGFC, HBEGF and SCF; VEGFC, HBEGF and PDGFA; VEGFC, HBEGF and PDGFB; VEGFC, HBEGF and PDGFC; VEGFC, HBEGF and VEGFA; VEGFC, HBEGF and VEGFB; VEGFC, HBEGF and VEGFD; VEGFC, HBEGF and TGFB1; VEGFC, HBEGF and IL-10; VEGFC, HBEGF and an anti-TNFa antibody; VEGFC, HGF and HRGB; VEGFC, HGF and BTC; VEGFC, HGF and EGF; VEGFC, HGF and TGFA; VEGFC, HGF and FGF1; VEGFC, HGF and FGF2; VEGFC, HGF and FGF4; VEGFC, HGF and FGF7; VEGFC, HGF and FGF9; VEGFC, HGF and FGF19; VEGFC, HGF and SCF; VEGFC, HGF and PDGFA; VEGFC, HGF and PDGFB; VEGFC, HGF and PDGFC; VEGFC, HGF and VEGFA; VEGFC, HGF and VEGFB; VEGFC, HGF and VEGFD; VEGFC, HGF and TGFB1; VEGFC, HGF and IL-10; VEGFC, HGF and an anti-TNFa antibody; VEGFC, HRGB and BTC; VEGFC, HRGB and EGF; VEGFC, HRGB and TGFA; VEGFC, HRGB and FGF1; VEGFC, HRGB and FGF2; VEGFC, HRGB and FGF4; VEGFC, HRGB and FGF7; VEGFC, HRGB and FGF9; VEGFC, HRGB and FGF19; VEGFC, HRGB and SCF; VEGFC, HRGB and PDGFA; VEGFC, HRGB and PDGFB; VEGFC, HRGB and PDGFC; VEGFC, HRGB and VEGFA; VEGFC, HRGB and VEGFB; VEGFC, HRGB and VEGFD; VEGFC, HRGB and TGFB1; VEGFC, HRGB and IL-10; VEGFC, HRGB and an anti-TNFα antibody; VEGFC, BTC and EGF; VEGFC, BTC and TGFA; VEGFC, BTC and FGF1; VEGFC, BTC and FGF2; VEGFC, BTC and FGF4; VEGFC, BTC and FGF7; VEGFC, BTC and FGF9; VEGFC, BTC and FGF19; VEGFC, BTC and SCF; VEGFC, BTC and PDGFA; VEGFC, BTC and PDGFB; VEGFC, BTC and PDGFC; VEGFC, BTC and VEGFA; VEGFC, BTC and VEGFB; VEGFC, BTC and VEGFD; VEGFC, BTC and TGFB1; VEGFC, BTC and IL-10; VEGFC, BTC and an anti-TNFα antibody; VEGFC, EGF and TGFA; VEGFC, EGF and FGF1; VEGFC, EGF and FGF2; VEGFC, EGF and FGF4; VEGFC, EGF and FGF7; VEGFC, EGF and FGF9; VEGFC, EGF and FGF 19; VEGFC, EGF and SCF; VEGFC, EGF and PDGFA; VEGFC, EGF and PDGFB; VEGFC, EGF and PDGFC; VEGFC, EGF and VEGFA; VEGFC, EGF and VEGFB; VEGFC, EGF and VEGFD; VEGFC, EGF and TGFB1; VEGFC, EGF and IL-10; VEGFC, EGF and an anti-TNFα antibody; VEGFC, TGFA and FGF1; VEGFC, TGFA and FGF2; VEGFC, TGFA and FGF4; VEGFC, TGFA and FGF7; VEGFC, TGFA and FGF9; VEGFC, TGFA and FGF19; VEGFC, TGFA and SCF; VEGFC, TGFA and PDGFA; VEGFC, TGFA and PDGFB; VEGFC, TGFA and PDGFC; VEGFC, TGFA and VEGFA; VEGFC, TGFA and VEGFB; VEGFC, TGFA and VEGFD; VEGFC, TGFA and TGFB1; VEGFC, TGFA and IL-10; VEGFC, TGFA and an anti-TNFα antibody; VEGFC, FGF1 and FGF2; VEGFC, FGF1 and FGF4; VEGFC, FGF1 and FGF7; VEGFC, FGF1 and FGF9; VEGFC, FGF1 and FGF19; VEGFC, FGF1 and SCF; VEGFC, FGF1 and PDGFA; VEGFC, FGF1 and PDGFB; VEGFC, FGF1 and PDGFC; VEGFC, FGF1 and VEGFA; VEGFC, FGF1 and VEGFB; VEGFC, FGF1 and VEGFD; VEGFC, FGF1 and TGFB1; VEGFC, FGF1 and IL-10; VEGFC, FGF1 and an anti-TNFα antibody; VEGFC, FGF2 and FGF4; VEGFC, FGF2 and FGF7; VEGFC, FGF2 and FGF9; VEGFC, FGF2 and FGF19; VEGFC, FGF2 and SCF; VEGFC, FGF2 and PDGFA; VEGFC, FGF2 and PDGFB; VEGFC, FGF2 and PDGFC; VEGFC, FGF2 and VEGFA; VEGFC, FGF2 and VEGFB; VEGFC, FGF2 and VEGFD; VEGFC, FGF2 and TGFB1; VEGFC, FGF2 and IL-10; VEGFC, FGF2 and an anti-TNFα antibody; VEGFC, FGF4 and FGF7; VEGFC, FGF4 and FGF9; VEGFC, FGF4 and FGF19; VEGFC, FGF4 and SCF; VEGFC, FGF4 and PDGFA; VEGFC, FGF4 and PDGFB; VEGFC, FGF4 and PDGFC; VEGFC, FGF4 and VEGFA; VEGFC, FGF4 and VEGFB; VEGFC, FGF4 and VEGFD; VEGFC, FGF4 and TGFB1; VEGFC, FGF4 and IL-10; VEGFC, FGF4 and an anti-TNFα antibody; VEGFC, FGF7 and FGF9; VEGFC, FGF7 and FGF19; VEGFC, FGF7 and SCF; VEGFC, FGF7 and PDGFA; VEGFC, FGF7 and PDGFB; VEGFC, FGF7 and PDGFC; VEGFC, FGF7 and VEGFA; VEGFC, FGF7 and VEGFB; VEGFC, FGF7 and VEGFD; VEGFC, FGF7 and TGFB1; VEGFC, FGF7 and IL-10; VEGFC, FGF7 and an anti-TNFα antibody; VEGFC, FGF9 and FGF19; VEGFC, FGF9 and SCF; VEGFC, FGF9 and PDGFA; VEGFC, FGF9 and PDGFB; VEGFC, FGF9 and PDGFC; VEGFC, FGF9 and VEGFA; VEGFC, FGF9 and VEGFB; VEGFC, FGF9 and VEGFD; VEGFC, FGF9 and TGFB1; VEGFC, FGF9 and IL-10; VEGFC, FGF9 and an anti-TNFα antibody; VEGFC, FGF19 and SCF; VEGFC, FGF19 and PDGFA; VEGFC, FGF19 and PDGFB; VEGFC, FGF19 and PDGFC; VEGFC, FGF19 and VEGFA; VEGFC, FGF19 and VEGFB; VEGFC, FGF19 and VEGFD; VEGFC, FGF19 and TGFB1; VEGFC, FGF19 and IL-10; VEGFC, FGF19 and an anti-TNFα antibody; VEGFC, SCF and PDGFA; VEGFC, SCF and PDGFB; VEGFC, SCF and PDGFC; VEGFC, SCF and VEGFA; VEGFC, SCF and VEGFB; VEGFC, SCF and VEGFD; VEGFC, SCF and TGFB1; VEGFC, SCF and IL-10; VEGFC, SCF and an anti-TNFα antibody; VEGFC, PDGFA and PDGFB; VEGFC, PDGFA and PDGFC; VEGFC, PDGFA and VEGFA; VEGFC, PDGFA and VEGFB; VEGFC, PDGFA and VEGFD; VEGFC, PDGFA and TGFB1; VEGFC, PDGFA and IL-10; VEGFC, PDGFA and an anti-TNFα antibody; VEGFC, PDGFB and PDGFC; VEGFC, PDGFB and VEGFA; VEGFC, PDGFB and VEGFB; VEGFC, PDGFB and VEGFD; VEGFC, PDGFB and TGFB1; VEGFC, PDGFB and IL-10; VEGFC, PDGFB and an anti-TNFα antibody; VEGFC, PDGFC and VEGFA; VEGFC, PDGFC and VEGFB; VEGFC, PDGFC and VEGFD; VEGFC, PDGFC and TGFB1; VEGFC, PDGFC and IL-10; VEGFC, PDGFC and an anti-TNFα antibody; VEGFC, VEGFA and VEGFB; VEGFC, VEGFA and VEGFD; VEGFC, VEGFA and TGFB1; VEGFC, VEGFA and IL-10; VEGFC, VEGFA and an anti-TNFα antibody; VEGFC, VEGFB and VEGFD; VEGFC, VEGFB and TGFB1; VEGFC, VEGFB and IL-10; VEGFC, VEGFB and an anti-TNFα antibody; VEGFC, VEGFD and TGFB1; VEGFC, VEGFD and IL-10; VEGFC, VEGFD and an anti-TNFα antibody; VEGFC, TGFB1 and IL-10; VEGFC, TGFB1 and an anti-TNFα antibody; VEGFC, IL-10 and an anti-TNFα antibody; VEGFD, AREG and EREG; VEGFD, AREG and HBEGF; VEGFD, AREG and HGF; VEGFD, AREG and HRGB; VEGFD, AREG and BTC; VEGFD, AREG and EGF; VEGFD, AREG and TGFA; VEGFD, AREG and FGF1; VEGFD, AREG and FGF2; VEGFD, AREG and FGF4; VEGFD, AREG and FGF7; VEGFD, AREG and FGF9; VEGFD, AREG and FGF19; VEGFD, AREG and SCF; VEGFD, AREG and PDGFA; VEGFD, AREG and PDGFB; VEGFD, AREG and PDGFC; VEGFD, AREG and VEGFA; VEGFD, AREG and VEGFB; VEGFD, AREG and VEGFC; VEGFD, AREG and TGFB1; VEGFD, AREG and IL-10; VEGFD, AREG and an anti-TNFα antibody; VEGFD, EREG and HBEGF; VEGFD, EREG and HGF; VEGFD, EREG and HRGB; VEGFD, EREG and BTC; VEGFD, EREG and EGF; VEGFD, EREG and TGFA; VEGFD, EREG and FGF1; VEGFD, EREG and FGF2; VEGFD, EREG and FGF4; VEGFD, EREG and FGF7; VEGFD, EREG and FGF9; VEGFD, EREG and FGF19; VEGFD, EREG and SCF; VEGFD, EREG and PDGFA; VEGFD, EREG and PDGFB; VEGFD, EREG and PDGFC; VEGFD, EREG and VEGFA; VEGFD, EREG and VEGFB; VEGFD, EREG and VEGFC; VEGFD, EREG and TGFB1; VEGFD, EREG and IL-10; VEGFD, EREG and an anti-TNFα antibody; VEGFD, HBEGF and HGF; VEGFD, HBEGF and HRGB; VEGFD, HBEGF and BTC; VEGFD, HBEGF and EGF; VEGFD, HBEGF and TGFA; VEGFD, HBEGF and FGF1; VEGFD, HBEGF and FGF2; VEGFD, HBEGF and FGF4; VEGFD, HBEGF and FGF7; VEGFD, HBEGF and FGF9; VEGFD, HBEGF and FGF19; VEGFD, HBEGF and SCF; VEGFD, HBEGF and PDGFA; VEGFD, HBEGF and PDGFB; VEGFD, HBEGF and PDGFC; VEGFD, HBEGF and VEGFA; VEGFD, HBEGF and VEGFB; VEGFD, HBEGF and VEGFC; VEGFD, HBEGF and TGFB1; VEGFD, HBEGF and IL-10; VEGFD, HBEGF and an anti-TNFα antibody; VEGFD, HGF and HRGB; VEGFD, HGF and BTC; VEGFD, HGF and EGF; VEGFD, HGF and TGFA; VEGFD, HGF and FGF1; VEGFD, HGF and FGF2; VEGFD, HGF and FGF4; VEGFD, HGF and FGF7; VEGFD, HGF and FGF9; VEGFD, HGF and FGF19; VEGFD, HGF and SCF; VEGFD, HGF and PDGFA; VEGFD, HGF and PDGFB; VEGFD, HGF and PDGFC; VEGFD, HGF and VEGFA; VEGFD, HGF and VEGFB; VEGFD, HGF and VEGFC; VEGFD, HGF and TGFB1; VEGFD, HGF and IL-10; VEGFD, HGF and an anti-TNFα antibody; VEGFD, HRGB and BTC; VEGFD, HRGB and EGF; VEGFD, HRGB and TGFA; VEGFD, HRGB and FGF1; VEGFD, HRGB and FGF2; VEGFD, HRGB and FGF4; VEGFD, HRGB and FGF7; VEGFD, HRGB and FGF9; VEGFD, HRGB and FGF19; VEGFD, HRGB and SCF; VEGFD, HRGB and PDGFA; VEGFD, HRGB and PDGFB; VEGFD, HRGB and PDGFC; VEGFD, HRGB and VEGFA; VEGFD, HRGB and VEGFB; VEGFD, HRGB and VEGFC; VEGFD, HRGB and TGFB1; VEGFD, HRGB and IL-10; VEGFD, HRGB and an anti-TNFα antibody; VEGFD, BTC and EGF; VEGFD, BTC and TGFA; VEGFD, BTC and FGF1; VEGFD, BTC and FGF2; VEGFD, BTC and FGF4; VEGFD, BTC and FGF7; VEGFD, BTC and FGF9; VEGFD, BTC and FGF19; VEGFD, BTC and SCF; VEGFD, BTC and PDGFA; VEGFD, BTC and PDGFB; VEGFD, BTC and PDGFC; VEGFD, BTC and VEGFA; VEGFD, BTC and VEGFB; VEGFD, BTC and VEGFC; VEGFD, BTC and TGFB1; VEGFD, BTC and IL-10; VEGFD, BTC and an anti-TNFa antibody; VEGFD, EGF and TGFA; VEGFD, EGF and FGF1; VEGFD, EGF and FGF2; VEGFD, EGF and FGF4; VEGFD, EGF and FGF7; VEGFD, EGF and FGF9; VEGFD, EGF and FGF19; VEGFD, EGF and SCF; VEGFD, EGF and PDGFA; VEGFD, EGF and PDGFB; VEGFD, EGF and PDGFC; VEGFD, EGF and VEGFA; VEGFD, EGF and VEGFB; VEGFD, EGF and VEGFC; VEGFD, EGF and TGFB1; VEGFD, EGF and IL-10; VEGFD, EGF and an anti-TNFα antibody; VEGFD, TGFA and FGF1; VEGFD, TGFA and FGF2; VEGFD, TGFA and FGF4; VEGFD, TGFA and FGF7; VEGFD, TGFA and FGF9; VEGFD, TGFA and FGF19; VEGFD, TGFA and SCF; VEGFD, TGFA and PDGFA; VEGFD, TGFA and PDGFB; VEGFD, TGFA and PDGFC; VEGFD, TGFA and VEGFA; VEGFD, TGFA and VEGFB; VEGFD, TGFA and VEGFC; VEGFD, TGFA and TGFB1; VEGFD, TGFA and IL-10; VEGFD, TGFA and an anti-TNFα antibody; VEGFD, FGF1 and FGF2; VEGFD, FGF1 and FGF4; VEGFD, FGF1 and FGF7; VEGFD, FGF1 and FGF9; VEGFD, FGF1 and FGF19; VEGFD, FGF1 and SCF; VEGFD, FGF1 and PDGFA; VEGFD, FGF1 and PDGFB; VEGFD, FGF1 and PDGFC; VEGFD, FGF1 and VEGFA; VEGFD, FGF1 and VEGFB; VEGFD, FGF1 and VEGFC; VEGFD, FGF1 and TGFB1; VEGFD, FGF1 and IL-10; VEGFD, FGF1 and an anti-TNFα antibody; VEGFD, FGF2 and FGF4; VEGFD, FGF2 and FGF7; VEGFD, FGF2 and FGF9; VEGFD, FGF2 and FGF19; VEGFD, FGF2 and SCF; VEGFD, FGF2 and PDGFA; VEGFD, FGF2 and PDGFB; VEGFD, FGF2 and PDGFC; VEGFD, FGF2 and VEGFA; VEGFD, FGF2 and VEGFB; VEGFD, FGF2 and VEGFC; VEGFD, FGF2 and TGFB1; VEGFD, FGF2 and IL-10; VEGFD, FGF2 and an anti-TNFα antibody; VEGFD, FGF4 and FGF7; VEGFD, FGF4 and FGF9; VEGFD, FGF4 and FGF19; VEGFD, FGF4 and SCF; VEGFD, FGF4 and PDGFA; VEGFD, FGF4 and PDGFB; VEGFD, FGF4 and PDGFC; VEGFD, FGF4 and VEGFA; VEGFD, FGF4 and VEGFB; VEGFD, FGF4 and VEGFC; VEGFD, FGF4 and TGFB1; VEGFD, FGF4 and IL-10; VEGFD, FGF4 and an anti-TNFα antibody; VEGFD, FGF7 and FGF9; VEGFD, FGF7 and FGF19; VEGFD, FGF7 and SCF; VEGFD, FGF7 and PDGFA; VEGFD, FGF7 and PDGFB; VEGFD, FGF7 and PDGFC; VEGFD, FGF7 and VEGFA; VEGFD, FGF7 and VEGFB; VEGFD, FGF7 and VEGFC; VEGFD, FGF7 and TGFB1; VEGFD, FGF7 and IL-10; VEGFD, FGF7 and an anti-TNFα antibody; VEGFD, FGF9 and FGF19; VEGFD, FGF9 and SCF; VEGFD, FGF9 and PDGFA; VEGFD, FGF9 and PDGFB; VEGFD, FGF9 and PDGFC; VEGFD, FGF9 and VEGFA; VEGFD, FGF9 and VEGFB; VEGFD, FGF9 and VEGFC; VEGFD, FGF9 and TGFB1; VEGFD, FGF9 and IL-10; VEGFD, FGF9 and an anti-TNFα antibody; VEGFD, FGF19 and SCF; VEGFD, FGF19 and PDGFA; VEGFD, FGF19 and PDGFB; VEGFD, FGF19 and PDGFC; VEGFD, FGF19 and VEGFA; VEGFD, FGF19 and VEGFB; VEGFD, FGF19 and VEGFC; VEGFD, FGF19 and TGFB1; VEGFD, FGF19 and IL-10; VEGFD, FGF19 and an anti-TNFa antibody; VEGFD, SCF and PDGFA; VEGFD, SCF and PDGFB; VEGFD, SCF and PDGFC; VEGFD, SCF and VEGFA; VEGFD, SCF and VEGFB; VEGFD, SCF and VEGFC; VEGFD, SCF and TGFB1; VEGFD, SCF and IL-10; VEGFD, SCF and an anti-TNFα antibody; VEGFD, PDGFA and PDGFB; VEGFD, PDGFA and PDGFC; VEGFD, PDGFA and VEGFA; VEGFD, PDGFA and VEGFB; VEGFD, PDGFA and VEGFC; VEGFD, PDGFA and TGFB1; VEGFD, PDGFA and IL-10; VEGFD, PDGFA and an anti-TNFa antibody; VEGFD, PDGFB and PDGFC; VEGFD, PDGFB and VEGFA; VEGFD, PDGFB and VEGFB; VEGFD, PDGFB and VEGFC; VEGFD, PDGFB and TGFB1; VEGFD, PDGFB and IL-10; VEGFD, PDGFB and an anti-TNFa antibody; VEGFD, PDGFC and VEGFA; VEGFD, PDGFC and VEGFB; VEGFD, PDGFC and VEGFC; VEGFD, PDGFC and TGFB1; VEGFD, PDGFC and IL-10; VEGFD, PDGFC and an anti-TNFα antibody; VEGFD, VEGFA and VEGFB; VEGFD, VEGFA and VEGFC; VEGFD, VEGFA and TGFB1; VEGFD, VEGFA and IL-10; VEGFD, VEGFA and an anti-TNFa antibody; VEGFD, VEGFB and VEGFC; VEGFD, VEGFB and TGFB1; VEGFD, VEGFB and IL-10; VEGFD, VEGFB and an anti-TNFa antibody; VEGFD, VEGFC and TGFB1; VEGFD, VEGFC and IL-10; VEGFD, VEGFC and an anti-TNFα antibody; VEGFD, TGFB1 and IL-10; VEGFD, TGFB1 and an anti-TNFa antibody; VEGFD, IL-10 and an anti-TNFα antibody; TGFB1, AREG and EREG; TGFB1, AREG and HBEGF; TGFB1, AREG and HGF; TGFB1, AREG and HRGB; TGFB1, AREG and BTC; TGFB1, AREG and EGF; TGFB1, AREG and TGFA; TGFB1, AREG and FGF1; TGFB1, AREG and FGF2; TGFB1, AREG and FGF4; TGFB1, AREG and FGF7; TGFB1, AREG and FGF9; TGFB1, AREG and FGF19; TGFB1, AREG and SCF; TGFB1, AREG and PDGFA; TGFB1, AREG and PDGFB; TGFB1, AREG and PDGFC; TGFB1, AREG and VEGFA; TGFB1, AREG and VEGFB; TGFB1, AREG and VEGFC; TGFB1, AREG and VEGFD; TGFB1, AREG and IL-10; TGFB1, AREG and an anti-TNFα antibody; TGFB1, EREG and HBEGF; TGFB1, EREG and HGF; TGFB1, EREG and HRGB; TGFB1, EREG and BTC; TGFB1, EREG and EGF; TGFB1, EREG and TGFA; TGFB1, EREG and FGF1; TGFB1, EREG and FGF2; TGFB1, EREG and FGF4; TGFB1, EREG and FGF7; TGFB1, EREG and FGF9; TGFB1, EREG and FGF19; TGFB1, EREG and SCF; TGFB1, EREG and PDGFA; TGFB1, EREG and PDGFB; TGFB1, EREG and PDGFC; TGFB1, EREG and VEGFA; TGFB1, EREG and VEGFB; TGFB1, EREG and VEGFC; TGFB1, EREG and VEGFD; TGFB1, EREG and IL-10; TGFB1, EREG and an anti-TNFα antibody; TGFB1, HBEGF and HGF; TGFB1, HBEGF and HRGB; TGFB1, HBEGF and BTC; TGFB1, HBEGF and EGF; TGFB1, HBEGF and TGFA; TGFB1, HBEGF and FGF1; TGFB1, HBEGF and FGF2; TGFB1, HBEGF and FGF4; TGFB1, HBEGF and FGF7; TGFB1, HBEGF and FGF9; TGFB1, HBEGF and FGF19; TGFB1, HBEGF and SCF; TGFB1, HBEGF and PDGFA; TGFB1, HBEGF and PDGFB; TGFB1, HBEGF and PDGFC; TGFB1, HBEGF and VEGFA; TGFB1, HBEGF and VEGFB; TGFB1, HBEGF and VEGFC; TGFB1, HBEGF and VEGFD; TGFB1, HBEGF and IL-10; TGFB1, HBEGF and an anti-TNFα antibody; TGFB1, HGF and HRGB; TGFB1, HGF and BTC; TGFB1, HGF and EGF; TGFB1, HGF and TGFA; TGFB1, HGF and FGF1; TGFB1, HGF and FGF2; TGFB1, HGF and FGF4; TGFB1, HGF and FGF7; TGFB1, HGF and FGF9; TGFB1, HGF and FGF19; TGFB1, HGF and SCF; TGFB1, HGF and PDGFA; TGFB1, HGF and PDGFB; TGFB1, HGF and PDGFC; TGFB1, HGF and VEGFA; TGFB1, HGF and VEGFB; TGFB1, HGF and VEGFC; TGFB1, HGF and VEGFD; TGFB1, HGF and IL-10; TGFB1, HGF and an anti-TNFα antibody; TGFB1, HRGB and BTC; TGFB1, HRGB and EGF; TGFB1, HRGB and TGFA; TGFB1, HRGB and FGF1; TGFB1, HRGB and FGF2; TGFB1, HRGB and FGF4; TGFB1, HRGB and FGF7; TGFB1, HRGB and FGF9; TGFB1, HRGB and FGF19; TGFB1, HRGB and SCF; TGFB1, HRGB and PDGFA; TGFB1, HRGB and PDGFB; TGFB1, HRGB and PDGFC; TGFB1, HRGB and VEGFA; TGFB1, HRGB and VEGFB; TGFB1, HRGB and VEGFC; TGFB1, HRGB and VEGFD; TGFB1, HRGB and IL-10; TGFB1, HRGB and an anti-TNFα antibody; TGFB1, BTC and EGF; TGFB1, BTC and TGFA; TGFB1, BTC and FGF1; TGFB1, BTC and FGF2; TGFB1, BTC and FGF4; TGFB1, BTC and FGF7; TGFB1, BTC and FGF9; TGFB1, BTC and FGF19; TGFB1, BTC and SCF; TGFB1, BTC and PDGFA; TGFB1, BTC and PDGFB; TGFB1, BTC and PDGFC; TGFB1, BTC and VEGFA; TGFB1, BTC and VEGFB; TGFB1, BTC and VEGFC; TGFB1, BTC and VEGFD; TGFB1, BTC and IL-10; TGFB1, BTC and an anti-TNFα antibody; TGFB1, EGF and TGFA; TGFB1, EGF and FGF1; TGFB1, EGF and FGF2; TGFB1, EGF and FGF4; TGFB1, EGF and FGF7; TGFB1, EGF and FGF9; TGFB1, EGF and FGF19; TGFB1, EGF and SCF; TGFB1, EGF and PDGFA; TGFB1, EGF and PDGFB; TGFB1, EGF and PDGFC; TGFB1, EGF and VEGFA; TGFB1, EGF and VEGFB; TGFB1, EGF and VEGFC; TGFB1, EGF and VEGFD; TGFB1, EGF and IL-10; TGFB1, EGF and an anti-TNFα antibody; TGFB1, TGFA and FGF1; TGFB1, TGFA and FGF2; TGFB1, TGFA and FGF4; TGFB1, TGFA and FGF7; TGFB1, TGFA and FGF9; TGFB1, TGFA and FGF19; TGFB1, TGFA and SCF; TGFB1, TGFA and PDGFA; TGFB1, TGFA and PDGFB; TGFB1, TGFA and PDGFC; TGFB1, TGFA and VEGFA; TGFB1, TGFA and VEGFB; TGFB1, TGFA and VEGFC; TGFB1, TGFA and VEGFD; TGFB1, TGFA and IL-10; TGFB1, TGFA and an anti-TNFα antibody; TGFB1, FGF1 and FGF2; TGFB1, FGF1 and FGF4; TGFB1, FGF1 and FGF7; TGFB1, FGF1 and FGF9; TGFB1, FGF1 and FGF19; TGFB1, FGF1 and SCF; TGFB1, FGF1 and PDGFA; TGFB1, FGF1 and PDGFB; TGFB1, FGF1 and PDGFC; TGFB1, FGF1 and VEGFA; TGFB1, FGF1 and VEGFB; TGFB1, FGF1 and VEGFC; TGFB1, FGF1 and VEGFD; TGFB1, FGF1 and IL-10; TGFB1, FGF1 and an anti-TNFα antibody; TGFB1, FGF2 and FGF4; TGFB1, FGF2 and FGF7; TGFB1, FGF2 and FGF9; TGFB1, FGF2 and FGF19; TGFB1, FGF2 and SCF; TGFB1, FGF2 and PDGFA; TGFB1, FGF2 and PDGFB; TGFB1, FGF2 and PDGFC; TGFB1, FGF2 and VEGFA; TGFB1, FGF2 and VEGFB; TGFB1, FGF2 and VEGFC; TGFB1, FGF2 and VEGFD; TGFB1, FGF2 and IL-10; TGFB1, FGF2 and an anti-TNFα antibody; TGFB1, FGF4 and FGF7; TGFB1, FGF4 and FGF9; TGFB1, FGF4 and FGF19; TGFB1, FGF4 and SCF; TGFB1, FGF4 and PDGFA; TGFB1, FGF4 and PDGFB; TGFB1, FGF4 and PDGFC; TGFB1, FGF4 and VEGFA; TGFB1, FGF4 and VEGFB; TGFB1, FGF4 and VEGFC; TGFB1, FGF4 and VEGFD; TGFB1, FGF4 and IL-10; TGFB1, FGF4 and an anti-TNFα antibody; TGFB1, FGF7 and FGF9; TGFB1, FGF7 and FGF19; TGFB1, FGF7 and SCF; TGFB1, FGF7 and PDGFA; TGFB1, FGF7 and PDGFB; TGFB1, FGF7 and PDGFC; TGFB1, FGF7 and VEGFA; TGFB1, FGF7 and VEGFB; TGFB1, FGF7 and VEGFC; TGFB1, FGF7 and VEGFD; TGFB1, FGF7 and IL-10; TGFB1, FGF7 and an anti-TNFα antibody; TGFB1, FGF9 and FGF19; TGFB1, FGF9 and SCF; TGFB1, FGF9 and PDGFA; TGFB1, FGF9 and PDGFB; TGFB1, FGF9 and PDGFC; TGFB1, FGF9 and VEGFA; TGFB1, FGF9 and VEGFB; TGFB1, FGF9 and VEGFC; TGFB1, FGF9 and VEGFD; TGFB1, FGF9 and IL-10; TGFB1, FGF9 and an anti-TNFα antibody; TGFB1, FGF19 and SCF; TGFB1, FGF19 and PDGFA; TGFB1, FGF19 and PDGFB; TGFB1, FGF19 and PDGFC; TGFB1, FGF19 and VEGFA; TGFB1, FGF19 and VEGFB; TGFB1, FGF19 and VEGFC; TGFB1, FGF19 and VEGFD; TGFB1, FGF19 and IL-10; TGFB1, FGF19 and an anti-TNFα antibody; TGFB1, SCF and PDGFA; TGFB1, SCF and PDGFB; TGFB1, SCF and PDGFC; TGFB1, SCF and VEGFA; TGFB1, SCF and VEGFB; TGFB1, SCF and VEGFC; TGFB1, SCF and VEGFD; TGFB1, SCF and IL-10; TGFB1, SCF and an anti-TNFα antibody; TGFB1, PDGFA and PDGFB; TGFB1, PDGFA and PDGFC; TGFB1, PDGFA and VEGFA; TGFB1, PDGFA and VEGFB; TGFB1, PDGFA and VEGFC; TGFB1, PDGFA and VEGFD; TGFB1, PDGFA and IL-10; TGFB1, PDGFA and an anti-TNFα antibody; TGFB1, PDGFB and PDGFC; TGFB1, PDGFB and VEGFA; TGFB1, PDGFB and VEGFB; TGFB1, PDGFB and VEGFC; TGFB1, PDGFB and VEGFD; TGFB1, PDGFB and IL-10; TGFB1, PDGFB and an anti-TNFα antibody; TGFB1, PDGFC and VEGFA; TGFB1, PDGFC and VEGFB; TGFB1, PDGFC and VEGFC; TGFB1, PDGFC and VEGFD; TGFB1, PDGFC and IL-10; TGFB1, PDGFC and an anti-TNFα antibody; TGFB1, VEGFA and VEGFB; TGFB1, VEGFA and VEGFC; TGFB1, VEGFA and VEGFD; TGFB1, VEGFA and IL-10; TGFB1, VEGFA and an anti-TNFα antibody; TGFB1, VEGFB and VEGFC; TGFB1, VEGFB and VEGFD; TGFB1, VEGFB and IL-10; TGFB1, VEGFB and an anti-TNFα antibody; TGFB1, VEGFC and VEGFD; TGFB1, VEGFC and IL-10; TGFB1, VEGFC and an anti-TNFα antibody; TGFB1, VEGFD and IL-10; TGFB1, VEGFD and an anti-TNFα antibody; TGFB1, IL-10 and an anti-TNFα antibody; IL-10, AREG andEREG; IL-10, AREG and HBEGF; IL-10, AREG and HGF; IL-10, AREG and HRGB; IL-10, AREG and BTC; IL-10, AREG and EGF; IL-10, AREG and TGFA; IL-10, AREG and FGF1; IL-10, AREG and FGF2; IL-10, AREG and FGF4; IL-10, AREG and FGF7; IL-10, AREG and FGF9; IL-10, AREG and FGF19; IL-10, AREG and SCF; IL-10, AREG and PDGFA; IL-10, AREG and PDGFB; IL-10, AREG and PDGFC; IL-10, AREG and VEGFA; IL-10, AREG and VEGFB; IL-10, AREG and VEGFC; IL-10, AREG and VEGFD; IL-10, AREG and TGFB1; IL-10, AREG and IL-10; IL-10, AREG and an anti-TNFα antibody; IL-10, EREG and HBEGF; IL-10, EREG and HGF; IL-10, EREG and HRGB; IL-10, EREG and BTC; IL-10, EREG and EGF; IL-10, EREG and TGFA; IL-10, EREG and FGF1; IL-10, EREG and FGF2; IL-10, EREG and FGF4; IL-10, EREG and FGF7; IL-10, EREG and FGF9; IL-10, EREG and FGF19; IL-10, EREG and SCF; IL-10, EREG and PDGFA; IL-10, EREG and PDGFB; IL-10, EREG and PDGFC; IL-10, EREG and VEGFA; IL-10, EREG and VEGFB; IL-10, EREG and VEGFC; IL-10, EREG and VEGFD; IL-10, EREG and TGFB1; IL-10, EREG and an anti-TNFα antibody; IL-10, HBEGF and HGF; IL-10, HBEGF and HRGB; IL-10, HBEGF and BTC; IL-10, HBEGF and EGF; IL-10, HBEGF and TGFA; IL-10, HBEGF and FGF1; IL-10, HBEGF and FGF2; IL-10, HBEGF and FGF4; IL-10, HBEGF and FGF7; IL-10, HBEGF and FGF9; IL-10, HBEGF and FGF19; IL-10, HBEGF and SCF; IL-10, HBEGF and PDGFA; IL-10, HBEGF and PDGFB; IL-10, HBEGF and PDGFC; IL-10, HBEGF and VEGFA; IL-10, HBEGF and VEGFB; IL-10, HBEGF and VEGFC; IL-10, HBEGF and VEGFD; IL-10, HBEGF and TGFB1; IL-10, HBEGF and an anti-TNFα antibody; IL-10, HGF and HRGB; IL-10, HGF and BTC; IL-10, HGF and EGF; IL-10, HGF and TGFA; IL-10, HGF and FGF1; IL-10, HGF and FGF2; IL-10, HGF and FGF4; IL-10, HGF and FGF7; IL-10, HGF and FGF9; IL-10, HGF and FGF19; IL-10, HGF and SCF; IL-10, HGF and PDGFA; IL-10, HGF and PDGFB; IL-10, HGF and PDGFC; IL-10, HGF and VEGFA; IL-10, HGF and VEGFB; IL-10, HGF and VEGFC; IL-10, HGF and VEGFD; IL-10, HGF and TGFB1; IL-10, HGF and an anti-TNFα antibody; IL-10, HRGB and BTC; IL-10, HRGB and EGF; IL-10, HRGB and TGFA; IL-10, HRGB and FGF1; IL-10, HRGB and FGF2; IL-10, HRGB and FGF4; IL-10, HRGB and FGF7; IL-10, HRGB and FGF9; IL-10, HRGB and FGF19; IL-10, HRGB and SCF; IL-10, HRGB and PDGFA; IL-10, HRGB and PDGFB; IL-10, HRGB and PDGFC; IL-10, HRGB and VEGFA; IL-10, HRGB and VEGFB; IL-10, HRGB and VEGFC; IL-10, HRGB and VEGFD; IL-10, HRGB and TGFB1; IL-10, HRGB and an anti-TNFα antibody; IL-10, BTC and EGF; IL-10, BTC and TGFA; IL-10, BTC and FGF1; IL-10, BTC and FGF2; IL-10, BTC and FGF4; IL-10, BTC and FGF7; IL-10, BTC and FGF9; IL-10, BTC and FGF19; IL-10, BTC and SCF; IL-10, BTC and PDGFA; IL-10, BTC and PDGFB; IL-10, BTC and PDGFC; IL-10, BTC and VEGFA; IL-10, BTC and VEGFB; IL-10, BTC and VEGFC; IL-10, BTC and VEGFD; IL-10, BTC and TGFB1; IL-10, BTC and an anti-TNFα antibody; IL-10, EGF and TGFA; IL-10, EGF and FGF1; IL-10, EGF and FGF2; IL-10, EGF and FGF4; IL-10, EGF and FGF7; IL-10, EGF and FGF9; IL-10, EGF and FGF19; IL-10, EGF and SCF; IL-10, EGF and PDGFA; IL-10, EGF and PDGFB; IL-10, EGF and PDGFC; IL-10, EGF and VEGFA; IL-10, EGF and VEGFB; IL-10, EGF and VEGFC; IL-10, EGF and VEGFD; IL-10, EGF and TGFB1; IL-10, EGF and an anti-TNFα antibody; IL-10, TGFA and FGF1; IL-10, TGFA and FGF2; IL-10, TGFA and FGF4; IL-10, TGFA and FGF7; IL-10, TGFA and FGF9; IL-10, TGFA and FGF19; IL-10, TGFA and SCF; IL-10, TGFA and PDGFA; IL-10, TGFA and PDGFB; IL-10, TGFA and PDGFC; IL-10, TGFA and VEGFA; IL-10, TGFA and VEGFB; IL-10, TGFA and VEGFC; IL-10, TGFA and VEGFD; IL-10, TGFA and TGFB1; IL-10, TGFA and an anti-TNFα antibody; IL-10, FGF1 and FGF2; IL-10, FGF1 and FGF4; IL-10, FGF1 and FGF7; IL-10, FGF1 and FGF9; IL-10, FGF1 and FGF19; IL-10, FGF1 and SCF; IL-10, FGF1 and PDGFA; IL-10, FGF1 and PDGFB; IL-10, FGF1 and PDGFC; IL-10, FGF1 and VEGFA; IL-10, FGF1 and VEGFB; IL-10, FGF1 and VEGFC; IL-10, FGF1 and VEGFD; IL-10, FGF1 and TGFB1; IL-10, FGF1 and an anti-TNFa antibody; IL-10, FGF2 and FGF4; IL-10, FGF2 and FGF7; IL-10, FGF2 and FGF9; IL-10, FGF2 and FGF19; IL-10, FGF2 and SCF; IL-10, FGF2 and PDGFA; IL-10, FGF2 and PDGFB; IL-10, FGF2 and PDGFC; IL-10, FGF2 and VEGFA; IL-10, FGF2 and VEGFB; IL-10, FGF2 and VEGFC; IL-10, FGF2 and VEGFD; IL-10, FGF2 and TGFB1; IL-10, FGF2 and an anti-TNFα antibody; IL-10, FGF4 and FGF7; IL-10, FGF4 and FGF9; IL-10, FGF4 and FGF19; IL-10, FGF4 and SCF; IL-10, FGF4 and PDGFA; IL-10, FGF4 and PDGFB; IL-10, FGF4 and PDGFC; IL-10, FGF4 and VEGFA; IL-10, FGF4 and VEGFB; IL-10, FGF4 and VEGFC; IL-10, FGF4 and VEGFD; IL-10, FGF4 and TGFB1; IL-10, FGF4 and an anti-TNFa antibody; IL-10, FGF7 and FGF9; IL-10, FGF7 and FGF19; IL-10, FGF7 and SCF; IL-10, FGF7 and PDGFA; IL-10, FGF7 and PDGFB; IL-10, FGF7 and PDGFC; IL-10, FGF7 and VEGFA; IL-10, FGF7 and VEGFB; IL-10, FGF7 and VEGFC; IL-10, FGF7 and VEGFD; IL-10, FGF7 and TGFB1; IL-10, FGF7 and an anti-TNFα antibody; IL-10, FGF9 and FGF19; IL-10, FGF9 and SCF; IL-10, FGF9 and PDGFA; IL-10, FGF9 and PDGFB; IL-10, FGF9 and PDGFC; IL-10, FGF9 and VEGFA; IL-10, FGF9 and VEGFB; IL-10, FGF9 and VEGFC; IL-10, FGF9 and VEGFD; IL-10, FGF9 and TGFB1; IL-10, FGF9 and an anti-TNFα antibody; IL-10, FGF19 and SCF; IL-10, FGF19 and PDGFA; IL-10, FGF19 and PDGFB; IL-10, FGF19 and PDGFC; IL-10, FGF19 and VEGFA; IL-10, FGF19 and VEGFB; IL-10, FGF19 and VEGFC; IL-10, FGF19 and VEGFD; IL-10, FGF19 and TGFB1; IL-10, FGF19 and an anti-TNFα antibody; IL-10, SCF and PDGFA; IL-10, SCF and PDGFB; IL-10, SCF and PDGFC; IL-10, SCF and VEGFA; IL-10, SCF and VEGFB; IL-10, SCF and VEGFC; IL-10, SCF and VEGFD; IL-10, SCF and TGFB1; IL-10, SCF and an anti-TNFα antibody; IL-10, PDGFA and PDGFB; IL-10, PDGFA and PDGFC; IL-10, PDGFA and VEGFA; IL-10, PDGFA and VEGFB; IL-10, PDGFA and VEGFC; IL-10, PDGFA and VEGFD; IL-10, PDGFA and TGFB1; IL-10, PDGFA and an anti-TNFα antibody; IL-10, PDGFB and PDGFC; IL-10, PDGFB and VEGFA; IL-10, PDGFB and VEGFB; IL-10, PDGFB and VEGFC; IL-10, PDGFB and VEGFD; IL-10, PDGFB and TGFB1; IL-10, PDGFB and an anti-TNFα antibody; IL-10, PDGFC and VEGFA; IL-10, PDGFC and VEGFB; IL-10, PDGFC and VEGFC; IL-10, PDGFC and VEGFD; IL-10, PDGFC and TGFB1; IL-10, PDGFC and an anti-TNFα antibody; IL-10, VEGFA and VEGFB; IL-10, VEGFA and VEGFC; IL-10, VEGFA and VEGFD; IL-10, VEGFA and TGFB1; IL-10, VEGFA and an anti-TNFα antibody; IL-10, VEGFB and VEGFC; IL-10, VEGFB and VEGFD; IL-10, VEGFB and TGFB1; IL-10, VEGFB and an anti-TNFα antibody; IL-10, VEGFC and VEGFD; IL-10, VEGFC and TGFB1; IL-10, VEGFC and an anti-TNFα antibody; IL-10, VEGFD and TGFB1; IL-10, VEGFD and an anti-TNFα antibody; IL-10, TGFB1 and an anti-TNFα antibody; an anti-TNFα antibody, AREG and EREG; an anti-TNFα antibody, AREG and HBEGF; an anti-TNFα antibody, AREG and HGF; an anti-TNFα antibody, AREG and HRGB; an anti-TNFα antibody, AREG and BTC; an anti-TNFα antibody, AREG and EGF; an anti-TNFα antibody, AREG and TGFA; an anti-TNFα antibody, AREG and FGF1; an anti-TNFα antibody, AREG and FGF2; an anti-TNFα antibody, AREG and FGF4; an anti-TNFα antibody, AREG and FGF7; an anti-TNFα antibody, AREG and FGF9; an anti-TNFα antibody, AREG and FGF19; an anti-TNFα antibody, AREG and SCF; an anti-TNFα antibody, AREG and PDGFA; an anti-TNFα antibody, AREG and PDGFB; an anti-TNFα antibody, AREG and PDGFC; an anti-TNFα antibody, AREG and VEGFA; an anti-TNFα antibody, AREG and VEGFB; an anti-TNFα antibody, AREG and VEGFC; an anti-TNFa antibody, AREG and VEGFD; an anti-TNFα antibody, AREG and TGFB1; an anti-TNFα antibody, AREG and IL-10; an anti-TNFα antibody, EREG and HBEGF; an anti-TNFα antibody, EREG and HGF; an anti-TNFα antibody, EREG and HRGB; an anti-TNFα antibody, EREG and BTC; an anti-TNFα antibody, EREG and EGF; an anti-TNFα antibody, EREG and TGFA; an anti-TNFα antibody, EREG and FGF1; an anti-TNFα antibody, EREG and FGF2; an anti-TNFα antibody, EREG and FGF4; an anti-TNFα antibody, EREG and FGF7; an anti-TNFα antibody, EREG and FGF9; an anti-TNFα antibody, EREG and FGF19; an anti-TNFα antibody, EREG and SCF; an anti-TNFα antibody, EREG and PDGFA; an anti-TNFα antibody, EREG and PDGFB; an anti-TNFα antibody, EREG and PDGFC; an anti-TNFα antibody, EREG and VEGFA; an anti-TNFα antibody, EREG and VEGFB; an anti-TNFa antibody, EREG and VEGFC; an anti-TNFα antibody, EREG and VEGFD; an anti-TNFα antibody, EREG and TGFB1; an anti-TNFα antibody, EREG and IL-10; an anti-TNFα antibody, HBEGF and HGF; an anti-TNFα antibody, HBEGF and HRGB; an anti-TNFα antibody, HBEGF and BTC; an anti-TNFα antibody, HBEGF and EGF; an anti-TNFa antibody, HBEGF and TGFA; an anti-TNFα antibody, HBEGF and FGF1; an anti-TNFα antibody, HBEGF and FGF2; an anti-TNFα antibody, HBEGF and FGF4; an anti-TNFα antibody, HBEGF and FGF7; an anti-TNFα antibody, HBEGF and FGF9; an anti-TNFα antibody, HBEGF and FGF19; an anti-TNFα antibody, HBEGF and SCF; an anti-TNFα antibody, HBEGF and PDGFA; an anti-TNFα antibody, HBEGF and PDGFB; an anti-TNFα antibody, HBEGF and PDGFC; an anti-TNFα antibody, HBEGF and VEGFA; an anti-TNFα antibody, HBEGF and VEGFB; an anti-TNFα antibody, HBEGF and VEGFC; an anti-TNFα antibody, HBEGF and VEGFD; an anti-TNFα antibody, HBEGF and TGFB1; an anti-TNFα antibody, HBEGF and IL-10; an anti-TNFα antibody, HGF and HRGB; an anti-TNFα antibody, HGF and BTC; an anti-TNFα antibody, HGF and EGF; an anti-TNFα antibody, HGF and TGFA; an anti-TNFα antibody, HGF and FGF1; an anti-TNFα antibody, HGF and FGF2; an anti-TNFa antibody, HGF and FGF4; an anti-TNFα antibody, HGF and FGF7; an anti-TNFα antibody, HGF and FGF9; an anti-TNFα antibody, HGF and FGF19; an anti-TNFα antibody, HGF and SCF; an anti-TNFα antibody, HGF and PDGFA; an anti-TNFα antibody, HGF and PDGFB; an anti-TNFα antibody, HGF and PDGFC; an anti-TNFα antibody, HGF and VEGFA; an anti-TNFα antibody, HGF and VEGFB; an anti-TNFα antibody, HGF and VEGFC; an anti-TNFα antibody, HGF and VEGFD; an anti-TNFα antibody, HGF and TGFB1; an anti-TNFα antibody, HGF and IL-10; an anti-TNFα antibody, HRGB and BTC; an anti-TNFα antibody, HRGB and EGF; an anti-TNFα antibody, HRGB and TGFA; an anti-TNFα antibody, HRGB and FGF1; an anti-TNFa antibody, HRGB and FGF2; an anti-TNFα antibody, HRGB and FGF4; an anti-TNFα antibody, HRGB and FGF7; an anti-TNFα antibody, HRGB and FGF9; an anti-TNFα antibody, HRGB and FGF19; an anti-TNFα antibody, HRGB and SCF; an anti-TNFα antibody, HRGB and PDGFA; an anti-TNFα antibody, HRGB and PDGFB; an anti-TNFα antibody, HRGB and PDGFC; an anti-TNFα antibody, HRGB and VEGFA; an anti-TNFα antibody, HRGB and VEGFB; an anti-TNFα antibody, HRGB and VEGFC; an anti-TNFα antibody, HRGB and VEGFD; an anti-TNFα antibody, HRGB and TGFB1; an anti-TNFα antibody, HRGB and IL-10; an anti-TNFα antibody, BTC and EGF; an anti-TNFα antibody, BTC and TGFA; an anti-TNFα antibody, BTC and FGF1; an anti-TNFα antibody, BTC and FGF2; an anti-TNFα antibody, BTC and FGF4; an anti-TNFα antibody, BTC and FGF7; an anti-TNFa antibody, BTC and FGF9; an anti-TNFα antibody, BTC and FGF19; an anti-TNFα antibody, BTC and SCF; an anti-TNFα antibody, BTC and PDGFA; an anti-TNFα antibody, BTC and PDGFB; an anti-TNFα antibody, BTC and PDGFC; an anti-TNFα antibody, BTC and VEGFA; an anti-TNFα antibody, BTC and VEGFB; an anti-TNFα antibody, BTC and VEGFC; an anti-TNFα antibody, BTC and VEGFD; an anti-TNFα antibody, BTC and TGFB1; an anti-TNFa antibody, BTC and IL-10; an anti-TNFα antibody, EGF and TGFA; an anti-TNFα antibody, EGF and FGF1; an anti-TNFα antibody, EGF and FGF2; an anti-TNFα antibody, EGF and FGF4; an anti-TNFα antibody, EGF and FGF7; an anti-TNFα antibody, EGF and FGF9; an anti-TNFα antibody, EGF and FGF19; an anti-TNFα antibody, EGF and SCF; an anti-TNFα antibody, EGF and PDGFA; an anti-TNFα antibody, EGF and PDGFB; an anti-TNFα antibody, EGF and PDGFC; an anti-TNFα antibody, EGF and VEGFA; an anti-TNFα antibody, EGF and VEGFB; an anti-TNFα antibody, EGF and VEGFC; an anti-TNFα antibody, EGF and VEGFD; an anti-TNFα antibody, EGF and TGFB1; an anti-TNFα antibody, EGF and IL-10; an anti-TNFα antibody, TGFA and FGF1; an anti-TNFα antibody, TGFA and FGF2; an anti-TNFα antibody, TGFA and FGF4; an anti-TNFα antibody, TGFA and FGF7; an anti-TNFα antibody, TGFA and FGF9; an anti-TNFα antibody, TGFA and FGF19; an anti-TNFα antibody, TGFA and SCF; an anti-TNFα antibody, TGFA and PDGFA; an anti-TNFα antibody, TGFA and PDGFB; an anti-TNFα antibody, TGFA and PDGFC; an anti-TNFα antibody, TGFA and VEGFA; an anti-TNFα antibody, TGFA and VEGFB; an anti-TNFα antibody, TGFA and VEGFC; an anti-TNFα antibody, TGFA and VEGFD; an anti-TNFα antibody, TGFA and TGFB1; an anti-TNFα antibody, TGFA and IL-10; an anti-TNFα antibody, FGF1 and FGF2; an anti-TNFα antibody, FGF1 and FGF4; an anti-TNFα antibody, FGF1 and FGF7; an anti-TNFα antibody, FGF1 and FGF9; an anti-TNFα antibody, FGF1 and FGF19; an anti-TNFα antibody, FGF1 and SCF; an anti-TNFα antibody, FGF1 and PDGFA; an anti-TNFα antibody, FGF1 and PDGFB; an anti-TNFα antibody, FGF1 and PDGFC; an anti-TNFα antibody, FGF1 and VEGFA; an anti-TNFa antibody, FGF1 and VEGFB; an anti-TNFα antibody, FGF1 and VEGFC; an anti-TNFα antibody, FGF1 and VEGFD; an anti-TNFα antibody, FGF1 and TGFB1; an anti-TNFa antibody, FGF1 and IL-10; an anti-TNFα antibody, FGF2 and FGF4; an anti-TNFα antibody, FGF2 and FGF7; an anti-TNFα antibody, FGF2 and FGF9; an anti-TNFα antibody, FGF2 and FGF19; an anti-TNFα antibody, FGF2 and SCF; an anti-TNFα antibody, FGF2 and PDGFA; an anti-TNFα antibody, FGF2 and PDGFB; an anti-TNFa antibody, FGF2 and PDGFC; an anti-TNFα antibody, FGF2 and VEGFA; an anti-TNFα antibody, FGF2 and VEGFB; an anti-TNFα antibody, FGF2 and VEGFC; an anti-TNFα antibody, FGF2 and VEGFD; an anti-TNFα antibody, FGF2 and TGFB1; an anti-TNFα antibody, FGF2 and IL-10; an anti-TNFα antibody, FGF4 and FGF7; an anti-TNFα antibody, FGF4 and FGF9; an anti-TNFα antibody, FGF4 and FGF19; an anti-TNFα antibody, FGF4 and SCF; an anti-TNFα antibody, FGF4 and PDGFA; an anti-TNFα antibody, FGF4 and PDGFB; an anti-TNFα antibody, FGF4 and PDGFC; an anti-TNFα antibody, FGF4 and VEGFA; an anti-TNFα antibody, FGF4 and VEGFB; an anti-TNFα antibody, FGF4 and VEGFC; an anti-TNFα antibody, FGF4 and VEGFD; an anti-TNFα antibody, FGF4 and TGFB1; an anti-TNFα antibody, FGF4 and IL-10; an anti-TNFα antibody, FGF7 and FGF9; an anti-TNFα antibody, FGF7 and FGF19; an anti-TNFα antibody, FGF7 and SCF; an anti-TNFα antibody, FGF7 and PDGFA; an anti-TNFα antibody, FGF7 and PDGFB; an anti-TNFα antibody, FGF7 and PDGFC; an anti-TNFα antibody, FGF7 and VEGFA; an anti-TNFα antibody, FGF7 and VEGFB; an anti-TNFα antibody, FGF7 and VEGFC; an anti-TNFα antibody, FGF7 and VEGFD; an anti-TNFα antibody, FGF7 and TGFB1; an anti-TNFα antibody, FGF7 and IL-10; an anti-TNFα antibody, FGF9 and FGF19; an anti-TNFα antibody, FGF9 and SCF; an anti-TNFα antibody, FGF9 and PDGFA; an anti-TNFα antibody, FGF9 and PDGFB; an anti-TNFα antibody, FGF9 and PDGFC; an anti-TNFα antibody, FGF9 and VEGFA; an anti-TNFα antibody, FGF9 and VEGFB; an anti-TNFα antibody, FGF9 and VEGFC; an anti-TNFα antibody, FGF9 and VEGFD; an anti-TNFα antibody, FGF9 and TGFB1; an anti-TNFα antibody, FGF9 and IL-10; an anti-TNFα antibody, FGF19 and SCF; an anti-TNFα antibody, FGF19 and PDGFA; an anti-TNFα antibody, FGF19 and PDGFB; an anti-TNFα antibody, FGF19 and PDGFC; an anti-TNFα antibody, FGF19 and VEGFA; an anti-TNFα antibody, FGF19 and VEGFB; an anti-TNFα antibody, FGF19 and VEGFC; an anti-TNFα antibody, FGF19 and VEGFD; an anti-TNFα antibody, FGF19 and TGFB1; an anti-TNFα antibody, FGF19 and IL-10; an anti-TNFα antibody, SCF and PDGFA; an anti-TNFα antibody, SCF and PDGFB; an anti-TNFα antibody, SCF and PDGFC; an anti-TNFα antibody, SCF and VEGFA; an anti-TNFα antibody, SCF and VEGFB; an anti-TNFα antibody, SCF and VEGFC; an anti-TNFα antibody, SCF and VEGFD; an anti-TNFα antibody, SCF and TGFB1; an anti-TNFα antibody, SCF and IL-10; an anti-TNFα antibody, PDGFA and PDGFB; an anti-TNFα antibody, PDGFA and PDGFC; an anti-TNFα antibody, PDGFA and VEGFA; an anti-TNFα antibody, PDGFA and VEGFB; an anti-TNFα antibody, PDGFA and VEGFC; an anti-TNFα antibody, PDGFA and VEGFD; an anti-TNFα antibody, PDGFA and TGFB1; an anti-TNFα antibody, PDGFA and IL-10; an anti-TNFα antibody, PDGFB and PDGFC; an anti-TNFα antibody, PDGFB and VEGFA; an anti-TNFα antibody, PDGFB and VEGFB; an anti-TNFα antibody, PDGFB and VEGFC; an anti-TNFα antibody, PDGFB and VEGFD; an anti-TNFα antibody, PDGFB and TGFB1; an anti-TNFα antibody, PDGFB and IL-10; an anti-TNFα antibody, PDGFC and VEGFA; an anti-TNFα antibody, PDGFC and VEGFB; an anti-TNFα antibody, PDGFC and VEGFC; an anti-TNFα antibody, PDGFC and VEGFD; an anti-TNFα antibody, PDGFC and TGFB1; an anti-TNFα antibody, PDGFC and IL-10; an anti-TNFα antibody, VEGFA and VEGFB; an anti-TNFα antibody, VEGFA and VEGFC; an anti-TNFα antibody, VEGFA and VEGFD; an anti-TNFα antibody, VEGFA and TGFB1; an anti-TNFα antibody, VEGFA and IL-10; an anti-TNFα antibody, VEGFB and VEGFC; an anti-TNFα antibody, VEGFB and VEGFD; an anti-TNFα antibody, VEGFB and TGFB1; an anti-TNFα antibody, VEGFB and IL-10; an anti-TNFα antibody, VEGFC and VEGFD; an anti-TNFα antibody, VEGFC and TGFB1; an anti-TNFα antibody, VEGFC and IL-10; an anti-TNFα antibody, VEGFD and TGFB1; an anti-TNFα antibody, VEGFD and IL-10; an anti-TNFα antibody, TGFB1 and IL-10; and the like. In some instances, the combination of at least three markers can further include at least 1, 2, 3, 4, 5, 6, 7, 8, 9, 10, 11, 12, 13, 14,15, 16, 17, 18, 19, 20, 21, or 22 of the other markers selected from the second set of markers to form a proliferation phase marker score.

In certain instances, the presence or level of various markers such as an inflammatory phase marker, a growth factor marker, a repair factor marker, an anti-inflammatory marker, a proliferation phase marker, or a serology marker is detected at the level of mRNA expression with an assay such as, for example, a hybridization assay or an amplification-based assay.

In other instances, the presence or level of various markers such as an inflammatory phase marker, a growth factor marker, a repair factor marker, an anti-inflammatory marker, an anti-drug antibody (ADA), a proliferation phase marker, a serology marker, or an anti-TNFα antibody is detected at the level of protein expression using, for example, an immunoassay (*e.g*., ELISA or CEER), a homogeneous mobility shift assay (HMSA) or an immunohistochemical assay.

Suitable ELISA kits for determining the presence or level of a growth factor, an inflammatory marker, or an anti-inflammatory marker in a serum, plasma, saliva, or urine sample are available from, *e.g*., Antigenix America Inc. (Huntington Station, NY), Promega (Madison, WI), R&D Systems, Inc. (Minneapolis, MN), Invitrogen (Camarillo, CA), CHEMICON International, Inc. (Temecula, CA), Neogen Corp. (Lexington, KY), PeproTech (Rocky Hill, NJ), Alpco Diagnostics (Salem, NH), Pierce Biotechnology, Inc. (Rockford, IL), and/or Abazyme (Needham, MA).

In other instances, the presence or level of various markers such as an inflammatory phase marker, a growth factor marker, a repair factor marker, an anti-inflammatory marker, a proliferation phase marker, or a serology marker is detected using a multiplexed immunoarray, such as a Collaborative Enzyme Enhanced Reactive ImmunoAssay (CEER), also known as the Collaborative Proximity Immunoassay (COPIA). CEER is described in the following patent documents which are herein incorporated by reference in their entirety for all purposes: PCT Publication Nos. WO 2008/036802, WO 2009/012140, WO 2009/108637, WO 2010/132723, WO 2011/008990, WO 2011/050069; WO 2012/088337; WO 2012/119113; and WO 2013/033623.

Suitable anti-repair factor antibodies useful in determining the level of a selected growth factor in a sample include, without limitation, an antibody that recognizes (binds to, forms a complex with, is specific for) a selected growth factor protein, a growth factor polypeptide having substantially the same amino acid sequence as the selected growth factor protein, or a fragment thereof such as an immunoreactive fragment thereof. A growth factor polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a growth factor protein, with the amino acid identity determined using a sequence alignment program such as CLUSTALW. Suitable antibodies for determining the level of a growth factor are available from, *e.g*., Promega (Madison, WI), R&D Systems, Inc. (Minneapolis, MN), Invitrogen (Camarillo, CA), CHEMICON International, Inc. (Temecula, CA), Abcam (Cambridge, MA), Santa Cruz Biotechnology (Santa Cruz, CA), and Dako (Carpinteria, CA).

### C. Inflammatory Phase Markers

In certain aspects, any of a variety of inflammatory phase markers, including but not limited to biochemical markers, serological markers, protein markers, and/or other clinical characteristics, are useful in the methods of the present invention. In particular embodiments, the methods herein facilitate predicting the likelihood of mucosal healing and monitoring the progression of mucosal healing based upon one or more inflammatory phase markers. In other embodiments, the methods herein facilitate selecting therapy, optimizing therapy, reducing toxicity, and/or monitoring the efficacy of therapeutic treatment with one or more therapeutic agents such as biologics (*e.g.,* anti-TNF drugs). In preferred embodiments, the methods herein utilize the determination of an inflammatory phase marker score based upon one or more (a plurality of) inflammatory phase markers (*e.g.,* alone or in combination with biomarkers from other categories) to aid or assist in predicting the likelihood of mucosal healing, monitoring the progression of mucosal healing, predicting disease course, selecting an appropriate anti-TNF drug therapy, optimizing anti-TNF drug therapy, reducing toxicity associated with anti-TNF drug therapy, and/or monitoring the efficacy of therapeutic treatment with an anti-TNF drug.

Non-limiting examples of inflammatory phase markers include cytokines, chemokines, acute phase proteins, cellular adhesion molecules, S100 proteins, serology markers, and/or other inflammatory markers.

Inflammatory phase markers that can be used to establish a marker score include, but are not limited to, at least one or a plurality (*e.g*., at least 2, 3, 4, 5, 6, 7, 8, 9, 10, 11, 12, 13, 14,15, 16, 17, 18, 19, 20, or 21, such as, *e.g.,* a panel or an array) of the following markers: TNF-α, IL-12p70, IL-1β, IL-2, IL-6, IL8, SDF-1, GM-CSF, IL-13, IFN-γ, SAA, CRP, ICAM, VCAM, TWEAK, ASCA-A, ASCA-G, Cbir, Fla2, FlaX, OmpC, and combinations thereof. In preferred embodiments, the inflammatory phase markers include one or more of GM-CSF, IL-2, VCAM, and combinations thereof.

### 1. Cytokines

The determination of the presence or level of at least one cytokine or chemokine in a sample is particularly useful in the present invention. As used herein, the term "cytokine" includes any of a variety of polypeptides or proteins secreted by immune cells that regulate a range of immune system functions and encompasses small cytokines such as chemokines. The term "cytokine" also includes adipocytokines, which comprise a group of cytokines secreted by adipocytes that function, for example, in the regulation of body weight, hematopoiesis, angiogenesis, wound healing, insulin resistance, the immune response, and the inflammatory response.

In certain embodiments, the presence or level of at least one cytokine including, but not limited to, granulocyte-macrophage colony-stimulating factor (GM-CSF), IFN-γ, IL-1β, IL-2, IL-6, IL-8, TNF-α, soluble tumor necrosis factor-α receptor II (sTNF RII), TNF-related weak inducer of apoptosis (TWEAK), osteoprotegerin (OPG), IFN-α, IFN-β, IL-1α, IL-1 receptor antagonist (IL-1ra), IL-4, IL-5, soluble IL-6 receptor (sIL-6R), IL-7, IL-9, IL-12, IL-13, IL-15, IL-17, IL-23, and IL-27 is determined in a sample.

In certain other embodiments, the presence or level of at least one chemokine such as, for example, CXCL1/GRO1/GROα, CXCL2/GRO2, CXCL3/GRO3, CXCL4/PF-4, CXCL5/ENA-78, CXCL6/GCP-2, CXCL7/NAP-2, CXCL9/MIG, CXCL10/IP-10, CXCL11/I-TAC, CXCL12/SDF-1, CXCL13/BCA-1, CXCL14/BRAK, CXCL15, CXCL16, CXCL17/DMC, CCL1, CCL2/MCP-1, CCL3/MIP-1α, CCL4/MIP-1β, CCL5/RANTES, CCL6/C10, CCL7/MCP-3, CCL8/MCP-2, CCL9/CCL10, CCL11/Eotaxin, CCL12/MCP-5, CCL13/MCP-4, CCL14/HCC-1, CCL15/MIP-5, CCL16/LEC, CCL17/TARC, CCL18/MIP-4, CCL19/MIP-3β, CCL20/MIP-3α, CCL21/SLC, CCL22/MDC, CCL23/MPIF1, CCL24/Eotaxin-2, CCL25/TECK, CCL26/Eotaxin-3, CCL27/CTACK, CCL28/MEC, CL1, CL2, and CX₃CL1 is determined in a sample. In certain further embodiments, the presence or level of at least one adipocytokine including, but not limited to, leptin, adiponectin, resistin, active or total plasminogen activator inhibitor-1 (PAI-1), visfatin, and retinol binding protein 4 (RBP4) is determined in a sample. Preferably, the presence or level of SDF-1, GM-CSF, IFN-γ, IL-1 β, IL-2, IL-6, IL-8, TNF-α, sTNF RII, and/or other cytokines or chemokines is determined.

In certain instances, the presence or level of a particular cytokine or chemokine marker is detected at the level of mRNA expression with an assay such as, for example, a hybridization assay or an amplification-based assay. In certain other instances, the presence or level of a particular cytokine or chemokine is detected at the level of protein expression using, for example, an immunoassay (*e.g.,* ELISA), an immunohistochemical assay, or a multiplexed immunoarray, such as a Collaborative Enzyme Enhanced Reactive ImmunoAssay (CEER), also known as the Collaborative Proximity Immunoassay (COPIA). Suitable ELISA kits for determining the presence or level of a cytokine or chemokine of interest in a serum, plasma, saliva, or urine sample are available from, *e.g.,* R&D Systems, Inc. (Minneapolis, MN), Neogen Corp. (Lexington, KY), Alpco Diagnostics (Salem, NH), Assay Designs, Inc. (Ann Arbor, MI), BD Biosciences Pharmingen (San Diego, CA), Invitrogen (Camarillo, CA), Calbiochem (San Diego, CA), CHEMICON International, Inc. (Temecula, CA), Antigenix America Inc. (Huntington Station, NY), QIAGEN Inc. (Valencia, CA), Bio-Rad Laboratories, Inc. (Hercules, CA), and/or Bender MedSystems Inc. (Burlingame, CA).

The human IL-1β polypeptide sequence is set forth in, *e.g*., Genbank Accession No. NP_000567. The human IL-1β mRNA (coding) sequence is set forth in, *e.g*., Genbank Accession No. NM_000576. One skilled in the art will appreciate that IL-1β is also known as IL1F2 and IL-1beta.

The human IL-2 polypeptide sequence is set forth in, *e.g*., Genbank Accession No. NP_000577. The human IL-2 mRNA (coding) sequence is set forth in, *e.g*., Genbank Accession No. NM_000586. One skilled in the art will appreciate that IL-2 is also known as TCGF and lymphokine.

The human IL-6 polypeptide sequence is set forth in, *e.g*., Genbank Accession No. NP_000591. The human IL-6 mRNA (coding) sequence is set forth in, *e.g*., Genbank Accession No. NM_000600. One skilled in the art will appreciate that IL-6 is also known as interferon beta 2 (IFNB2), HGF, HSF, and BSF2.

The human IL-8 polypeptide sequence is set forth in, *e.g*., Genbank Accession No. NP_000575. The human IL-8 mRNA (coding) sequence is set forth in, *e.g*., Genbank Accession No. NM_000584. One skilled in the art will appreciate that IL-8 is also known as CXCL8, K60, NAF, GCP1, LECT, LUCT, NAP1, 3-10C, GCP-1, LYNAP, MDNCF, MONAP, NAP-1, SCYB8, TSG-1, AMCF-I, and b-ENAP.

The human GM-CSF polypeptide sequence is set forth in, *e.g*., Genbank Accession No. NP_000749. The human GM-CSF mRNA (coding) sequence is set forth in, *e.g*., Genbank Accession No. NM_000758. One skilled in the art will appreciate that GM-CSF is also known as granulocyte-macrophage colony stimulating factor, colony stimulating factor 2 (granulocyte-macrophage), GSF2 and GMCSF.

The human IFNγ polypeptide sequence is set forth in, *e.g*., Genbank Accession No. NP_000610. The human IFNγ mRNA (coding) sequence is set forth in, *e.g*., Genbank Accession No. NM_000619. One skilled in the art will appreciate that GM-CSF is also known as interferon gamma, IFNG, IFG, IFI, and IFN gamma.

The human TNFα polypeptide sequence is set forth in, *e.g*., Genbank Accession No. NP_000585. The human TNFα mRNA (coding) sequence is set forth in, *e.g*., Genbank Accession No. NM_000594. One skilled in the art will appreciate that TNFα is also known as tumor necrosis factor, TNF, DIF, TNF-alpha, TNFA, and TNFSF2.

The human TNF-related weak inducer of apoptosis (TWEAK) polypeptide sequence is set forth in, *e.g*., Genbank Accession No. NP_003800.1. The human TWEAK mRNA (coding) sequence is set forth in, *e.g*., Genbank Accession No. NM_003809.2. One skilled in the art will appreciate that TWEAK is also known as TNF12, APO3 ligand, APO3L, DR3LG, and UNQ181/PRO207.

### 2. Acute Phase Proteins

The determination of the presence or level of one or more acute-phase proteins in a sample is also useful in the present invention. Acute-phase proteins are a class of proteins whose plasma concentrations increase (positive acute-phase proteins) or decrease (negative acute-phase proteins) in response to inflammation. This response is called the acute-phase reaction (also called acute-phase response). Examples of positive acute-phase proteins include, but are not limited to, C-reactive protein (CRP), D-dimer protein, mannose-binding protein, alpha 1 -antitrypsin, alpha 1-antichymotrypsin, alpha 2-macroglobulin, fibrinogen, prothrombin, factor VIII, von Willebrand factor, plasminogen, complement factors, ferritin, serum amyloid P component, serum amyloid A (SAA), orosomucoid (alpha 1-acid glycoprotein, AGP), ceruloplasmin, haptoglobin, and combinations thereof. Non-limiting examples of negative acute-phase proteins include albumin, transferrin, transthyretin, transcortin, retinol-binding protein, and combinations thereof. Preferably, the presence or level of CRP and/or SAA is determined.

In certain instances, the presence or level of a particular acute-phase protein is detected at the level of mRNA expression with an assay such as, for example, a hybridization assay or an amplification-based assay. In certain other instances, the presence or level of a particular acute-phase protein is detected at the level of protein expression using, for example, an immunoassay (*e.g.,* ELISA), an immunohistochemical assay, or a multiplexed immunoarray, such as a Collaborative Enzyme Enhanced Reactive ImmunoAssay (CEER), also known as the Collaborative Proximity Immunoassay (COPIA). For example, a sandwich colorimetric ELISA assay available from Alpco Diagnostics (Salem, NH) can be used to determine the level of CRP in a serum, plasma, urine, or stool sample. Similarly, an ELISA kit available from Biomeda Corporation (Foster City, CA) can be used to detect CRP levels in a sample. Other methods for determining CRP levels in a sample are described in, *e.g.,* U.S. Patent Nos. 6,838,250; 6,406,862; 7,439,019; and U.S. Patent Publication No. 20060019410. Additional methods for determining CRP levels include, *e.g.,* immunoturbidimetry assays, rapid immunodiffusion assays, and visual agglutination assays. Suitable ELISA kits for determining the presence or level of SAA in a sample such as serum, plasma, saliva, urine, or stool are available from, *e.g.,* Antigenix America Inc. (Huntington Station, NY), Abazyme (Needham, MA), USCN Life (Missouri City, TX), and/or U.S. Biological (Swampscott, MA).

C-reactive protein (CRP) is a protein found in the blood in response to inflammation (an acute-phase protein). CRP is typically produced by the liver and by fat cells (adipocytes). It is a member of the pentraxin family of proteins. The human CRP polypeptide sequence is set forth in, *e.g*., Genbank Accession No. NP_000558. The human CRP mRNA (coding) sequence is set forth in, *e.g*., Genbank Accession No. NM_000567. One skilled in the art will appreciate that CRP is also known as PTX1, MGC88244, and MGC149895.

Serum amyloid A (SAA) proteins are a family of apolipoproteins associated with high-density lipoprotein (HDL) in plasma. Different isoforms of SAA are expressed constitutively (constitutive SAAs) at different levels or in response to inflammatory stimuli (acute phase SAAs). These proteins are predominantly produced by the liver. The conservation of these proteins throughout invertebrates and vertebrates suggests SAAs play a highly essential role in all animals. Acute phase serum amyloid A proteins (A-SAAs) are secreted during the acute phase of inflammation. The human SAA polypeptide sequence is set forth in, *e.g.,* Genbank Accession No. NP_000322. The human SAA mRNA (coding) sequence is set forth in, *e.g.,* Genbank Accession No. NM_000331. One skilled in the art will appreciate that SAA is also known as PIG4, TP53I4, MGC111216, and SAA1.

### 3. Immunoglobulin Proteins

The determination of the presence or level of one or more immunoglobulin superfamily cellular adhesion molecules in a sample is also useful in the present invention. As used herein, the term "immunoglobulin superfamily cellular adhesion molecule" (IgSF CAM) includes any of a variety of polypeptides or proteins located on the surface of a cell that have one or more immunoglobulin-like fold domains, and which function in intercellular adhesion and/or signal transduction. In many cases, IgSF CAMs are transmembrane proteins. Non-limiting examples of IgSF CAMs include Neural Cell Adhesion Molecules (NCAMs; *e.g*., NCAM-120, NCAM-125, NCAM-140, NCAM-145, NCAM-180, NCAM-185, *etc.*)*,* Intercellular Adhesion Molecules (ICAMs, *e.g.,* ICAM-1, ICAM-2, ICAM-3, ICAM-4, and ICAM-5), Vascular Cell Adhesion Molecule-1 (VCAM-1), Platelet-Endothelial Cell Adhesion Molecule-1 (PECAM-1), L1 Cell Adhesion Molecule (L1CAM), cell adhesion molecule with homology to L1CAM (close homolog of L1) (CHL1), sialic acid binding Ig-like lectins (SIGLECs; *e.g*., SIGLEC-1, SIGLEC-2, SIGLEC-3, SIGLEC-4, *etc.*)*,* Nectins (*e.g.,* Nectin-1, Nectin-2, Nectin-3, *etc.*)*,* and Nectin-like molecules (*e.g*., Necl-1, Necl-2, Necl-3, Necl-4, and Necl-5). Preferably, the presence or level of ICAM-1 and/or VCAM-1 is determined.

ICAM-1 (ICAM) is a transmembrane cellular adhesion protein that is continuously present in low concentrations in the membranes of leukocytes and endothelial cells. Upon cytokine stimulation, the concentrations greatly increase. ICAM-1 can be induced by IL-1 and TNFα and is expressed by the vascular endothelium, macrophages, and lymphocytes. In IBD, proinflammatory cytokines cause inflammation by upregulating expression of adhesion molecules such as ICAM-1 and VCAM-1. The increased expression of adhesion molecules recruit more lymphocytes to the infected tissue, resulting in tissue inflammation (*see,* Goke et al., J., Gastroenterol., 32:480 (1997); and Rijcken et al., Gut, 51:529 (2002)). ICAM-1 is encoded by the intercellular adhesion molecule 1 gene (ICAM1; Entrez GeneID:3383; Genbank Accession No. NM_000201) and is produced after processing of the intercellular adhesion molecule 1 precursor polypeptide (Genbank Accession No. NP_000192).

VCAM-1 (VCAM) is a transmembrane cellular adhesion protein that mediates the adhesion of lymphocytes, monocytes, eosinophils, and basophils to vascular endothelium. Upregulation of VCAM-1 in endothelial cells by cytokines occurs as a result of increased gene transcription (*e.g*., in response to tumor necrosis factor-alpha (TNFα) and Interleukin-1 (IL-1)). VCAM-1 is encoded by the vascular cell adhesion molecule 1 gene (VCAM1; Entrez GeneID:7412) and is produced after differential splicing of the transcript (Genbank Accession No. NM_001078 (variant 1) or NM_080682 (variant 2)), and processing of the precursor polypeptide splice isoform (Genbank Accession No. NP_001069 (isoform a) or NP_542413 (isoform b)).

In certain instances, the presence or level of an IgSF CAM is detected at the level of mRNA expression with an assay such as, for example, a hybridization assay or an amplification-based assay. In certain other instances, the presence or level of an IgSF CAM is detected at the level of protein expression using, for example, an immunoassay (e.g., ELISA), an immunohistochemical assay, or a multiplexed immunoarray, such as a Collaborative Enzyme Enhanced Reactive ImmunoAssay (CEER), also known as the Collaborative Proximity Immunoassay (COPIA). Suitable antibodies and/or ELISA kits for determining the presence or level of ICAM-1 and/or VCAM-1 in a sample such as a tissue sample, biopsy, serum, plasma, saliva, urine, or stool are available from, *e.g*., Invitrogen (Camarillo, CA), Santa Cruz Biotechnology, Inc. (Santa Cruz, CA), and/or Abcam Inc. (Cambridge, MA).

### 4. Anti-Inflammatory Markers

In certain embodiments, a variety of anti-inflammatory markers are particularly useful in the methods of the present invention. In particular embodiments, the methods herein utilize the determination of an inflammatory phase marker score based upon one or more (a plurality of) anti-inflammatory markers (*e.g*., alone or in combination with biomarkers from other categories) to aid or assist in predicting the likelihood of mucosal healing, monitoring the progression of mucosal healing, predicting disease course, selecting an appropriate anti-TNF drug therapy, optimizing anti-TNF drug therapy, reducing toxicity associated with anti-TNF drug therapy, and/or monitoring the efficacy of therapeutic treatment with an anti-TNF drug.

In certain instances, the presence or level of a particular anti-inflammatory marker is detected at the level of mRNA expression with an assay such as, for example, a hybridization assay or an amplification-based assay. In certain other instances, the presence or level of a particular anti-inflammatory marker is detected at the level of protein expression using, for example, an immunoassay (*e.g*., ELISA), an immunohistochemical assay, or a multiplexed immunoarray, such as a Collaborative Enzyme Enhanced Reactive ImmunoAssay (CEER), also known as the Collaborative Proximity Immunoassay (COPIA).

The human IL-12p70 polypeptide is a heterodimer made up of two subunits of IL-12 proteins: one is 40kDa (IL-12p40) and one is 35kDa (IL-12p35). Suitable ELISA kits for determining the presence or level of IL-12p70 in a serum, plasma, saliva, or urine sample are available from, *e.g.,* Gen-Probe Diaclone SAS (France), Abazyme (Needham, MA), BD Biosciences Pharmingen (San Diego, CA), Cell Sciences (Canton, MA), eBioscience (San Diego, CA), Invitrogen (Camarillo, CA), R&D Systems, Inc. (Minneapolis, MN), and Thermo Scientific Pierce Protein Research Products (Rockford, IL).

### 5. Serology Markers

In certain aspects, the methods of the present invention utilize the determination of an inflammatory phase marker score based upon one or more (a plurality of) serological immune markers (*e.g.,* alone or in combination with biomarkers from other categories) to aid or assist in predicting the likelihood of mucosal healing, monitoring the progression of mucosal healing, predicting disease course, selecting an appropriate anti-TNF drug therapy, optimizing anti-TNF drug therapy, reducing toxicity associated with anti-TNF drug therapy, and/or monitoring the efficacy of therapeutic treatment with an anti-TNF drug.

Non-limiting examples of serological immune markers suitable for use in the present invention include anti-neutrophil antibodies, *anti-Saccharomyces cerevisiae* antibodies, and/or other anti-microbial antibodies. Mucosal healing can also result in a decrease in the antibody titer of antibodies to bacterial antigens such as, *e.g.,* OmpC, flagellins (cBir-1, Fla-A, Fla-X, *etc.*), I2, and others (pANCA, ASCA, *etc.*)*.*

The determination of ASCA (*e.g*., ASCA-IgA and/or ASCA-IgG) levels in a sample is useful in the present invention. As used herein, the term *"anti-Saccharomyces cerevisiae* immunoglobulin A" or "ASCA-IgA" includes antibodies of the immunoglobulin A isotype that react specifically with *S. cerevisiae.* Similarly, the term *"anti-Saccharomyces cerevisiae* immunoglobulin G" or "ASCA-IgG" includes antibodies of the immunoglobulin G isotype that react specifically with *S. cerevisiae.*

The determination of whether a sample is positive for ASCA-IgA or ASCA-IgG is made using an antigen specific for ASCA. Such an antigen can be any antigen or mixture of antigens that is bound specifically by ASCA-IgA and/or ASCA-IgG. Although ASCA antibodies were initially characterized by their ability to bind *S*. *cerevisiae,* those of skill in the art will understand that an antigen that is bound specifically by ASCA can be obtained from *S. cerevisiae* or from a variety of other sources so long as the antigen is capable of binding specifically to ASCA antibodies. Accordingly, exemplary sources of an antigen specific for ASCA, which can be used to determine the levels of ASCA-IgA and/or ASCA-IgG in a sample, include, without limitation, whole killed yeast cells such as *Saccharomyces* or *Candida* cells; yeast cell wall mannan such as phosphopeptidomannan (PPM); oligosachharides such as oligomannosides; neoglycolipids; anti-ASCA idiotypic antibodies; and the like. Different species and strains of yeast, such as *S*. *cerevisiae* strain Su1, Su2, CBS 1315, or BM 156, or *Candida albicans* strain VW32, are suitable for use as an antigen specific for ASCA-IgA and/or ASCA-IgG. Purified and synthetic antigens specific for ASCA are also suitable for use in determining the levels of ASCA-IgA and/or ASCA-IgG in a sample. Examples of purified antigens include, without limitation, purified oligosaccharide antigens such as oligomannosides. Examples of synthetic antigens include, without limitation, synthetic oligomannosides such as those described in U.S. Patent Publication No. 20030105060, *e.g*., D-Man β(1-2) D-Man β(1-2) D-Man β(1-2) D-Man-OR, D-Man α(1-2) D-Man α(1-2) D-Man α(1-2) D-Man-OR, and D-Man α(1-3) D-Man α(1-2) D-Man α(1-2) D-Man-OR, wherein R is a hydrogen atom, a C₁ to C₂₀ alkyl, or an optionally labeled connector group.

Preparations of yeast cell wall mannans, *e.g*., PPM, can be used in determining the levels of ASCA-IgA and/or ASCA-IgG in a sample. Such water-soluble surface antigens can be prepared by any appropriate extraction technique known in the art, including, for example, by autoclaving, or can be obtained commercially (*see, e.g.,* Lindberg et al., Gut, 33:909-913 (1992)). The acid-stable fraction of PPM is also useful in the present invention (Sendid et al., Clin. Diag. Lab. Immunol., 3:219-226 (1996)). An exemplary PPM that is useful in determining ASCA levels in a sample is derived from *S. uvarum* strain ATCC #38926.

Purified oligosaccharide antigens such as oligomannosides can also be useful in determining the levels of ASCA-IgA and/or ASCA-IgG in a sample. The purified oligomannoside antigens are preferably converted into neoglycolipids as described in, for example, Faille et al., Eur. J. Microbiol. Infect. Dis., 11:438-446 (1992). One skilled in the art understands that the reactivity of such an oligomannoside antigen with ASCA can be optimized by varying the mannosyl chain length (Frosh et al., Proc Natl. Acad. Sci. USA, 82:1194-1198 (1985)); the anomeric configuration (Fukazawa et al., In "Immunology of Fungal Disease," E. Kurstak (ed.), Marcel Dekker Inc., New York, pp. 37-62 (1989); Nishikawa et al., Microbiol. Immunol., 34:825-840 (1990); Poulain et al., Eur. J. Clin. Microbiol., 23:46-52 (1993); Shibata et al., Arch. Biochem. Biophys., 243:338-348 (1985); Trinel et al., Infect. Immun., 60:3845-3851 (1992)); or the position of the linkage (Kikuchi et al., Planta, 190:525-535 (1993)).

Suitable oligomannosides for use in the methods of the present invention include, without limitation, an oligomannoside having the mannotetraose Man(1-3) Man(1-2) Man(1-2) Man. Such an oligomannoside can be purified from PPM as described in, *e.g.,* Faille *et al., supra.* An exemplary neoglycolipid specific for ASCA can be constructed by releasing the oligomannoside from its respective PPM and subsequently coupling the released oligomannoside to 4-hexadecylaniline or the like.

The determination of anti-OmpC antibody levels in a sample is also useful in the present invention. As used herein, the term "anti-outer membrane protein C antibody" or "anti-OmpC antibody" includes antibodies directed to a bacterial outer membrane porin as described in, *e.g.,* PCT Patent Publication No. WO 01/89361. The term "outer membrane protein C" or "OmpC" refers to a bacterial porin that is immunoreactive with an anti-OmpC antibody.

The level of anti-OmpC antibody present in a sample from an individual can be determined using an OmpC protein or a fragment thereof such as an immunoreactive fragment thereof. Suitable OmpC antigens useful in determining anti-OmpC antibody levels in a sample include, without limitation, an OmpC protein, an OmpC polypeptide having substantially the same amino acid sequence as the OmpC protein, or a fragment thereof such as an immunoreactive fragment thereof. As used herein, an OmpC polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with an OmpC protein, with the amino acid identity determined using a sequence alignment program such as CLUSTALW. Such antigens can be prepared, for example, by purification from enteric bacteria such as *E. coli,* by recombinant expression of a nucleic acid such as Genbank Accession No. K00541, by synthetic means such as solution or solid phase peptide synthesis, or by using phage display.

The determination of anti-flagellin antibody levels in a sample is also useful in the present invention. As used herein, the term "anti-flagellin antibody" includes antibodies directed to a protein component of bacterial flagella as described in, *e.g.,* PCT Patent Publication Nos. WO 03/053220 and WO 07/087576; and U.S. Patent Nos. 7,361,733 and 7,868,139. The term "flagellin" refers to a bacterial flagellum protein that is immunoreactive with an anti-flagellin antibody. Microbial flagellins are proteins found in bacterial flagellum that arrange themselves in a hollow cylinder to form the filament.

The level of anti-flagellin antibody present in a sample from an individual can be determined using a flagellin protein or a fragment thereof such as an immunoreactive fragment thereof. Suitable flagellin antigens useful in determining anti-flagellin antibody levels in a sample include, without limitation, a flagellin protein such as CBir-1 flagellin, flagellin X (FlaX), flagellin A, flagellin B, Fla2 (A4-Fla2), FliC, fragments thereof such as immunoreactive fragments thereof, flagellin polypeptides having substantially the same amino acid sequence as a flagellin protein, and combinations thereof. As used herein, a flagellin polypeptide generally describes polypeptides having an amino acid sequence with greater than about 50% identity, preferably greater than about 60% identity, more preferably greater than about 70% identity, still more preferably greater than about 80%, 85%, 90%, 95%, 96%, 97%, 98%, or 99% amino acid sequence identity with a naturally-occurring flagellin protein, with the amino acid identity determined using a sequence alignment program such as CLUSTALW. Such flagellin antigens can be prepared, *e.g.,* by purification from bacterium such as *Helicobacter Bilis, Helicobacter mustelae, Helicobacter pylori, Butyrivibrio fibrisolvens,* and bacterium found in the cecum, by recombinant expression of a nucleic acid encoding a flagellin antigen, by synthetic means such as solution or solid phase peptide synthesis, or by using phage display.

### D. Proliferation Phase Markers

In certain aspects, the determination of the presence and/or level of at least one or more proliferation phase markers (*e.g.,* repair factor markers, anti-inflammatory markers, anti-TNFα antibody) in a sample is useful in the present invention. As used herein, the term "repair factor" includes any of a variety of peptides, polypeptides, or proteins capable of stimulating cellular proliferation and/or cellular differentiation.

In particular embodiments, at least one or a plurality (*e.g.,* at least 2, 3, 4, 5, 6, 7, 8, 9, 10, 11, 12, 13, 14, 15, 16, 17, 18, 19, 20, 21, 22, 23, or 24, such as, *e.g.,* a panel or an array) of the following repair factor markers can be detected or measured (*e.g.,* alone or in combination with biomarkers from other categories) and used to form a proliferation phase marker score to aid or assist in predicting the likelihood of mucosal healing, monitoring the progression of mucosal healing, predicting disease course, and/or to improve the accuracy of selecting therapy, optimizing therapy, reducing toxicity, and/or monitoring the efficacy of therapeutic treatment to anti-TNF drug therapy: EGF, AREG, EREG, HBEGF, HGF, HRGB, BTC, TGFA, FGF1, FGF2, FGF4, FGF7, FGF9, FGF19, SCF, PDGFA, PDGFB, PDGFC, VEGFA, VEGFB, VEGFC, VEGFD, TGFB1, TWEAK, and combinations thereof.

Non-limiting examples of repair factors include epidermal growth factor (EGF), heparin binding epidermal growth factor (HB-EGF), amphiregulin (AREG), betacellulin (BTC), epiregulin (EREG), heregulin and variants thereof (HRGα, HRGβ1, HRGβ2, HRGβ3, HRGγ), neuregulin 1 (NRG1) and isoforms thereof (*e.g.* type I NRG1 (also known as neu differentiation factor (NDF), heregulin, or acetylcholine receptor inducing activity), type II NRG1 (also known as glial growth factor 2 (GGF2)), type III NRG1 (also knowns as sensory and motor neuron-derived factor (SMDF)), type IV NRG1, type V NRG1, type VI NRG1), neuregulin 2 (NRG2), neuregulin 3 (NRG3), neuregulin 4 (NRG4), vascular endothelial growth factors (VEGF-A, VEGF-B, VEGF-C, VEGF-D), platelet derived growth factors (PDGF-A, PDGF-B, PDGF-C, PDGF-D), fibroblast growth factors (FGF1, FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF22, FGF23, *etc.*), stem cell factor (SCF), transforming growth factor (TGFα, TGFβ (TGFB)), endothelin 3 (EDN3), hepatocye growth factor (HGF), insulin growth factor-1 (IGF-1), interleukin 10 (IL-10) and combinations thereof.

In other embodiments, repair factors also include platelet-derived growth factor (PDGF), soluble fms-like tyrosine kinase 1 (sFlt1), placenta growth factor (PIGF, PLGF or PGF), pigment epithelium-derived factor (PEDF, also known as SERPINF1), endothelin-1 (ET-1), keratinocyte growth factor (KGF), bone morphogenetic proteins (*e.g.,* BMP1-BMP15), platelet-derived growth factor (PDGF), nerve growth factor (NGF), β-nerve growth factor (β-NGF), neurotrophic factors (*e.g.,* brain-derived neurotrophic factor (BDNF), neurotrophin 3 (NT3), neurotrophin 4 (NT4), *etc.),* growth differentiation factor-9 (GDF-9), granulocyte-colony stimulating factor (G-CSF), myostatin (GDF-8), erythropoietin (EPO), thrombopoietin (TPO), and combinations thereof.

The human amphiregulin (AREG) polypeptide sequence is set forth in, *e.g.,* NCBI Accession No. AAA51781.1. The human AREG mRNA (coding) sequence is set forth in, *e.g.,* Genbank Accession Nos. NM_001657.2 and XM_001125684.3. One skilled in the art will appreciate that AREG is also known as AR, colorectum cell-derived growth factor, CRDGF, SDGF, and AREGB.

The human epiregulin (EREG) polypeptide sequence is set forth in, *e.g.,* NCBI Accession No. BAA22146.1. The human EREG mRNA (coding) sequence is set forth in, *e.g.,* Genbank Accession No. NM_001432.2. One skilled in the art will appreciate that EREG is also known as EPR.

The human heparin-binding EGF-like growth factor (HB-EGF) polypeptide sequence is set forth in, *e.g.,* NCBI Accession No. AAA35956.1. The human HB-EGF mRNA (coding) sequence is set forth in, *e.g.,* Genbank Accession No. NM_001945.2. One skilled in the art will appreciate that HB-EGF is also known as diphtheria toxin receptor, DT-R, HBEGF, DTR, DTS, and HEGFL.

The human hepatocyte growth factor (HGF) polypeptide sequence is set forth in, *e.g.,* ncbi Accession No. NP_000592.3. The human HGF mRNA (coding) sequence is set forth in, *e.g.,* Genbank Accession Nos. NM_000601.4, NM_001010931.1, NM_001010932.1, NM_001010933.1 and NM_001010934.1. One skilled in the art will appreciate that HGF is also known as scatter factor, SF, HPTA and hepatopoietin-A. One of skill will also appreciate that HGF includes all isoform variants.

The human heregulin β (HRGβ) polypeptide sequence is set forth in, *e.g.,* NCBI Accession No. ABY70644.1. One of skill will also appreciate that HRGβ includes all isoform variants.

The human betacellulin (BTC) polypeptide sequence is set forth in, *e.g.,* NCBI Accession No. AAB25452.1. The human BTC mRNA (coding) sequence is set forth in, *e.g.,* Genbank Accession No. NM_001729.2. One skilled in the art will appreciate that BTC includes all isoform variants.

The human epidermal growth factor (EGF) polypeptide sequence is set forth in, *e.g.,* Genbank Accession No. AAI13462.1. The human EGF mRNA (coding) sequence is set forth in, *e.g.,* Genbank Accession Nos. NM_001963.4 and NM_001178131.1.. One skilled in the art will appreciate that EGF is also known as beta-urogastrone, urogastrone, URG, and HOMG4. One skilled in the art will appreciate that EGF includes all isoform variants.

The human transforming growth factor alpha (TGF-α) polypeptide sequence is set forth in, *e.g.,* NCBI Accession No. AAA61159.1. The human TGF-α mRNA (coding) sequence is set forth in, *e.g.,* Genbank Accession Nos. NM_003236.3 and NM_001099691.2. One skilled in the art will appreciate that TGF-α includes all isoform variants. One skilled in the art will also appreciate that TGF-α is also known as TGFA, EGF-like TGF, ETGF, and TGF type 1.

The human transforming growth factor alpha (TGF-β or TGFB) polypeptide sequence is set forth in, *e.g.,* NCBI Accession No. AAH00125.1. One skilled in the art will appreciate that TGF- β includes all isoform variants.

The human vascular endothelial growth factor (VEGF-A) polypeptide sequence is set forth in, *e.g.,* NCBI Accession No. AAA35789.1. The human VEGF-A mRNA (coding) sequence is set forth in, *e.g.,* Genbank Accession No. NM_001025366, NM_001025367, NM_001025368, NM_001025369, NM_001025370, NM_001033756, and NM_003376. One skilled in the art will appreciate that VEGF-A is also known as VPF, VEGFA, VEGF, and MGC70609. One skilled in the art will appreciate that VEGF-A includes all isoform variants.

The human vascular endothelial growth factor (VEGF-B) polypeptide sequence is set forth in, *e.g.,* NCBI Accession No. AAH08818.1. The human VEGF-B mRNA (coding) sequence is set forth in, *e.g.,* Genbank Accession Nos. NM_001243733 and NM_003377. One skilled in the art will appreciate that VEGF-B is also known as VEGFB, VEGF-related factor, and VRF. One skilled in the art will appreciate that VEGF-B includes all isoform variants.

The human vascular endothelial growth factor (VEGF-C) polypeptide sequence is set forth in, *e.g.,* NCBI Accession No. AAH63685.1. The human VEGF-C mRNA (coding) sequence is set forth in, *e.g.,* Genbank Accession No. NM_005429. One skilled in the art will appreciate that VEGF-C is also known as VEGFC, Flt4 ligand, Flt4-L, VRP and vascular endothelial growth factor-related protein. One skilled in the art will appreciate that VEGF-C includes all isoform variants.

The human vascular endothelial growth factor (VEGF-D) polypeptide sequence is set forth in, *e.g.,* NCBI Accession No. NP_004460.1. One skilled in the art will appreciate that VEGF-D is also known as VEGFD, c-Fos induced growth factor or FIGF. One skilled in the art will appreciate that VEGF-D includes all isoform variants.

The human platelet-dervied growth factor subunit A (PDGF-A) polypeptide sequence is set forth in, *e.g.,* NCBI Accession No. AAI09247.1. One skilled in the art will appreciate that PDGF-A is also known as PDGFA, PDGF subunit A, PDGF-1, platelet-dervied growth factor A chain, and platelet-dervied growth factor alpha polypeptide. One skilled in the art will appreciate that PDGF-A includes all isoform variants.

The human platelet-dervied growth factor subunit B (PDGF-B) polypeptide sequence is set forth in, *e.g.,* NCBI Accession No. NP_002599.1. One skilled in the art will appreciate that PDGF-B is also known as PDGFB, PDGF subunit B, PDGF-2, platelet-dervied growth factor B chain, proto-oncogene c-Sos, and platelet-dervied growth factor beta polypeptide. One skilled in the art will appreciate that PDGF-B includes all isoform variants.

The human platelet-dervied growth factor subunit C (PDGF-C) polypeptide sequence is set forth in, *e.g.,* NCBI Accession No. AAK51637.1. One skilled in the art will appreciate that PDGF-C is also known as PDGFC, PDGF subunit C, fallotein, spinal cord-dervied growth factor, SCDGF, and VEGF-E. One skilled in the art will appreciate that PDGF-C includes all isoform variants.

The human platelet-dervied growth factor subunit D (PDGF-D) polypeptide sequence is set forth in, *e.g.,* NCBI Accession No. AAK38840.1. One skilled in the art will appreciate that PDGF-D is also known as PDGFD, PDGF subunit D, iris-expressed growth factor, spinal cord-dervied growth factor B, and SCDGF-B. One skilled in the art will appreciate that PDGF-D includes all isoform variants.

The human fibroblast growth factor 1 (FGF1) polypeptide sequence is set forth in, *e.g.,* NCBI Accession No. AAH32697.1. The human FGF1 mRNA (coding) sequence is set forth in, *e.g*., Genbank Accession Nos. NM_000800, NM_001144892, NM_001144934, NM_001144934, NM_001144935, NM_033136 and NM_033137. One skilled in the art will appreciate that FGF1 is also known as FGFA, FGF-1, acidic fibroblast growth factor, aFGF, endothelial cell growth factor, ECGF, heparin-binding growth factor 1, and HB-EGF1. One skilled in the art will appreciate that FGF1 includes all isoform variants.

The human fibroblast growth factor (FGF2) polypeptide sequence is set forth in, *e.g.,* Genbank Accession No. NP_001997.5. The human FGF2 mRNA (coding) sequence is set forth in, *e.g*., Genbank Accession No. NM_002006.4. One skilled in the art will appreciate that FGF2 is also known as basic FGF, bFGF, and FGFB.

The human fibroblast growth factor (FGF4) polypeptide sequence is set forth in, *e.g.,* Genbank Accession No. NP_001998.1. One skilled in the art will appreciate that FGF4 is also known as heparin-secretory transforming protein 1, HST, HST-1, HSTF-1, heparin-binding growth factor 4, HBGF-4, and KS3.

The human fibroblast growth factor 7 (FGF7) polypeptide sequence is set forth in, *e.g.,* NCBI Accession No. CAG46799.1. The human FGF7 mRNA (coding) sequence is set forth in, *e.g*., Genbank Accession No. NM_002009.3. One skilled in the art will appreciate that FGF7 is also known as FGF-7, heparin-binding growth factor 7, HBGF-7 and keratinocyte growth factor.

The human fibroblast growth factor 9 (FGF9) polypeptide sequence is set forth in, *e.g.,* NCBI Accession No. AAT74624.1. The human FGF9 mRNA (coding) sequence is set forth in, *e.g*., Genbank Accession No. NM_002010.2. One skilled in the art will appreciate that FGF9 is also known as heparin-binding growth factor 9, GAF, and HBGF-9.

The human fibroblast growth factor 19 (FGF19) polypeptide sequence is set forth in, *e.g.,* NCBI Accession No. AAQ88669.1. One skilled in the art will appreciate that FGF19 is also known as zFGF5. One skilled in the art will appreciate that FGF19 includes all isoform variants.

The human stem cell factor (SCF) polypeptide sequence is set forth in, *e.g.,* NCBI Accession No. AAI26167.1. One skilled in the art will appreciate that PDGF-A is also known as kit ligand, c-kit ligand, mast cell growth factor and MGF. One skilled in the art will appreciate that SCF includes all isoform variants.

The interleukin 10 (IL-10) polypeptide sequence is set forth in, *e.g.,* NCBI Accession No. AAI04253.1. One skilled in the art will appreciate that IL-10 includes all isoform variants.

### E. Anti-TNF Drug Levels & Anti-Drug Antibody (ADA) Levels

In some embodiments, the method comprises determining the presence and/or level of a drug analyte in a patient sample (*e.g.,* a serum sample from a patient on anti-TNF drug therapy) to form an inflammatory phase marker score and/or proliferation phase marker score. In certain instances, the measurements can be made at multiple time points, *e.g*., before, during, and/or after the course of therapy. In some embodiments, the drug analyte is an anti-TNF drug (*e.g.,* level of free anti-TNFa therapeutic antibody such as infliximab) and/or an anti-drug antibody (ADA) (*e.g.,* level of autoantibody to the anti-TNF drug such as HACA, HAHA, HAMA, and combinations thereof).

In particular embodiments, the presence and/or level of an anti-TNF drug and/or ADA is determined with a homogeneous mobility shift assay using size exclusion chromatography. These methods and related technology are described in PCT Publication Nos. WO 2011/056590, WO 2012/054532, WO 2012/154253 and WO 2013/006810, and in US Provisional Application No. 61/683,681, filed August 15, 2012, the disclosures of which are incorporated by reference in their entirety for all purposes. These methods are particularly advantageous for measuring the presence or level of TNFα inhibitors as well as autoantibodies (*e.g.,* HACA, HAHA, *etc.*) that are generated against them.

The method for determining the presence or level of an anti-TNFa drug in a sample can comprise: (a) contacting a labeled TNFα with a sample having an anti-TNFa drug to form a labeled complex with the anti-TNFa drug; (b) subjecting the labeled complex to size exclusion chromatography to separate the labeled complex from free labeled TNFα and to detect an amount of the labeled complex and an amount of the free labeled TNFα; and (c) comparing the amount of the labeled complex and the amount of the free labeled TNFα detected in step (b) to a standard curve of known amounts of the anti-TNFa drug, thereby determining the presence or level of the anti-TNFa drug.

The method for determining the presence or level of an autoantibody to an anti-TNFa drug in a sample can comprise: (a) contacting a labeled anti-TNFa drug with the sample to form a labeled complex with the autoantibody; (b) subjecting the labeled complex to size exclusion chromatography to separate the labeled complex from free labeled anti-TNFa drug and to detect an amount of the labeled complex and an amount of the free labeled anti-TNFa drug; and (c) comparing the amount of the labeled complex and the amount of the free labeled anti-TNFa drug detected in step (b) to a standard curve of known amounts of the autoantibody, thereby determining the presence or level of the autoantibody.

In certain embodiments, the methods are especially useful for the following anti-TNFa drugs: REMICADE™ (infliximab), ENBREL™ (etanercept), HUMIRA™ (adalimvumab), and CIMZIA® (certolizumab pegol).

In other embodiments, the methods are especially useful for the following anti-drug antibodies (ADA): human anti-chimeric antibody (HACA), human anti-humanized antibody (HAHA), and human anti-mouse antibody (HAMA).

In some embodiments, the method of detecting an anti-drug antibody includes determining the presence or level of anti-drug antibody (ADA) isotypes in ADA-positive patients receiving anti-TNF drug therapy. Non-limiting examples of antibody isotypes include IgA, IgD, IgE, IgG, and IgM. In certain aspects, the detection of the presence or level of a specific ADA isotype or a particular combination of ADA isotypes is associated with different clinical outcomes.

Non-limiting examples of other methods for determining the presence and/or level of a drug analyte (*e.g.*, anti-TNF drug and/or anti-drug antibodies (ADA)) include enzyme-linked immunosorbent assays (ELISAs) such as bridging ELISAs. For example, the Infliximab ELISA from Matriks Biotek Laboratories detects free infliximab in serum and plasma samples, and the HACA ELISA from PeaceHealth Laboratories detects HACA in serum samples.

### F. Generating a Marker Score

In some embodiments, the methods of predicting the presence of mucosal healing and/or monitoring the progression of mucosal healing in an individual utilize an empirically derived score (*e.g.*, inflammatory phase score and proliferation phase score).

In addition, the methods of selecting an appropriate therapy, optimizing therapeutic efficiency and the like, include the use of the marker score to select, for example, a dose of drug, an appropriate drug, a course or length of therapy, a therapy regimen, or the maintenance of an existing drug or dose. In certain aspects, a derived or measured score can be used for selecting an appropriate therapeutic regimen that promotes mucosal healing.

In certain aspects, each marker is assigned a value based upon the concentration and level of the marker relative to a standard or a set of standards. In some embodiments, the value is selected from 0 to 6, *e.g.,* 0, 1, 2, 3, 4, 5, or 6. For example, if the level of a marker is below the level of the lowest standard, the marker is assigned a value of 0. If the level of a marker is between the first lowest standard and the second standard (from low to high), the marker is given a value of 1. If the level of a marker is between the second standard and the third standard, the marker is given a value of 2. If the level of a marker is between the third standard and the fourth standard, the marker is given a value of 3. If the level of a marker is between the fourth standard and the fifth standard, the marker is given a value of 4. If the level of a marker is between the fifth standard and the sixth standard, the marker is given a value of 5. If the level of a marker is even with the sixth standard or higher, the marker is given a value of 6. In certain embodiments, each marker can be assigned a value selected from 0 to 1, 2, 3, 4, 5, 6, 7, 8, 9, 10, 11, 12, 13, 14, 15, 16, 17, 18, 19, 20, 25, 30, or any range therein (*e.g.*, from 1 to 6).

In certain aspects, each marker is assigned a value based upon the quantile level of the marker. In some embodiments, the value is selected from 0 to 6, *e.g.,* 0, 1, 2, 3, 4, 5, or 6. For instance, the values are split into 7 groups ("a septile") and the markers are assigned a value from 0 to 6 based on its quantile group. In certain embodiments, each marker can be assigned a value selected from 0 to 1, 2, 3, 4, 5, 6, 7, 8, 9, 10, 11, 12, 13, 14, 15, 16, 17, 18, 19, 20, 25, 30, or any range therein (e.g., from 1 to 6). For instance, the values are split into 12 groups and the markers are assigned a value from 0 to 11 based on its quantile group.

In some aspects, the concentration or level of each marker is relative to the level of the same marker in a control patient population, *e.g*., a population or group of IBD patients that do not exhibit mucosal healing.

In some embodiments, the inflammatory phase marker score is the summation of the value of each marker in the first set of markers, such as one or more of TWEAK, CRP, ICAM, SAA, VCAM, IL-2, IL-8, IL-12p70, IL-1β, GMCSF, IFNγ, IL-6, TNFα, ASCA-A, ASCA-G, CBirl, Fla2, FlaX, OmpC, and an anti-drug antibody (ADA). In other embodiments, the value of each marker in the first set of markers includes one or more of GMCSF, IL-2, and VCAM.

In some embodiments, the proliferation phase marker score is the summation of the value of each marker in the second set of markers, such as one or more of AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFa antibody. In other embodiments, the value of the marker in the second set of markers includes HGF.

In some embodiments, an algorithm is applied to the inflammatory marker score and the proliferation phase marker score. In particular embodiments, the algorithm allows for the comparison of the inflammatory marker score and the proliferation phase marker score to form or generate a biomarker score. As a non-limiting example, the biomarker score comprises the proliferation phase marker score (*e.g.*, sum of the proliferation phase marker values) minus the inflammatory phase marker score (*e.g.*, sum of the inflammatory phase marker values).

In some embodiments, the algorithm predicts the likelihood of mucosal healing and/or monitors the progression of mucosal healing independent of clinical confounders. For instance, one or more of the clinical confounders, *e.g*., age of diagnosis, age of last sample, disease location, anal involvement, smoking, and surgery does not contribute to the algorithm for determining the likelihood of mucosal healing.

In some embodiments, the algorithm predicts the likelihood of mucosal healing and/or monitors the progression of mucosal healing without one or more serology markers, *e.g*., ASCA-A, ASCA-G, CBirl, Fla2, FlaX, and OmpC, that are used to form the inflammatory phase marker score. The algorithm remains predictive with the exclusion of one or more serology markers, *e.g*., ASCA-A, ASCA-G, CBirl, Fla2, FlaX, and OmpC.

In some embodiments, the subject's biomarker score is compared to the biomarker score of a control patient population, such as a population wherein the patients have not undergone the inflammatory phase of mucosal healing, the proliferation phase of mucosal healing, a progression of mucosal healing, incomplete improvement of mucosal healing, and/or complete improvement of mucosal healing. For instance, if a subject's biomarker score is higher than the biomarker score of a patient population without mucosal healing, then the subject has an increased likelihood of having complete improvement of mucosal healing without relapse. If a subject's biomarker score is lower than the biomarker score of a patient population without mucosal healing, then the subject has a decreased likelihood of having complete improvement of mucosal healing without relapse, thereby indicating the need for selecting an appropriate therapy (*e.g.*, an increase in dose of anti-TNFα therapy, surgery, combination therapy, and the like) for effective treatment.

### G. Statistical Algorithms for Mucosal Healing

In certain aspects, the present invention provides an algorithmic-based analysis that incorporates the inflammatory phase marker score and the proliferation phase marker score to improve the sensitivity, specificity, and/or accuracy of predicting and/or monitoring mucosal healing, selecting therapy, optimizing therapy, reducing toxicity, and/or monitoring the efficacy of therapeutic treatment to anti-TNFa drug therapy.

In some aspects, the present invention provides methods for identifying the phase of mucosal healing, monitoring mucosal healing, predicting the likelihood of mucosal healing and/or selecting a therapeutic regimen for a subject by applying a statistical algorithm to a proliferation phase marker score and an inflammatory phase marker score to generate a mucosal healing measurement.

The term "statistical analysis" or "statistical algorithm" or "statistical process" includes any of a variety of statistical methods and models used to determine relationships between variables. In the present invention, the variables are the presence and/or level of at least one marker of interest. Any number of markers can be analyzed using a statistical analysis described herein. For example, the presence or level of 1, 2, 3, 4, 5, 6, 7, 8, 9, 10, 11, 12, 13, 14, 15, 16, 17, 18, 19, 20, 25, 30, 35, 40, 45, 50, 55, 60, or more markers can be included in a statistical analysis. In one embodiment, logistic regression is used. In another embodiment, linear regression is used. In yet another embodiment, ordinary least squares regression or unconditional logistic regression is used.

In some embodiments, the statistical analyses of the present invention comprise a quantile measurement of one or more markers, *e.g*., within a given population, as a variable. Quantiles are a set of "cut points" that divide a sample of data into groups containing (as far as possible) equal numbers of observations. For example, quartiles are values that divide a sample of data into four groups containing (as far as possible) equal numbers of observations. The lower quartile is the data value a quarter way up through the ordered data set; the upper quartile is the data value a quarter way down through the ordered data set. Quintiles are values that divide a sample of data into five groups containing (as far as possible) equal numbers of observations. The present invention can also include the use of percentile ranges of marker levels (*e.g.*, tertiles, quartile, quintiles, *etc.*), or their cumulative indices (*e.g.,* quartile sums of marker levels to obtain quartile sum scores (QSS), *etc.*) as variables in the statistical analyses (just as with continuous variables).

In some embodiments, the statistical analyses of the present invention comprise one or more learning statistical classifier systems. As used herein, the term "learning statistical classifier system" includes a machine learning algorithmic technique capable of adapting to complex data sets (*e.g.,* panel of markers of interest) and making decisions based upon such data sets. In some embodiments, a single learning statistical classifier system such as a decision/classification tree (*e.g.*, random forest (RF) or classification and regression tree (C&RT)) is used. In other embodiments, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.*, decision/classification trees such as random forests, classification and regression trees (C&RT), boosted trees, *etc.*), Probably Approximately Correct (PAC) learning, connectionist learning (*e.g.*, neural networks (NN), artificial neural networks (ANN), neuro fuzzy networks (NFN), network structures, the Cox Proportional-Hazards Model (CPHM), perceptrons such as multi-layer perceptrons, multi-layer feed-forward networks, applications of neural networks, Bayesian learning in belief networks, *etc.*), reinforcement learning (*e.g.,* passive learning in a known environment such as naive learning, adaptive dynamic learning, and temporal difference learning, passive learning in an unknown environment, active learning in an unknown environment, learning action-value functions, applications of reinforcement learning, *etc.*), and genetic algorithms and evolutionary programming. Other learning statistical classifier systems include support vector machines (e.g., Kernel methods), multivariate adaptive regression splines (MARS), Levenberg-Marquardt algorithms, Gauss-Newton algorithms, mixtures of Gaussians, gradient descent algorithms, and learning vector quantization (LVQ).

Random forests are learning statistical classifier systems that are constructed using an algorithm developed by Leo Breiman and Adele Cutler. Random forests use a large number of individual decision trees and decide the class by choosing the mode (*i.e.,* most frequently occurring) of the classes as determined by the individual trees. Random forest analysis can be performed, *e.g*., using the RandomForests software available from Salford Systems (San Diego, CA). *See, e.g.,* Breiman, Machine Learning, 45:5-32 (2001); and http://stat-www.berkeley.edu/users/breiman/RandomForests/cc_home.htm, for a description of random forests.

Classification and regression trees represent a computer intensive alternative to fitting classical regression models and are typically used to determine the best possible model for a categorical or continuous response of interest based upon one or more predictors. Classification and regression tree analysis can be performed, *e.g*., using the C&RT software available from Salford Systems or the Statistica data analysis software available from StatSoft, Inc. (Tulsa, OK). A description of classification and regression trees is found, *e.g.,* in Breiman et al. "Classification and Regression Trees," Chapman and Hall, New York (1984); and Steinberg et al., "CART: Tree-Structured Non-Parametric Data Analysis," Salford Systems, San Diego, (1995).

Neural networks are interconnected groups of artificial neurons that use a mathematical or computational model for information processing based on a connectionist approach to computation. Typically, neural networks are adaptive systems that change their structure based on external or internal information that flows through the network. Specific examples of neural networks include feed-forward neural networks such as perceptrons, single-layer perceptrons, multi-layer perceptrons, backpropagation networks, ADALINE networks, MADALINE networks, Learnmatrix networks, radial basis function (RBF) networks, and self-organizing maps or Kohonen self-organizing networks; recurrent neural networks such as simple recurrent networks and Hopfield networks; stochastic neural networks such as Boltzmann machines; modular neural networks such as committee of machines and associative neural networks; and other types of networks such as instantaneously trained neural networks, spiking neural networks, dynamic neural networks, and cascading neural networks. Neural network analysis can be performed, *e.g*., using the Statistica data analysis software available from StatSoft, Inc. *See, e.g.,* Freeman et al., In "Neural Networks: Algorithms, Applications and Programming Techniques," Addison-Wesley Publishing Company (1991); Zadeh, Information and Control, 8:338-353 (1965); Zadeh, "IEEE Trans. on Systems, Man and Cybernetics," 3:28-44 (1973); Gersho et al., In "Vector Quantization and Signal Compression," Kluywer Academic Publishers, Boston, Dordrecht, London (1992); and Hassoun, "Fundamentals of Artificial Neural Networks," MIT Press, Cambridge, Massachusetts, London (1995), for a description of neural networks.

Support vector machines are a set of related supervised learning techniques used for classification and regression and are described, *e.g.,* in Cristianini et al., "An Introduction to Support Vector Machines and Other Kernel-Based Learning Methods," Cambridge University Press (2000). Support vector machine analysis can be performed, *e.g.,* using the SVM*^{light}* software developed by Thorsten Joachims (Cornell University) or using the LIBSVM software developed by Chih-Chung Chang and Chih-Jen Lin (National Taiwan University).

The various statistical methods and models described herein can be trained and tested using a cohort of samples (e.g., serological samples) from healthy or non-IBD individuals and patients with a TNFα-mediated disease or disorder such as, *e.g.,* IBD (*e.g.,* CD and/or UC). For example, samples from patients diagnosed by a physician, preferably by a gastroenterologist, as having IBD or a clinical subtype thereof using a biopsy, colonoscopy, or an immunoassay as described in, *e.g.,* U.S. Patent No. 6,218,129, are suitable for use in training and testing the statistical methods and models of the present invention. Samples from patients diagnosed with IBD can also be stratified into Crohn's disease or ulcerative colitis using an immunoassay as described in, *e.g.,* U.S. Patent Nos. 5,750,355 and 5,830,675. Samples from healthy individuals can include those that were not identified as IBD samples. One skilled in the art will know of additional techniques and diagnostic criteria for obtaining a cohort of patient samples that can be used in training and testing the statistical methods and models of the present invention.

### H. Predicting Therapeutic Response/Therapeutic Efficacy

The present invention provides non-invasive methods for predicting the likelihood of mucosal healing and/or monitoring mucosal healing in patients, such as patients receiving anti-TNF therapy. In addition, the present invention provides methods of predicting therapeutic response, risk of relapse, and risk of surgery in patients with IBD (*e.g.,* Crohn's disease and ulcerative colitis) based upon the progression of mucosal healing in the subject. In particular, the methods of the present invention find utility for selecting an appropriate anti-TNFa therapy for continued treatment, for determining when or how to adjust or modify (*e.g.*, increase or decrease) the subsequent dose of an anti-TNFa drug to optimize therapeutic efficacy and/or to reduce toxicity, for determining when or how to combine an anti-TNFa drug (*e.g.,* at an initial, increased, decreased, or same dose) with one or more immunosuppressive agents such as methotrexate (MTX) or azathioprine (AZA), and/or for determining when or how to change the current course of therapy (*e.g.*, switch to a different anti-TNFa drug or to a drug that targets a different mechanism). The present invention also provides methods for selecting an appropriate therapy for patients diagnosed with IBD, wherein the therapy promotes mucosal healing (*e.g.,* complete improvement of mucosal healing without relapse).

In some embodiments, selecting an appropriate therapy comprises maintaining, increasing, or decreasing a subsequent dose of the course of therapy for the subject. In other embodiments, the method further comprises determining a different course of therapy for the subject. In certain instances, the different course of therapy comprises treatment with a different anti-TNFa antibody. In other instances, the different course of therapy comprises the current course of therapy along with another therapeutic agent, such as, but not limited to, an immunosuppressive agent, a corticosteroid, a drug that targets a different mechanism, nutrition therapy, and combinations thereof.

In some embodiments, selecting an appropriate therapy comprises selecting an appropriate therapy for initial treatment. In some instances, the therapy comprises an anti-TNFa antibody therapy.

In certain embodiments, the methods disclosed herein can be used as confirmation that a proposed new drug or therapeutic is the same as or is sufficiently or substantially similar to an approved drug product, such that the proposed new drug or therapeutic can be used as a "biosimilar" therapeutic. For example, if the proposed new drug has only a slightly different disease activity profile compared to the branded drug product, this would be apparent using the methods disclosed herein. If the proposed new drug has a significantly different disease activity profile compared to the branded drug product, then the new drug would not be biosimilar. Advantageously, the methods disclosed herein can be used in clinical trials of proposed new drugs in order to assess the effective therapeutic value of the drug.

In some embodiments, selecting an appropriate therapy comprises maintaining, increasing, or decreasing a subsequent dose of the course of therapy for the subject. In other embodiments, the method further comprises determining a different course of therapy for the subject. In certain instances, the different course of therapy comprises treatment with a different anti-TNFa antibody. In other instances, the different course of therapy comprises the current course of therapy along with another therapeutic agent, such as, but not limited to, an immunosuppressive agent, a corticosteroid, a drug that targets a different mechanism, nutrition therapy, and combinations thereof).

Accordingly, in some aspects, the methods of the invention provide information useful for guiding treatment decisions for patients receiving or about to receive anti-TNFa drug therapy, *e.g.,* by selecting an appropriate anti-TNFa therapy for initial treatment, by determining when or how to adjust or modify (*e.g.*, increase or decrease) the subsequent dose of an anti-TNFa drug, by determining when or how to combine an anti-TNFa drug (*e.g.,* at an initial, increased, decreased, or same dose) with one or more immunosuppressive agents such as methotrexate (MTX) or azathioprine (AZA), and/or by determining when or how to change the current course of therapy (*e.g.*, switch to a different anti-TNFa drug or to a drug that targets a different mechanism such as an IL-6 receptor-inhibiting monoclonal antibody, anti-integrin molecule (*e.g.*, Tysabri, Vedaluzamab), JAK-2 inhibitor, and tyrosine kinase inhibitor, or to a nutrition therapy (*e.g.*, special carbohydrate diet)).

In other embodiments, the methods of the present invention can be used to predict responsiveness to a TNFα inhibitor, especially to an anti-TNFa antibody in a subject having an autoimmune disorder (*e.g.*, rheumatoid arthritis, Crohn's disease, ulcerative colitis and the like.). In this method, by assaying the subject for the correct or therapeutic dose of anti-TNFa antibody, *i.e.,* the therapeutic concentration level, it is possible to predict whether the individual will be responsive to the therapy.

In another embodiment, the present invention provides methods for monitoring IBD (*e.g.,* Crohn's disease and ulcerative colitis) in a subject having the IBD disorder, wherein the method comprises assaying the subject for the correct or therapeutic dose of anti-TNFa antibody, *i.e.,* the therapeutic concentration level, overtime. In this manner, it is possible to predict whether the individual will be responsive to the therapy over the given time period.

Once the diagnosis or prognosis of a subject receiving anti-TNFa drug therapy has been determined or the likelihood of response to an anti-TNFa drug has been predicted in a subject diagnosed with a disease in which TNFα has been implicated in the pathophysiology, including, but not limited to, IBD (*e.g.,* Crohn's disease and ulcerative colitis), shock, sepsis, infections, autoimmune diseases, RA, transplant rejection and graft-versus-host disease, according to the methods described herein, the present invention may further comprise recommending a course of therapy based upon the diagnosis, prognosis, or prediction. In certain instances, the present invention may further comprise administering to a subject a therapeutically effective amount of an anti-TNFa drug useful for treating one or more symptoms associated with the TNF-mediated disease or disorder. For therapeutic applications, the anti-TNF drug can be administered alone or co-administered in combination with one or more additional anti-TNF drugs and/or one or more drugs that reduce the side-effects associated with the anti-TNF drug (*e.g.,* an immunosuppressive agent). As such, the present invention advantageously enables a clinician to practice "personalized medicine" by guiding treatment decisions and informing therapy selection and optimization for anti-TNFa drugs such that the right drug is given to the right patient at the right time.

Understanding the clinical course of the disease enables physicians to make better informed treatment decisions for their inflammatory disease patients (*e.g.,* IBD (*e.g.,* Crohn's disease and ulcerative colitis), rheumatoid arthritis (RA), others) and helps to direct new drug development. The biomarkers described herein are able to detect mucosal healing phases and monitor the effect of treatment; and have a predictive value towards healing or recurrence of the disease. The present invention is particularly advantageous because it provides indicators of the phases of mucosal healing and enables a prediction of mucosal improvement in patients. In addition, the inflammatory phase score and the proliferation phase score of the present invention have enormous implications for patient management, as well as therapeutic decision-making, and aid or assist in directing the appropriate therapy to patients who most likely will benefit from it and avoid the expense and potential toxicity of chronic maintenance therapy in those who have a low risk of recurrence.

### V. Examples

The following examples are offered for illustrative purposes, and are not intended to limit the invention in any manner. Those of skill in the art will readily recognize a variety of noncritical parameters which can be changed or modified to yield essentially the same results.

### Example 1. Per-Marker Analysis and Individual Patient Analysis of Mucosal Healing.

This example illustrates that the phases of mucosal healing (e.g., inflammatory phase, proliferation phase, and remodeling phase) in an IBD patient are associated with the levels of various inflammatory markers and repair markers. Described herein is a biomarker analysis of IBD patients who showed either complete healing or no healing.

In this study serum samples were obtained from 197 IBD patients with repeated endoscopic scores. At least one sample from each patient was available for evaluation. 131 patients had more than 1 sample available for testing and 49 subjects has more than 2 samples. In total, 386 samples were analyzed.

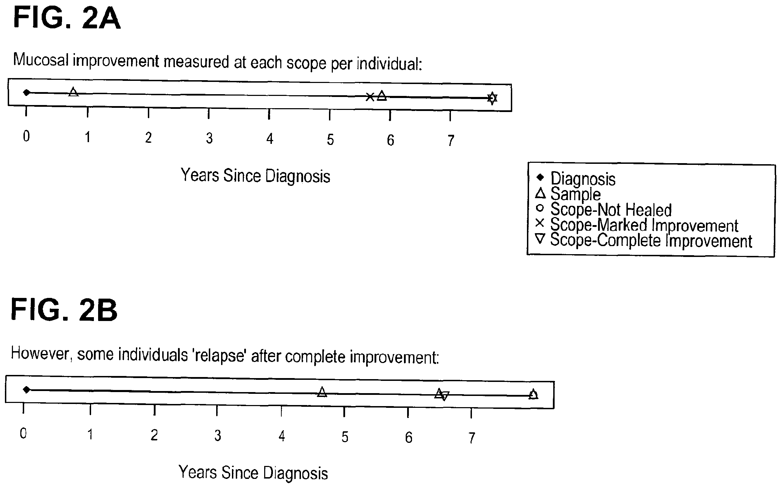

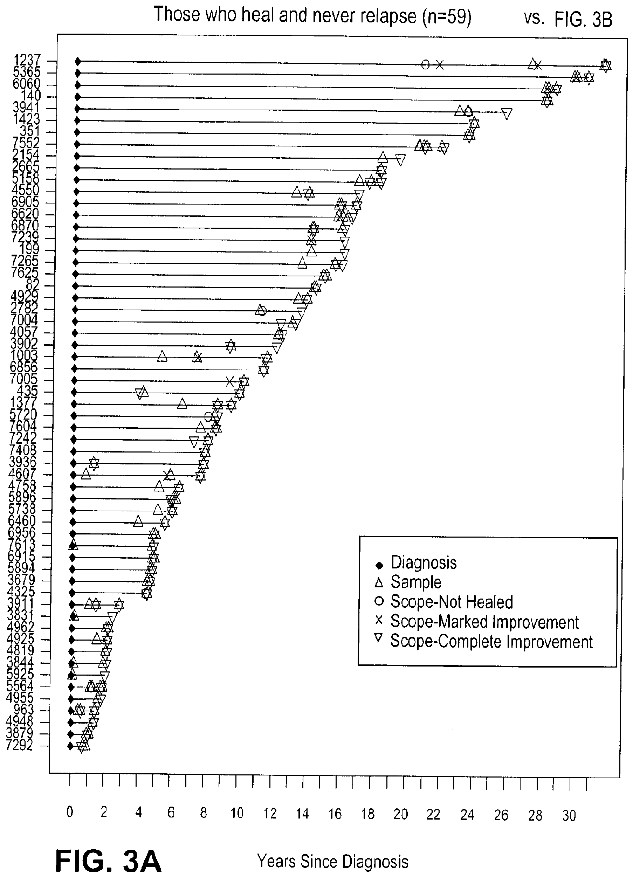

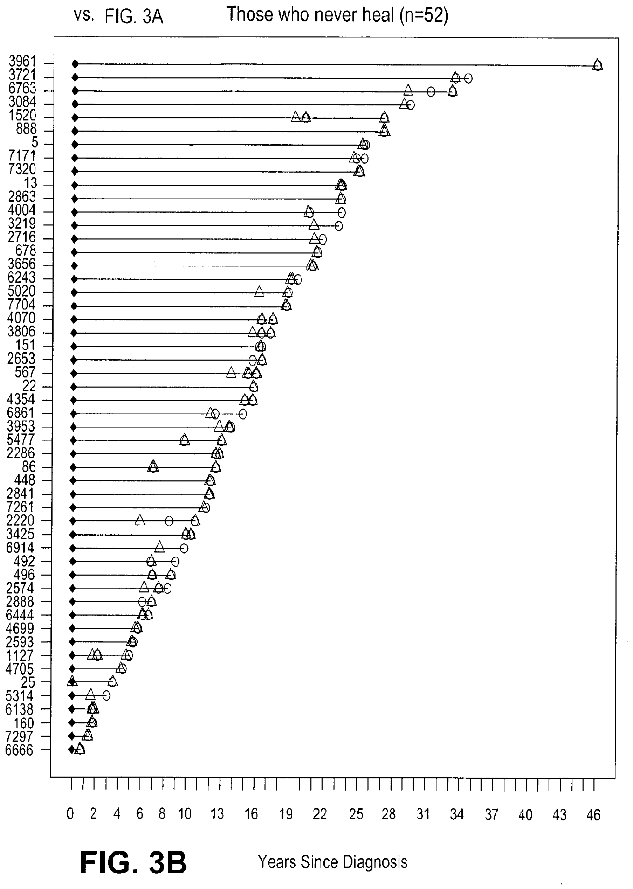

Endoscopy remains the standard method for determining the clinical status of patients with IBD. It is used to assess mucosal changes (*e.g.*, improvements) and to determine whether a patient exhibits complete improvement. Unfortunately, some patients with an endoscopic score of complete improvement can relapse (FIG. 2A). This suggests that endoscopic scoring does not provide an adequate assessment of mucosal healing in IBD patients. In one aspect, this study investigated whether mucosal healing and its three phases (*e.g.*, inflammatory, proliferation, and remodeling) can be detected in serum samples from IBD patients. Two distinct populations were analyzed: patients who showed complete healing (FIG. 3A); and patients who never healed (FIG. 3B).

To further investigate the differences between the phases of mucosal healing, biomarker profiles were compared between the two patient populations. In particular, forty-five markers were measured including 22 repair factors (*e.g.,* 7 HER ligands, 14 angiogenesis ligands, and 1 hematopoiesis ligand), 15 inflammatory markers (*e.g.*, 13 pro-inflammatory markers and 2 antiinflammatory markers), 6 serological markers, infliximab (IFX), and anti-infliximab antibodies (ATI).

The markers EGF, AREG, EREG, HBEGF, HGF, HRGB, BTC, TGFA, FGF1, FGF2, FGF, FGF7, FGF9, FGF19, SCF, PDGFA, PDGFB, PDGFC, VEGFA, VEGFB, VEGFC, VEGFD, TGFB1, and TWEAK were measured by CEER. IFX and ATI levels were measured by HMSA. IL10, CRP, ICAM, SAA, VCAM, IL2, IL8, IL12p70, IL1B, GMCSF, IFNgamma, IL6, TNF alpha were measured by ELISA array (*e.g.*, Meso Scale Discovery assay kit). And, the markers ASCAA, ASCAG, CBirl, Fla2, FLAX, OmpC were measured by ELISA (*e.g.,* Prometheus IBD sgi Diagnostic test).

59 samples were analyzed from patients who healed and never relapsed. 52 samples were from patients who never healed. Samples used in the study were obtained within 3 years before and no more than 30 days after the patient's last endoscopy.

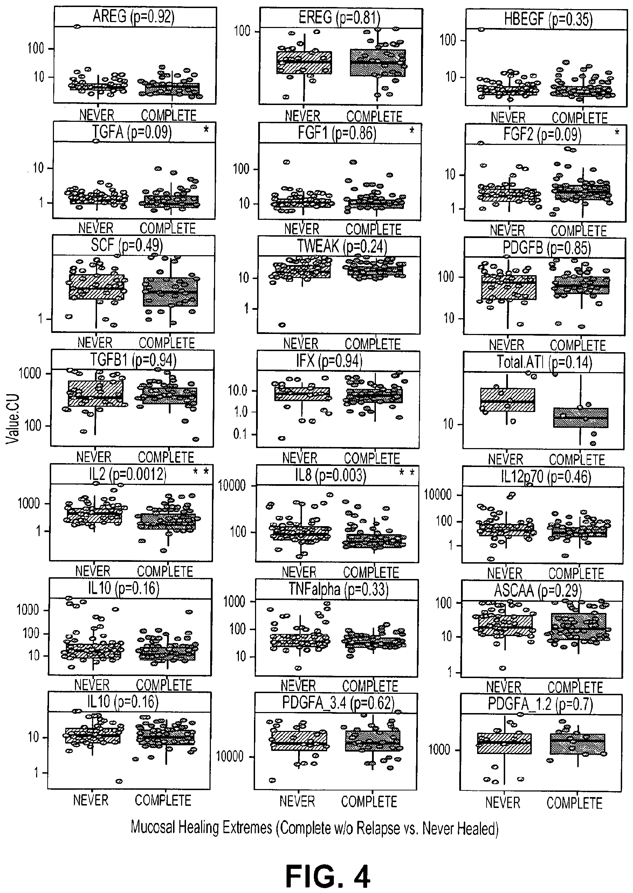

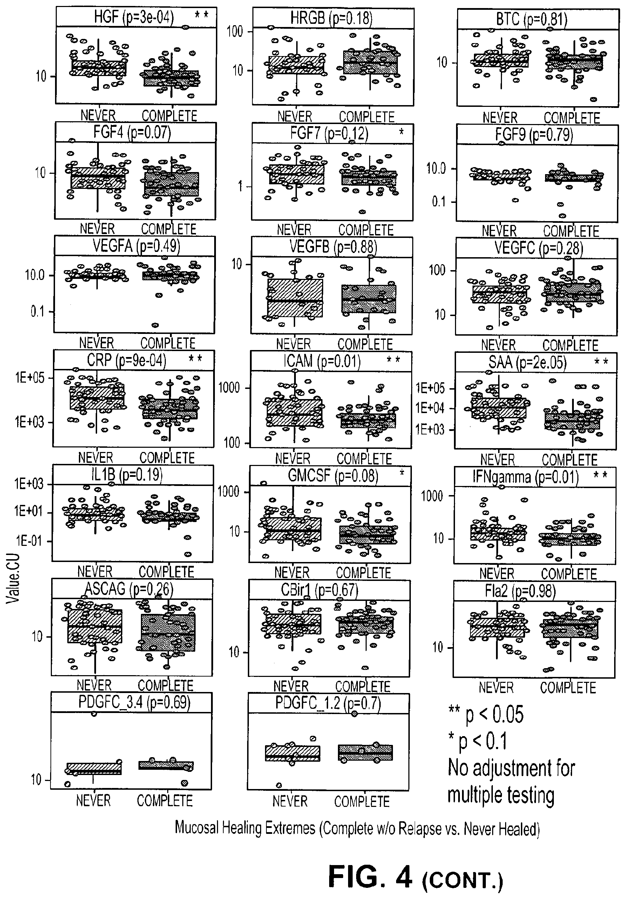

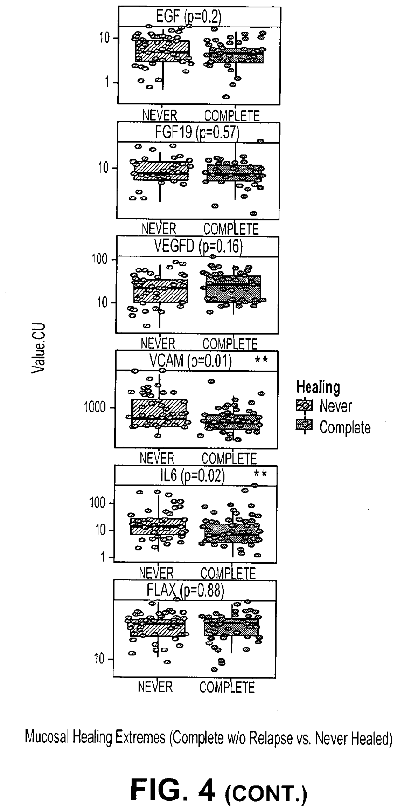

FIG. 4 shows the distribution of the markers in patients who never healed (left bar in each plot) and those who had complete healing (right bar in each plot).

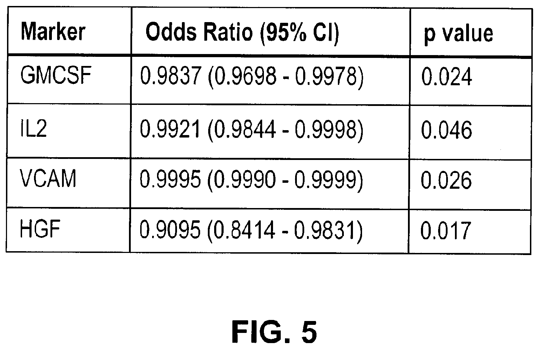

Logistic regression of per-marker analysis was performed to determine if a particular marker was associated with a clinical outcome. Adjustments were made for various clinical variables, such as, location of disease, anal involvement in disease, age of patient at the time of sampling, age at diagnosis, smoking, and surgery. The associations of GM-CSF, IL2, VCAM and HGF with clinical outcome were significant (FIG. 5). These markers were significantly associated with mucosal healing after controlling for clinical variables related to severity. Lower values of the markers were predictive of mucosal healing. The value of each marker in a patient sample was relative to the level of the same marker in patients with inflammatory bowel disease (IBD) but no mucosal healing.

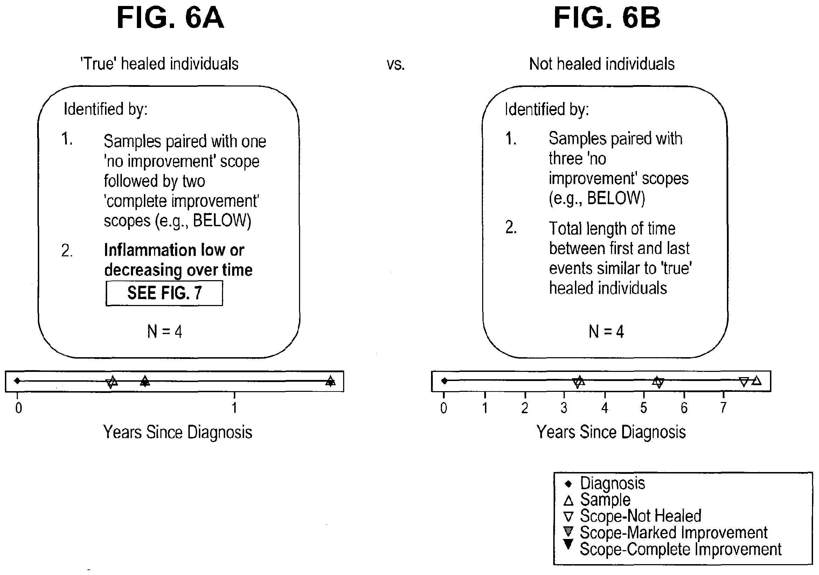

To further elucidate the marker association with mucosal healing, an individual patient analysis strategy was employed (*see,* FIG. 6A-D). 4 "true" healed individuals (Patients #1-4) were compared to 4 not healed individuals (Patients #5-8). "True" healed individuals were selected according to the following criteria: 1) having low or decreasing inflammation over time and 2) having samples characterized as being from one "no improvement" scope followed by two "complete improvement" scopes. Not healed individuals were selected according to the following criteria: 1) having samples paired with three "no improvement" scopes and 2) having a length of time between first and second events similar to the "true" healed individuals.

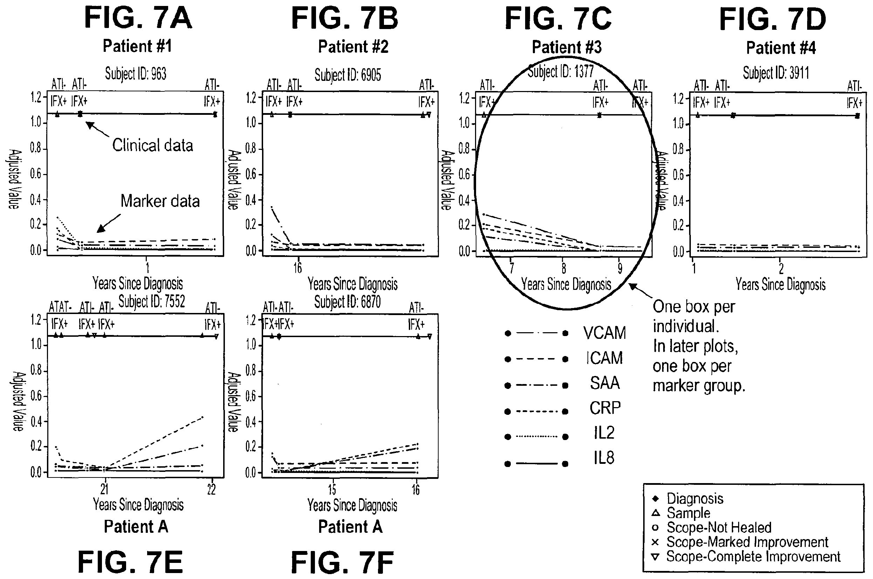

FIG. 7A-F shows representative data from the individuals in the study. Each box represents one patient. The top line shows the clinical data (*e.g.*, ATI and IFX status) and the plots at the bottom show the marker data, *e.g.,* data of VCAM, ICAM, SAA, CRP, IL2, and IL8. The marker values were scaled to the highest non-outlier value for each marker. The plots are used to determine if the marker value is increasing or decreasing with time (*e.g.*, years since diagnosis).

Patients #1-4 showed decreasing or low inflammation over time, indicating that these patients were more likely to be healed (FIG. 7A-D). Patients A and B (FIG. 7E & 7F) were determined to be completely healed by endoscopic scoring, yet these patients showed increasing inflammation in the marker analysis. The study revealed that Patients A and B were not completely healed and may be in the inflammatory phase of mucosal healing (FIG. 7).

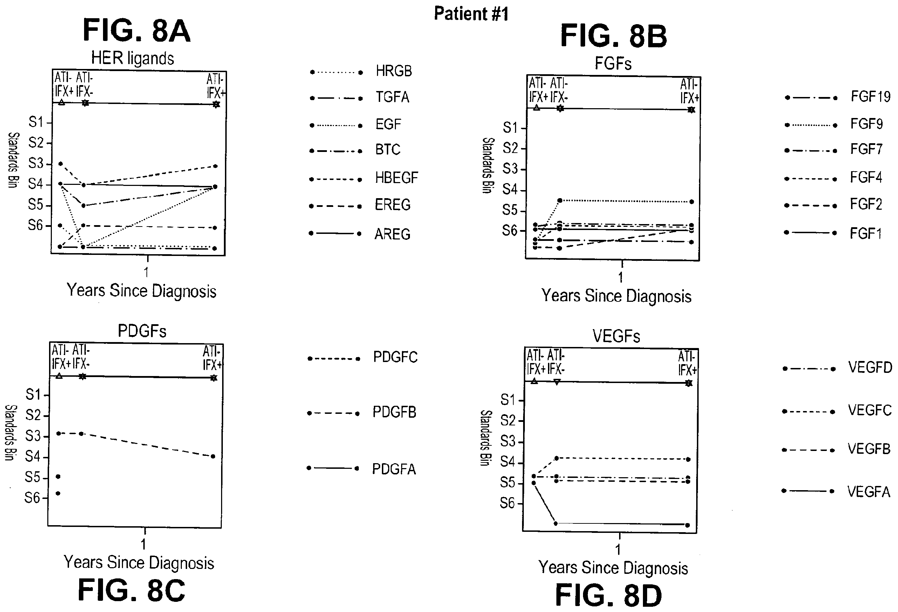

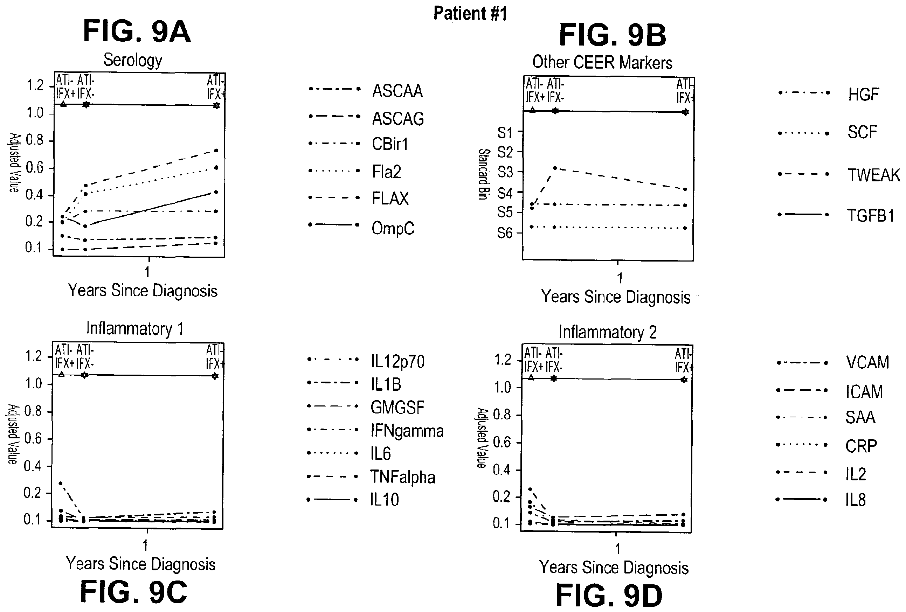

FIGS. 8-9 show the marker profile for Patient #1. Repair factors (*e.g.*, HER ligands and FGFs) (FIG. 8A-D) and serologic markers (FIG. 9A-D) increased over time. TWEAK spiked when Patient #1 was ATI negative and IFX negative at time point 2 (t2). The patient was ATI-, IFX+ at time point 1 (t1) and time point 3 (t3). The data shows that the patient is likely in the proliferation phase of healing.

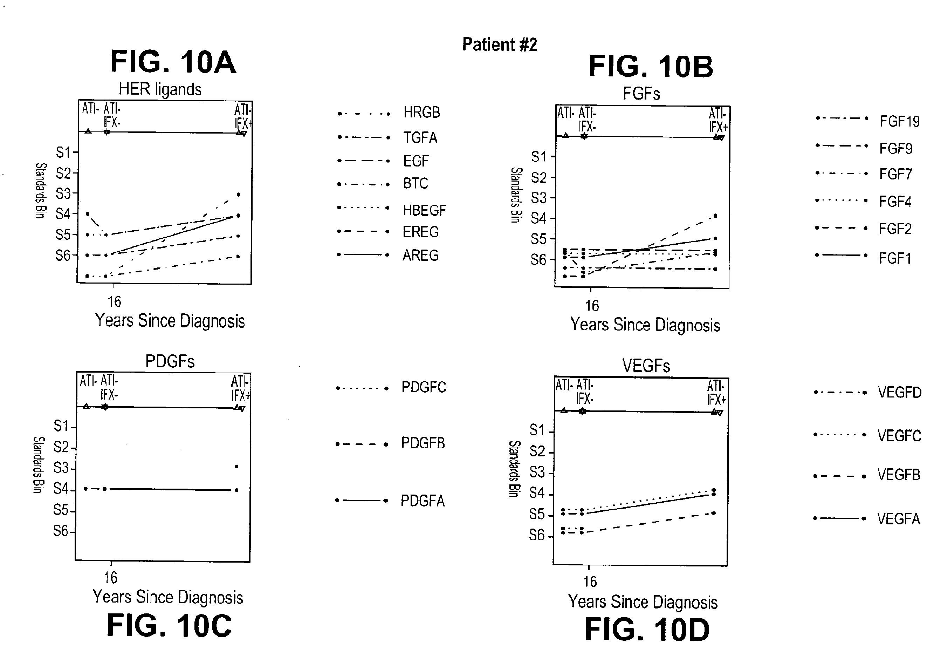

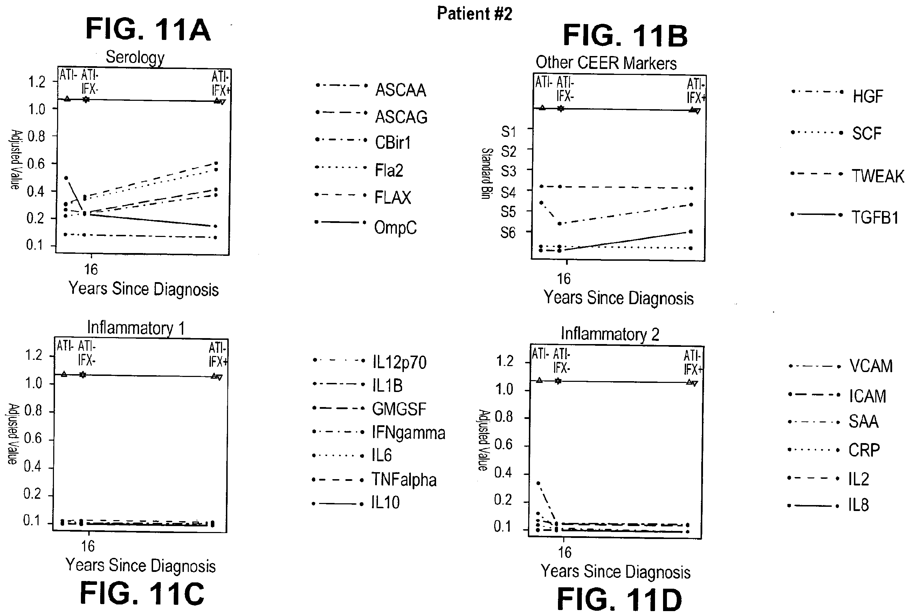

FIGS. 10-11 show the marker profile for Patient #2. Repair factors (FIG. 10A-D) and serological markers increased over time as inflammatory markers decreased (FIG. 11A-D). Patient #2 was ATI- at t1 and ATI-, IFX+ at t2 and t3. The results indicated that the patient is not fully healed and is likely in the proliferation phase of mucosal healing.

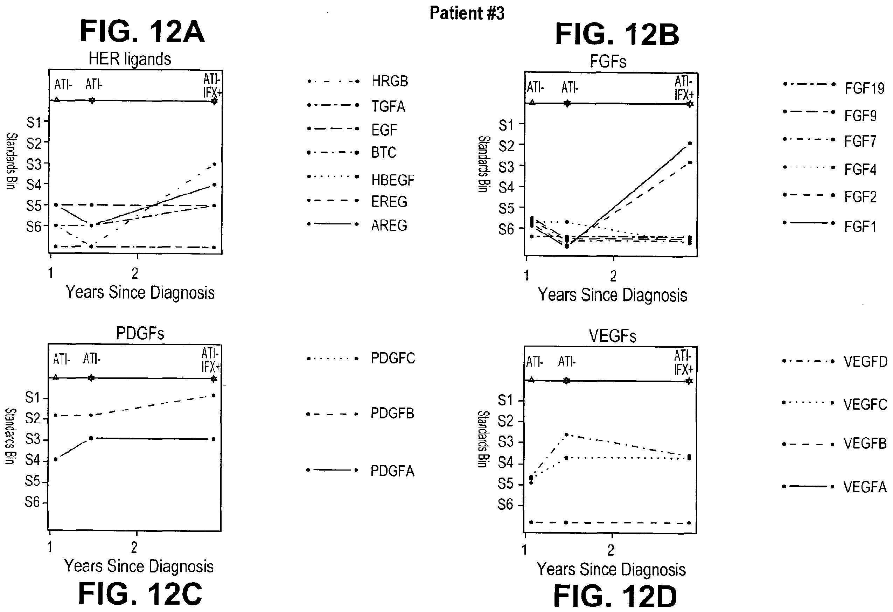

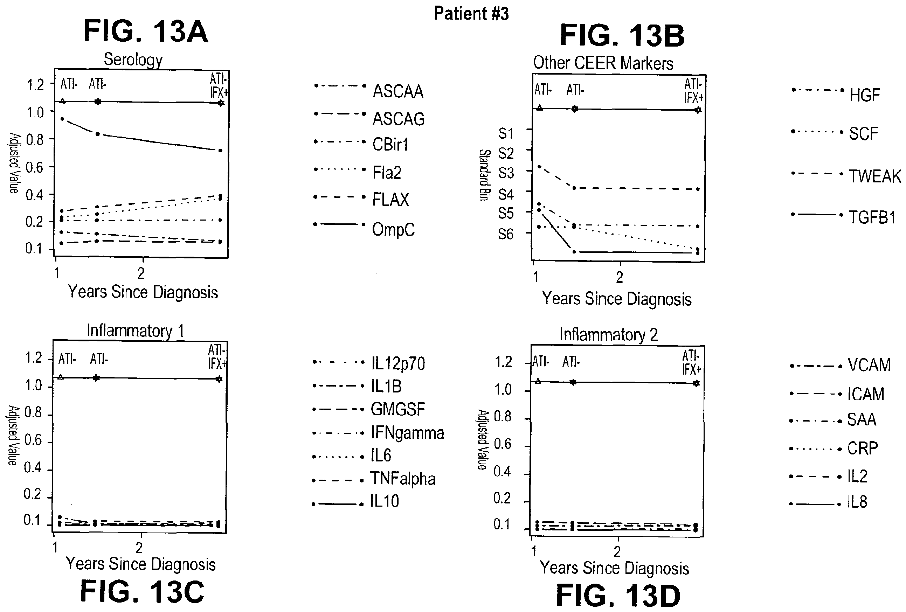

The marker profile for Patient #3 is shown in FIGS. 12- 13. Repair factors (FIG. 12A-D) and serolocial markers increased over time as inflammatory markers decreased (FIG. 13A-D). Patient #3 was ATI- at t1 and t2, and progressed to ATI-, IFX+ at t3. As with Patients #1 and 2, this patient is likely in the proliferation phase of mucosal healing and should not be considered to be completely healed.

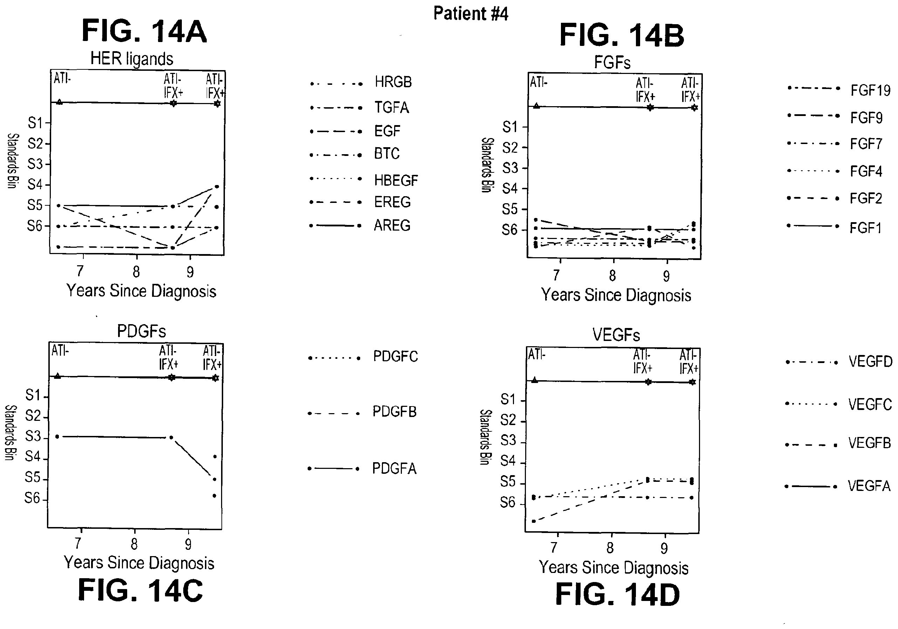

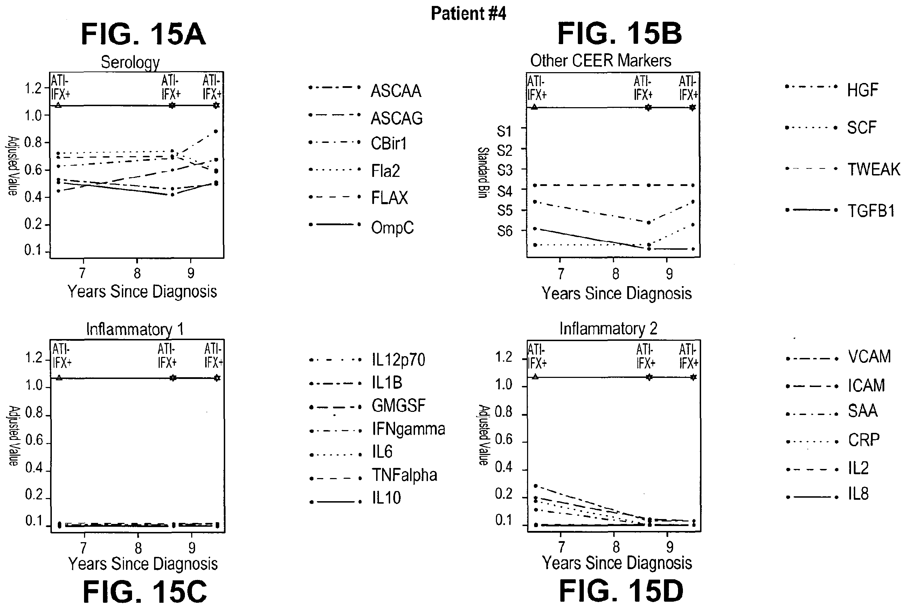

FIGS. 14-15 show the marker profile for Patient #4. Compared to the profile of Patient #3, the repair factors (FIG. 14A-D), serologic markers and inflammatory markers (FIG. 15A-D) were lower for Patient #4, indicating that this patient is closer to being fully healed. Patient #4 was ATI- at t1, and progressed to ATI-, IFX+ at t2 and t3.

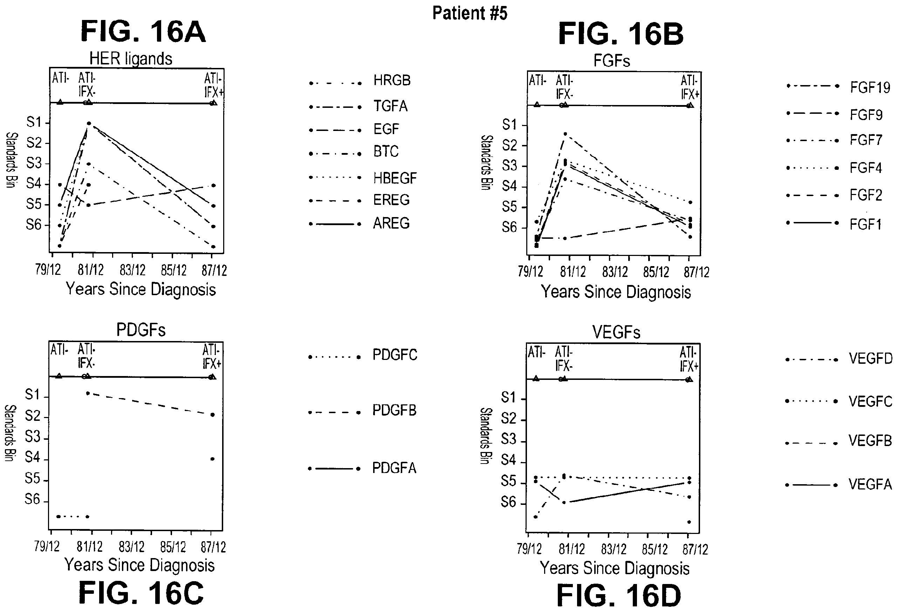

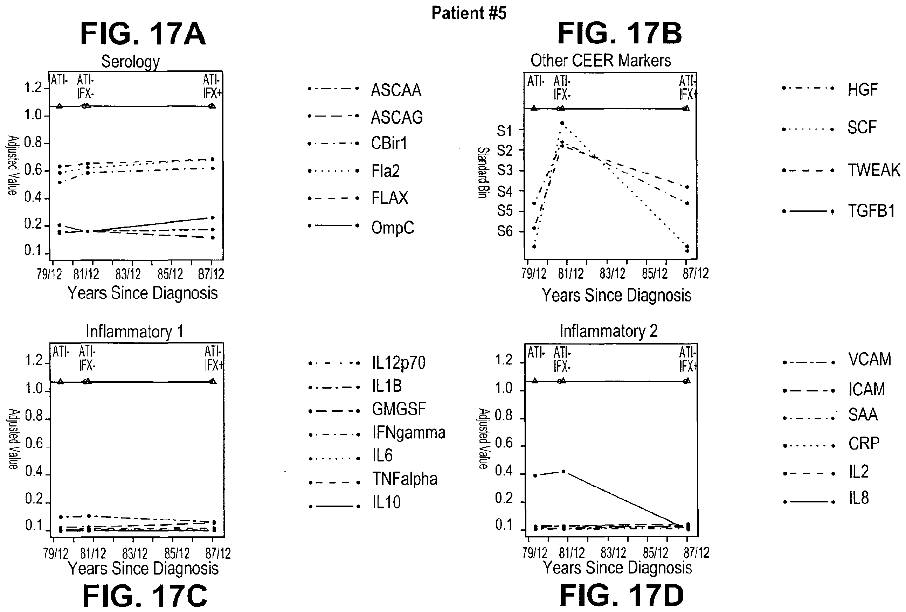

FIGS. 16-17 show the marker profile for Patient #5. The repair factors, serological markers (FIG. 16A-D) and inflammatory markers (FIG. 17A-D) increased or remained high over time. For instance, IL8 and TWEAK were still high when the repair factors and the flagellin markers (*e.g.*,CBir1, Fla2 and FLAX) were high. At t1, Patient #5 was ATI- and ATI-, IFX- at t2. By t3, the patient was ATI-, IFX+.

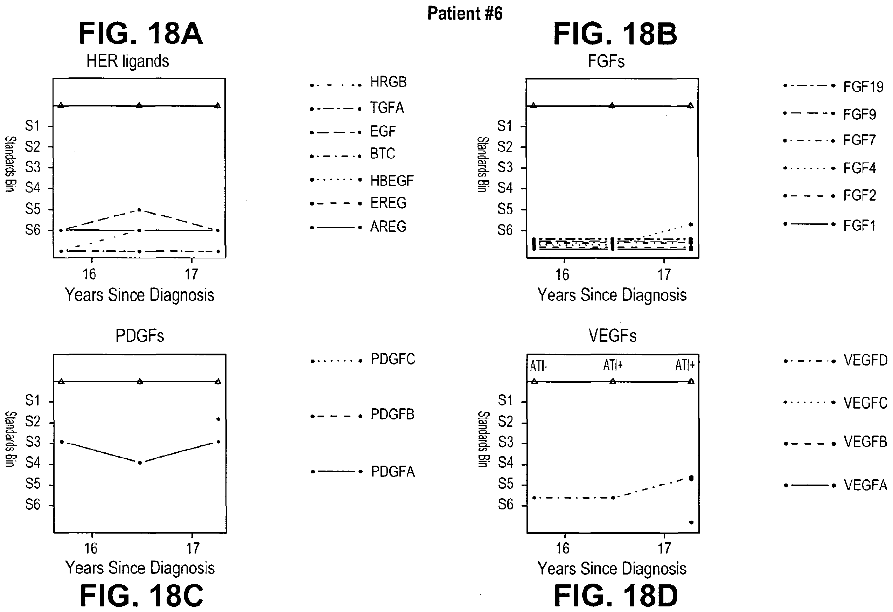

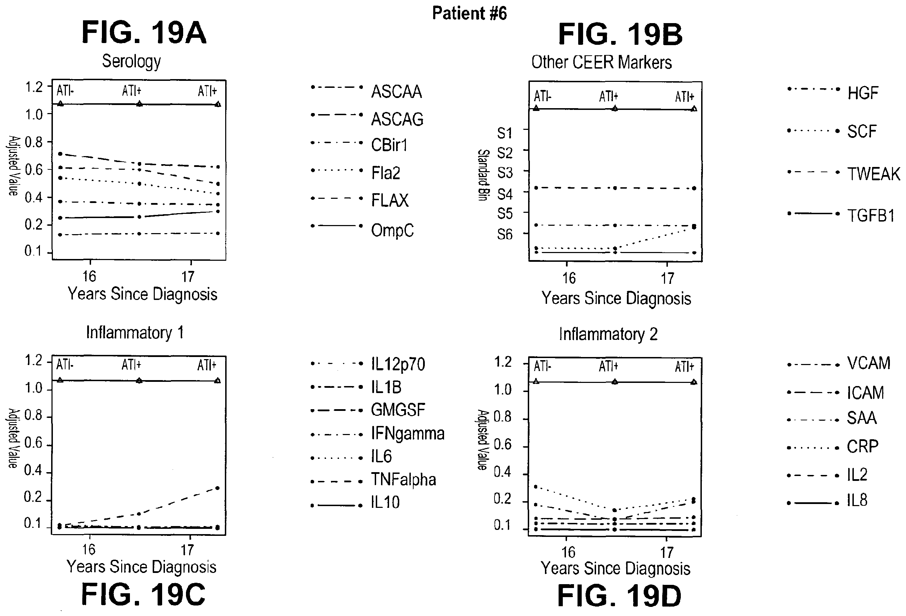

FIGS. 18-19 show the marker profile for Patient #6. The repair factors were not increased (FIG. 18A-D), but some of the inflammatory markers and serological markers were high (FIG. 19 A-D). By the time the second sample was obtained, the patient developed ATI and elevated levels of TNFα (FIG. 18).

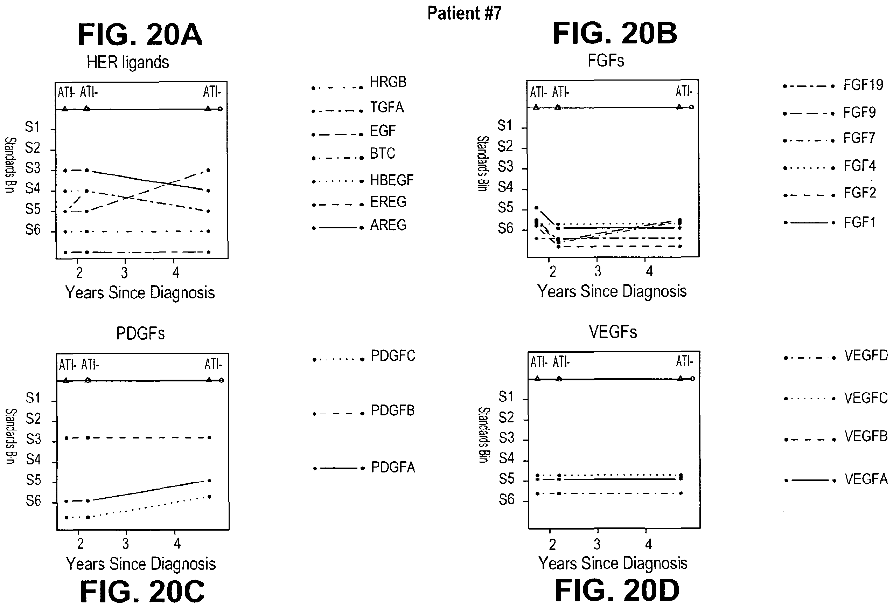

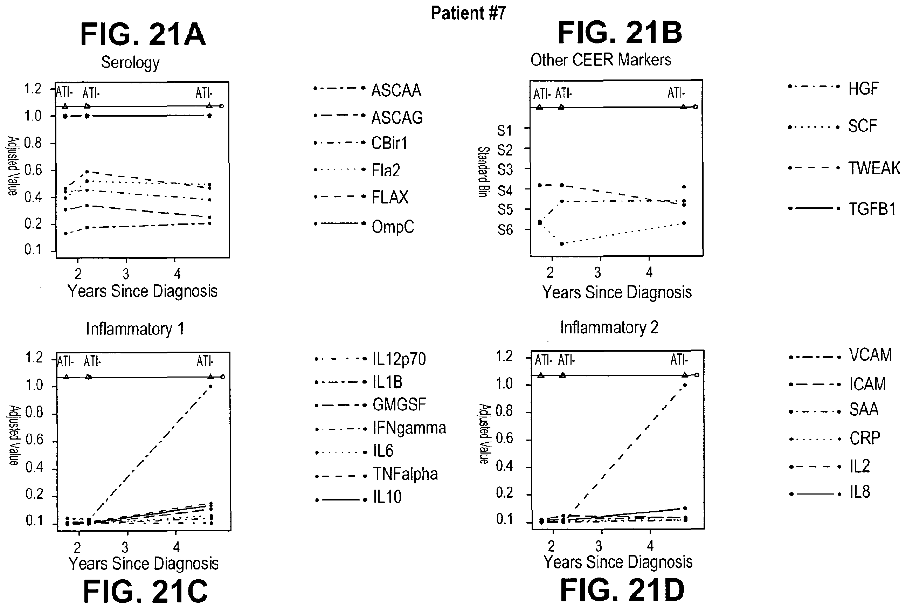

FIGS. 20-21 show the marker profile for Patient #7. Several repair factors (*e.g.*, AREG, HBEGF, TGFA and PDGFB) are high in the first two samples from the patient, but these markers decreased at the third time point (FIG. 20A-D) when the inflammation markers IL1β and IL2 increased. OmpC levels were very high in all samples tested (FIG. 21A-D). The patient was ATI-at all time points.

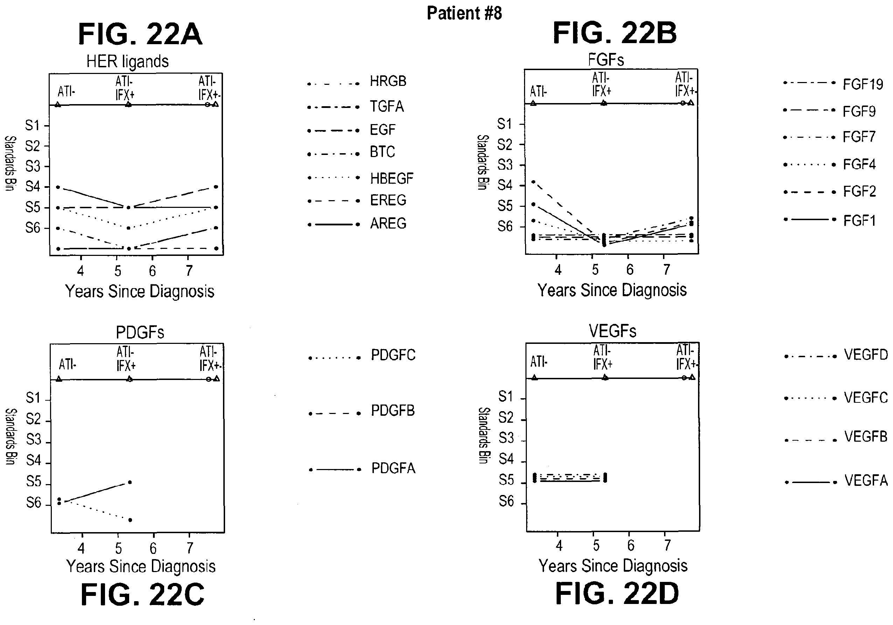

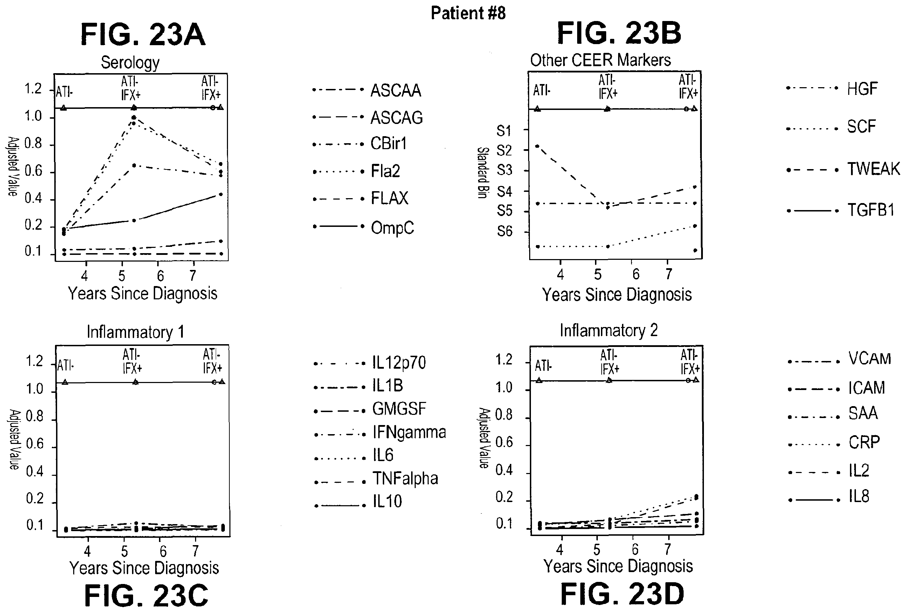

FIGS. 22-23 show the marker profile for Patient #8. In this patient, repair factors were high at times (FIG. 22A-D), while some inflammation markers and serologic markers were always high (FIG. 23A-D). Patient #8 was ATI- at t1, ATI-, IFX+ at t2, and ATI-, IFX+ at t3.

The individual patient comparison analysis showed that even when endoscopic analysis reported complete improvement, some patients expressed high levels of repair factors and low inflammation. The results indicate that these patients were still in the proliferation phase of mucosal healing. Furthermore, patients with high levels of repair factors and high levels of inflammatory markers were determined to be not completely healed.

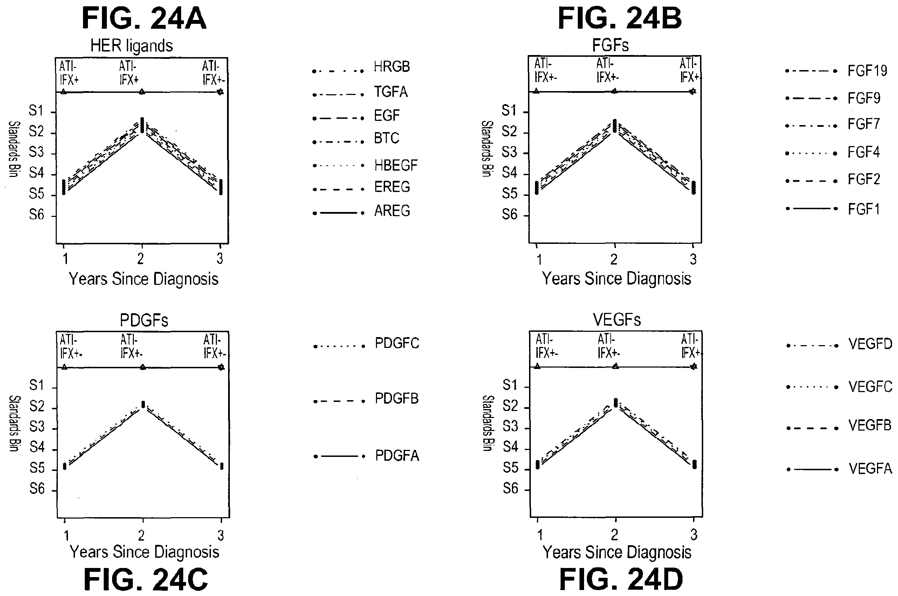

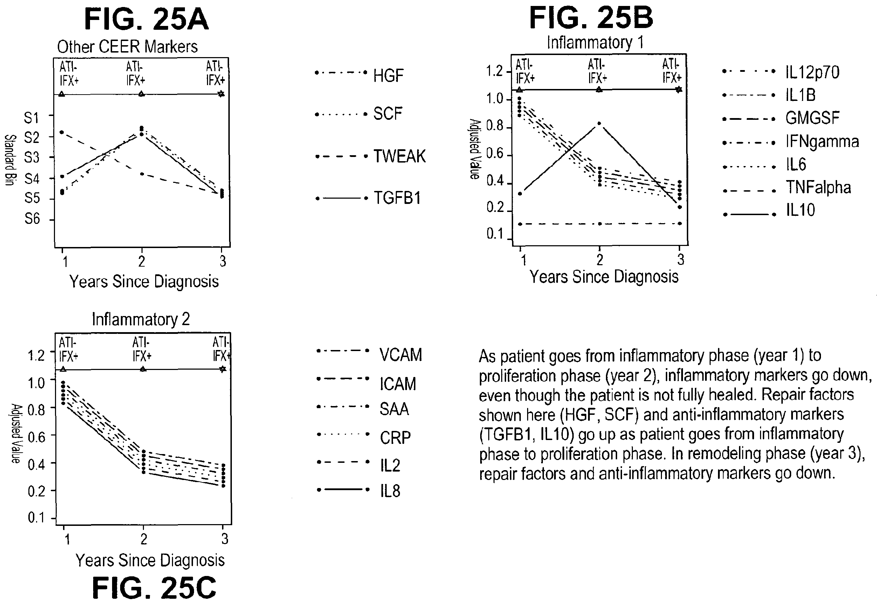

The data illustrates that marker expression in a patient's serum changes during progression through the phases of healing. As a patient goes from inflammatory phase (year 1) to proliferation phase (year 2), the levels of inflammatory markers decrease even though the patient is not fully healed (FIG. 25A-C). Repair factors and inflammatory markers increase as the patient progresses from the inflammatory phase to the proliferation phase (FIG. 24A-D). In the remodeling phase (year 3), repair factors and inflammatory markers decrease (FIGS. 24-25).

In summary, this example shows that measuring inflammatory markers, serology markers, and repair markers can indicate when a patient's intestinal mucosa is undergoing mucosal healing. Likewise, these markers can be used to determine whether the patient is healed and in the remodeling phase.

### Example 2. Combined Marker Analysis for Predicting Phases of Mucosal Healing.

This example shows a method for selecting markers that are predictive of the different phases of mucosal healing. In this study two sets of markers (e.g., proliferation phase markers and inflammatory phase markers) was analyzed in two patient populations, *e.g*., individuals with complete improvement without relapse and individuals who never healed. Statistical analysis was conducted to determine whether the marker sets (or a subset of the marker sets) were associated with a particular clinical outcome. Initially, the full marker sets were analyzed. Secondly, the analysis was serially repeated with the selective exclusion of one marker in each round until each marker was tested.

Samples from 111 individuals who showed either complete improvement (remodeling phase of mucosal healing) or no healing (inflammatory phase) were obtained and assayed. The markers used included proliferation phase markers such as proliferation markers, antiinflammatory markers and IFX (*e.g.,* AREG, EREG, HBEGF, HGF, HRGB, BTC, EGF, TGFA, FGF1, FGF2, FGF4, FGF7, FGF9, FGF19, SCF, PDGFA, PDGFB, PDGFC, VEGFA, VEGFB, VEGFC, VEGFD, TGFB1, IL10, and IFX) and inflammatory phase markers such as inflammatory markers, serology markers and ATI (*e.g.*, TWEAK, CRP, ICAM, SAA, VCAM, IL2, IL8, IL12p70, IL1β, GMCSF, IFNγ, IL6, TNFα, ASCAA, ASCAG, CBirl, Fla2, FlaX, OmpC, and ATI). The levels (*e.g.*, concentrations) of the markers were assayed by CEER or other methods, such as, ELISA, HMSA, and protein array. Each marker value was assigned a score of 0 to 6, depending on the level detected relative to either a series of standards or quantiles.

For CEER markers (*e.g.*, EGF, AREG, EREG, HBEGF, HGF, HRGB, BTC, TGFA, FGF1, FGF2, FGF, FGF7, FGF9, FGF19, SCF, PDGFA, PDGFB, PDGFC, VEGFA, VEGFB, VEGFC, VEGFD, TGFB1, and TWEAK), the score was based on the individual's marker value relative to six standards. For example, if the value was below the lowest standard, the marker was given a 0 score. If the value was between the lowest standard and the second lowest standard, the marker was given a 1 score. For some markers, the score was determined using a lower dilution factor such as, *e.g*., 1:5, 1:25, 1:100, 1:260, or 1:1250.

For non-CEER markers, the score was based on the quantiles of each marker in the data set. The samples are split into 7 groups and assigned a score of 0 to 6 based on the group that the marker value was associated with. It was assumed that there was a wide distribution of each marker in the data set.

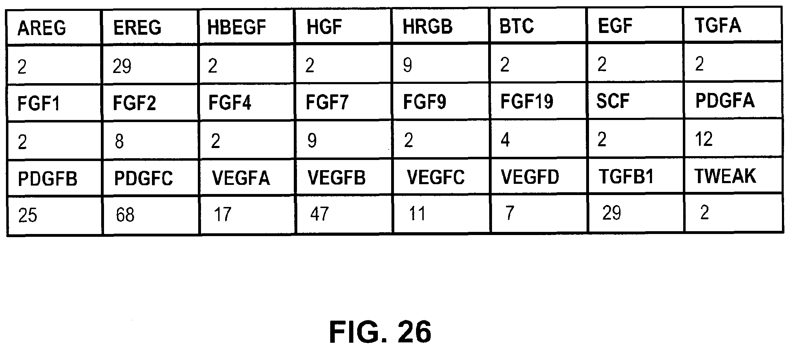

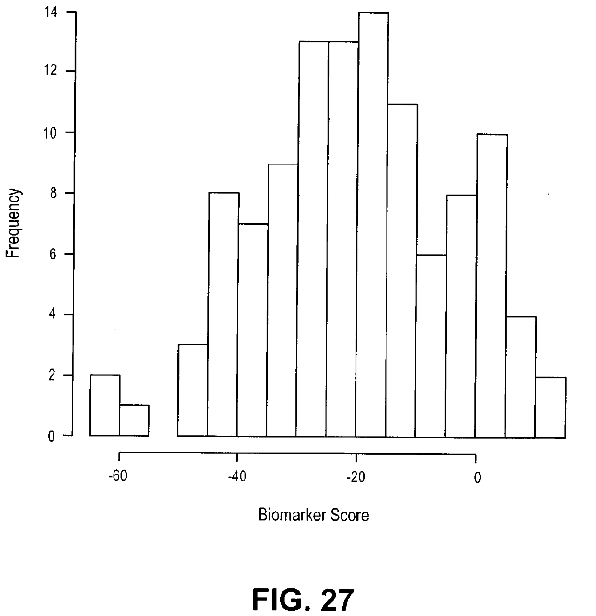

If an individual was missing data for a particular marker, the individual was given the average value of the marker. FIG. 26 shows the number of individuals with missing biomarker values. A biomarker score was calculated as the sum of the scores for all the proliferation phase markers minus the sum of the scores for all the inflammatory markers. The distribution of the biomarker scores is presented in FIG. 27.

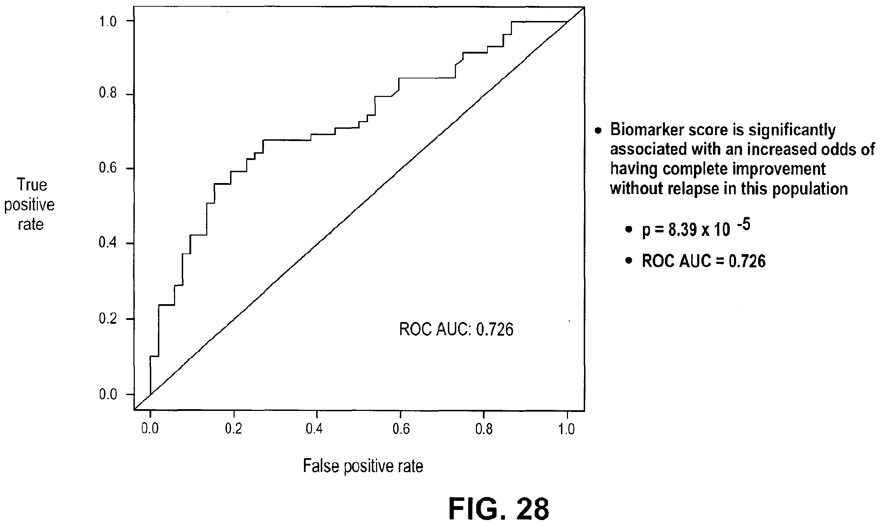

Logistic regression of mucosal improvement status on the biomarker score was used to test for association. The data shows that the biomarker score is significantly associated with an increased odds of having complete improvement without relapse (FIG. 28).

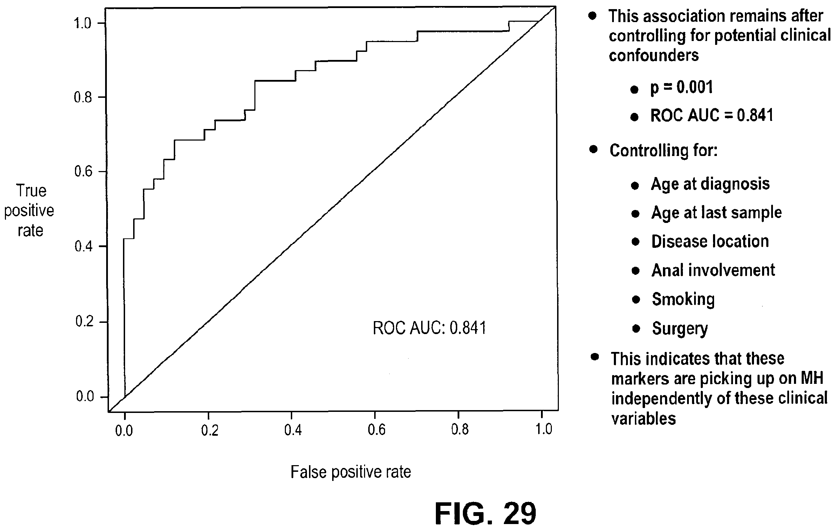

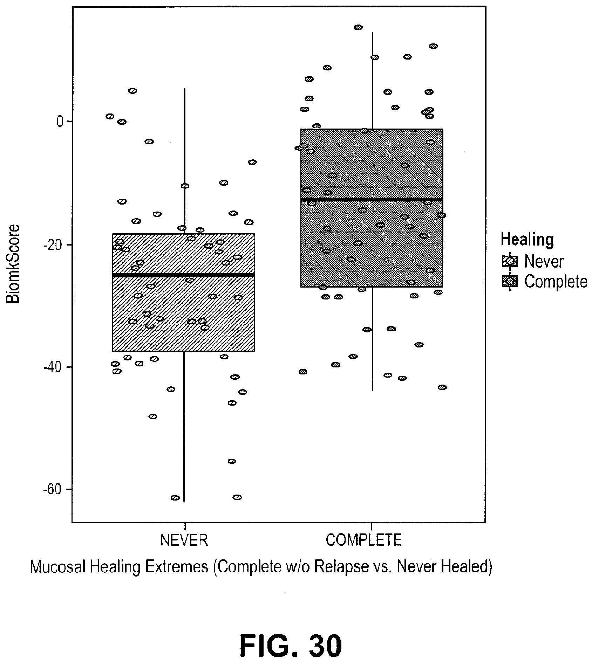

The analysis was also performed by controlling for potential clinical confounders, such as age of diagnosis, age of last sample, disease location, anal involvement, smoking and surgery. Data from 79 individuals were used, in particular, 41 individuals who never healed and 38 patient who showed complete improvement with no relapse). Even with the adjustment, the biomarker score was significantly associated with an increased odds of having complete improvement without relapse (FIG. 29). Thus, the proliferation phase markers and inflammatory phase markers are predictive of mucosal healing and independent of clinical variables. FIG. 30 shows the distribution of the biomarker score segregated by mucosal healing status.

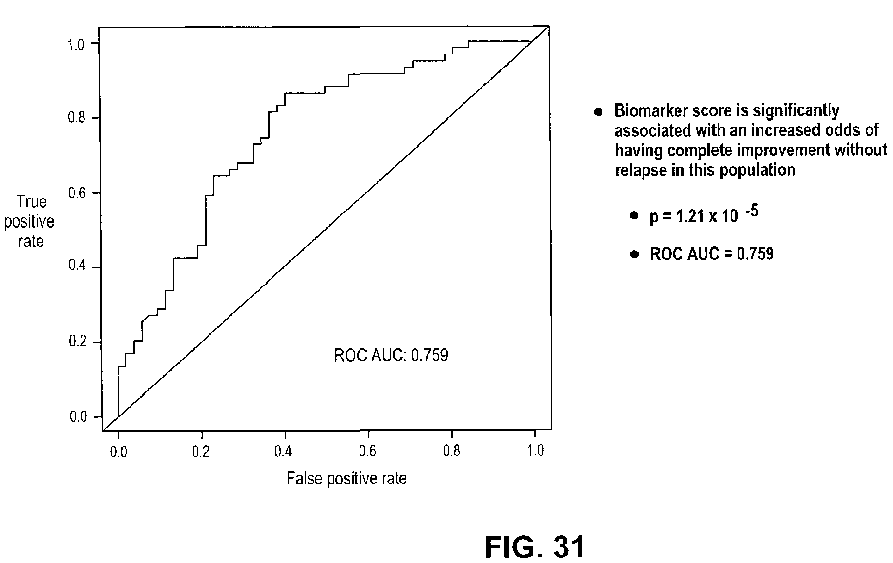

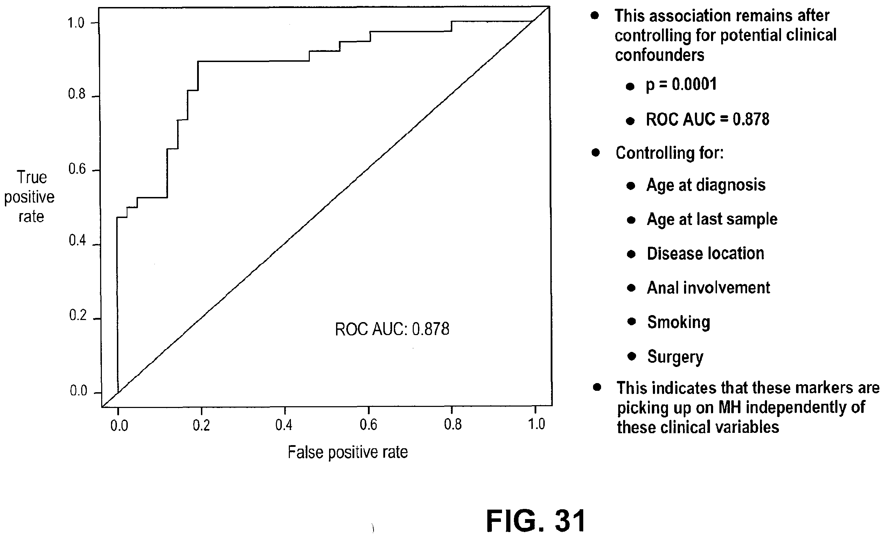

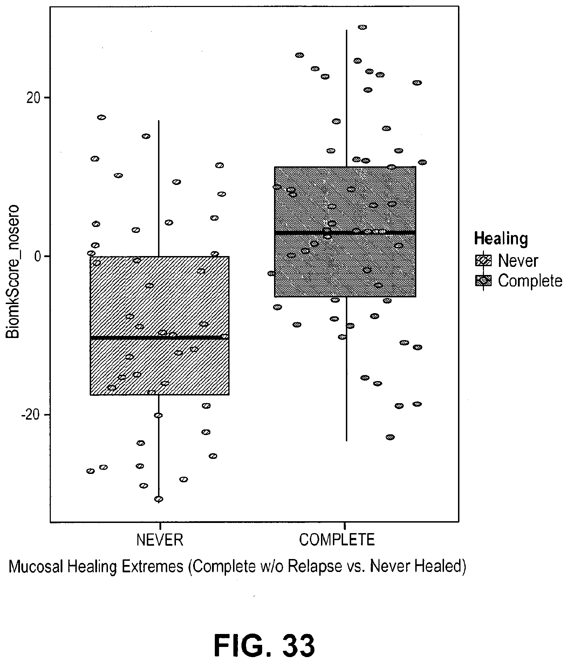

Next, the analysis of the biomarker score was performed by excluding the serology markers. Once again, the biomarker score was significantly associated with an increased odd of having complete improvement (FIG. 31). This was also the case when the data was adjusted to control for potential clinical confounders (FIG. 32). FIG. 33 shows the distribution of the biomarker score without serology markers and partitioned by mucosal healing status.

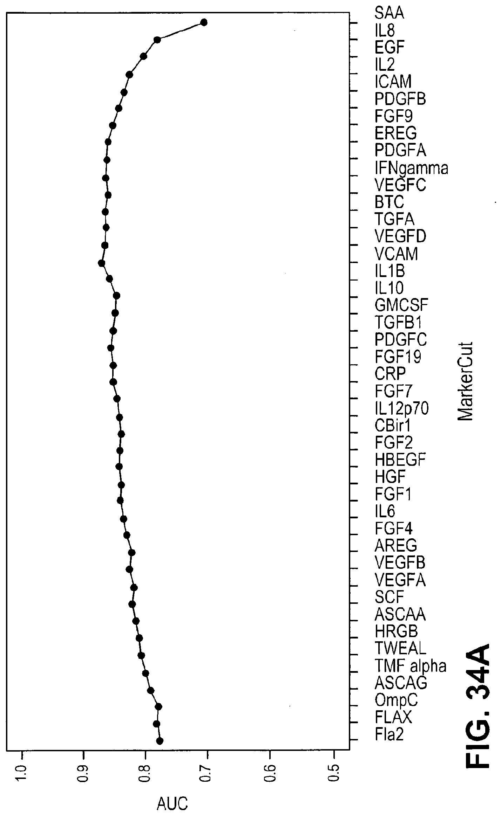

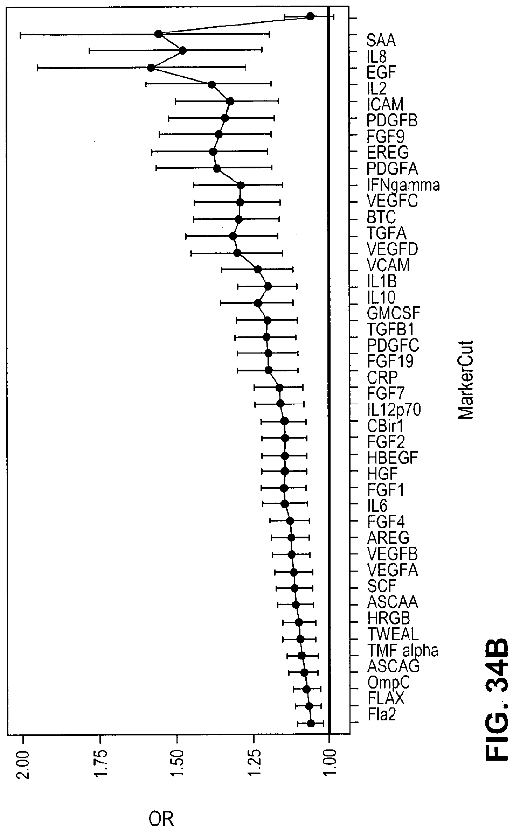

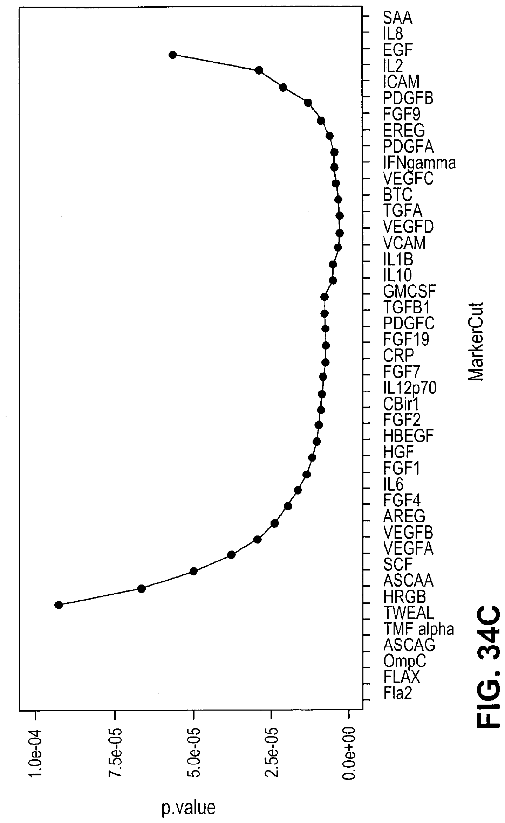

This example describes a biomarker score approach that can be used when multivariate modeling cannot be used with the dataset. The method involves the following steps: 1) selecting an experimental biomarker set (e.g., proliferation phase markers and inflammatory phase markers except ATI and IFX) to be tested; 2) calculating the p-value, OR and ROC AUC of the biomarker score as a single marker is excluded from the analysis; 3) eliminating any marker from the experimental biomarker set if that marker results in the lowest p-value when excluded in the analysis; 4) plotting the biomarker score, p-value, OR and ROC AUC; and 5) repeating steps 1-4. The trends in the statistical analysis can be used to determine which markers of the set are more predictive of the remodeling phase of mucosal healing (e.g., complete improvement without relapse) (FIG. 34A-C). This example provides a method for selecting informative markers that are predictive of the different phases of mucosal healing.

Although the foregoing invention has been described in some detail by way of illustration and example for purposes of clarity of understanding, one of skill in the art will appreciate that certain changes and modifications may be practiced within the scope of the appended claims.

## Claims

1. An in vitro method for predicting the likelihood of mucosal healing in a subject having or suspected of having an inflammatory bowel disease (IBD), the method comprising:
(a) measuring the level of a first set of markers to form a first marker score, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, and an anti-drug antibody (ADA), wherein lower levels of SAA and VCAM relative to the level of the same marker in patients with IBD but no mucosal healing are predictive of mucosal healing;
(b) measuring the level of a second set of markers to form a second marker score, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFα antibody,wherein a lower level of TGFA relative to the level of the same marker in patients with IBD but no mucosal healing is predictive of mucosal healing;
(c) comparing the first marker score to the second marker score; and
(d) predicting the likelihood of mucosal healing based upon the comparison in step (c).

2. The method of claim 1, wherein the subject has an inflammatory bowel disease (IBD) and wherein IBD is Crohn's disease or ulcerative colitis.

3. The method of any one of claims 1 to 2, wherein the comparison in step (c) comprises applying an algorithm incorporating the first marker score and the second marker score and, wherein the algorithm predicts
(i) the likelihood of mucosal healing independent of clinical confounders, wherein the clinical confounders comprise one or more selected from the group consisting of age of diagnosis, age of last sample, disease location, anal involvement, smoking, and surgery; or
(ii) the likelihood of mucosal healing excluding serology markers, wherein the excluded serology markers comprise one or more selected from the group consisting of ASCA-A, ASCA-G, CBirl, Fla2, FlaX, and OmpC.

4. The method of any one of claims 1 to 3
wherein the subject is receiving an anti-TNFα antibody
comprising one or more of REMICADE™ (infliximab), ENBREL™ (etanercept), HUMIRA™ (adalimumab), and CIMZIA® (certolizumab pegol).

5. The method of any one of claims 1 to 4, wherein the level of the markers is
(i) measured in a sample selected from the group consisting of serum, plasma, whole blood, stool, peripheral blood mononuclear cells (PBMC), polymorphonuclear (PMN) cells, and a tissue biopsy; and/or
(ii) is detected at the level of protein expression using an immunoassay, a homogeneous mobility shift assay (HMSA), or an immunohistochemical assay; and/or
(iii) is detected at the level of mRNA expression with a hybridization assay or an amplification-based assay.

6. An in vitro method for monitoring the progression of mucosal healing in a subject, the method comprising:
(a) measuring the level of a first set of markers at a plurality of time points to form a plurality of first marker scores, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, and an anti-drug antibody (ADA), wherein lower levels of SAA and VCAM relative to the level of the same marker in patients with IBD but no mucosal healing are predictive of mucosal healing;
(b) measuring the level of a second set of markers at a plurality of time points to form a plurality of second marker scores, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFα antibody, wherein a lower level of TGFA relative to the level of the same marker in patients with IBD but no mucosal healing is predictive of mucosal healing;
(c) comparing the first marker score to the second marker score at each time point and across the plurality of time points; and
(d) monitoring the progression of mucosal healing based upon the comparison in step (c).

7. The method of claim 6, wherein the subject has an inflammatory bowel disease (IBD), wherein the IBD is Crohn's disease or ulcerative colitis.

8. The method of any one of claims 6 to 7, wherein the comparison in step (c) comprises applying an algorithm incorporating the first marker score and the second marker score.

9. The method of claim 8, wherein the algorithm
(i) monitors the progression of mucosal healing independent of clinical confounders, wherein the clinical confounders comprise one or more selected from the group consisting of age of diagnosis, age of last sample, disease location, anal involvement, smoking, and surgery ; or
(ii) monitors the progression of mucosal healing excluding serology markers, wherein the excluded serology markers comprise one or more selected from the group consisting of ASCA-A, ASCA-G, CBirl, Fla2, FlaX, and OmpC.

10. The method of any one of claims 6 to 9, wherein the subject is receiving an anti-TNFα antibody comprising one or more of REMICADE™ (infliximab), ENBREL™ (etanercept), HUMIRA™ (adalimumab), and CIMZIA® (certolizumab pegol).

11. The method of any one of claims 6 to 10, wherein the level of the markers at each time point is measured in a sample selected from the group consisting of serum, plasma, whole blood, stool, peripheral blood mononuclear cells (PBMC), polymorphonuclear (PMN) cells, and a tissue biopsy.

12. The method of any one of claims 6 to 11, wherein the level of the markers is detected at the level of protein expression using an immunoassay, a homogeneous mobility shift assay (HMSA), or an immunohistochemical assay.

13. The method of any one of claims 6 to 11, wherein the level of the markers is detected at the level of mRNA expression with a hybridization assay or an amplification-based assay.

14. An anti-TNFα antibody for the use in the treatment of IBD, wherein the amount of anti-TNFα antibody is based upon the progression of mucosal healing in the subject as determined by the in vitro method comprising:
a) measuring the level of a first set of markers at a plurality of time points to form a plurality of first marker scores, wherein the first set of markers comprises SAA and VCAM, and optionally further comprises one or more of TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, and an anti-drug antibody (ADA);
(b) measuring the level of a second set of markers at a plurality of time points to form a plurality of second marker scores, wherein the second set of markers comprises TGFA, and optionally further comprises one or more of AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, and an anti-TNFα antibody, wherein a lower level of TGFA relative to the level of the same marker in patients with IBD but no mucosal healing is predictive of mucosal healing;
(c) comparing the first marker score to the second marker score at each time point and across the plurality of time points; and
(d) monitoring the progression of mucosal healing based upon the comparison in step (c).

## Patentansprüche

1. In-vitro-Verfahren zur Vorhersage der Wahrscheinlichkeit von mukosaler Heilung bei einem Subjekt mit oder mit Verdacht auf eine entzündliche Darmerkrankung (IBD), das Verfahren umfassend:
(a) Messen des Spiegels eines ersten Satzes von Markern, um eine erste Markerbewertung zu bilden, wobei der erste Satz von Markern SAA und VCAM umfasst und optional ferner eines oder mehrere von TWEAK, CRP, ICAM, IL-2, IL-8, IL-12p70, IL-1β, GMCSF, IFNy, IL-6, TNFα, ASCA-A, ASCA-G, CBirl, Fla2, FlaX, OmpC, und einen Anti-Arzneimittel-Antikörper (ADA) umfasst, wobei niedrigere Spiegel von SAA und VCAM in Bezug auf den Spiegel desselben Markers in Patienten mit IBD, die jedoch keine mukosale Heilung aufweisen, eine mukosale Heilung vorhersagen.
(b) Messen des Spiegels eines zweiten Satzes von Markern, um eine zweite Markerbewertung zu bilden, wobei der zweite Satz von Markern TGFA umfasst und optional ferner eines oder mehrere von AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, und einen Anti-TNFa-Antikörper umfasst, wobei ein niedrigerer Spiegel von TGFA in Bezug auf den Spiegel desselben Markers in Patienten mit IBD, die jedoch keine mukosale Heilung aufweisen, eine mukosale Heilung vorhersagen.
(c) Vergleichen der ersten Markerbewertung mit der zweiten Markerbewertung; und
(d) Vorhersagen der Wahrscheinlichkeit von mukosaler Heilung einer basierend auf dem Vergleichs in Schritt (c).

2. Verfahren nach Anspruch 1, wobei das Subjekt eine entzündliche Darmerkrankung (IBD) hat und wobei IBD Morbus Crohn oder Colitis ulcerosa ist.

3. Verfahren nach einem der Ansprüche 1 bis 2, wobei der Vergleich in Schritt (c) das Anwenden eines Algorithmus umfasst, der die erste Markerbewertung und die zweite Markerbewertung enthält, und wobei der Algorithmus Folgendes vorhersagt
(i) die Wahrscheinlichkeit der mukosalen Heilung unabhängig von klinischen Störfaktoren, wobei die klinischen Störfaktoren einen oder mehrere umfassen, ausgewählt aus der Gruppe bestehend aus Diagnosealter, Alter der letzten Probe, Krankheitsort, Analbeteiligung, Rauchen und Operation; oder
(ii) die Wahrscheinlichkeit der mukosalen Heilung unter Ausschluss von Serologiemarkern, wobei die ausgeschlossenen Serologiemarker einen oder mehrere umfassen, ausgewählt aus der Gruppe bestehend aus ASCA-A, ASCA-G, CBirl, Fla2, FlaX und OmpC.

4. Verfahren nach einem der Ansprüche 1 bis 3,
wobei das Subjekt einen Anti-TNFa-Antikörper erhält, der eines oder mehrere von REMICADE™ (Infliximab), ENBREL™ (Etanercept), HUMIRA™ (Adalimumab) und CIMIZIA® (Certolizumab Pegol) umfasst.

5. Verfahren nach einem der Ansprüche 1 bis 4, wobei der Spiegel des Markers
(i) in einer Probe, ausgewählt aus der Gruppe bestehend aus Serum, Plasma, Vollblut, Stuhl, mononuklearen Zellen des peripheren Blutes (PBMC), polymorphnuklearen Zellen (PMN) und einer Gewebebiopsie gemessen wird; und/oder
(ii) auf der Ebene der Proteinexpression unter Verwendung eines Immunoassays, eines homogenen Mobilitätsverschiebungsassays (HMSA) oder eines immunhistochemischen Assays nachgewiesen wird; und/oder
(iii) auf der Ebene der mRNA-Expression mit einem Hybridisierungsassay oder einem Assay auf Amplifikationsbasis nachgewiesen wird.

6. In-vitro-Verfahren zur Überwachung des Fortschreitens mukosaler Heilung bei einem Subjekt, Verfahren umfassend:
(a) Messen des Spiegels eines ersten Satzes von Markern zu einer Vielzahl von Zeitpunkten, um eine Vielzahl von ersten Markerbewertungen zu bilden, wobei der erste Satz von Markern SAA und VCAM umfasst und optional ferner eines oder mehrere von TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, und einen Anti-Arzneimittel-Antikörper (ADA) umfasst, wobei niedrigere Spiegel von SAA und VCAM in Bezug auf den Spiegel desselben Markers in Patienten mit IBD, die jedoch keine mukosale Heilung aufweisen, eine mukosale Heilung vorhersagen;
(b) Messen des Spiegels eines zweiten Satzes von Markern zu einer Vielzahl von Zeitpunkten, um eine Vielzahl von zweiten Markerbewertungen zu bilden, wobei der zweite Satz von Markern TGFA umfasst und optional ferner eines oder mehrere von AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, und einen Anti-TNFa-Antikörper umfasst, wobei ein niedrigerer Spiegel von TGFA in Bezug auf den Spiegel desselben Markers in Patienten mit IBD, die jedoch keine mukosale Heilung aufweisen, eine mukosale Heilung vorhersagen.
(c) Vergleichen der ersten Markerbewertung mit der zweiten Markerbewertung zu jedem Zeitpunkt und über die Vielzahl von Zeitpunkten hinweg; und
(d) Überwachen des Fortschreitens der mukosalen Heilung basierend auf dem Vergleich in Schritt (c).

7. Verfahren nach Anspruch 6, wobei das Subjekt eine entzündliche Darmerkrankung (IBD) hat und wobei die IBD Morbus Crohn oder Colitis ulcerosa ist.

8. Verfahren nach einem der Ansprüche 6 bis 7, wobei der Vergleich in Schritt (c) das Anwenden eines Algorithmus umfasst, der die erste Markerbewertung und die zweite Markerbewertung enthält.

9. Verfahren nach Anspruch 8, wobei der Algorithmus
(i) das Fortschreiten der mukosalen Heilung unabhängig von klinischen Störfaktoren überwacht, wobei die klinischen Störfaktoren einen oder mehrere umfassen, ausgewählt aus der Gruppe bestehend aus Diagnosealter, Alter der letzten Probe, Krankheitsort, Analbeteiligung, Rauchen und Operation; oder
(ii) das Fortschreiten der mukosalen Heilung unter Ausschluss von Serologiemarkern überwacht, wobei die ausgeschlossenen Serologiemarker einen oder mehrere umfassen, ausgewählt aus der Gruppe bestehend aus ASCA-A, ASCA-G, CBirl, Fla2, FlaX und OmpC.

10. Verfahren nach einem der Ansprüche 6 bis 9, wobei das Subjekt einen Anti-TNFa-Antikörper erhält, der eines oder mehrere von REMICADE™ (Infliximab), ENBREL™ (Etanercept), HUMIRA™ (Adalimumab) und CIMIZIA® (Certolizumab Pegol) umfasst.

11. Verfahren nach einem der Ansprüche 6 bis 10, wobei der Spiegel der Marker zu jedem Zeitpunkt in einer Probe gemessen wird, ausgewählt aus der Gruppe bestehend aus Serum, Plasma, Vollblut, Stuhl, mononuklearen Zellen des peripheren Blutes (PBMC) und polymorphnuklearen Zellen (PMN), und einer Gewebebiopsie.

12. Verfahren nach einem der Ansprüche 6 bis 11, wobei der Spiegel der marker auf der Ebene der Proteinexpression unter Verwendung eines Immunoassays, eines homogenen Mobilitätsverschiebungsassays (HMSA) oder eines immunhistochemischen Assays nachgewiesen wird.

13. Verfahren nach einem der Ansprüche 6 bis 11, wobei der Spiegel der Marker auf der Ebene der mRNA-Expression mit einem Hybridisierungsassay oder einem Assay auf Amplifikationsbasis nachgewiesen wird.

14. Anti-TNFa-Antikörper zur Verwendung bei der Behandlung von IBD, wobei die Menge an Anti-TNFa-Antikörper auf dem Fortschriit der mukosalen Heilung bei dem Subjekt basiert, wie durch das in vitro-Verfahren bestimmt, umfassend:
(a) Messen des Spiegels eines ersten Satzes von Markern an einer Vielzahl von Zeitpunkten, um eine Vielzahl von ersten Markerbewertungen zu bilden, wobei der erste Satz von Markern SAA und VCAM umfasst und optional ferner eines oder mehrere von TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, und einen Anti-Arzneimittel-Antikörper (ADA);
(b) Messen des Spiegels eines zweiten Satzes von Markern an einer Vielzahl von Zeitpunkten, um eine Vielzahl von zweiten Markerbewertungen zu bilden, wobei der zweite Satz von Markern TGFA umfasst und optional ferner eines oder mehrere von AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, und einen Anti-TNFa-Antikörper umfasst, wobei ein niedrigerer Spiegel von TGFA in Bezug auf den Spiegel desselben Markers in Patienten mit IBD, die jedoch keine mukosale Heilung aufweisen, eine mukosale Heilung vorhersagen.
(c) Vergleichen der ersten Markerbewertung mit der zweiten Markerbewertung zu jedem Zeitpunkt und über die Vielzahl von Zeitpunkten hinweg; und
(d) Überwachen des Fortschreitens der mukosalen Heilung basierend auf dem Vergleich in Schritt (c).

## Revendications

1. Procédé in vitro permettant de prédire la probabilité d'une cicatrisation de muqueuse chez un sujet présentant ou suspecté de présenter une maladie intestinale inflammatoire (IBD), le procédé comprenant :
(a) la mesure du taux d'un premier ensemble des marqueurs pour former un premier score de marqueurs, le premier ensemble de marqueurs comprenant SAA et VCAM, et comprenant éventuellement en outre au moins l'un de TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, et un anticorps anti-médicament (ADA), des taux inférieurs de SAA et VCAM par rapport au taux du même marqueur chez des patients atteints d'IBD, mais sans cicatrisation de muqueuse, étant prédictifs d'une cicatrisation de muqueuse ;
(b) la mesure du taux d'un deuxième ensemble de marqueurs pour former un deuxième score de marqueurs, le deuxième ensemble de marqueurs comprenant TGFA, et comprenant en outre éventuellement en outre au moins l'un de AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, et un anticorps anti-TNFα, un taux inférieur de TGFA par rapport au taux du même marqueur chez des patients atteints d'IBD, mais sans cicatrisation de muqueuse, étant prédictif d'une cicatrisation de muqueuse ;
(c) la comparaison du premier score de marqueurs au deuxième score de marqueurs ; et
(d) la prédiction de la probabilité de cicatrisation de muqueuse sur la base de la comparaison de l'étape (c).

2. Procédé de la revendication 1, dans lequel le sujet est atteint d'une maladie intestinale inflammatoire (IBD) et dans lequel l'IBD est la maladie de Crohn ou la rectocolite hémorragique.

3. Procédé de l'une quelconque des revendications 1 à 2, dans lequel la comparaison à l'étape (c) comprend l'application d'un algorithme incorporant le premier score de marqueurs et le deuxième score de marqueurs, et dans lequel l'algorithme prédit
(i) la probabilité de cicatrisation de muqueuse indépendamment d'éléments de confusion cliniques, les éléments de confusion cliniques comprenant au moins l'un des éléments choisis dans le groupe constitué par l'ancienneté du diagnostic, l'ancienneté du dernier échantillon, l'emplacement de la maladie, l'implication anale, le tabagisme et la chirurgie ; ou
(ii) la probabilité de cicatrisation de muqueuse excluant les marqueurs sérologiques, le marqueurs sérologiques comprenant au moins un élément choisi dans le groupe constitué par ASCA-A, ASCA-G, CBirl, Fla2, FlaX et OmpC.

4. Procédé de l'une quelconque des revendications 1 à 3
dans lequel le sujet reçoit un anticorps anti-TNFα comprenant au moins l'un de REMICADE™ (infliximab), ENBREL™ (étanercept), HUMIRA™ (adalimumab) et CIMZIA® (certolizumab pégol).

5. Procédé de l'une quelconque des revendications 1 à 4, dans lequel le taux du marqueur est
(i) mesuré dans un échantillon choisi dans le groupe constitué par le sérum, le plasma, le sang total, les selles, les cellules mononuclées de sang périphérique (PBMC), les cellules polymorphonucléaires (PMN) et une biopsie de tissu ; et/ou
(ii) est détecté au niveau de l'expression protéique à l'aide d'un immunodosage, d'un dosage de décalage de mobilité homogène (HMSA) ou d'un dosage immunohistochimique ; et/ou
(iii) est détecté au niveau de l'expression d'ARNm avec un dosage d'hybridation ou un dosage basé sur l'amplification.

6. Procédé in vitro permettant de surveiller la progression de la cicatrisation de muqueuse chez un sujet, le procédé comprenant :
(a) la mesure du taux d'un premier ensemble des marqueurs à une pluralité de points temporels pour former une pluralité de premiers scores de marqueurs, le premier ensemble de marqueurs comprenant SAA et VCAM, et comprenant éventuellement en outre au moins l'un de TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, et un anticorps anti-médicament (ADA), des taux inférieurs de SAA et VCAM par rapport au taux du même marqueur chez des patients atteints d'IBD, mais sans cicatrisation de muqueuse, étant prédictifs d'une cicatrisation de muqueuse ;
(b) la mesure du taux d'un deuxième ensemble de marqueurs à une pluralité de points temporels pour former une pluralité de deuxièmes scores de marqueurs, le deuxième ensemble de marqueurs comprenant TGFA, et comprenant en outre éventuellement en outre au moins l'un de AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, et un anticorps anti-TNFα, un taux inférieur de TGFA par rapport au taux du même marqueur chez des patients atteints d'IBD, mais sans cicatrisation de muqueuse, étant prédictif d'une cicatrisation de muqueuse ;
(c) la comparaison du premier score de marqueurs eu deuxième score de marqueurs à chaque point temporel de la pluralité de points temporels ; et
(d) la surveillance de la progression de la cicatrisation de muqueuse sur la base de la comparaison de l'étape (c).

7. Procédé de la revendication 6, dans lequel le sujet est atteint d'une maladie intestinale inflammatoire (IBD), dans lequel l'IBD est la maladie de Crohn ou la rectocolite hémorragique.

8. Procédé de l'une quelconque des revendications 6 ou 7, dans lequel la comparaison à l'étape (c) comprend l'application d'un algorithme incorporant le premier score de marqueurs et le deuxième score de marqueurs.

9. Procédé de la revendication 8, dans lequel l'algorithme
(i) surveille la progression de la cicatrisation de muqueuse indépendamment d'éléments de confusion cliniques, les éléments de confusion cliniques comprenant au moins l'un des éléments choisis dans le groupe constitué par l'ancienneté du diagnostic, l'ancienneté du dernier échantillon, l'emplacement de la maladie, l'implication anale, le tabagisme et la chirurgie ; ou
(ii) surveille la progression de la cicatrisation de muqueuse en excluant les marqueurs sérologiques, le marqueurs sérologiques comprenant au moins un élément choisi dans le groupe constitué par ASCA-A, ASCA-G, CBirl, Fla2, FlaX et OmpC.

10. Procédé de l'une quelconque des revendications 6 à 9, dans lequel le sujet reçoit un anticorps anti-TNFa comprenant au moins l'un de REMICADE™ (infliximab), ENBREL™ (étanercept), HUMIRA™ (adalimumab) et CIMZIA® (certolizumab pégol).

11. Procédé de l'une quelconque des revendications 6 à 10, dans lequel le taux des marqueurs à chaque point temporel est mesuré dans un échantillon choisi dans le groupe constitué par le sérum, le plasma, le sang total, les selles, les cellules mononuclées de sang périphérique (PBMC), les cellules polymorphonucléaires (PMN) et une biopsie de tissu.

12. Procédé de l'une quelconque des revendications 6 à 11, dans lequel le taux des marqueurs est détecté au niveau de l'expression protéique à l'aide d'un immunodosage, d'un dosage de décalage de mobilité homogène (HMSA) ou d'un dosage immunohistochimique.

13. Procédé de l'une quelconque des revendications 6 à 11, dans lequel le taux des marqueurs est détecté au niveau de l'expression d'ARNm avec un dosage d'hybridation ou un dosage basé sur l'amplification.

14. Anticorps anti-TNFα pour une utilisation dans le traitement d'une IBD, la quantité d'anticorps anti-TNFα étant basée sur la progression de la cicatrisation de muqueuse chez le sujet telle que déterminée par le procédé in vitro comprenant :
(a) la mesure du taux d'un premier ensemble des marqueurs à une pluralité de points temporels pour former une pluralité de premiers scores de marqueurs, le premier ensemble de marqueurs comprenant SAA et VCAM, et comprenant éventuellement en outre au moins l'un de TWEAK, CRP, ICAM, IL-2, IL-8, IL-12p70, IL-1β, GMCSF, IFNγ, IL-6, TNFα, ASCA-A, ASCA-G, CBirl, Fla2, FlaX, OmpC, et un anticorps anti-médicament (ADA) ;
(b) la mesure du taux d'un deuxième ensemble de marqueurs à une pluralité de points temporels pour former une pluralité de deuxièmes scores de marqueurs, le deuxième ensemble de marqueurs comprenant TGFA, et comprenant en outre éventuellement en outre au moins l'un de AREG, EREG, HBEGF, HGF, HRGB, BTC, EGF, FGF1, FGF2, FGF4, FGF7, FGF9, FGF19, SCF, PDGFA, PDGFB, PDGFC, VEGFA, VEGFB, VEGFC, VEGFD, TGFB1, IL-10, et un anticorps anti-TNFα, un taux inférieur de TGFA par rapport au taux du même marqueur chez des patients atteints d'IBD, mais sans cicatrisation de muqueuse, étant prédictif d'une cicatrisation de muqueuse ;
(c) la comparaison du premier score de marqueurs eu deuxième score de marqueurs à chaque point temporel de la pluralité de points temporels ; et
(d) la surveillance de la progression de la cicatrisation de muqueuse sur la base de la comparaison de l'étape (c).
